# FASCICULE DE BREVET EUROPEEN

(11) **EP 2 121 601 B1**
(45) Date de publication et mention de la délivrance du brevet: **15.06.2016**
(21) Numéro de dépôt: 08761826.0
(22) Date de dépôt: 31.01.2008
(51) Int. Cl.: C07D 209/14

(54) **NOUVEAUX DERIVES INDOLIQUES, LEURS PROCEDES DE PREPARATION ET LEURS UTILISATIONS NOTAMMENT EN TANT QU'ANTIBACTERIENS**
NEUARTIGE INDOLDERIVATE, VERFAHREN ZU DEREN HERSTELLUNG UND VERWENDUNG DAVON, INSBESONDERE ALS ANTIBAKTERIELLE WIRKSTOFFE
NOVEL INDOLE DERIVATIVES, METHODS FOR PREPARING SAME, AND USE THEREOF PARTICULARLY AS ANTIBACTERIAL AGENTS

(30) Priorité: 01.02.2007 FR 0700717
(43) Date de publication de la demande: 25.11.2009
(73) Titulaire: Université Joseph Fourier, 38041 Grenoble Cedex 09 (FR); Centre National de la Recherche Scientifique, 75794 Paris Cedex 16 (FR)
(72) Inventeur: DENIS, Jean-Noël, Marie, Léon, F-38560 Jarrie (FR); GUINCHARD, Xavier, Jean, Georges, Marie, 92160 Antony (FR); MOREAU née LAFFONT, Nicole, Jeanne, F-94220 Charenton Le Pont (FR); NEUVILLE, Luc, F-75013 Paris (FR); VALLEE, Yannick, F-38190 Laval (FR)
(74) Mandataire: Grosset-Fournier, Chantal Catherine
(86) Numéro de dépôt international: PCT/FR2008/000118
(87) Numéro de publication internationale: WO 2008/110690

(56) Documents cités:
- EP-A- 0 122 157
- WO-A-03/088897
- WO-A-2004/082586
- WO-A-2004/099124

## Description

La présente invention a pour objet de fournir de nouveaux dérivés indoliques présentant notamment des propriétés antibactériennes.

La présente invention a également pour objet de fournir un procédé de préparation desdits nouveaux dérivés indoliques.

La recherche de nouveaux composés biologiquement actifs suscite un intérêt de plus en plus croissant, notamment dans le domaine de la médecine (M. Hibert, J. Haiech Médecine/Sciences 2000, 16, 1332-1339). Parmi la multitude de produits naturels isolés, les composés azotés hétéroaromatiques occupent une place importante, notamment ceux qui sont dérivés de l'indole. Bon nombre de ces composés indoliques possède le motif "nortryptamine" (3'-indolyl méthylamine) de formule **1a** (**1**, R = R¹ = H) ou le motif "tryptamine" (2-(3'-indolyl)éthylamine) de formule **2a** (**2**, R = H). Afin de trouver des molécules bio-actives toujours plus originales, le milieu marin est devenu un domaine de recherche incontournable de par la grande diversité de ses hôtes : plus de 500 000 espèces d'organismes (D. J. Faulkner Nat. Prod. Rep. 2001, 18, 1-49 et revues rapportées dedans ; C.-G. Yang, H. Huang, B. Jiang Cur. Org. Chem. 2004, 8, 1691-1720; W. Gul, M. T. Hamann Life Sciences 2005, 78, 442-453). Leur étude a permis de découvrir des nouveaux alcaloïdes indoliques de structure originale caractérisée par un motif 1,2-diamine indolique de formule **3a** (**3**, R = H), appelé 1-(3'-indolyl)-1,2-diaminoéthane.

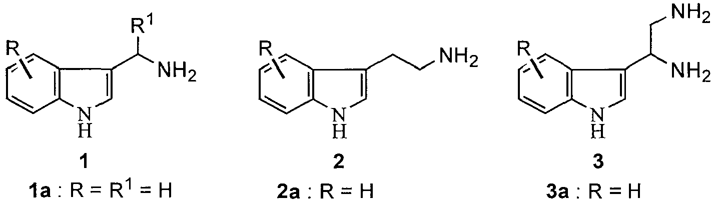

L'importance stratégique d'accéder efficacement à ces trois structures **1a, 2a** et **3a** et aux dérivés correspondants **1, 2** et **3,** motifs-clefs d'intermédiaires dans la synthèse de nombreux composés indoliques bio-actifs, a conduit de nombreux groupes de recherche à développer des voies de synthèse efficaces de ces intermédiaires. En particulier, l'introduction d'une chaîne carbonée azotée directement en position 3 d'un noyau indolique, la position la plus réactive vis-à-vis de la substitution électrophile aromatique lorsqu'elle n'est pas substituée, a fait l'objet d'une étude particulière car le choix judicieux du partenaire électrophile azoté du noyau indolique permet l'accès direct au motif souhaité.

Ainsi, la réaction la plus courante pour accéder aux dérivés indoliques **1** possédant le motif (3'-indolyl)méthylamine **1a** est la réaction de Mannich. Elle consiste à faire réagir un aldéhyde et une amine avec un noyau indolique en milieu acide. Son domaine d'application étant limité (A. Heydari, H. Tavakol, S. Aslanzadeh, J. Azarnia, N. Ahmadi Synthesis 2005, 622-626), d'autres méthodes dérivées d'aminométhylation et d'aminoalkylation ont été développées (H.-J. Grumbach, M. Arend, N. Risch Synthesis 1996, 883-887 et références citées ; Y. Gong, K. Kato, H. Kimoto Bull. Chem. Soc. Jpn. 2002, 75, 2637-3645 ; N. Sakai, M. Hirasawa, M. Hamajima, T. Konakahara J. Org. Chem. 2003, 68, 483-488 et références citées).

La synthèse de dérivés indoliques **2** possédant le motif 2-(3'-indolyl)éthylamine **2a** à partir de l'indole nécessite plusieurs étapes pour introduire les deux carbones. D'autres voies, notamment à partir du tryptophane, sont également décrites.

Concernant la synthèse des dérivés indoliques possédant le motif 1-(3'-indolyl)-1,2-diaminoéthane **3a,** à notre connaissance, seule une méthode a été décrite avant 1997. En 1965, Rajagopalan et Advani ont décrit une stratégie de synthèse de 1,2-diamines indoliques basée sur la réaction de Strecker (P. Rajagopalan, B. G. Advani Tetrahedron Lett. 1965, 2197-2200). Elle nécessite plusieurs étapes à partir de l'indole et elle conduit à des dérivés protégés dont le groupement protecteur des amines ne peut pas être enlevé. Cette méthode nécessite une protection préalable de l'azote indolique et ne permet pas d'accéder aux dérivés indoliques 1,2-diaminés non protégés **3.**

Les demandes EP 0 122 157, WO 2004/099124, WO 2004/082586 et WO 03/088897 décrivent des composés comprenant un groupement indolique, en tant qu'antibactériens. Cependant, la structure de ces composés est différente de celle des composés de la présente invention.

Ainsi, un des buts de la présente invention consiste à fournir des stratégies de synthèse permettant d'accéder à des dérivés indoliques possédant le motif 1-(3'-indolyl)-1,2-diaminoéthane **3a.**

La présente invention a également pour but de fournir des composés antibactériens de structures nouvelles issues de ces stratégies, présentant soit une activité antibactérienne intrinsèque soit une activité inhibitrice des pompes d'efflux.

La présente description concerne l'utilisation d'au moins un composé de formule (I) suivante : 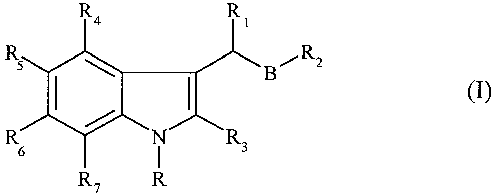 dans laquelle :
- R représente un groupe choisi parmi l'un des groupes suivants :
   * un atome d'hydrogène ;
   * un groupe alkyle comprenant de 1 à 7 atomes de carbone, le cas échéant substitué par un halogène (notamment chlore ou brome), tel que le groupe 2-chloroéthyle (-CH₂CH₂Cl) ;
   * un groupe alcoxyle comprenant de 1 à 7 atomes de carbone, notamment un groupe alcoxyméthyle tel que -OCH₂OMe ou le groupe méthoxyle (-OMe) ;
   * un groupe méthylaryle comprenant de 7 à 11 atomes de carbone, notamment un groupe benzyle ou un groupe -CH₂-C₆H₄-X' avec X' = Cl, Br ou OH) ;
   * un groupe -CH₂-NRₐR_{b}, Rₐ et R_{b} représentant indépendamment l'un de l'autre un atome d'hydrogène ou un groupe alkyle comprenant de 1 à 4 atomes de carbone, notamment un groupe méthyle ; ledit groupe -CH₂-NRₐR_{b} représentant notamment l'un des groupes suivants : -CH₂NH₂, -CH₂-NHMe ou -CH₂-NMe₂ ;
   * un groupe -CH₂CH₂NRₐR_{b}, Rₐ et R_{b} étant tels que définis ci-dessus, ledit groupe -CH₂CH₂-NRₐR_{b} représentant notamment l'un des groupes suivants : -CH₂CH₂-NH₂, -CH₂CH₂-NHMe ou -CH₂CH₂-NMe₂ ;
   * un groupe NRₐR_{b}, Rₐ et R_{b} étant tels que définis ci-dessus ;
   * un groupe -SO₂Ar, Ar représentant un groupe aryle comprenant de 6 à 10 atomes de carbone, Ar représentant notamment un groupe phényle, le cas échéant substitué par un groupe méthyle, notamment le groupe -SO₂Ph ou un groupe -SO₂C₆H₄Me, le groupe méthyle étant de préférence en para ;
   * un groupe choisi parmi : Boc, Fmoc, Cbz, Ac, CF₃CO, C₆H₅CO ;
   * un groupe hydroxyle ou alkoxyle OR_{f}, R_{f} représentant un groupe alkyle comprenant de 1 à 7 atomes de carbone, ledit groupe alkyle pouvant être le cas échéant substitué par un groupe aryle comprenant de 6 à 10 atomes de carbone ou par un groupement NH₂, R_{f} représentant de préférence un groupe méthyle ou un groupe benzyle ; ou
   * un groupe silylé Si(Rg)₃, les groupes Rg pouvant être identiques ou différents et représentant indépendamment les uns des autres un groupe alkyle comprenant de 1 à 6 atomes de carbone, R pouvant représenter par exemple un groupe Me₂*t*-BuSi, *t*-BuPh₂Si ou Si(Et)₃ ;
- R₃ représente un atome d'hydrogène ou un atome d'halogène, notamment Br, Cl, F ou I ;
- R₄, R₅, R₆ et R₇ représentent indépendamment l'un de l'autre un groupe choisi parmi l'un des groupes suivants :
   * un atome d'hydrogène ;
   * un groupe alkyle comprenant de 1 à 4 atomes de carbone, notamment un groupe méthyle ;
   * un groupe trifluorométhyle ;
   * un groupe hydroxyle ;
   * un groupe alcoxyle comprenant de 1 à 7 atomes de carbone, notamment un groupe méthoxyle ou un groupe benzyloxyle ;
   * un groupe hydroxyméthyle (-CH₂OH) ou alcoxyméthyle (-CH₂OR_{f}), R_{f} étant défini tel que ci-dessus ;
   * un groupe trifluorométhoxyle ;
   * un atome d'halogène, notamment Br, Cl, F ou I ;
   * un groupe amino NH₂ ;
   * un groupe *N*-alkylamino NHRₐ, Rₐ étant tel que défini ci-dessus ;
   * un groupe *N,N*-dialkylamino NRₐR_{b}, notamment un groupe *N,N-*diméthylamino, Rₐ et R_{b} étant tels que définis ci-dessus ; ou
- R₁ représente un groupe choisi parmi l'un des groupes suivants :
   * un atome d'hydrogène,
   * un groupe alkyle comprenant de 1 à 6 atomes de carbone, notamment un groupe méthyle, éthyle ou isobutyle ;
   * un groupe méthylaryle comprenant de 7 à 11 atomes de carbone, notamment un groupe benzyle ;
   * un groupe -(CH₂)ₘNH-GP, m étant égal à 1 ou 2, et GP représentant un groupe notamment choisi parmi les groupes suivants : Boc, Cbz, un autre groupe carbamate tel que Me₃SiCH₂CH₂OCO (Teoc), un groupe alkyle comprenant de 1 à 4 atomes de carbone, notamment un groupe méthyle, un groupe benzyle, un groupe acyle comprenant de 1 à 7 atomes de carbone, notamment un groupe acétyle, un groupe benzoyle ou un groupe trifluoroacétyle ;
   * un groupe -(CH₂)ₘNH₂ ou -(CH₂)ₘNH₂.X, X représentant notamment HCl, HCOOH ou HOOCCOOH, m étant tel que défini ci-dessus ;
   * un groupe -(CH₂)ₘN(GP)(GP'), m et GP étant tels que définis ci-dessus, GP' répondant à la même définition que GP, et GP et GP' pouvant être identiques ou différents,
   * un groupe aryle comprenant de 6 à 10 atomes de carbone, éventuellement substitué par un groupe NO₂ ou méthoxyle, et représentant notamment le groupe *p*-nitrophényle 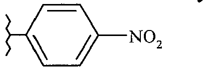
   * un groupe CH₂OH ou CH₂SH,
   * un groupe CH₂O-GP ou CH₂S-GP, GP représentant un groupe choisi parmi : Ac, CF₃CO, C₆H₅CO, CONH₂, *O*- ou *S*-benzyle et CSNH₂,
- R₂ représente un groupe choisi parmi l'un des groupes suivants :
   * un atome d'hydrogène ;
   * un groupe O⁻ ;
   * un groupe OH ;
   * un groupe COCH₂ONH-GP, COCH₂ONH₂, ou COCH₂ONH₂Y, GP représentant l'un des groupes suivants : Boc, Fmoc, Ac, Bz ou CF₃CO et Y représentant HCl, CF₃COOH, HCOOH ou HOOCCOOH; cette fonction oxyamine étant très utilisée pour préparer, après déprotection, des oximes qui peuvent être très fonctionnalisées ;
   * un groupe acyle comprenant de 1 à 10 atomes de carbone, notamment un groupe acétyle, trifluoroacétyle ou benzoyle ;
   * un groupe alcoxyle OR_{c}, R_{c} représentant un groupe alkyle comprenant de 1 à 10 atomes de carbone, notamment un groupe méthyle, ou un groupe benzyle ;
   * un groupe acyloxyle OCOR_{c}, R_{c} étant tel que défini ci-dessus ;
   * un groupe uréido OCONH₂ ;
   * un groupe thiouréido OCSNH₂ ;
- B représente un groupe choisi parmi l'un des groupes suivants : 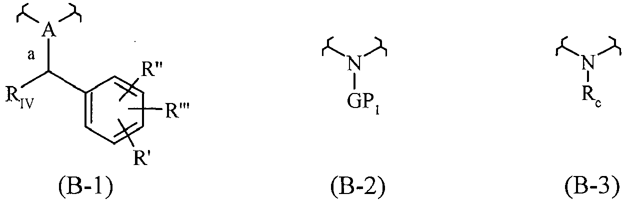
   * a représentant une liaison simple ou une double liaison ;
   * A représentant N ou N⁺ ;
      - a représentant une liaison simple lorsque A représente N ;
      - a représentant une double liaison lorsque A représente N⁺ et R₂ représente O⁻;
   * R', R" et R'" représentant, indépendamment les uns des autres, un groupe choisi parmi l'un des groupes suivants :
      * un atome d'hydrogène ;
      * un groupe OH ;
      * un groupe alcoxyle comprenant de 1 à 4 atomes de carbone, notamment o-méthoxyle, *m*-méthoxyle et *p*-méthoxyle ;
      * un groupe NH₂ ;
      * un groupe *N*-alkylamino NHRₐ, Rₐ étant tel que défini ci-dessus ;
      * un groupe *N,N*-dialkylamino NRₐR_{b}, notamment un groupe *N,N-*diméthylamino, Rₐ et R_{b} étant tels que définis ci-dessus,
      les groupes R' et R" représentant de préférence ensemble l'un des groupes suivants : *o,p*-diméthoxyle (2,4-diméthoxyle) ; di-*m*-diméthoxyle (3,5-diméthoxyle) ; *m,p*-diméthoxyle (3,4-diméthoxyle) ou OCH₂O (méthylènedioxyle), R'" représentant alors un atome d'hydrogène ;
      les groupes R', R" et R'" représentant de préférence ensemble l'un des groupes suivants : 3,4,5-triméthoxyle ; 3,5-diméthoxy-4-hydroxyle ou 3,4,5-trihydroxyle ;
      étant entendu que, de préférence, les groupes R', R" et R'" ne représentent pas de groupes NO₂,
   * R_{IV} représentant l'un des groupes suivants :
      * un atome d'hydrogène,
      * un groupe alkyle comprenant de 1 à 4 atomes de carbone, notamment un groupe méthyle ;
      * un groupe aryle comprenant de 6 à 10 atomes de carbone, notamment un groupe phényle,
      * un groupe CH₂OR_{d}, R_{d} représentant un groupe choisi parmi :
         - un groupe alkyle comprenant de 1 à 7 atomes de carbone, notamment un groupe méthyle,
         - un groupe méthylaryle comprenant de 7 à 11 atomes de carbone, notamment un groupe benzyle, ou
         - un groupe Si(Rₑ)₃, les groupes Rₑ pouvant être identiques ou différents et représentant, indépendamment les uns des autres, un groupe alkyle comprenant de 1 à 6 atomes de carbone, Si(Rₑ)₃ étant notamment un groupe Me₂*t*-BuSi ou Ph₂*t*-BuSi ;
   * GP₁ représentant un groupe Boc ou Cbz ;
   * R_{c} représentant un atome d'hydrogène ou un groupe alkyle comprenant de 1 à 4 atomes de carbone, notamment un groupe méthyle ou *t*-butyle ;
lesdits composés de formule (I) pouvant être sous forme d'isomères optiques, à savoir sous forme d'énantiomères et de diastéréoisomères ou de mélanges de ces différentes formes, y compris de mélanges racémiques, ou sous forme, le cas échéant, de sels d'acides physiologiquement acceptables tels que des chlorhydrates, des formiates ou des oxalates (HOOCCOOH),
pour la préparation d'un médicament destiné au traitement de pathologies associées à des infections bactériennes, en particulier pour le traitement de maladies bactériennes.

La présente invention concerne l'utilisation d'au moins un composé de formule (I) suivante : 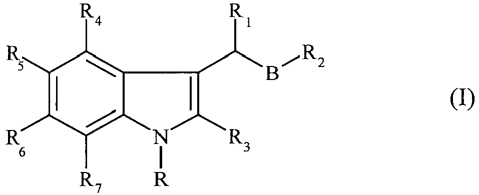 dans laquelle :
- R représente un atome d'hydrogène;
- R₃ représente un atome d'hydrogène;
- R₄ et R₇ repésentent un atome d'hydrogène;
- R₅ et R₆ représentent indépendamment l'un de l'autre un groupe choisi parmi l'un des groupes suivants:
   * un atome d'hydrogène;
   * un groupe alcoxyle comprenant de 1 à 7 atomes de carbone ou un groupe benzyloxyle;
   * un atome d'halogène choisi parmi Br, Cl, F ou I;
- R₁ représente un groupe choisi parmi l'un des groupes suivants:
   * un groupe -CH₂₋NH-GP, GP représentant un groupe choisi parmi les groupes suivants : Boc, Cbz, ou Me₃SiCH₂CH₂OCO (Teoc);
   * un groupe -CH₂-NH₂ ou -CH₂-NH₂.X, X représentant HCl, HCOOH ou HOOCCOOH;
- R₂ représente un groupe choisi parmi l'un des groupes suivants :
   * un atome d'hydrogène;
   * un groupe O⁻ ;
   * un groupe OH;
   * un groupe acyle comprenant de 1 à 10 atomes de carbone;
- B représente un groupe choisi parmi l'un des groupes suivants: 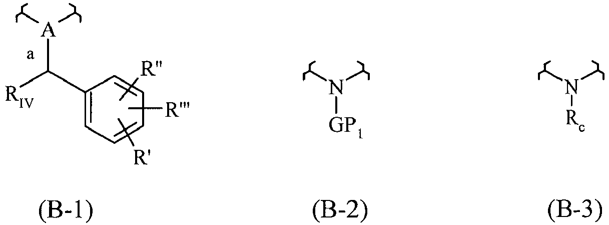
   * a représentant une liaison simple ou une double liaison;
   * A représentant N ou N⁺ ;
      - a représentant une liaison simple lorsque A représente N;
      - a représentant une double liaison lorsque A représente N⁺ et R₂ représente O⁻ ;
   * R', R" et R"' représentant, indépendamment les uns des autres, un groupe choisi parmi l'un des groupes suivants:
      * un atome d'hydrogène;
      * un groupe alcoxyle comprenant de 1 à 4 atomes de carbone;
      * R_{IV} représentant un atome d'hydrogène;
      * GP₁ représentant un groupe Boc ou Cbz;
      * R_{c} représentant un atome d'hydrogène;
lesdits composés de formule (I) pouvant être sous forme d'isomères optiques, à savoir sous forme d'énantiomères ou de mélanges de ces énantiomères, y compris le mélange racémique, ou sous forme, le cas échéant, de sels d'acides physiologiquement acceptables,
pour la préparation d'un médicament destiné au traitement de pathologies associées à des infections bactériennes.

Les composés de formule (I) peuvent donc être utilisés comme médicaments pour le traitement des maladies choisies notamment parmi la liste suivante : infection à germes sensibles, infection urinaire, cystite aiguë, pyélonéphrite, infection bronchopulmonaire, infection à staphylocoques, dysenterie bacillaire, sinusite, otite, infection à méningocoques, diarrhée des voyageurs, charbon et choléra.

Les produits de l'invention peuvent être utilisés comme médicaments dans le traitement des affections à germes sensibles provoquées par des bactéries à Gram (+) et notamment dans celui des staphylococcies, telles que septicémies à staphylocoques, staphylococcies malignes de la face ou cutanées, pyodermites, plaies septiques ou suppurantes, anthrax, phlegmons, érysipèles, staphylococcies aiguës primitives ou post grippales, broncho-pneumonies, suppurations pulmonaires.

Ces produits peuvent également être utilisés comme médicaments dans le traitement des colibacilloses et infections associées, dans les infections à proteus, à klebsiella et à salmonella et dans d'autres affections provoquées par des bactéries à Gram (-).

Dans la formule (I) telle que définie ci-dessus, les substituants R₄, R₅, R₆ et R₇ peuvent également représenter un groupe CHO.

Lorsque R représente un groupe -SO₂Ar, Boc, Fmoc, Cbz, Ac, CF₃CO, C₆H₅CO, ces groupes sont des groupes protecteurs dans la synthèse mais ils peuvent également être nécessaires à l'activité biologique.

De même, lorsque R₁ représente un groupe -(CH₂)ₘNH-GP, GP est un groupe protecteur dans la synthèse mais celui-ci peut également être nécessaire à l'activité biologique.

De préférence, les composés de formule (I) ne répondent pas à la formule suivante :

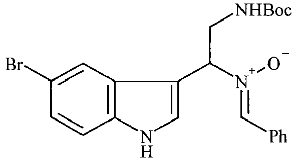

Comme exemples de sels d'addition avec des acides pharmaceutiquement acceptables, on peut citer les sels formés avec les acides minéraux (chlorhydrates, bromhydrates, sulfates, nitrates, phosphates) ou avec les acides organiques (oxalates, succinates, fumarates, tartrates, formiates, acétates, propionates, maléates, citrates, méthanesulfonates, éthanesulfonates, phénylsulfonates, *p*-toluènesulfonates, iséthionates (ou éthanolsulfonates), napthylsulfonates ou camphresulfonates, ou avec des dérivés de substitution de ces composés).

La présente invention concerne donc, à titre de médicaments et notamment de médicaments destinés au traitement des infections bactériennes chez l'homme ou l'animal, les composés de formule (I) telle que définie ci-dessus.

Afin de préparer les composés de formule (I) susmentionnée, dans laquelle R représente un groupe alkyle comprenant de 1 à 7 atomes de carbone, on peut procéder par une *N*-alkylation des indoles selon l'article Y. Kikugawa, Y. Miyake Synthesis 1981, 461-462. Lorsque R représente un groupe benzyle, on peut faire référence au même article : Y. Kikugawa, Y. Miyake Synthesis 1981, 461-462.

Afin de préparer les composés de formule (I) susmentionnée, dans laquelle R représente un groupe alkyle comprenant de 1 à 7 atomes de carbone substitué par un chlore, un groupe hydroxyle ou alcoxyle, on peut se référer à : T. W. Greene, P. M. Wuts, « Protective Groups in Organic Synthesis », 3rd Edition, John Wiley & Sons, Inc., 1999 : chapitre 7, pages 615-631, en particulier pages 619 et 624-626 ; lorsque R représente un groupe 2-chloroéthyle, on peut se référer à la publication suivante : M. A. de la Mora, E. Cuevas, J. M. Muchowski, R. Cruz-Almanza Tetrahedron Lett. 2001, 42, 5351-5353.

Afin de préparer les composés de formule (I) susmentionnée, dans laquelle R représente un groupe -CH₂CH₂-NRₐR_{b}, on peut faire référence à R. A. Glennon, J. M. Jacyno, R. Young, J. D. McKenney, D. Nelson J. Med. Chem. 1984, 27, 41-45.

Afin de préparer les composés de formule (I) susmentionnée, dans laquelle R représente un groupe -NRₐR_{b}, on peut faire référence à :
- J. Hynes, Jr., W. W. Doubleday, A. J. Dyckman, J. D. Godfrey, Jr., J. A. Grosso, S. Kiau, K. Leftheris J. Org. Chem. 2004, 69, 1368-1371 et J. T. Klein, L. Davis, G. E. Olsen, G. S. Wong, F. P. Huger, C. P. Smith, W. W. Petko, M. Cornfeldt, J. C. Wilker, R. D. Blitzer, E. Landau, V. Haroutunian, L. L. Martin, R. C. Effland J. Med. Chem. 1996, 39, 570-581 pour le groupe -NH₂,
- M. Somei, M. Natsume Tetrahedron Lett. 1974, 3605-3608 pour le groupe -NHRₐ et
- M. Watanabe, T. Yamamoto, M. Nishiyama Angew. Chem. Int. Ed. 2000, 39, 2501-2504 et J. T. Klein, L. Davis, G. E. Olsen, G. S. Wong, F. P. Huger, C. P. Smith, W. W. Petko, M. Cornfeldt, J. C. Wilker, R. D. Blitzer, E. Landau, V. Haroutunian, L. L. Martin, R. C. Effland J. Med. Chem. 1996, 39, 570-581 pour le groupe -NRₐR_{b}.

Afin de préparer les composés de formule (I) susmentionnée, dans laquelle R représente un groupe -CH₂-NRₐR_{b}, on peut faire référence à B. E. Love, B. T. Nguyen Synlett 1998, 1123-1125 et à K. S. Jandu, V. Barrett, M. Brockwell, D. Cambridge, D. R. Farrant, C. Foster, H. Giles, R. C. Glen, A. P. Hill, H. Hobbs, A. Honey, G. R. Martin, J. Salmon, D. Smith, P. Woollard, D. L Selwood J. Med. Chem. 2001, 44, 681-693 ; pour R= -CH₂-NHRₐ, on peut se référer à L. E. Overman, R. M. Burk Tetrahedron Lett. 1984, 25, 1635-1638 ; ou pour R=-CH₂-NMe₂ à la publication suivante : A. R. Katritzky, P. Lue, Y.-X. Chen J. Org. Chem. 1990, 55, 3688-3691. En ce qui concerne la monoalkylation (notamment monométhylation) des -CH₂-NHRₐ, on peut se référer à M. Kurosu, S. S. Dey, D. C. Crick Tetrahedron Lett. 2006, 47, 4871-4875.

Afin de préparer les composés de formule (I) susmentionnée, dans laquelle R représente un groupe -SO₂Ar, on peut se référer aux publications décrites dans : T. W. Greene, P. M. Wuts, « Protective Groups in Organic Synthesis », Third Edition, John Wiley & Sons, Inc., 1999 : chapitre 7, pages 616-617. On peut également se référer à S. Roy, G. W. Gribble Tetrahedron Lett. 2005, 46, 1325-1328 ou à R. Liu, P. Zhang, T. Gan, J. M. Cook J. Org. Chem. 1997, 62, 7447-7456, pour la *N*-phénylsulfonylation et à Y. Kikugawa Synthesis 1981, 460-461 ou à E. V. Sadanandan, S. K. Pillai, M. V. Lakshmikantham, A. D. Billimoria, J. S. Culpepper, M. P. Cava J. Org. Chem. 1995, 60, 1800-1805, pour la *N*-(*p*-toluène)sulfonylation.

Afin de préparer les composés de formule (I) susmentionnée, dans laquelle R représente un groupe Boc, on peut se référer aux publications suivantes : D. Dhanak, C. B. Reese J. Chem. Soc., Perkin Trans. 1 1986, 2181-218 ; P. Zhang, R. Liu, J. M. Cook Tetrahedron Lett. 1995, 36, 9133-9136 ; L. Grehn, U. Ragnarsson Angew. Chem. Int. Ed. Engl. 1984, 23, 296-301, S. Roy, G. W. Gribble Tetrahedron Lett. 2005, 46, 1325-1328 ; R. Liu, P. Zhang, T. Gan, J. M. Cook J. Org. Chem. 1997, 62, 7447-7456 ; lorsque R représente Ac ou C₆H₅CO, on peut se référer à la publication suivante : Y. Kikugawa Synthesis 1981, 460-461.

Afin de préparer les composés de formule (I) susmentionnée, dans laquelle R représente un groupe hydroxyle, on peut se référer à la publication suivante : M. Somei, F. Yamada, T. Kurauchi, Y. Nagahama, M. Hasegawa, K. Yamada, S. Teranishi, H. Sato, C. Kaneko Chem. Pharm. Bull. 2001, 49, 87-96.

Afin de préparer les composés de formule (I) susmentionnée, dans laquelle R représente un groupe méthoxyle, on peut se référer à la publication R. M. Acheson, P. G. Hunt, D. M. Littlewood, B. A. Murrer, H. E. Rosenberg J. Chem. Soc., Perkin Trans. I 1978, 1117-1125.

Afin de préparer les composés de formule (I) susmentionnée, dans laquelle R représente un groupe trialkylsilyle, on peut se référer aux publications décrites dans : T. W. Greene, P. M. Wuts, « Protective Groups in Organic Synthesis », 3rd Edition, John Wiley & Sons, Inc., 1999 : chapitre 7, page 620. Ainsi, par exemple, lorsque R représente un groupe Me₂*t*-BuSi, on peut se référer à D. Dhanak, C. B. Reese J. Chem. Soc., Perkin Trans.1 1986, 2181-2186; P. Ashworth, B. Broadbelt, P. Jankowski, P. Kocienski, A. Pimm, R. Bell Synthesis 1995, 199-206 ou Y. Hirai, K. Yokota, T. Momose Heterocycles 1994, 39, 603-612.

Afin de préparer les composés de formule (I) susmentionnée, dans laquelle R₃ représente un atome d'halogène, on peut se référer aux publications suivantes : Pour la synthèse du 2-chloroindole, du 2-bromoindole et du 2-iodoindole, on peut se référer à J. Bergman, L. Venemalm J. Org. Chem. 1992, 57, 2495-2497 ; pour la synthèse de 2-iodoindoles : T. Kline J. Heterocyclic Chem. 1985, 22, 505-509 ; pour la synthèse de 2-bromoindoles : R. Liu, P. Zhang, T. Gan, J. M. Cook J. Org. Chem. 1997, 62, 7447-7456 ; P. Zhang, R. Liu, J. M. Cook Tetrahedron Lett. 1995, 36, 3103-3106 et P. Zhang, R. Liu, J. M. Cook Tetrahedron Lett. 1995, 36, 9133-9136 ; pour les halogénations en position 2 du noyau indolique (Cl, Br, I) : G. Palmisano, B. Danieli, G. Lesma, G. Fiori Synthesis 1987, 137-139 ; pour la monobromation en position 2 du noyau indolique ou dibromation en positions 2 et 6 du noyau indolique : A. G. Mistry, K. Smith, M. R. Bye Tetrahedron Lett. 1986, 27, 1051-1054.

Pour les composés de formule (I) dans laquelle R₅, R₆ ou R₇ représente un groupe méthyle, il y a lieu de noter que les 5-, 6- et 7-méthylindoles sont commercialisés par Alfa Aesar, a Johnson Matthey Company.

De même, les 4-, 5- et 6-méthoxyindoles sont commercialisés par Alfa Aesar, a Johnson Matthey Company (pour les composés de formule (I) dans laquelle R₄, R₅ ou R₆ représente un groupe méthoxyle).

Afin de préparer les composés de formule (I) susmentionnée, dans laquelle R₅, R₆ ou R₇ représente un groupe trifluorométhyle, on peut se référer aux publications suivantes pour préparer les noyaux indoliques de départ : 5-trifluorométhylindole : A. Walkington, M. Gray, F. Hossner, J. Kitteringham, M. Voyle Synth. Commun. 2003, 33, 2229-2233 ; 6-trifluorométhylindole : A. Walkington, M. Gray, F. Hossner, J. Kitteringham, M. Voyle Synth. Commun. 2003, 33, 2229-2233 (il y a lieu de noter qu'il est commercialisé chez Alfa Aesar, a Johnson Matthey Company) ; 7-trifluorométhylindole : A. P. Dobbs, M. Voyle, N. Whittall Synlett 1999, 1594-1596.

Pour les composés de formule (I) dans laquelle R₄, R₅, R₆ ou R₇ représente Br, il y a lieu de noter que les 4-, 5-, 6- et 7-bromoindoles sont commercialisés par Alfa Aesar, a Johnson Matthey Company. Par ailleurs, la synthèse des 4-, 5-, 6-, ou 7-bromoindoles est décrite dans : M. P. Moyer, J. F. Shiurba, H. Rapoport J. Org. Chem. 1986, 51, 5106-5110. Les 4-, 5- et 6-chloroindoles, ainsi que les 5-, 6- et 7-fluoroindoles sont commercialisés par Alfa Aesar, a Johnson Matthey Company. Le 5-iodoindole est commercialisé par Aldrich.

Pour les composés de formule (I) dans laquelle R₄, R₅, R₆ ou R₇ représente I, ils pourraient être préparés à partir des nitroindoles correspondants selon la procédure décrite dans l'article K. Kato, M. Ono, H. Akita Tetrahedron lett. 1997, 38, 1805-1808. Les 4-, 5-, 6- et 7-nitroindoles sont commercialisés par Alfa Aesar, a Johnson Matthey Company.

De même, les composés de formule (I) dans laquelle R₄, R₅, R₆ ou R₇ représente un groupe amino (NH₂) sont commercialisés chez Alfa Aesar, a Johnson Matthey Company. Pour la synthèse du groupe *N*-alkylamino (-NHRₐ) à partir de l'amine primaire par amination réductrice, on peut se référer à la publication suivante : R. F. Borch, M. D. Bernstein, H. D. Durst J. Am. Chem. Soc. 1971, 93, 2897-2904. On peut également se référer à A. R. Katritzky, S. Rachwal, B. Rachwal J. Chem. Soc., Perkin Trans. 1 1987, 805-809. Pour la synthèse du groupe *N*-éthylamino à partir de l'amine primaire par amination réductrice, on peut se référer à la publication suivante : K. C. Nicolaou, R. D. Groneberg, N. A. Stylianides, T. Miyazaki J. Chem. Soc., Chem. Commun. 1990, 1275-1277 et pour la monométhylation : R. N. Salvatore, A. S. Nagle, S. E. Schmidt, K. W. Jung Org. Lett. 1999, 1, 1893-1896 ; Revue : R. N. Savatore, C. H. Yoon, K. W. Jung Tetrahedron 2001, 57, 7785-7811.

Le groupe *N,N*-diméthylamino peut être préparé directement à partir de l'amine correspondante selon l'article suivant : K. S. Jandu, V. Barrett, M. Brockwell, D. Cambridge, D. R. Farrant, C. Foster, H. Giles, R. C. Glen, A. P. Hill, H. Hobbs, A. Honey, G. R. Martin, J. Salmon, D. Smith, P. Woollard, D. L Selwood J. Med. Chem. 2001, 44, 681-693, ou à partir du groupe *N*-méthylamino : M. Kurosu, S. S. Dey, D. C. Crick Tetrahedron Lett. 2006, 47, 4871-4875. Cette méthode permet la synthèse d'amines tertiaires différemment substituées (Rₐ ≠ R_{b}) par *N*-alkylation d'amines secondaires).

Selon un mode de réalisation préféré, la présente description concerne l'utilisation telle que définie ci-dessus d'un composé de formule (I-1') suivante : 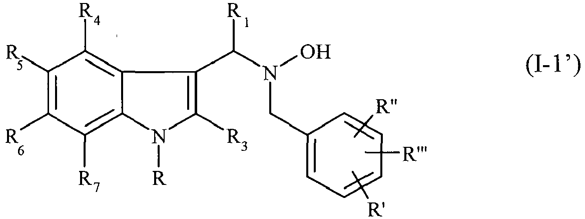 R, R₁, R₃, R₄, R₅, R₆, R₇, R', R" et R'" étant tels que définis ci-dessus.

Les composés de formule (I-1') sont des *N*-hydroxylamines indoliques, correspondant à des composés de formule (I) dans laquelle :
- R₂ représente un groupe OH ;
- B représente un groupe de formule (B-1) avec R_{IV} = H ;
- A représente un atome d'azote N ; et
- a représente une liaison simple.

Selon un autre mode de réalisation préféré, la présente description concerne l'utilisation telle que définie ci-dessus d'un composé de formule (I-1) suivante : 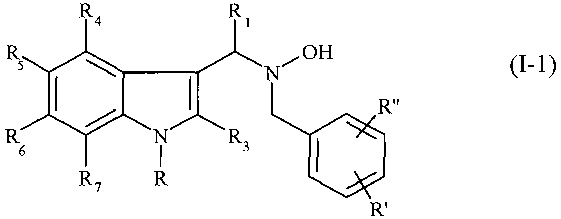 R, R₁, R₃, R₄, R₅, R₆, R₇, R' et R" étant tels que définis ci-dessus.

Les composés de formule (I-1) sont des *N*-hydroxylamines indoliques, correspondant à des composés de formule (I) dans laquelle :
- R₂ représente un groupe OH ;
- B représente un groupe de formule (B-1) avec R_{IV} = R"' = H ;
- A représente un atome d'azote N ; et
- a représente une liaison simple.

Ainsi, les composés de formule (I-1) correspondent à des composés de formule (I-1') dans laquelle R'" représente un atome d'hydrogène.

Selon un mode de réalisation préféré, les composés de formule (I-1) répondent à la formule donnée ci-dessus dans laquelle :
- R, R₃, R₄ et R₇ représentent un atome d'hydrogène ;
- R₅ représente un atome d'hydrogène, un groupe méthoxyle, un atome de brome ou un atome de chlore ;
- R₆ représente un atome d'hydrogène ou un atome de brome ;
- R₁ représente un groupe méthyle, isobutyle, *p*-nitrophényle ou CH₂NHBoc ;
- R' et R" représentent indépendamment l'un de l'autre un atome d'hydrogène ou un groupe méthoxyle ou R' et R" représentent ensemble un groupe *o,p-*diméthoxyle ou un groupe di-*m*-méthoxyle,
étant entendu, de préférence, que lorsque R₆ représente un atome de brome, R₅ représente alors un atome d'hydrogène,
et que lorsque R₅ représente un groupe méthoxyle, un atome de brome ou un atome de chlore, R₆ représente alors un atome d'hydrogène.

De tels composés forment une sous-famille répondant à la formule générale (I-1-a) suivante : 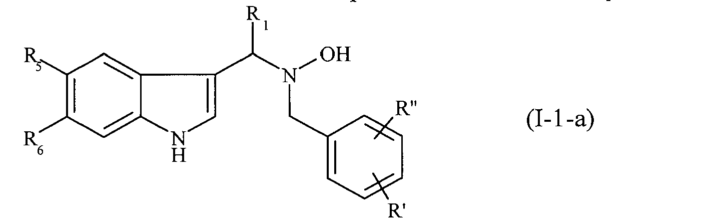 R₁, R₅, R₆, R' et R" étant tels que définis ci-dessus.

Selon un autre mode de réalisation préféré, la présente invention concerne l'utilisation telle que définie ci-dessus d'un composé de formule (I-1) suivante : 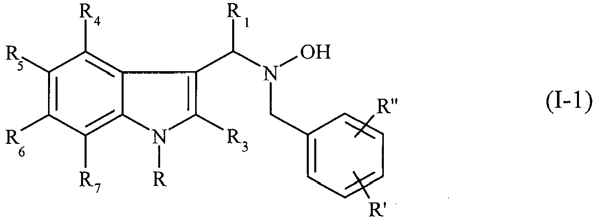 ledit composé de formule (I-1) étant caractérisé en ce que:
- R, R₃, R₄ et R₇ représentent un atome d'hydrogène;
- R₅ représente un atome d'hydrogène, un groupe méthoxyle, un atome de brome ou un atome de chlore;
- R₆ représente un atome d'hydrogène ou un atome de brome;
- R₁ représente CH₂NHBoc;

R' et R" représentent indépendamment l'un de l'autre un atome d'hydrogène ou un groupe méthoxyle ou R' et R" représentent ensemble un groupe *o,p*-diméthoxyle ou un groupe di-*m*-méthoxyle.

La présente description concerne l'utilisation telle que définie ci-dessus d'au moins un composé répondant à l'une des formules suivantes :

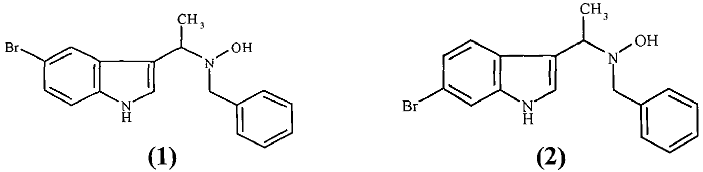

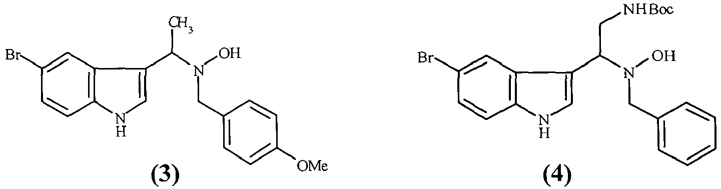

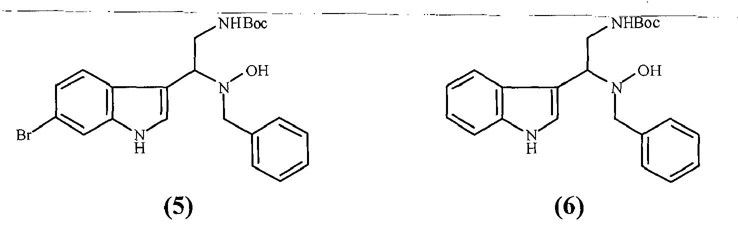

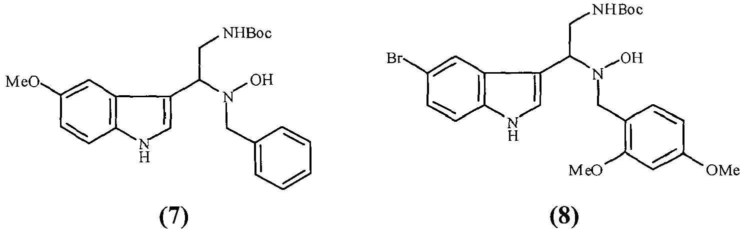

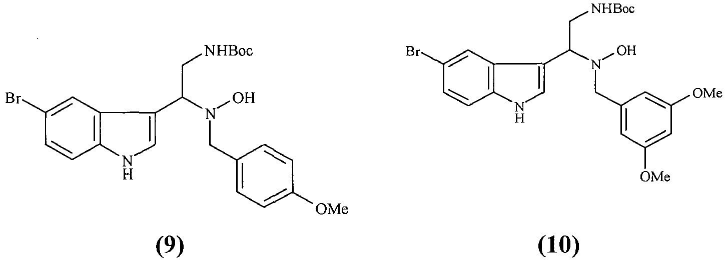

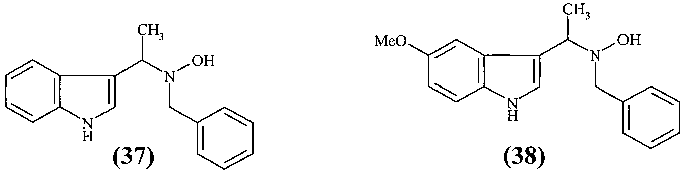

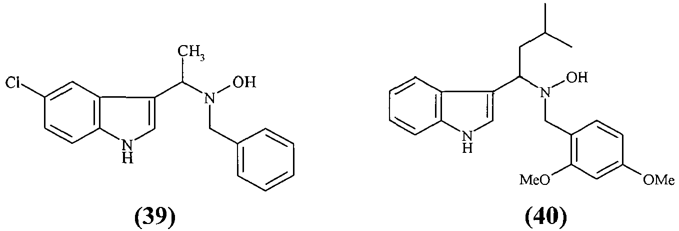

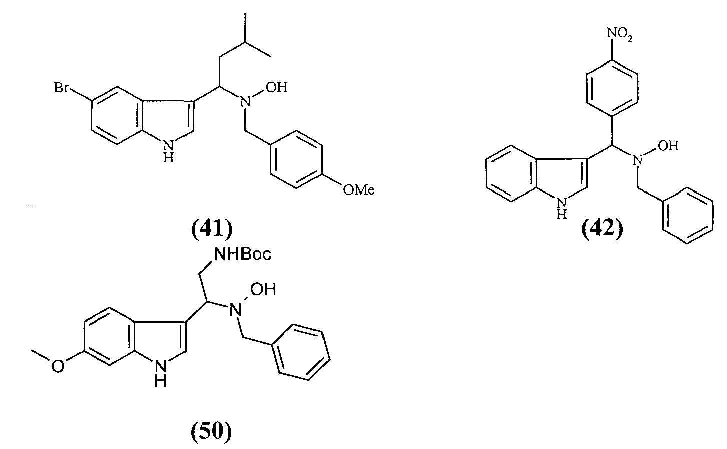

La présente invention concerne l'utilisation telle que définie ci-dessus d'au moins un composé répondant à l'une des formules suivantes :

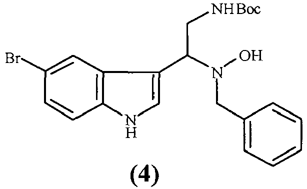

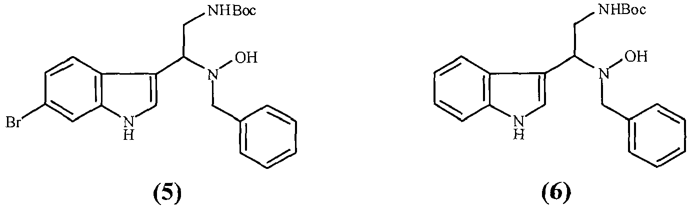

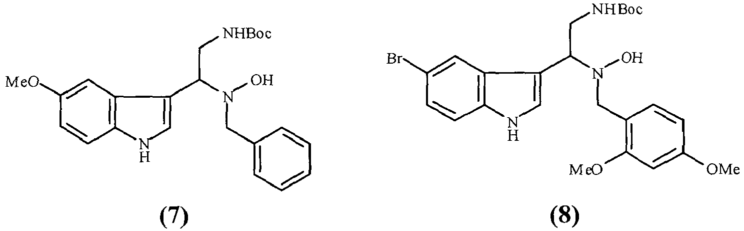

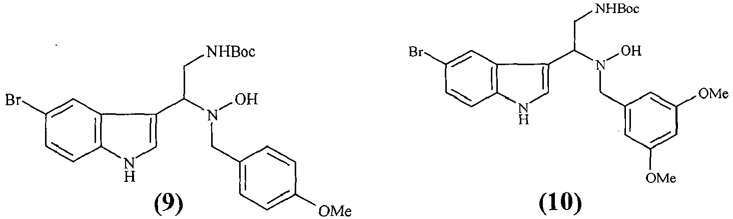

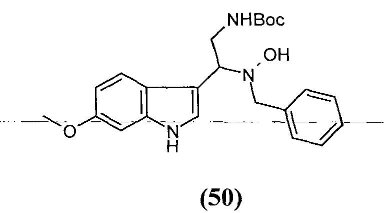

Selon un mode de réalisation préféré, la présente description concerne l'utilisation telle que définie ci-dessus d'un composé de formule (I-2-bis) suivante : 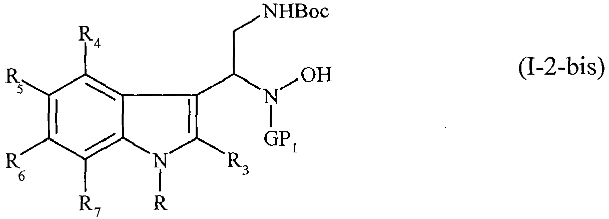 R, R₃, R₄, R₅, R₆, R₇ et GP₁ étant tels que définis ci-dessus.

Les composés de formule (I-2-bis) sont des *N*-(Boc)hydroxylamines indoliques β-aminées, correspondant à des composés de formule (I) dans laquelle :
- R₁ représente un groupe CH₂NHBoc ;
- R₂ représente un groupe OH ; et
- B représente un groupe de formule (B-2).

Selon un mode de réalisation préféré, la présente invention concerne l'utilisation telle que définie ci-dessus d'un composé de formule (I-2-bis) suivante : 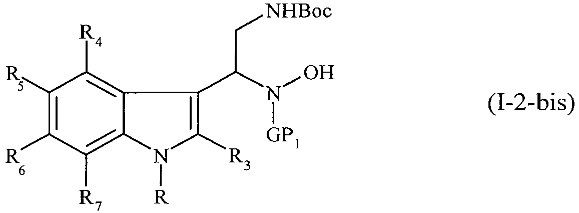 GP₁ représentant un groupe Boc, ledit composé de formule (I-2-bis) étant caractérisé en ce que :
- R, R₃, R₄ et R₇ représentent un atome d'hydrogène;
- R₅ représente un atome d'hydrogène, un atome de brome ou un atome de chlore;
- R₆ représente un atome d'hydrogène ou un atome de brome.

Selon un autre mode de réalisation préféré, la présente description concerne l'utilisation telle que définie ci-dessus d'un composé de formule (I-2) suivante : 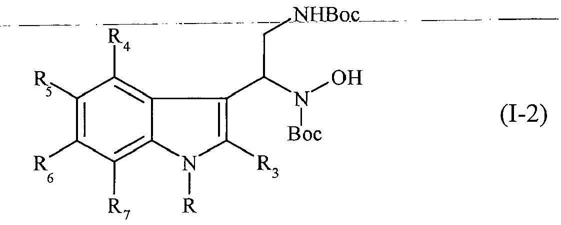 R, R₃, R₄, R₅, R₆ et R₇ étant tels que définis ci-dessus.

Les composés de formule (I-2) correspondent à des composés de formule (I) dans laquelle :
- R₁ représente un groupe CH₂NHBoc ;
- R₂ représente un groupe OH ; et
- B représente un groupe de formule (B-2) avec GP₁ = Boc.

La présente invention concerne également l'utilisation telle que définie ci-dessus, d'un composé de formule (I-2) dans laquelle :
- R, R₃, R₄ et R₇ représentent un atome d'hydrogène ;
- R₅ représente un atome d'hydrogène, un atome de brome ou un atome de chlore ;
- R₆ représente un atome d'hydrogène ou un atome de brome,
étant entendu, de préférence, que lorsque R₆ représente un atome de brome, R₅ représente alors un atome d'hydrogène,
et que lorsque R₅ représente un atome de brome ou un atome de chlore, R₆ représente alors un atome d'hydrogène.

De tels composés forment une sous-famille répondant à la formule générale (I-2-a) suivante : 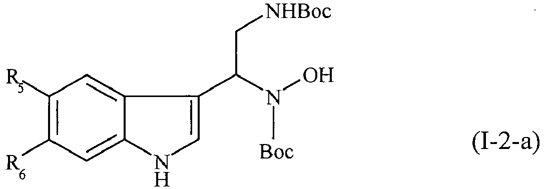 R₅ et R₆ étant tels que définis ci-dessus.

Parmi les composés de formule (I-2-a) telle que définie ci-dessus, la présente invention concerne l'utilisation telle que définie ci-dessus d'au moins un composé répondant à l'une des formules suivantes :

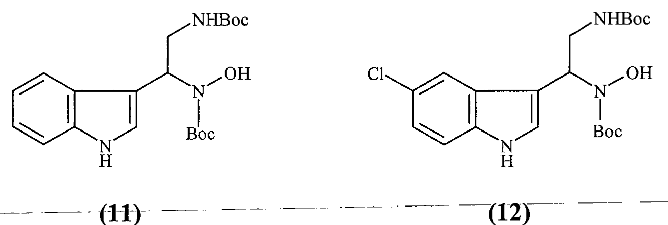

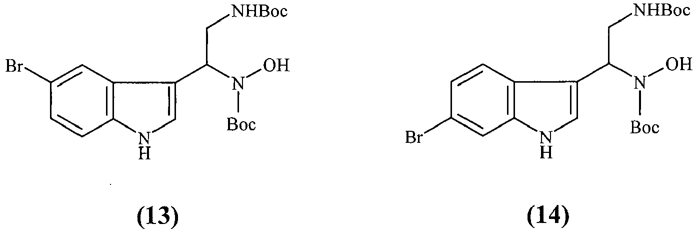

La présente description concerne également l'utilisation telle que définie ci-dessus d'un composé de formule (I-3-bis) suivante : 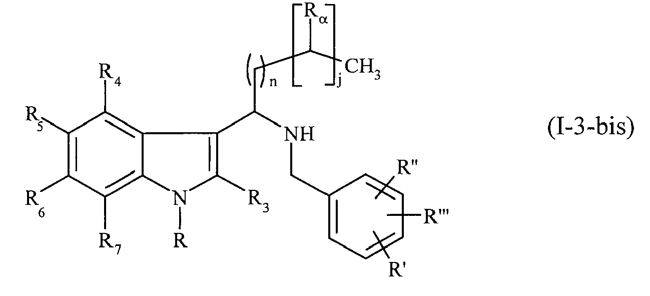 dans laquelle :
- n est égal à 0, 1 ou 2 ;
- j est égal à 0 ou 1 ;
- R, R₃, R₄, R₅, R₆ et R₇ sont tels que définis ci-dessus dans la formule (I),
- R_{α} représentant un atome d'hydrogène ou un groupe méthyle ou éthyle,
- R', R" et R'" sont tels que définis ci-dessus dans la formule (I).

De préférence, dans la formule (I-3-bis), R' est un groupe méthoxyle, de préférence en para, et R" et R"' sont des atomes d'hydrogène.

Selon un autre mode de réalisation, dans la formule (I-3-bis), les groupes R' et R" représentent de préférence ensemble l'un des groupes suivants : *o,p*-diméthoxyle (2,4-diméthoxyle) ; di-*m*-diméthoxyle (3,5-diméthoxyle) ; *m,p*-diméthoxyle (3,4-diméthoxyle) ou OCH₂O et R"' est alors un atome d'hydrogène.

Selon un autre mode de réalisation, dans la formule (I-3-bis), les groupes R', R" et R"' représentent ensemble les groupes 3,4,5-triméthoxyle, 3,5-diméthoxy-4-hydroxyle ou 3,4,5-trihydroxyle.

Les composés de formule (I-3-bis) sont des amines indoliques protégées, correspondant à des composés de formule (I) dans laquelle :
- R₁ représente un groupe alkyle ;
- R₂ représente un atome d'hydrogène ;
- B représente un groupe de formule (B-1) avec R_{IV} = H ;
- A représente un atome d'azote N ; et
- a représente une liaison simple.

Selon un mode de réalisation avantageux, la présente description concerne également l'utilisation telle que définie ci-dessus d'un composé de formule (I-3) suivante : dans laquelle : 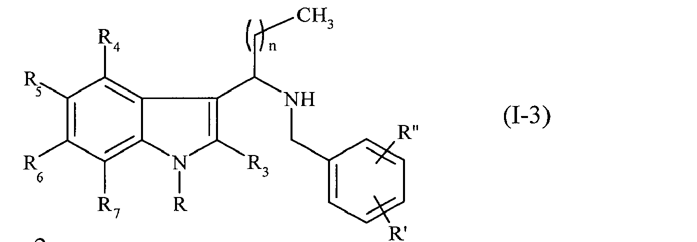
- n est égal à 0, 1 ou 2,
- R, R₃, R₄, R₅, R₆ et R₇ sont tels que définis ci-dessus dans la formule (I),
- R' et R" sont tels que définis ci-dessus dans la formule (I).

De préférence, dans la formule (I-3), R' est un groupe méthoxyle, de préférence en para, et R" est un atome d'hydrogène.

Selon un autre mode de réalisation, dans la formule (I-3), les groupes R' et R" représentent de préférence ensemble l'un des groupes suivants : *o,p*-diméthoxyle (2,4-diméthoxyle) ; di-*m*-diméthoxyle (3,5-diméthoxyle) ; *m,p*-diméthoxyle (3,4-diméthoxyle) ou OCH₂O.

Les composés de formule (I-3) sont des amines indoliques protégées, correspondant à des composés de formule (I) dans laquelle :
- R₁ représente un groupe alkyle ;
- R₂ représente un atome d'hydrogène ;
- B représente un groupe de formule (B-1) avec R_{IV} = R"' = H ;
- A représente un atome d'azote N ; et
- a représente une liaison simple.

Les composés de formule (I-3) correspondent à des composés de formule (I-3-bis) dans laquelle j = 0 et R"' = H.

Selon un mode de réalisation préféré, la description concerne l'utilisation telle que définie ci-dessus d'un composé de formule (I-3) dans laquelle :
- R' et R" représentent un atome d'hydrogène ;
- R, R₃, R₄ et R₇ représentent un atome d'hydrogène ;
- R₅ représente un atome d'hydrogène ou un atome de brome ;
- R₆ représente un atome d'hydrogène ou un atome de brome ;
- étant entendu, de préférence, que lorsque R₆ représente un atome de brome, R₅ représente alors un atome d'hydrogène,
et que lorsque R₅ représente un atome de brome, R₆ représente alors un atome d'hydrogène.

De tels composés forment une sous-famille répondant à la formule générale (I-3-a) suivante : 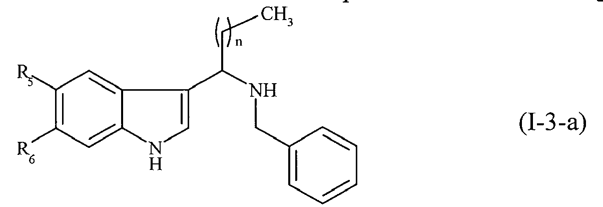 R₅ et R₆ étant tels que définis ci-dessus.

La présente description concerne également l'utilisation telle que définie ci-dessus d'au moins un composé répondant à l'une des formules suivantes :

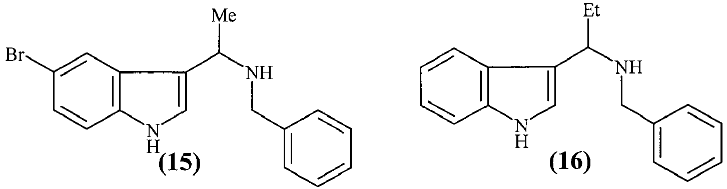

La présente description concerne également l'utilisation telle que définie ci-dessus, d'un composé de formule (I-4-bis) suivante : dans laquelle : 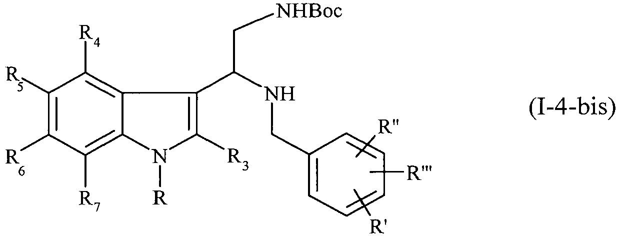
- R, R₃, R₄, R₅, R₆ et R₇ sont tels que définis ci-dessus dans la formule (I),
- R', R" et R"' sont tels que définis ci-dessus dans la formule (I).

De préférence, dans la formule (I-4-bis), R' est un groupe méthoxyle, de préférence en para, et R" et R"' sont des atomes d'hydrogène.

Selon un autre mode de réalisation, dans la formule (I-4-bis), les groupes R' et R" représentent de préférence ensemble l'un des groupes suivants : *o,p*-diméthoxyle (2,4-diméthoxyle) ; di-*m*-diméthoxyle (3,5-diméthoxyle) ; *m*,*p*-diméthoxyle (3,4-diméthoxyle) ou OCH₂O et R"' est alors un atome d'hydrogène.

Selon un autre mode de réalisation, dans la formule (I-3-bis), les groupes R', R" et R'" représentent ensemble les groupes 3,4,5-triméthoxyle, 3,5-diméthoxy-4-hydroxyle ou 3,4,5-trihydroxyle.

Les composés de formule (I-4-bis) sont des 1,2-diamines indoliques diprotégées, correspondant à des composés de formule (I) dans laquelle :
- R₁ représente un groupe CH₂NHBoc ;
- R₂ représente un atome d'hydrogène ;
- B représente un groupe de formule (B-1) avec R_{IV} = H ;
- A représente un atome d'azote N ; et
- a représente une liaison simple.

Selon un mode de réalisation avantageux, la présente description concerne l'utilisation telle que définie ci-dessus d'un composé de formule (I-4) suivante : 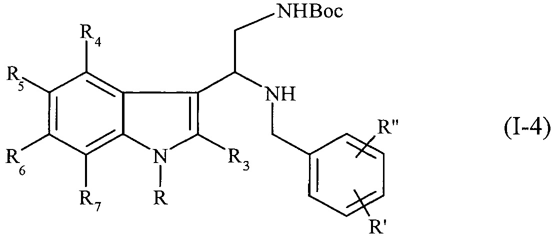 dans laquelle :
- R, R₃, R₄, R₅, R₆ et R₇ sont tels que définis ci-dessus dans la formule (I),
- R' et R" sont tels que définis ci-dessus dans la formule (I),

De préférence, dans la formule (I-4), R' est un groupe méthoxyle, de préférence en para, et R" et R'" sont des atomes d'hydrogène.

Selon un autre mode de réalisation, dans la formule (I-4), les groupes R' et R" représentent de préférence ensemble l'un des groupes suivants : *o*,*p*-diméthoxyle (2,4-diméthoxyle) ; di-*m*-diméthoxyle (3,5-diméthoxyle) ; *m*,*p*-diméthoxyle (3,4-diméthoxyle) ou OCH₂O et R"' est alors un atome d'hydrogène.

Les composés de formule (I-4) sont des 1,2-diamines indoliques diprotégées, correspondant à des composés de formule (I) dans laquelle :
- R₁ représente un groupe CH₂NHBoc ;
- R₂ représente un atome d'hydrogène ;
- B représente un groupe de formule (B-1) avec R_{IV} = R"' = H ;
- A représente un atome d'azote N ; et
- a représente une liaison simple.

Les composés de formule (I-4) correspondent à des composés de formule (I-4-bis) dans laquelle R"' = H.

Selon un mode de réalisation préféré, l'invention concerne l'utilisation telle que définie ci-dessus d'un composé de formule (I-4) dans laquelle :
- R' et R" représentent un atome d'hydrogène ;
- R, R₃, R₄ et R₇ représentent un atome d'hydrogène ;
- R₅ représente un atome d'hydrogène, un groupe méthoxyle ou un atome de brome ;
- R₆ représente un atome d'hydrogène ou un atome de brome ;
étant entendu, de préférence, que lorsque R₆ représente un atome de brome, R₅ représente alors un atome d'hydrogène,
que lorsque R₅ représente un atome de brome ou un groupe méthoxyle, R₆ représente alors un atome d'hydrogène,
ou R₅ et R₆ représentent un atome d'hydrogène.

De tels composés forment une sous-famille répondant à la formule générale (I-4-a) suivante : R₅ et R₆ étant tels que

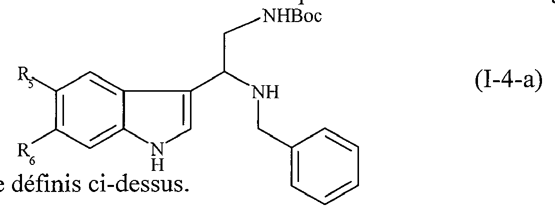

La présente invention concerne également l'utilisation telle que définie ci-dessus d'au moins un composé répondant à l'une des formules suivantes :

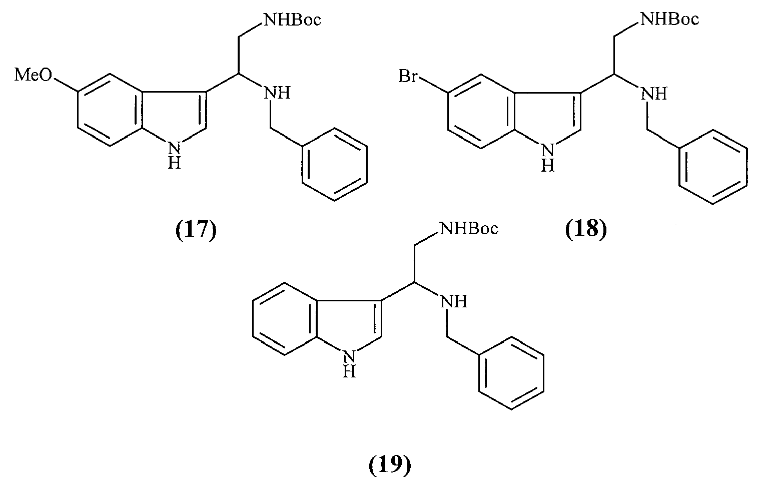

La présente description concerne également l'utilisation telle que définie ci-dessus, d'un composé de formule (I-5') suivante : 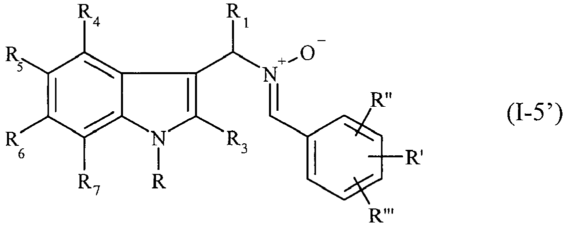 R, R₁, R₃, R₄, R₅, R₆, R₇, R', R" et R"' étant tels que définis ci-dessus.

De préférence, le composé de formule (I-5') est différent du composé suivant :

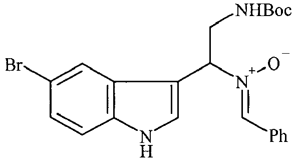

Les composés de formule (I-5') sont des nitrones indoliques, correspondant à des composés de formule (I) dans laquelle :
- R₂ représente O⁻ ;
- B représente un groupe de formule (B-1) avec R_{IV} = H ;
- A représente N⁺ ; et
- a représente une double liaison.

La présente invention concerne également l'utilisation telle que définie ci-dessus, d'un composé de formule formule (I) dans laquelle :
- R₂ représente O⁻ ;
- B représente un groupe de formule (B-1) avec R_{IV} = H;
- A représente N⁺ ; et
- a représente une double liaison.
- R, R₃, R₄ et R₇ représentent un atome d'hydrogène;
- R₅ représente un atome d'hydrogène, un groupe méthoxyle, un atome de brome ou un atome de chlore;
- R₆ représente un atome d'hydrogène ou un atome de brome;
- R₁ représente CH₂NHBoc;

R' et R" représentent indépendamment l'un de l'autre un atome d'hydrogène ou un groupe méthoxyle ou R' et R" représentent ensemble un groupe *o,p*-diméthoxyle ou un groupe di-*m*-méthoxyle,
les dits composés répondant à la formule (I-5'):

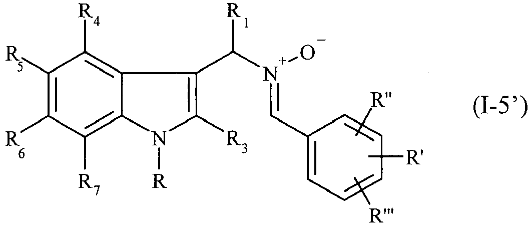

Selon un mode de réalisation préféré, la présente description concerne l'utilisation telle que définie ci-dessus, d'un composé de formule (I-5) suivante : 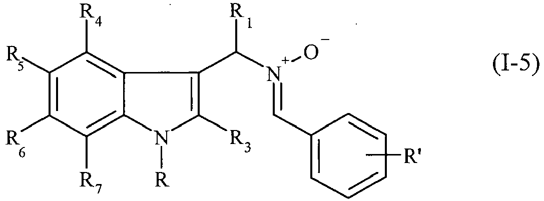 R, R₁, R₃, R₄, R₅, R₆, R₇ et R' étant tels que définis ci-dessus.

Les composés de formule (I-5) sont des nitrones indoliques, correspondant à des composés de formule (I) dans laquelle :
- R₂ représente O⁻ ;
- B représente un groupe de formule (B-1) avec R" = R"' = R_{IV} = H ;
- A représente N⁺ ; et
- a représente une double liaison.

Les composés de formule (I-5) correspondent à des composés de formule (I-5') dans laquelle R" = R'" = H.

Selon un autre mode de réalisation préféré, les composés utilisés selon la description répondent à la formule (I-5) dans laquelle :
- R' représente un atome d'hydrogène ou un groupe *p*-méthoxyle ;
- R, R₃, R₄ et R₇ représentent un atome d'hydrogène ;
- R₁ représente un groupe CH₂NHBoc, méthyle, éthyle ou isobutyle ;
- R₅ représente un atome d'hydrogène ou un atome de brome ;
- R₆ représente un atome d'hydrogène ou un atome de brome ;
étant entendu, de préférence, que lorsque R₆ représente un atome de brome, R₅ représente alors un atome d'hydrogène,
et que lorsque R₅ représente un atome de brome, R₆ représente alors un atome d'hydrogène.

De tels composés forment une sous-famille répondant à la formule générale (I-5-a) suivante : 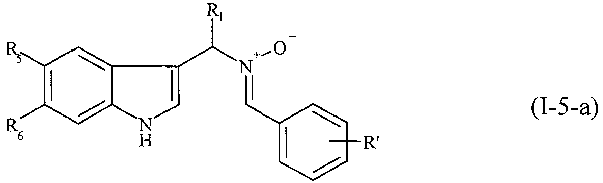 R', R₅ et R₆ étant tels que définis ci-dessus.

Selon un autre mode de réalisation préféré, les composés utilisés selon l'invention répondent à la formule (I-5) dans laquelle :
- R' représente un atome d'hydrogène ou un groupe *p*-méthoxyle;
- R, R₃, R₄ et R₇ représentent un atome d'hydrogène;
- R₁ représente un groupe CH₂NHBoc;
- R₅ représente un atome d'hydrogène ou un atome de brome;
- R₆ représente un atome d'hydrogène ou un atome de brome.

De préférence, la présente description concerne l'utilisation telle que définie ci-dessus, d'au moins un composé répondant à l'une des formules suivantes :

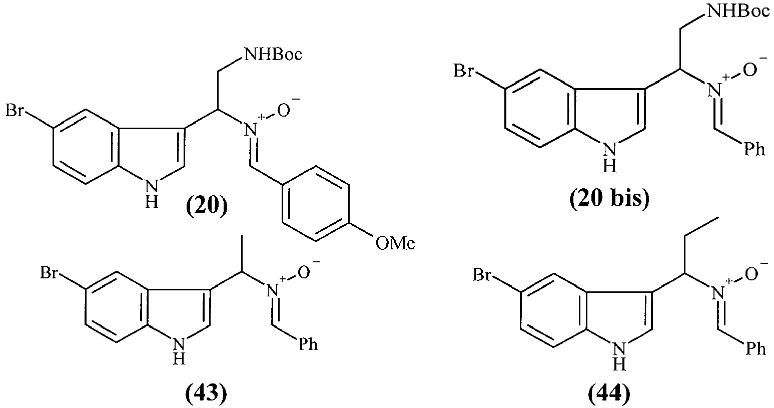

De préférence, la présente invention concerne l'utilisation telle que définie ci-dessus, d'au moins un composé répondant à l'une des formules suivantes :

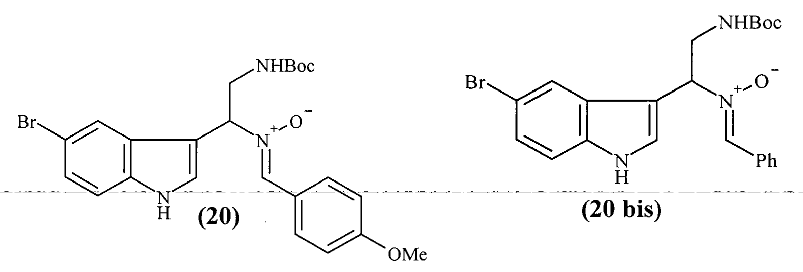

La présente description concerne l'utilisation telle que définie ci-dessus, d'un composé de formule (I-6) suivante : 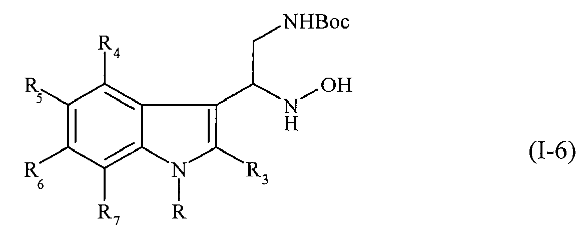 R, R₃, R₄, R₅, R₆ et R₇ étant tels que définis dans la formule (I),
lesdits composés de formule (I-6) pouvant être sous forme, le cas échéant, de sels d'acides physiologiquement acceptables tels que des chlorhydrates, des formiates ou des oxalates.

Les composés de formule (I-6) sont des β-(*N*-Boc)amino *N*-hydroxylamines indoliques, correspondant à des composés de formule (I) dans laquelle :
- R₁ représente un groupe CH₂NHBoc ;
- R₂ représente un groupe OH ; et
- B représente un groupe de formule (B-3) avec R_{c} = H.

Parmi les composés de formule (I-6) telle que définie ci-dessus, les composés de l'invention sont ceux où :
- R, R₃, R₄ et R₇ représentent un atome d'hydrogène ;
- R₅ représente un atome d'hydrogène ou un atome de brome ;
- R₆ représente un atome d'hydrogène ou un atome de brome,
étant entendu, de préférence, que lorsque R₆ représente un atome de brome, R₅ représente alors un atome d'hydrogène,
et que lorsque R₅ représente un atome de brome, R₆ représente alors un atome d'hydrogène.

De tels composés forment une sous-famille répondant à la formule générale (I-6-a) suivante : 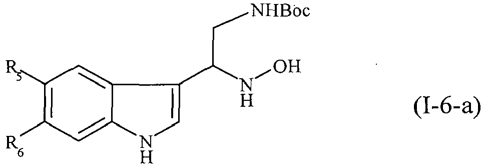 R₅ et R₆ étant tels que définis ci-dessus.

La présente invention concerne également l'utilisation telle que définie ci-dessus d'au moins un composé répondant à l'une des formules suivantes :

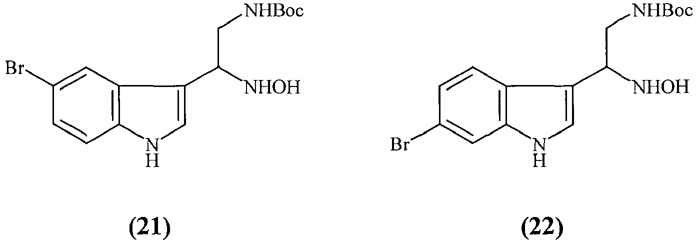

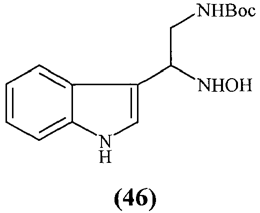

La présente description concerne également l'utilisation telle que définie ci-dessus, d'un composé de formule (I-7) suivante : 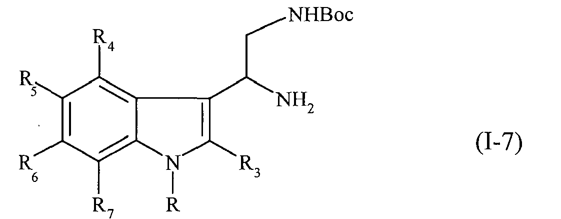 R, R₃, R₄, R₅, R₆ et R₇ étant tels que définis ci-dessus dans la formule (I),
lesdits composés de formule (I-7) pouvant être sous forme, le cas échéant, de sels d'acides physiologiquement acceptables tels que des chlorhydrates, des formiates ou des oxalates.

Les composés de formule (I-7) sont des 1,2-diamines indoliques monoprotégées, correspondant à des composés de formule (I) dans laquelle :
- R₁ représente un groupe CH₂NHBoc ;
- R₂ représente H ; et
- B représente un groupe de formule (B-3) avec R_{c} = H.

Parmi les composés de formule (I-7) telle que définie ci-dessus, les composés de l'invention sont ceux où :
- R, R₃, R₄ et R₇ représentent un atome d'hydrogène ;
- R₅ représente un atome d'hydrogène, un groupe méthoxyle ou un atome de brome ;
- R₆ représente un atome d'hydrogène, un groupe méthoxyle ou un atome de brome,
étant entendu, de préférence, que lorsque R₆ représente un atome de brome ou un groupe méthoxyle, R₅ représente alors un atome d'hydrogène,
et que lorsque R₅ représente un atome de brome ou un groupe méthoxyle, R₆ représente alors un atome d'hydrogène,
ou R₅ et R₆ représentent un atome d'hydrogène.

De tels composés forment une sous-famille répondant à la formule générale (I-7-a) suivante : 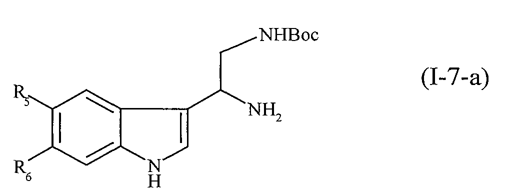 R₅ et R₆ étant tels que définis ci-dessus.

La présente invention concerne l'utilisation telle que définie ci-dessus d'au moins un composé répondant à l'une des formules suivantes :

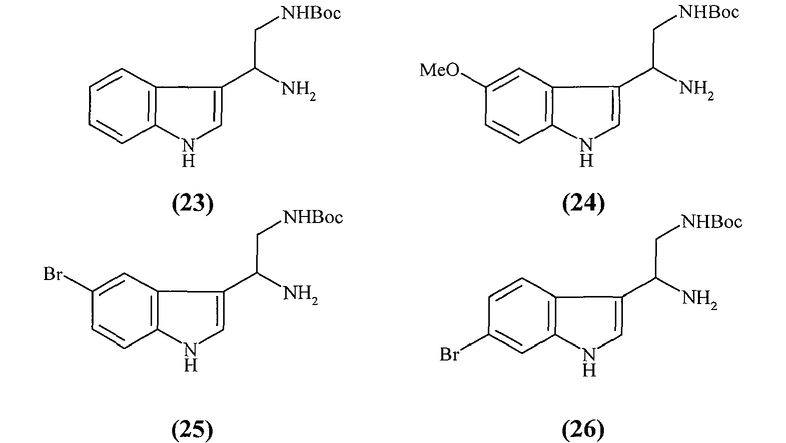

La présente description concerne également l'utilisation telle que définie ci-dessus, d'un composé de formule (I-8) suivante : dans laquelle : 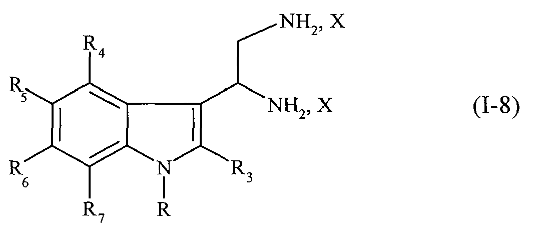
- R, R₃, R₄, R₅, R₆ et R₇ sont tels que définis ci-dessus dans la formule (I) ; et
- X représente HCl, HCOOH ou HOOCCOOH.

Les composés de formule (I-8) sont des sels de 1,2-diamines indoliques, notamment des dichlorhydrates de 1,2-diamines indoliques (lorsque X = Cl), correspondant à des composés de formule (I) dans laquelle :
- R₁ représente un groupe CH₂NH₂ (ou CH₂NH₂.X) ;
- R₂ représente H ; et
- B représente un groupe de formule (B-3) avec R_{c} = H,
lesdits composés étant sous forme de sels, notamment de dichlorhydrates.

Selon un mode de réalisation avantageux, les composés utilisés selon l'invention sont des composés de formule (I-8) dans laquelle :
- R, R₃, R₄ et R₇ représentent un atome d'hydrogène ;
- R₅ représente un atome d'hydrogène, un groupe méthoxyle ou un atome de brome ;
- R₆ représente un atome d'hydrogène, un groupe méthoxyle ou un atome de brome ;
- X représente HCl,
étant entendu, de préférence, que lorsque R₆ représente un atome de brome ou un groupe méthoxyle, R₅ représente alors un atome d'hydrogène,
et que lorsque R₅ représente un atome de brome ou un groupe méthoxyle, R₆ représente alors un atome d'hydrogène,
ou R₅ et R₆ représentent un atome d'hydrogène.

De tels composés forment une sous-famille répondant à la formule générale (I-8-a) suivante : 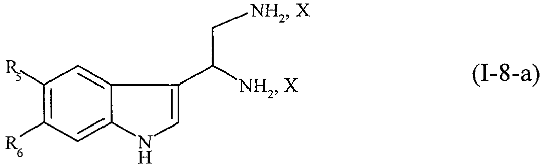 R₅ et R₆ étant tels que définis ci-dessus.

Selon un mode de réalisation avantageux, les composés utilisés selon l'invention sont des composés de formule (I-8) dans laquelle :
- R représente un atome d'hydrogène;
- R₃ représente un atome d'hydrogène;
- R₄ et R₇ repésentent un atome d'hydrogène;
- R₅ et R₆ représentent indépendamment l'un de l'autre un groupe choisi parmi l'un des groupes suivants:
   * un atome d'hydrogène;
   * un groupe alcoxyle comprenant de 1 à 7 atomes de carbone ou un groupe benzyloxyle;
   * un atome d'halogène choisi parmi Br, Cl, F ou I;
- X représente HCl, HCOOH ou HOOCCOOH.

La présente invention concerne également l'utilisation telle que définie ci-dessus d'au moins un composé répondant à l'une des formules suivantes :

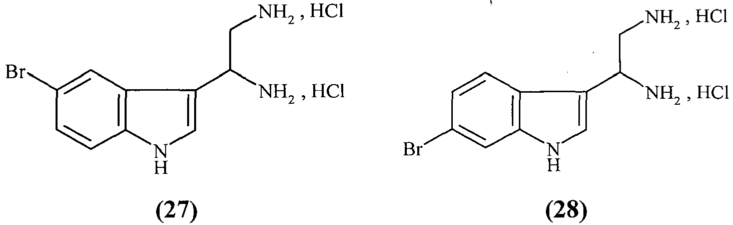

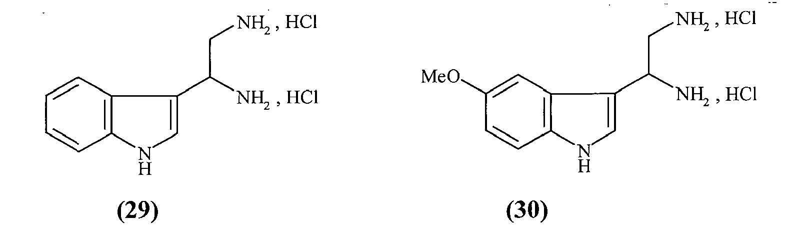

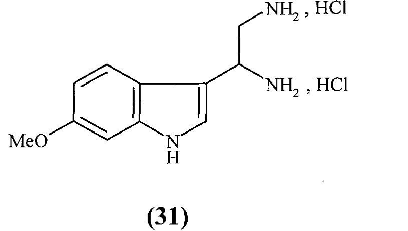

La présente description concerne également l'utilisation telle que définie ci-dessus, d'un composé de formule (I-9) suivante : 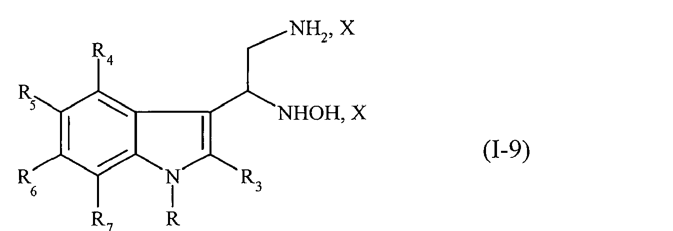 dans laquelle :
- R, R₃, R₄, R₅, R₆ et R₇ sont tels que définis ci-dessus dans la formule (I) ; et
- X représente HCl, HCOOH ou HOOCCOOH.

Les composés de formule (I-9) sont des *N*-hydroxylamines primaires indoliques *β*-aminées, correspondant à des composés de formule (I) dans laquelle :
- R₁ représente un groupe CH₂NH₂ (ou CH₂NH₂.X) ;
- R₂ représente un groupe OH ; et
- B représente un groupe de formule (B-3) avec R_{c} = H,
lesdits composés étant sous forme de sels, notamment dichlorhydrates.

Selon un mode de réalisation avantageux, les composés utilisés selon l'invention sont des composés de formule (I-9) dans laquelle :
- R, R₃, R₄ et R₇ représentent un atome d'hydrogène ;
- R₅ représente un atome d'hydrogène ou un atome de brome ;
- R₆ représente un atome d'hydrogène ou un atome de brome ;
- X représente HCl.
étant entendu, de préférence, que lorsque R₆ représente un atome de brome, R₅ représente alors un atome d'hydrogène,
et que lorsque R₅ représente un atome de brome, R₆ représente alors un atome d'hydrogène.

De tels composés forment une sous-famille répondant à la formule générale (I-9-a) suivante : 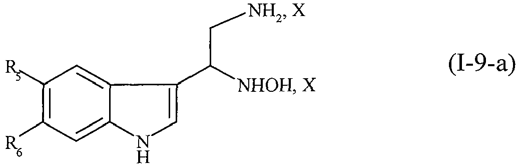 R₅ et R₆ étant tels que définis ci-dessus.

Selon un mode de réalisation avantageux, les composés utilisés selon l'invention sont des composés de formule (I-9) dans laquelle :
- R représente un atome d'hydrogène;
- R₃ représente un atome d'hydrogène;
- R₄ et R₇ repésentent un atome d'hydrogène;
- R₅ et R₆ représentent indépendamment l'un de l'autre un groupe choisi parmi l'un des groupes suivants:
   * un atome d'hydrogène;
   _{*} un groupe alcoxyle comprenant de 1 à 7 atomes de carbone ou un groupe benzyloxyle;
   * un atome d'halogène choisi parmi Br, Cl, F ou I;
- X représente HCl, HCOOH ou HOOCCOOH.

La présente invention concerne l'utilisation telle que définie ci-dessus d'au moins un composé répondant à l'une des formules suivantes :

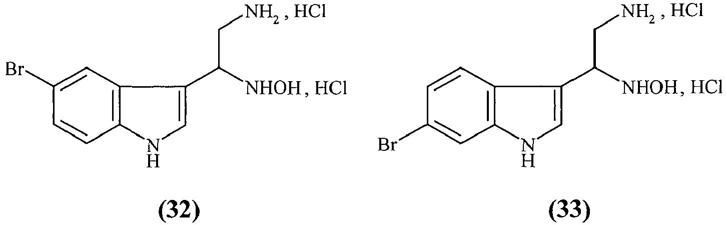

La présente description concerne également l'utilisation telle que définie ci-dessus, d'un composé de formule (I-10) suivante : 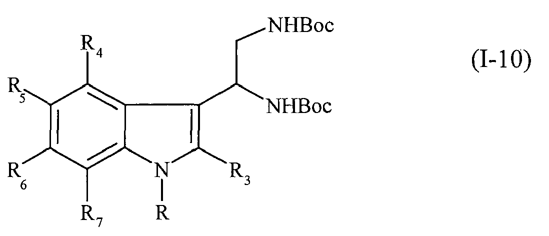 R, R₃, R₄, R₅, R₆ et R₇ étant tels que définis ci-dessus dans la formule (I).

Les composés de formule (I-10) sont des di-*N*-(Boc)-diamines indoliques, correspondant à des composés de formule (I) dans laquelle :
- R₁ représente un groupe CH₂NHBoc ;
- R₂ représente H ; et
- B représente un groupe de formule (B-2) avec GP₁ = Boc.

Selon un mode de réalisation avantageux, les composés utilisés selon l'invention sont des composés de formule (I-10) dans laquelle :
- R, R₃, R₄ et R₇ représentent un atome d'hydrogène ;
- R₅ représente un atome d'hydrogène, un atome de brome ou un groupe méthoxyle ;
- R₆ représente un atome d'hydrogène, un atome de brome ou un groupe méthoxyle ;
étant entendu, de préférence, que lorsque R₆ représente un atome de brome ou un groupe méthoxyle, R₅ représente alors un atome d'hydrogène,
et que lorsque R₅ représente un atome de brome ou un groupe méthoxyle, R₆ représente alors un atome d'hydrogène,
ou R₅ et R₆ représentent un atome d'hydrogène.

De tels composés forment une sous-famille répondant à la formule générale (I-10-a) suivante : 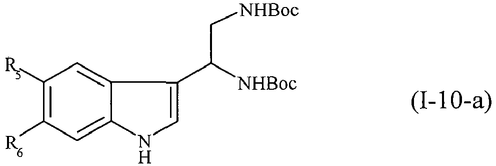 R₅ et R₆ étant tels que définis ci-dessus.

La présente invention concerne l'utilisation telle que définie ci-dessus d'au moins un composé répondant à l'une des formules suivantes :

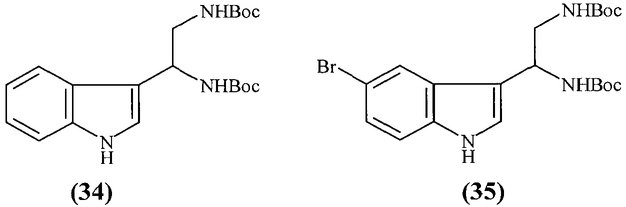

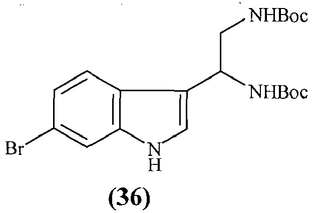

La présente description concerne également l'utilisation telle que définie ci-dessus d'un composé de formule 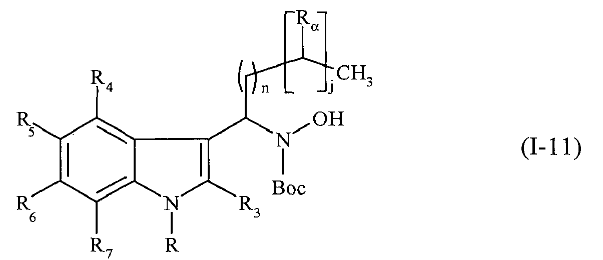 dans laquelle :
- n est égal à 0, 1 ou 2 ;
- j est égal à 0 ou 1 ;
- R, R₃, R₄, R₅, R₆ et R₇ sont tels que définis ci-dessus dans la formule (I) ;
- R_{α} représentant un atome d'hydrogène ou un groupe méthyle ou éthyle.

Les composés de formule (I-11) correspondent à des composés de formule (I) dans laquelle :
- R₁ représente un groupe -(CH₂)ₙ-(CHR_{α})ⱼ-CH₃ ;
- R₂ représente un groupe OH ; et
- B représente un groupe de formule (B-2) avec GP₁ = Boc.

Selon un mode de réalisation préféré, les composés de formule (I-11) répondent à la formule donnée ci-dessus dans laquelle R, R₃, R₄ et R₇ représentent un atome d'hydrogène. De tels composés forment une sous famille répondant à la formule générale (I-11-a) suivante: 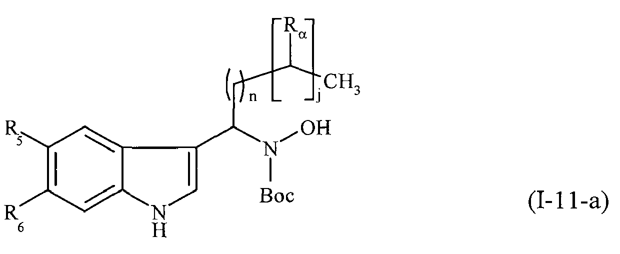 dans laquelle n , j, R_{α}, R₅ et R₆ sont tels que définis ci-dessus.

La présente description concerne l'utilisation telle que définie ci-dessus d'un composé de formule suivante :

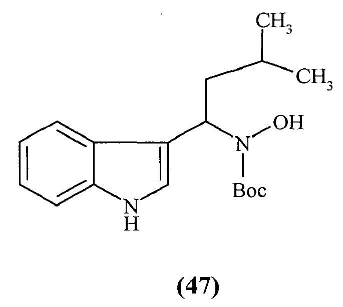

La présente description concerne également l'utilisation telle que définie ci-dessus d'un composé de formule (I-12) suivante: dans laquelle : 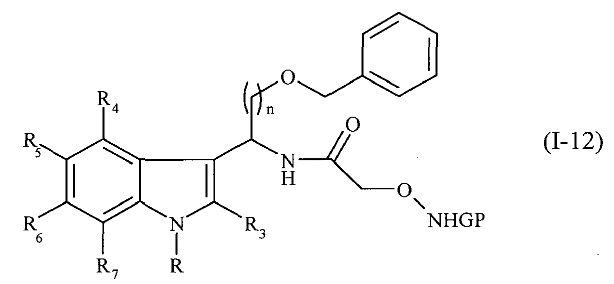
- n est égal à 1 ou 2 ;
- R, R₃, R₄, R₅, R₆ et R₇ sont tels que définis ci-dessus dans la formule (I) ;
- GP représentant un groupe Boc, Fmoc, Ac, Bz ou CF₃CO.

Les composés de formule (I-12) correspondent à des composés de formule (I) dans laquelle :
- R₁ représente un groupe -(CH₂)ₙ-O-CH₂-Ph;
- R₂ représente un groupe COCH₂ONHGP ; et
- B représente un groupe de formule (B-3) avec R_{c} = H.

Selon un mode de réalisation préféré, les composés de formule (I-12) répondent à la formule donnée ci-dessus dans laquelle R, R₃, R₄ et R₇ représentent un atome d'hydrogène. De tels composés forment une sous famille répondant à la formule générale (I-12-a) suivante: 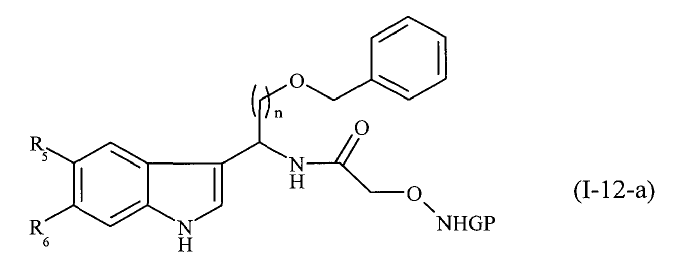 dans laquelle n , GP, R₅ et R₆ sont tels que définis ci-dessus.

La présente description concerne en particulier l'utilisation telle que définie ci-dessus d'un composé de formule suivante :

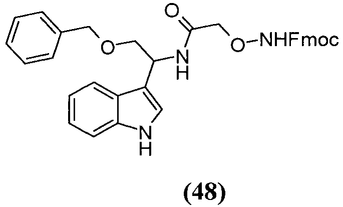

La présente description concerne l'utilisation telle que définie ci-dessus, d'au moins un composé de formule (I), présentant une activité antibactérienne intrinsèque, choisi parmi l'un des composés de formule suivante :

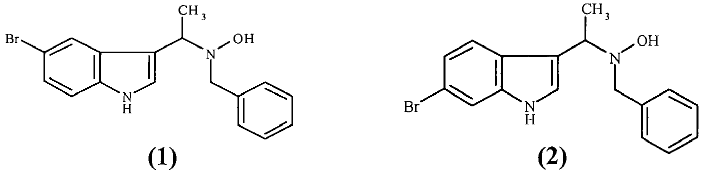

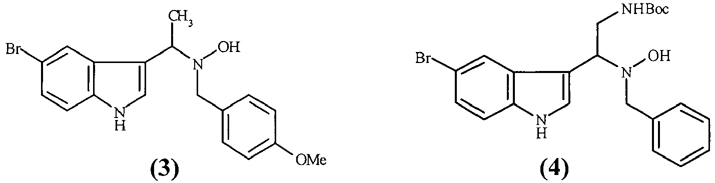

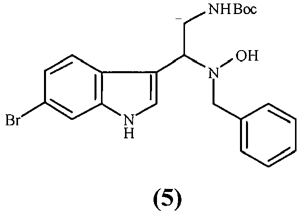

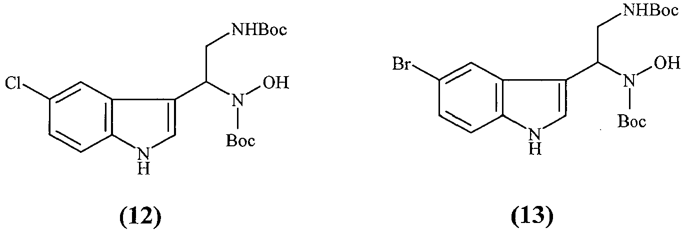

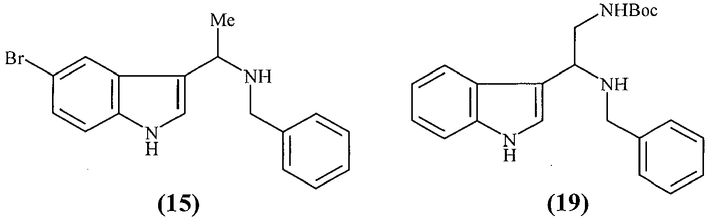

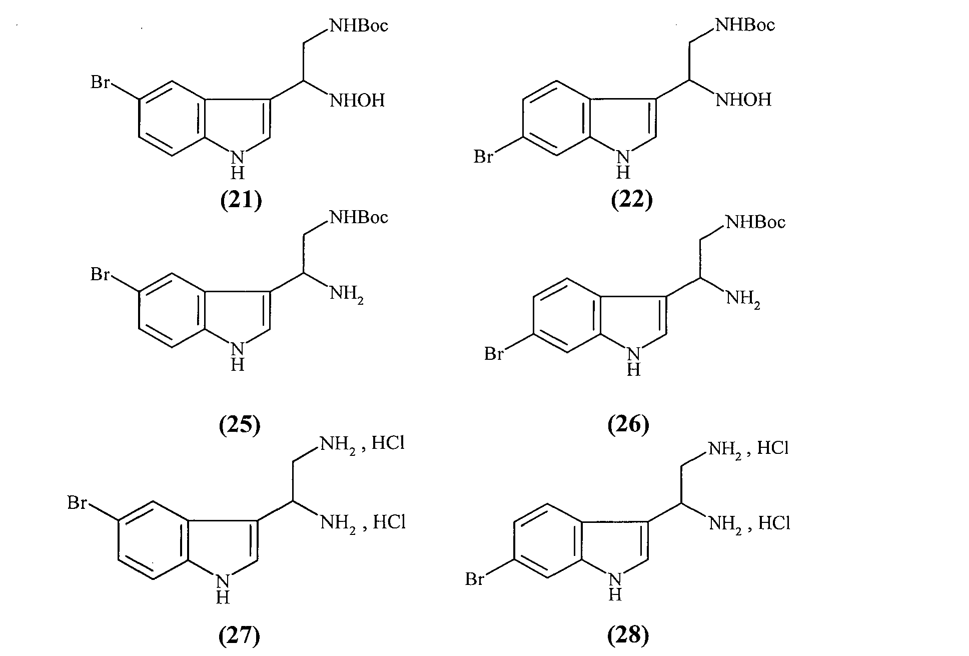

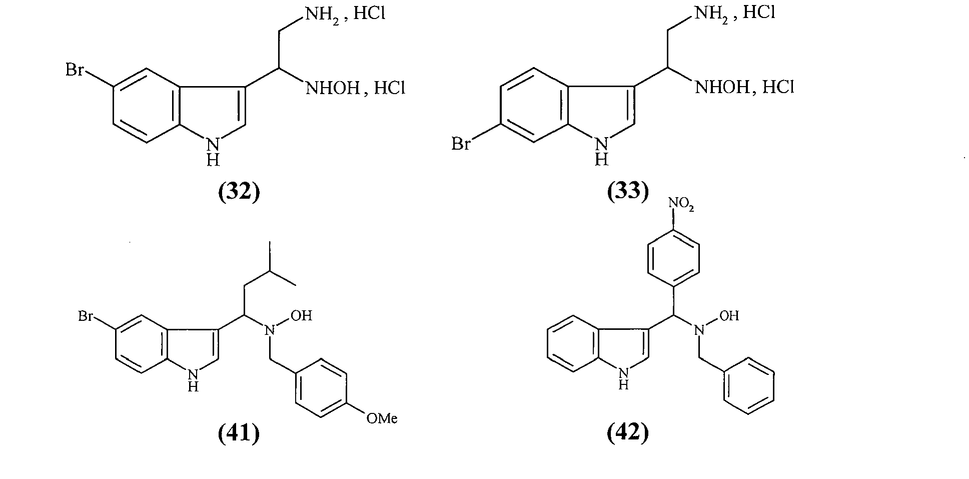

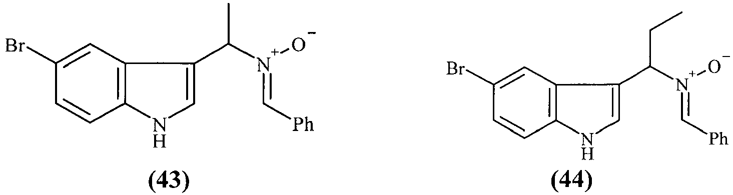

La présente invention concerne l'utilisation telle que définie ci-dessus, d'au moins un composé de formule (I), présentant une activité antibactérienne intrinsèque, choisi parmi l'un des composés de formule suivante :

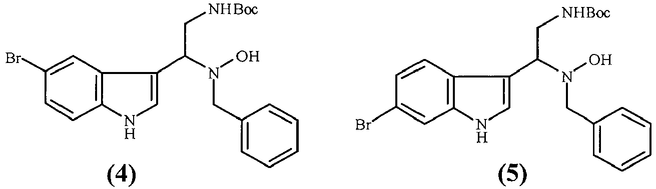

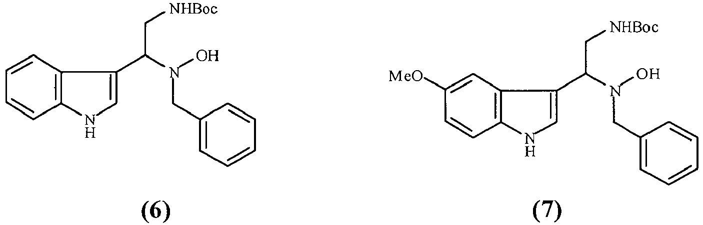

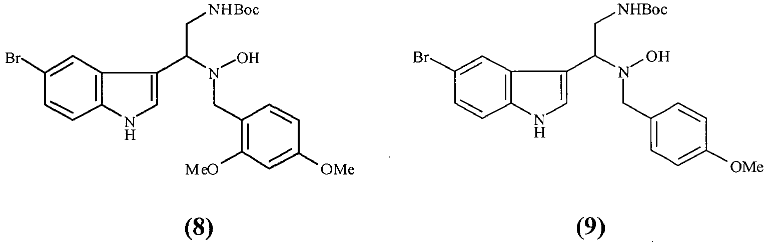

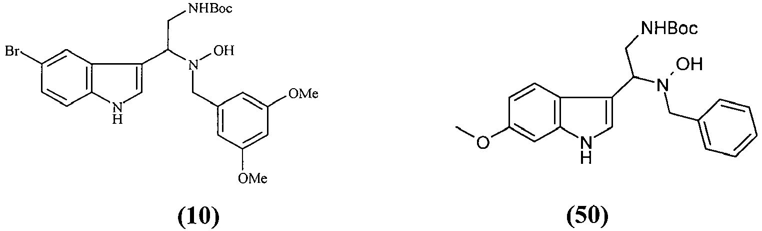

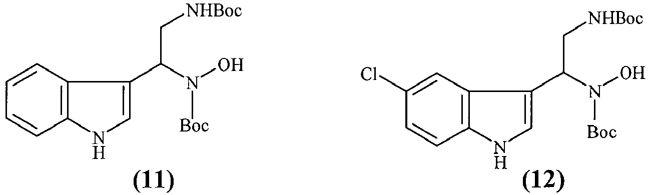

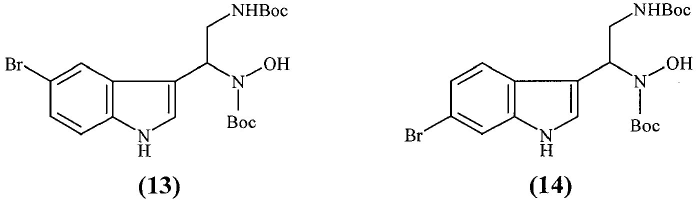

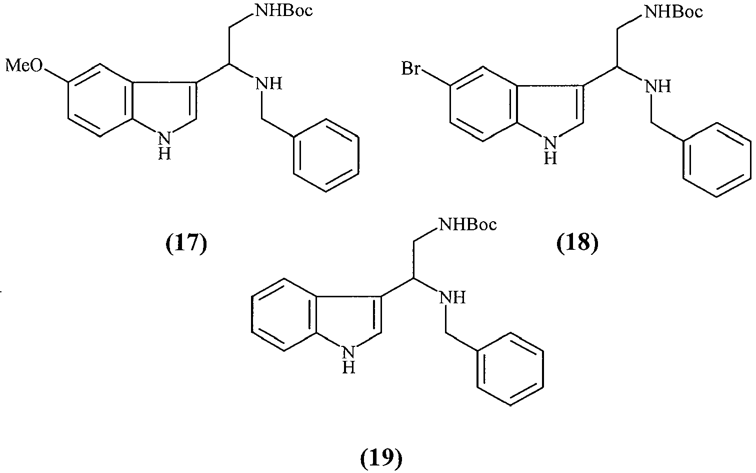

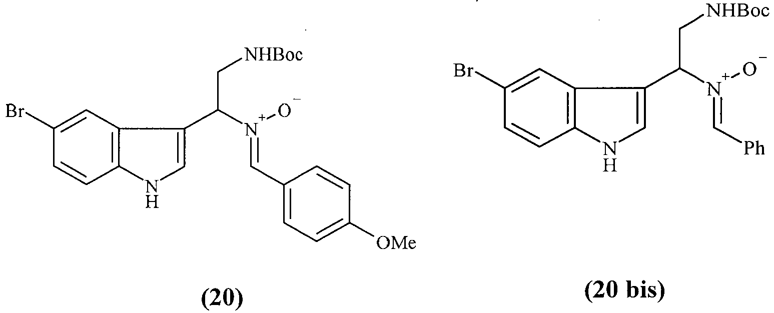

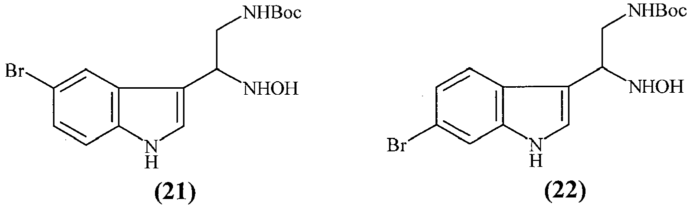

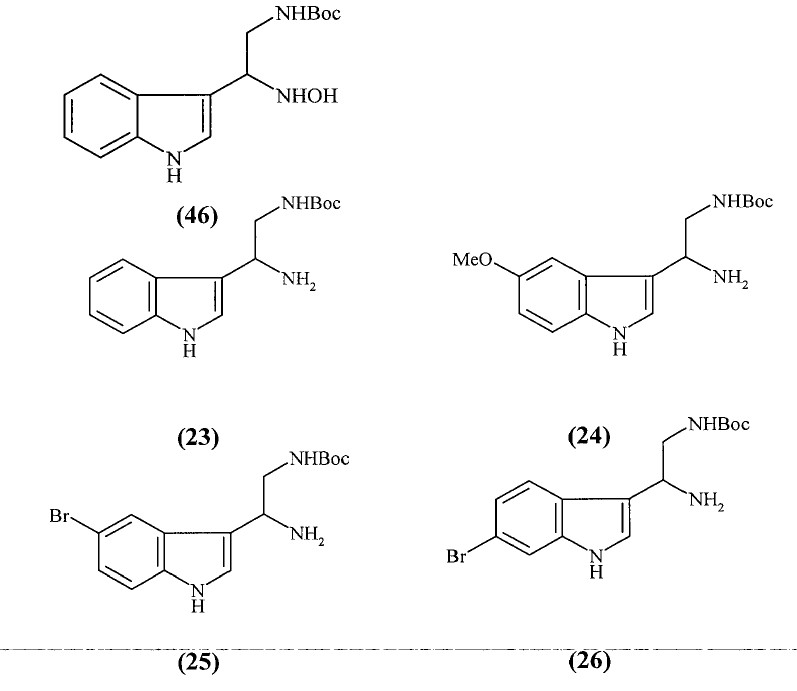

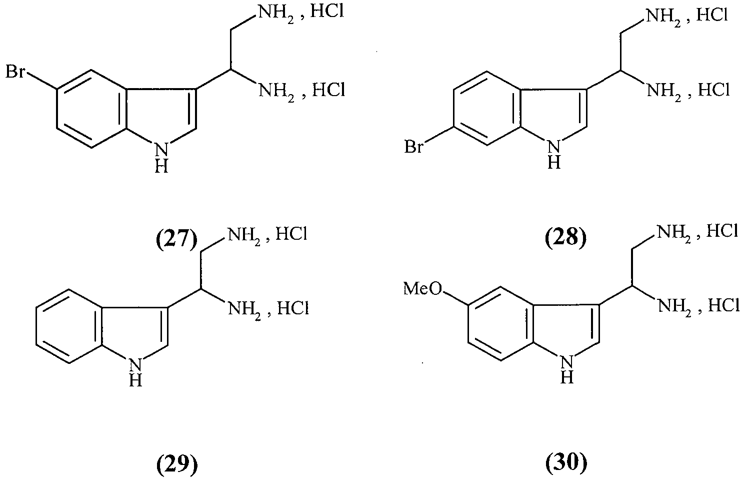

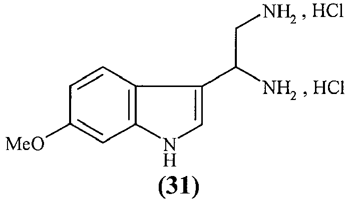

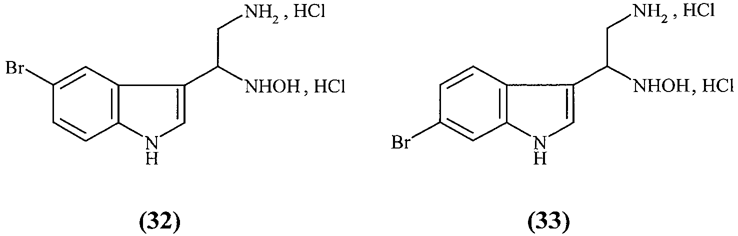

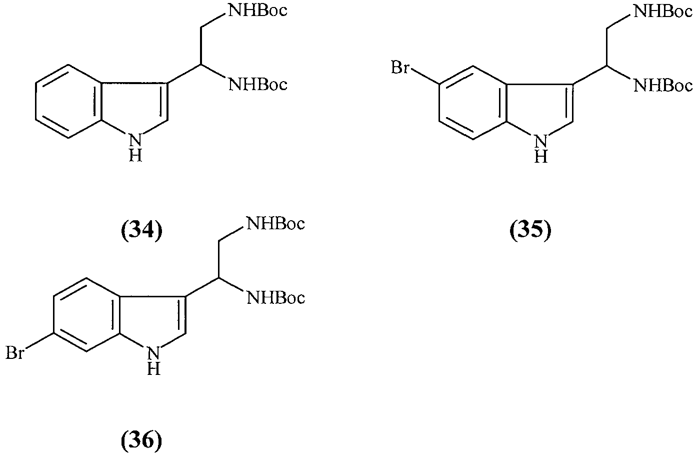

L'expression "activité antibactérienne intrinsèque" désigne l'activité résultant du seul composé (ou activité propre - à opposer à activité en association), c'est-à-dire la capacité du produit seul à inhiber la croissance bactérienne.

Les bactéries contre lesquelles les composés susmentionnés ont une activité sont notamment choisies parmi : *Pseudomonas, Pneumococcus, Staphylococcus, E. coli, Acine tobacter, Klebsiella, Haemophilus,*

La présente invention concerne l'utilisation telle que définie ci-dessus, d'au moins un composé de formule (I), présentant une activité d'inhibition d'une pompe d'efflux, notamment de la pompe d'efflux NorA, en association avec un antibiotique, notamment de la famille des fluoroquinolones, tel que la ciprofloxacine, la norfloxacine, la péfloxacine, l'énofloxacine, l'ofloxacine, la lévofloxacine et la moxifloxacine, pour le traitement de pathologies associées à des infections bactériennes vis-à-vis desquelles il existe une résistance aux antibactériens.

L'expression "activité d'inhibition d'une pompe d'efflux" désigne l'activité du composé qui permet de restaurer l'activité antibactérienne d'un second composé identifié comme antibactérien sur les souches non résistantes. Ainsi, un tel composé ne démontre pas ou démontre très peu d'activité intrinsèque mais permet à une molécule antibactérienne de redevenir active sur une bactérie résistante par pompes d'efflux de cette molécule. En d'autres termes, l'activité d'inhibition d'une pompe d'efflux désigne la capacité du produit à rendre son activité à un antibactérien auquel la bactérie du test était devenue résistante. Dans le cas de la pompe NorA, cet antibiotique appartient à la classe des quinolones.

Les pompes d'efflux concernées sont notamment choisies parmi les pompes de type MDR ("multi drug résistance") : *Bcr,* AcrB, AcrD, AcrF *(E. coli), Mef*(A), (D) (Streptocoques), TetA-TetE (gram négatif), NorA, NorB, Vga(A), Vga(B) (*S. Aureus*), MsrA (*S. epidermidis)* et MexB, MexD, MexF, MexI *(Pseudomonas aeruginosa*).

La présente description concerne l'utilisation telle que définie ci-dessus, caractérisée en ce que le composé de formule (I), en association avec un antibiotique, est choisi parmi l'un des composés suivants :

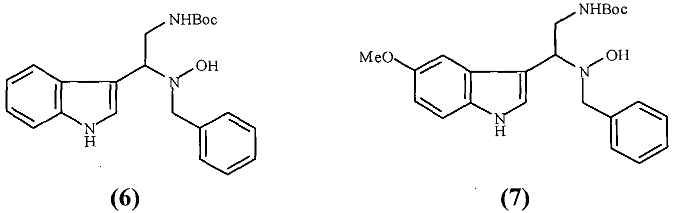

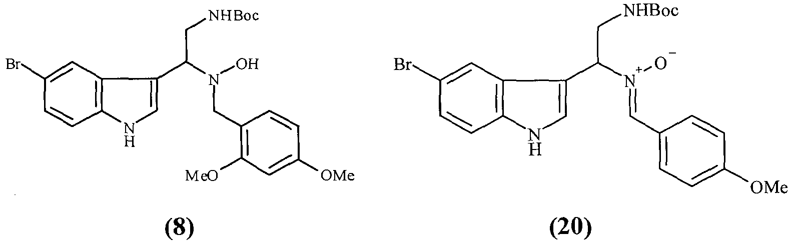

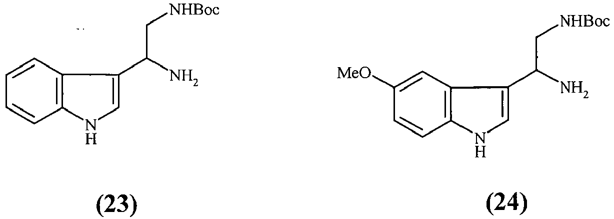

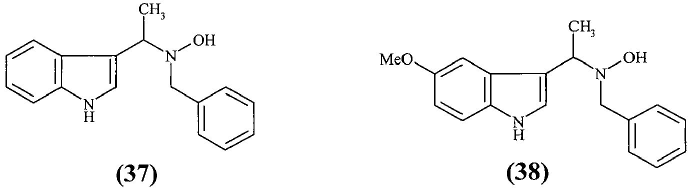

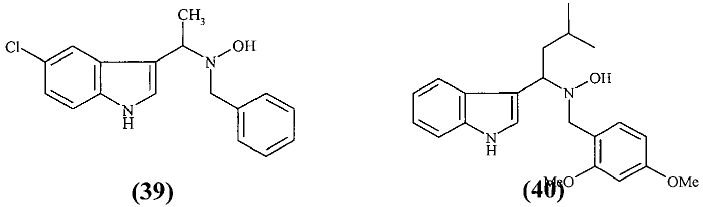

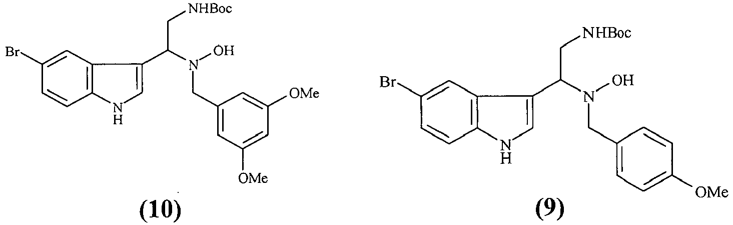

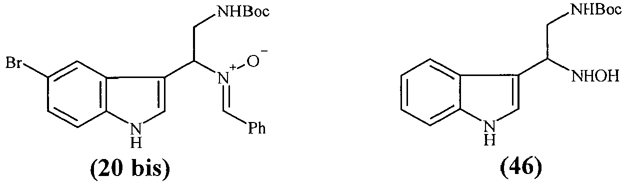

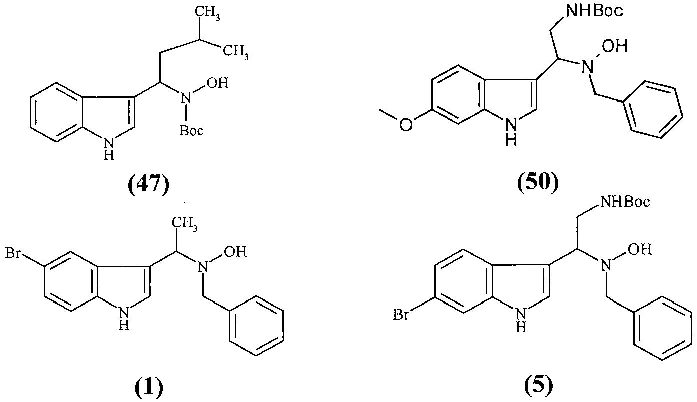

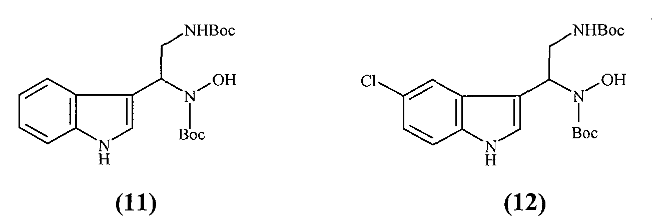

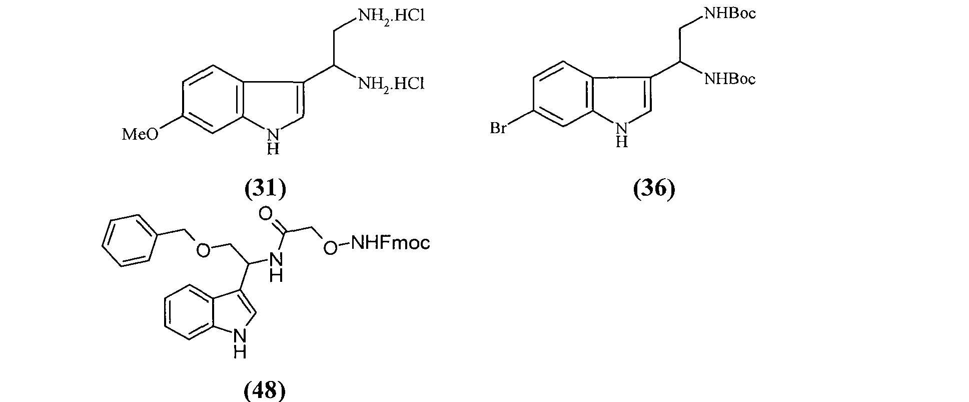

La présente invention concerne l'utilisation telle que définie ci-dessus, caractérisée en ce que le composé de formule (I), en association avec un antibiotique, est choisi parmi l'un des composés suivants :

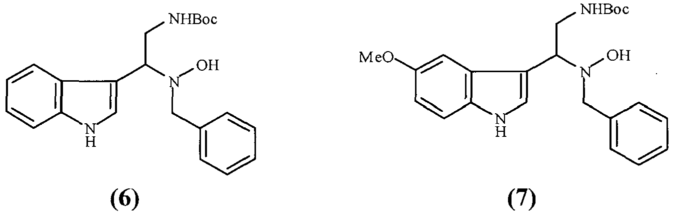

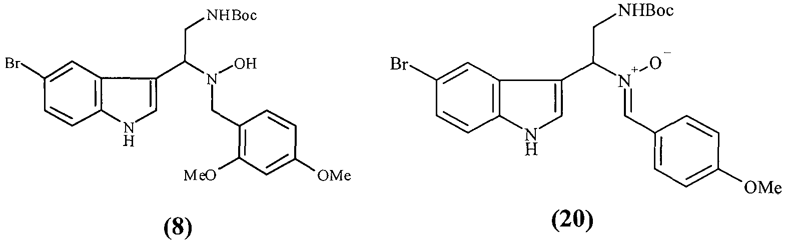

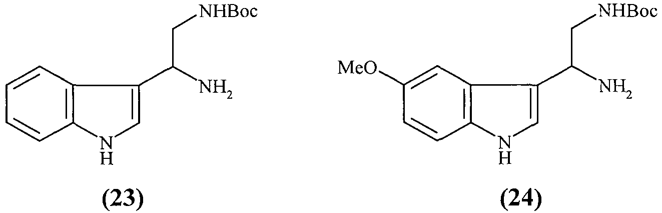

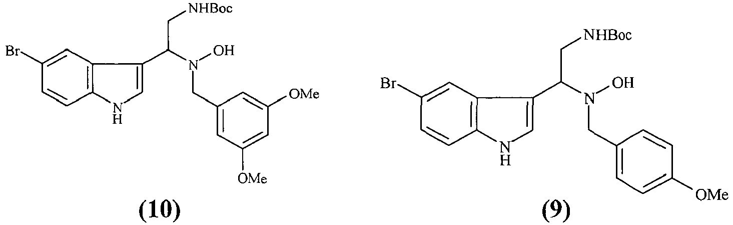

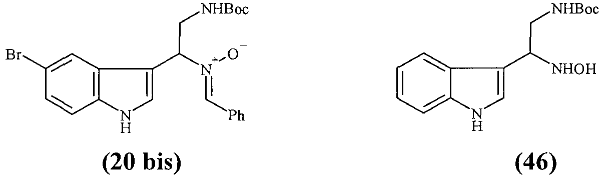

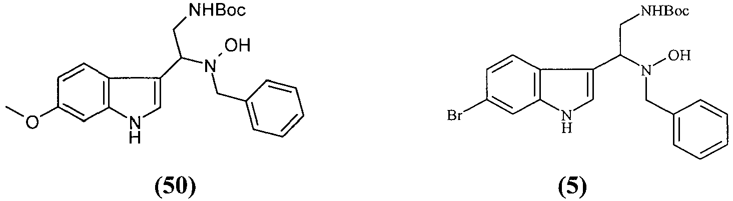

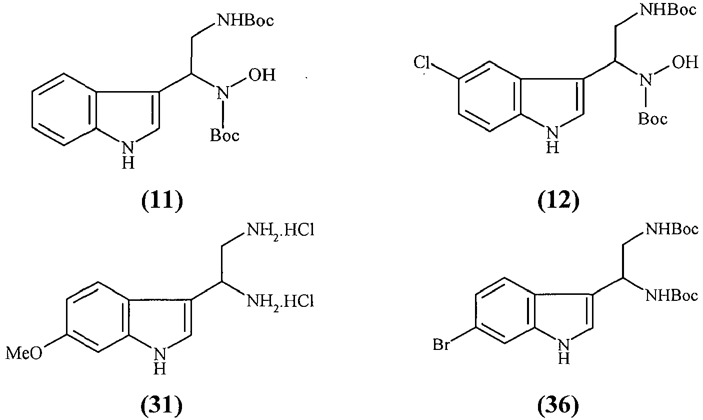

La présente invention concerne également l'utilisation d'un composé de formule (I) telle que définie ci-dessus, pour la fabrication de prothèses à base de matériaux composites.

La présente description concerne également une composition pharmaceutique comprenant, en association avec un véhicule pharmaceutiquement acceptable :
- au moins un composé de formule (I) telle que définie ci-dessus, et
- au moins un composé antibiotique, notamment de la famille des fluoroquinolones, tel que la ciprofloxacine, la norfloxacine, la péfloxacine, l'énofloxacine, l'ofloxacine, la lévofloxacine et la moxifloxacine.

L'expression "véhicule pharmaceutiquement acceptable" désigne notamment la cellulose, l'amidon, l'alcool benzylique, le polyéthylène glycol, la gélatine, le lactose, le polysorbate, le stéarate de magnésium ou de calcium, la gomme xanthane, le guar, l'alginate, la silice colloïdale.

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions pour administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols.

Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 pm, par exemple le dextrane, le mannitol ou le lactose.

Une composition pharmaceutique avantageuse selon la présente invention est une composition telle que définie ci-dessus, pour une utilisation simultanée, séparée ou étalée dans le temps destiné au traitement de pathologies associées à des infections bactériennes vis-à-vis desquelles il existe une résistance aux antibactériens.

Selon un mode de réalisation avantageux, la composition pharmaceutique selon la description est caractérisée en ce que le composé de formule (I) est choisi parmi l'un des composés suivants :

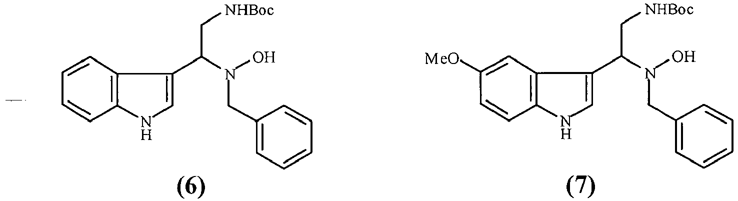

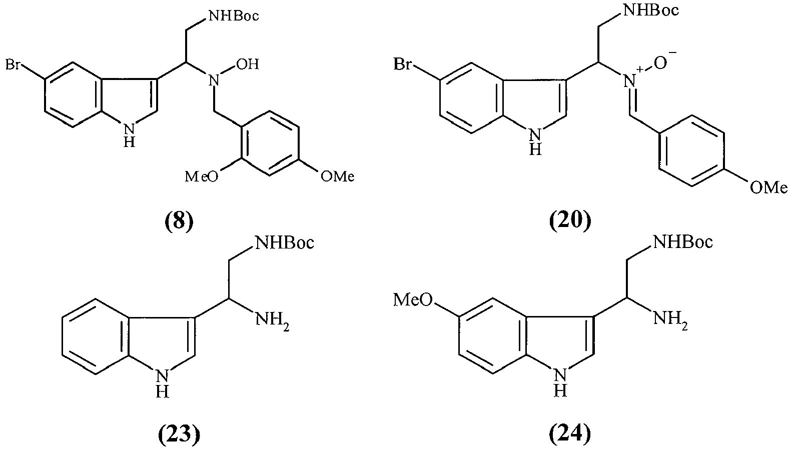

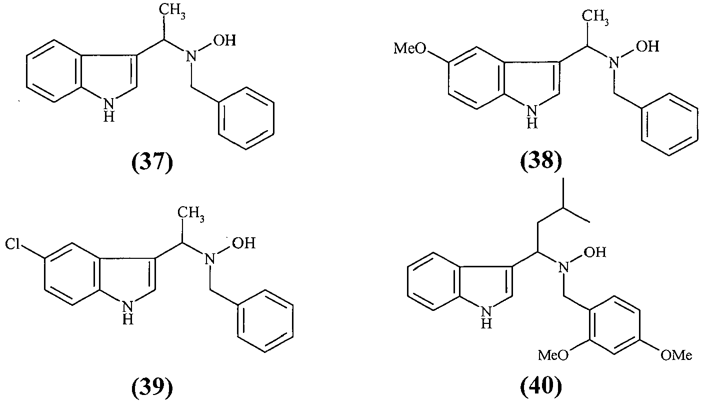

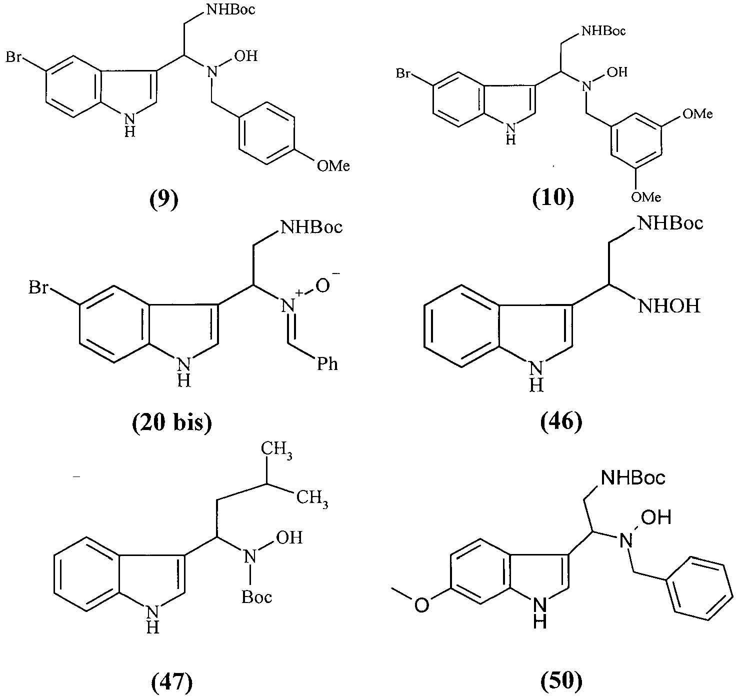

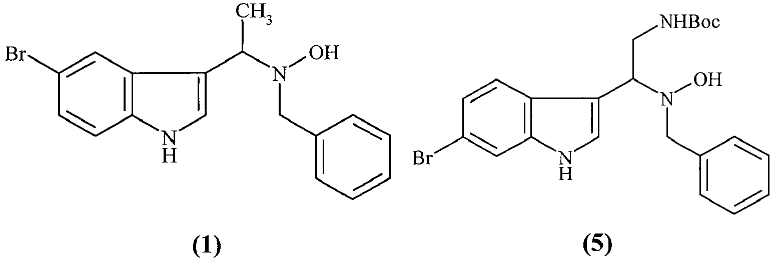

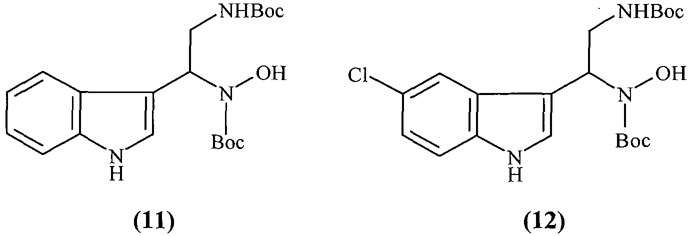

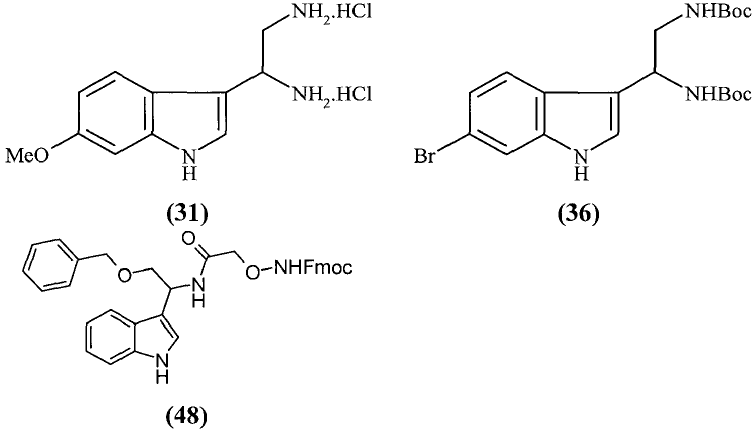

La présente invention concerne également une composition pharmaceutique comprenant, en association avec un véhicule pharmaceutiquement acceptable :
- au moins un composé de formule (I) telle que définie dans l'une quelconque des revendications 1 à 12, et
- au moins un composé antibiotique de la famille des fluoroquinolones, choisi parmi la ciprofloxacine, la norfloxacine, la péfloxacine, l'énofloxacine, l'ofloxacine, la lévofloxacine et la moxifloxacine,
ladite composition pharmaceutique étant utilisée pour une utilisation simultanée, séparée ou étalée dans le temps destiné au traitement de pathologies associées à des infections bactériennes vis-à-vis desquelles il existe une résistance aux antibactériens, ledit composé de formule (I) étant choisi parmi l'un des composés suivants:

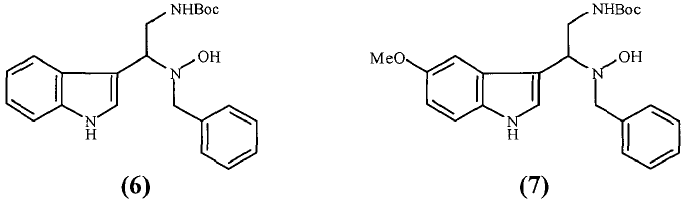

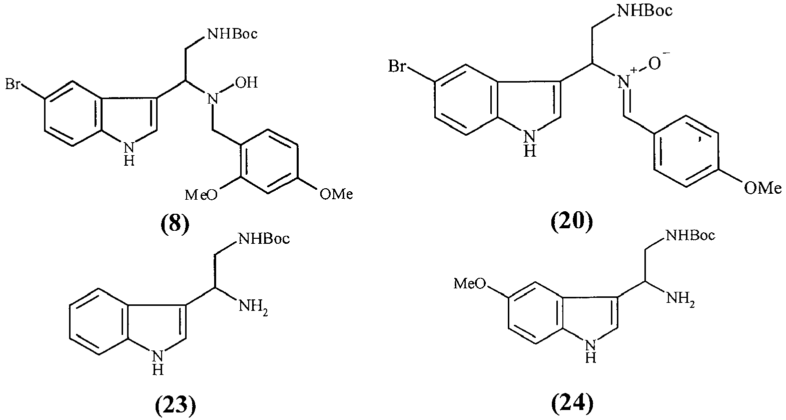

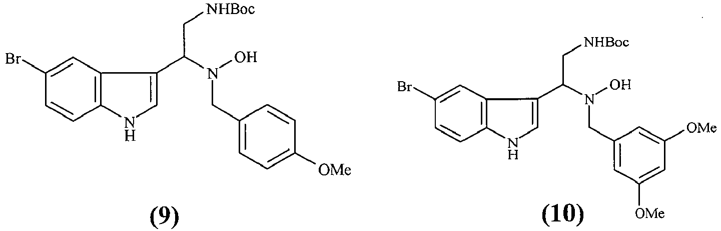

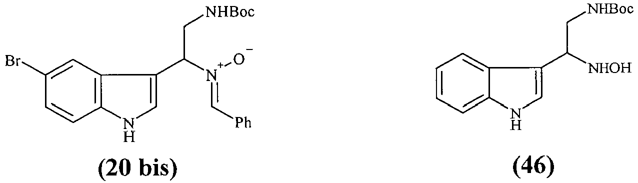

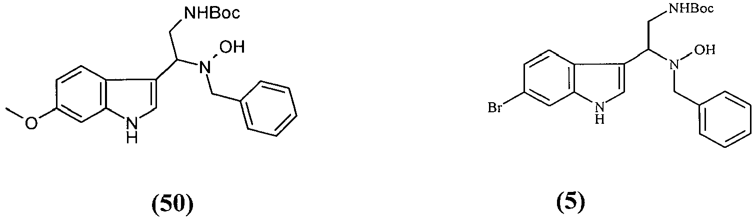

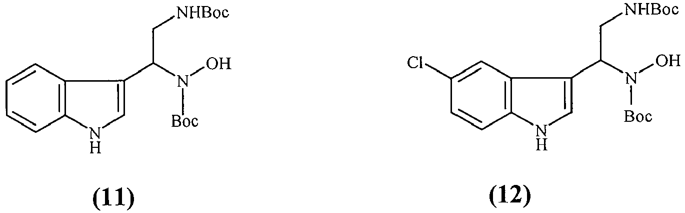

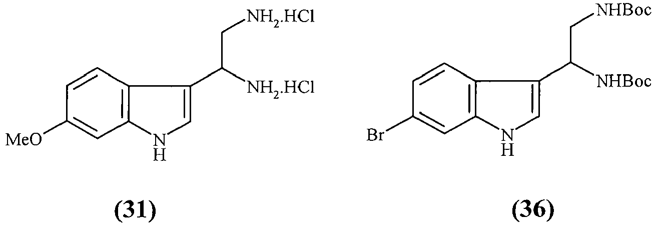

Selon un mode de réalisation avantageux, la composition pharmaceutique de l'invention telle que définie ci-dessus comprend d'environ 350 à environ 2 000 mg, de préférence d'environ 1 000 à environ 1 500 mg, de composé de formule (I) selon l'invention en 1 à 4 prises par jour et d'environ 350 à environ 2 000 mg, de préférence d'environ 1 000 à environ 1 500 mg, de composé antibiotique, notamment de la famille des fluoroquinolones, tel que la ciprofloxacine en 1 à 4 prises par jour, de préférence en 2 prises par jour.

Les doses indiquées ici sont pour un adulte de 70 kg (poids moyen utilisé). Ainsi, dans la composition pharmaceutique de l'invention, les doses de composé de formule (I) et de composé antibiotique varient de préférence d'environ 15 à environ 25 mg/kg/jour.

En thérapeutique humaine, les composés selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 750 mg et 3 g de produit actif en 2 ou 3 prises par jour par voie orale ou entre 400 mg et 1,2 g par voie intraveineuse pour un adulte.

La présente description concerne également un composé de formule (I-6-a) suivante : 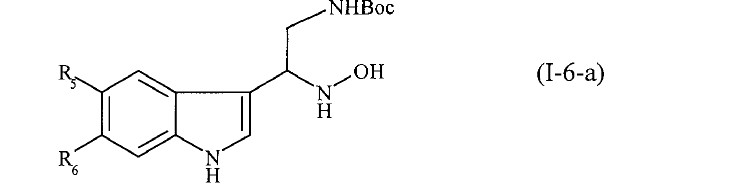 dans laquelle R₅ et R₆ représentent indépendamment l'un de l'autre un groupe choisi parmi l'un des groupes suivants :
* un atome d'hydrogène ;
* un groupe alkyle comprenant de 1 à 4 atomes de carbone, notamment un groupe méthyle ;
* un groupe trifluorométhyle ;
* un groupe trifluorométhoxyle ;
* un groupe hydroxyle ;
* un groupe alcoxyle comprenant de 1 à 7 atomes de carbone, notamment un groupe méthoxyle ou un groupe benzyloxyle ;
* un atome d'halogène, notamment Br, Cl, F ou I ;
* un groupe amino NH₂ ;
* un groupe *N*-alkylamino NHRₐ, Rₐ étant tel que défini ci-dessus ;
* un groupe *N,N-*dialkylamino NRₐR_{b}, notamment un groupe *N*,*N-*diméthylamino, Rₐ et R_{b} étant tels que définis ci-dessus,
lesdits composés de formule (I-6-a) pouvant être sous forme d'isomères optiques, à savoir sous forme d'énantiomères et de diastéréoisomères ou de mélanges de ces différentes formes, y compris de mélanges racémiques, ou sous forme, le cas échéant, de sels d'acides physiologiquement acceptables tels que des chlorhydrates, des formiates ou des oxalates (HOOCCOOH).

Les composés de formule (I-6-a) susmentionnée appartiennent à la famille des β-(*N*-Boc)amino *N*-hydroxylamines indoliques.

La présente invention concerne un composé de formule (I-6-a) telle que définie ci-dessus, caractérisé en ce que :
- R₅ représente un atome d'hydrogène ou un atome de brome ;
- R₆ représente un atome d'hydrogène ou un atome de brome,
étant entendu, de préférence, que lorsque R₆ représente un atome de brome, R₅ représente alors un atome d'hydrogène,
et que lorsque R₅ représente un atome de brome, R₆ représente alors un atome d'hydrogène.

La présente description concerne également un composé de formule (I-9-a) suivante : 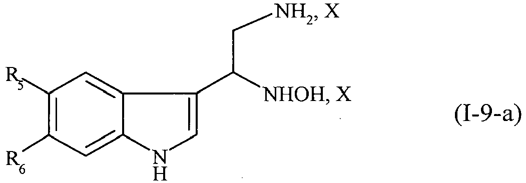 dans laquelle :
- X représente HCl, HCOOH ou HOOCCOOH ;
- R₅ et R₆ représentent indépendamment l'un de l'autre un groupe choisi parmi l'un des groupes suivants :
   * un atome d'hydrogène ;
   * un groupe alkyle comprenant de 1 à 4 atomes de carbone, notamment un groupe méthyle ;
   * un groupe trifluorométhyle ;
   * un groupe trifluorométhoxyle ;
   * un groupe hydroxyle ;
   * un groupe alcoxyle comprenant de 1 à 7 atomes de carbone, notamment un groupe méthoxyle ou un groupe benzyloxyle ;
   * un atome d'halogène, notamment Br, Cl, F ou I ;
   * un groupe amino NH₂ ;
   * un groupe *N*-alkylamino NHRₐ, Rₐ étant tel que défini ci-dessus ;
   * un groupe *N,N-*dialkylamino NRₐR_{b}, notamment un groupe *N*,*N-*diméthylamino, Rₐ et R_{b} étant tels que définis ci-dessus,
lesdits composés de formule (I-9-a) pouvant être sous forme d'isomères optiques, à savoir sous forme d'énantiomères et de diastéréoisomères ou de mélanges de ces différentes formes, y compris de mélanges racémiques, ou sous forme, le cas échéant, de sels d'acides physiologiquement acceptables tels que des chlorhydrates, des formiates ou des oxalates (HOOCCOOH).

Les composés de formule (I-9-a) susmentionnée appartiennent à la famille des *N*-hydroxylamines primaires indoliques β-aminées.

Un composé selon l'invention est un composé de formule (I-9-a) telle que définie ci-dessus, caractérisé en ce que :
- X représente HCl,
- R₅ représente un atome d'hydrogène ou un atome de brome ;
- R₆ représente un atome d'hydrogène ou un atome de brome,
étant entendu, de préférence, que lorsque R₆ représente un atome de brome, R₅ représente alors un atome d'hydrogène,
et que lorsque R₅ représente un atome de brome, R₆ représente alors un atome d'hydrogène.

La présente description concerne également un composé de formule (I-10-a) suivante : 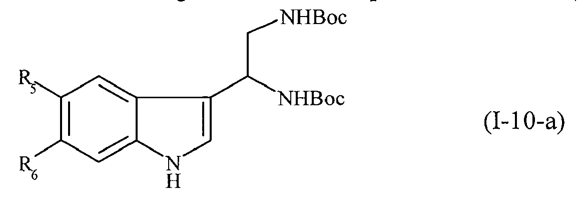 dans laquelle R₅ et R₆ représentent indépendamment l'un de l'autre un groupe choisi parmi l'un des groupes suivants :
* un atome d'hydrogène ;
* un groupe alkyle comprenant de 1 à 4 atomes de carbone, notamment un groupe méthyle ;
* un groupe trifluorométhyle ;
* un groupe trifluorométhoxyle ;
* un groupe hydroxyle ;
* un groupe alcoxyle comprenant de 1 à 7 atomes de carbone, notamment un groupe méthoxyle ou un groupe benzyloxyle ;
* un atome d'halogène, notamment Br, Cl, F ou I ;
* un groupe amino NH₂ ;
* un groupe *N*-alkylamino NHRₐ, Rₐ étant tel que défini ci-dessus ;
* un groupe *N,N-*dialkylamino NRₐR_{b}, notamment un groupe *N*,*N-*diméthylamino, Rₐ et R_{b} étant tels que définis ci-dessus,
lesdits composés de formule (I-10-a) pouvant être sous forme d'isomères optiques, à savoir sous forme d'énantiomères et de diastéréoisomères ou de mélanges de ces différentes formes, y compris de mélanges racémiques.

Les composés de formule (I-10-a) susmentionnée appartiennent à la famille des di-*N*-(Boc)-diamines indoliques.

Un composé selon l'invention est un composé de formule (I-10-a) telle que définie ci-dessus, caractérisé en ce que :
- R₅ représente un atome d'hydrogène ou un atome de brome ;
- R₆ représente un atome d'hydrogène ou un atome de brome,
étant entendu, de préférence, que lorsque R₆ représente un atome de brome, R₅ représente alors un atome d'hydrogène,
et que lorsque R₅ représente un atome de brome, R₆ représente alors un atome d'hydrogène.

La présente description concerne également un composé de formule (I-11) suivante : dans laquelle : 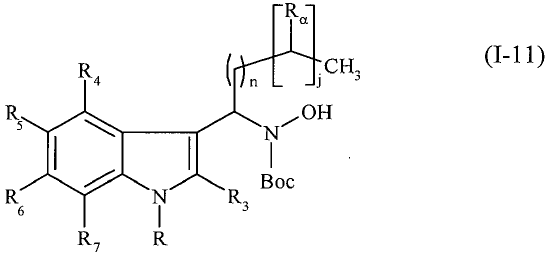
- n est égal à 0, 1 ou 2 ;
- j est égal à 0 ou 1 ;
- R, R₃, R₄, R₅, R₆ et R₇ sont tels que définis ci-dessus dans la formule (I) ;
- R_{α} représentant un atome d'hydrogène ou un groupe méthyle ou éthyle.

Les composés de formule (I-11) correspondent à des composés de formule (I) dans laquelle :
- R₁ représente un groupe -(CH₂)ₙ-(CHR_{α})ⱼ-CH3 ;
- R₂ représente un groupe OH ; et
- B représente un groupe de formule (B-2) avec GP₁ = Boc.

Selon un mode de réalisation préféré, les composés de formules (I-11) répondent à la formule donnée ci-dessus dans laquelle R, R₃, R₄ et R₇ représentent un atome d'hydrogène. De tels composés forment une sous famille répondant à la formule générale (I-11-a) suivante : 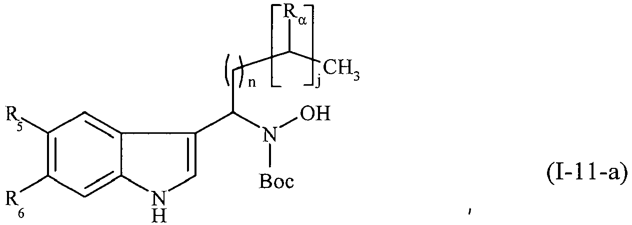 dans laquelle n , j, R_{α}, R₅ et R₆ sont tels que définis ci-dessus.

La présente description concerne en particulier un composé de formule suivante : 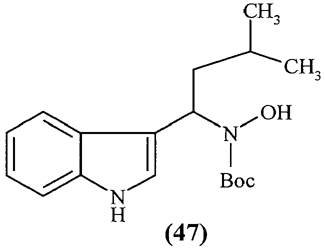 La présente description concerne également un composé de formule (I-12) suivante : dans laquelle :
- n est égal à 1 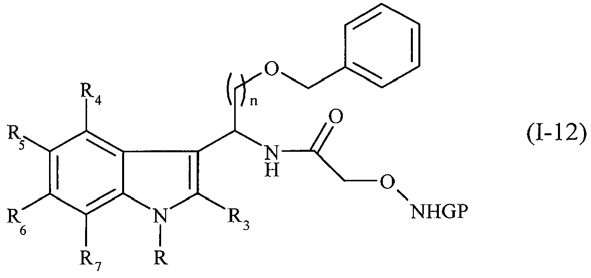
- R, R₃, R₄, R₅, R₆ et R₇ sont tels que définis ci-dessus dans la formule (I) ;
- GP représentant un groupe Boc, Fmoc, Ac, Bz ou CF₃CO.

Les composés de formule (I-12) correspondent à des composés de formule (I) dans laquelle :
- R₁ représente un groupe -(CH₂)ₙ-O-CH₂-Ph;
- R₂ représente un groupe COCH₂ONHGP ; et
- B représente un groupe de formule (B-3) avec R_{c} = H.

Selon un mode de réalisation préféré, les composés de formule (I-12) répondent à la formule donnée ci-dessus dans laquelle R, R₃, R₄ et R₇ représentent un atome d'hydrogène. De tels composés forment une sous famille répondant à la formule générale (I-12-a) suivante: 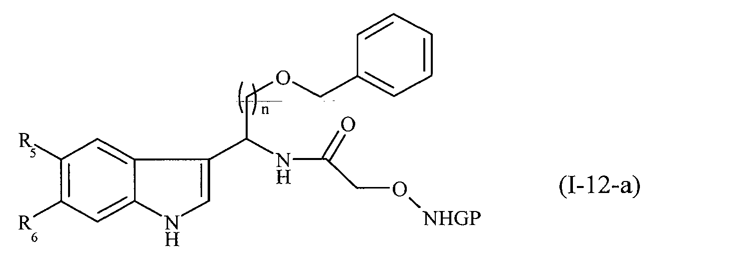 dans laquelle n , GP, R₅ et R₆ sont tels que définis ci-dessus.

La présente description concerne en particulier un composé de formule suivante :

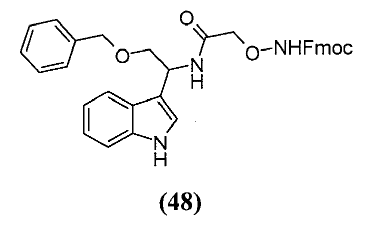

La présente description concerne également les composés répondant à l'une des formules suivantes :

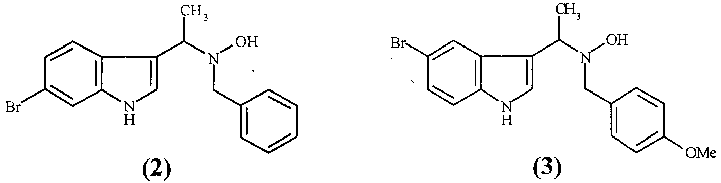

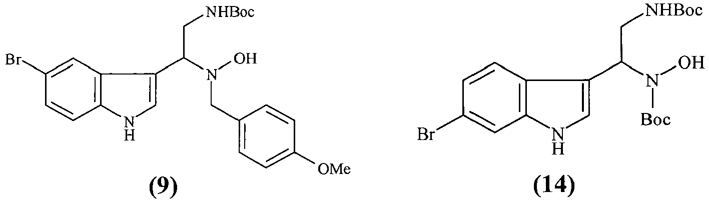

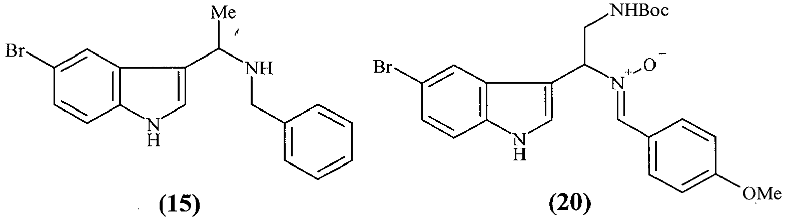

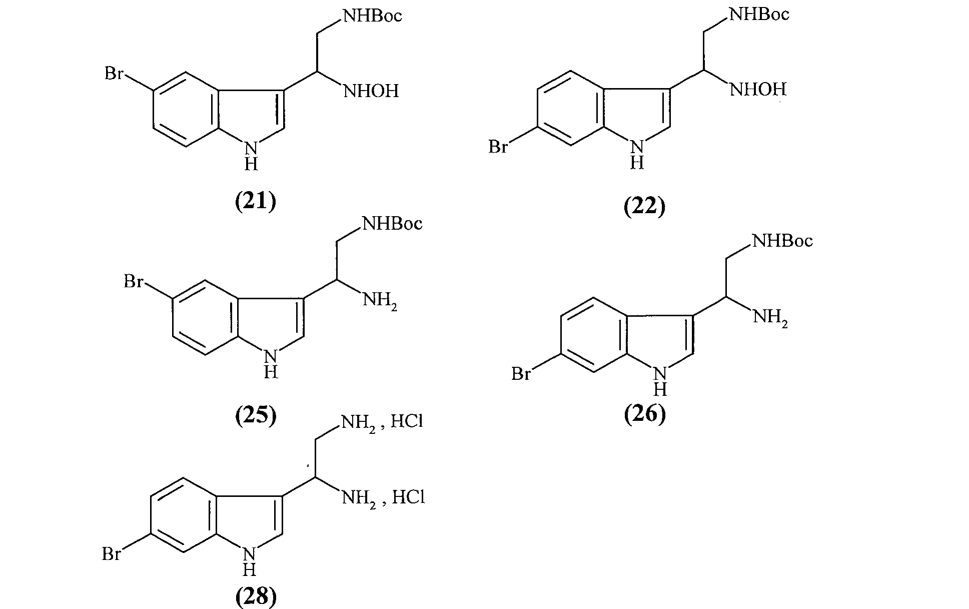

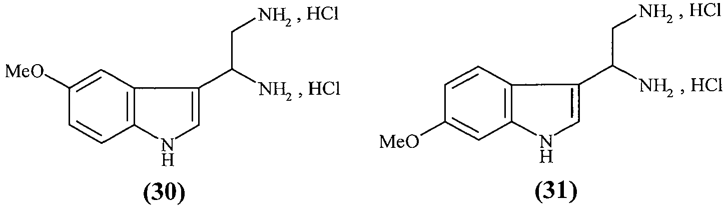

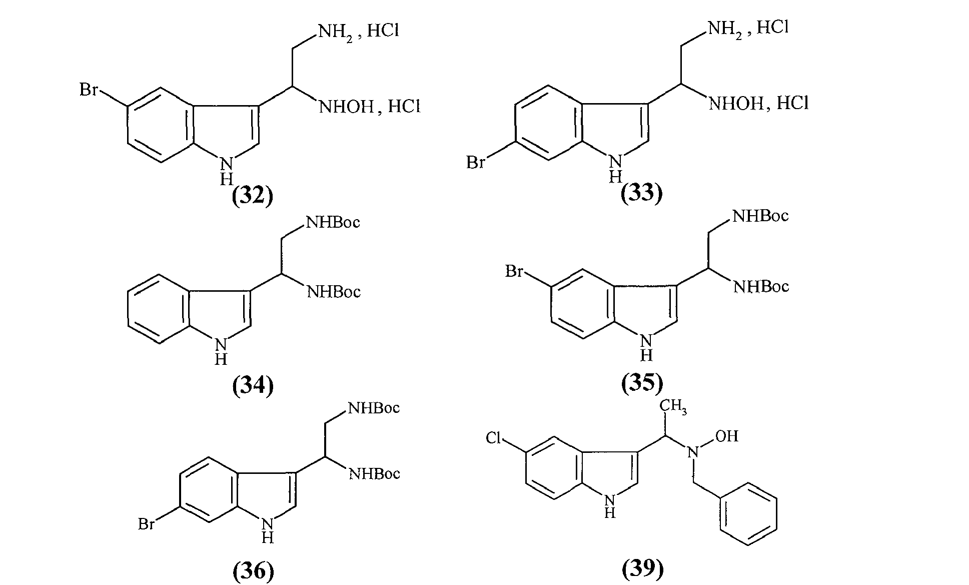

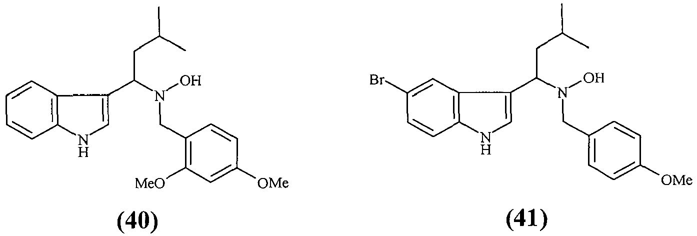

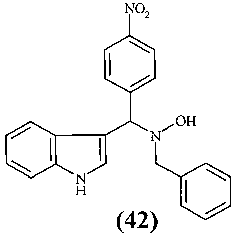

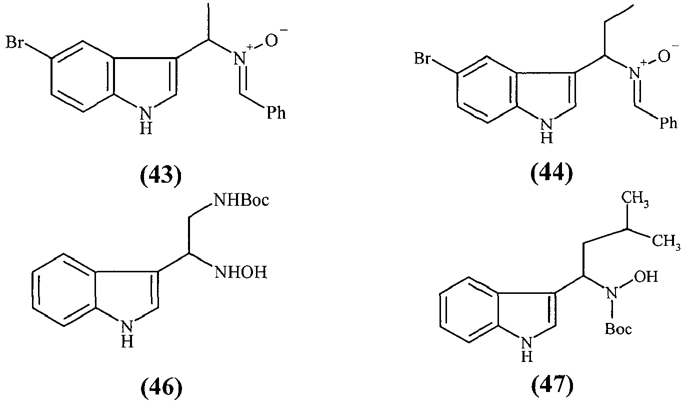

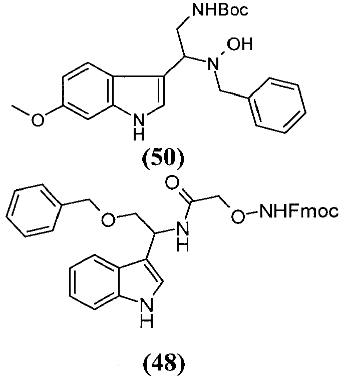

La présente invention concerne également les composés répondant à l'une des formules suivantes :

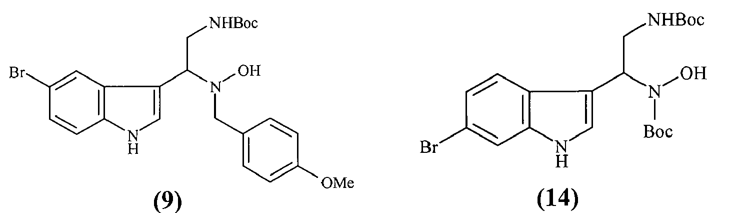

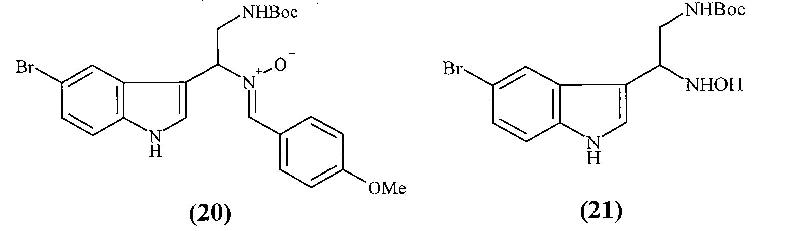

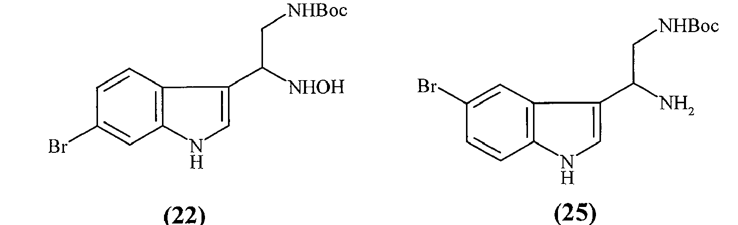

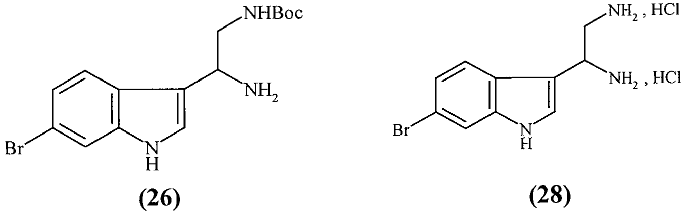

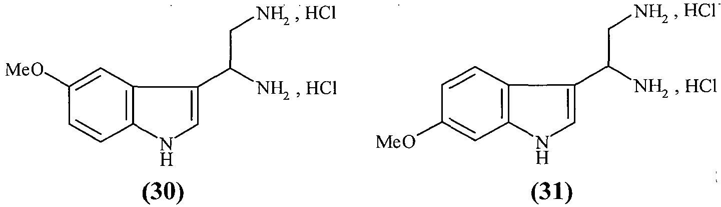

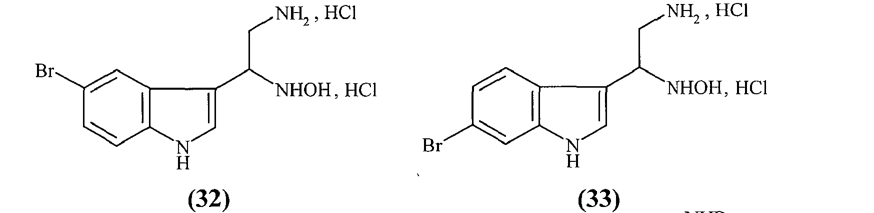

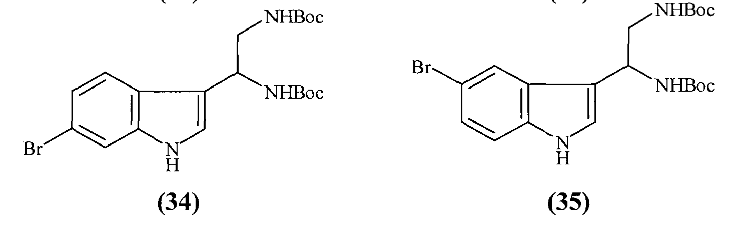

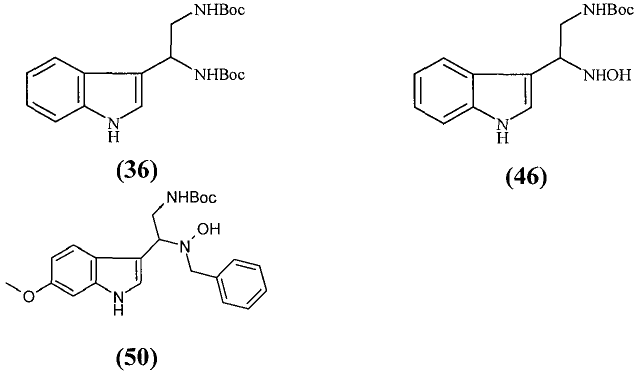

La présente description concerne également une composition pharmaceutique comprenant un composé tel que défini ci-dessus, répondant à l'une des formules (I-6-a), (I-9-a), (I-10-a), (I-11-a) ou **(50)** en association avec un composé pharmaceutiquement acceptable.

La présente invention concerne également une composition pharmaceutique comprenant un composé tel que défini ci-dessus, répondant à l'une des formules (I-6-a), (I-9-a), (I-10-a) ou **(50)** en association avec un composé pharmaceutiquement acceptable.

Selon un mode de réalisation, la composition pharmaceutique telle que définie ci-dessus et comprenant un composé tel que défini ci-dessus, répondant à l'une des formules (I-6-a), (I-9-a), (I-10-a), (I-11-a) ou **(50),** contient d'environ 350 à environ 2 000 mg, de préférence d'environ 1 000 à environ 1 500 mg, dudit composé de formule (I-6-a), (I-9-a), (I-10-a), (I-11-a) ou **(50)** en 1 à 4 prises par jour, et notamment en 2 prises par jour.

La présente description concerne également un procédé de préparation d'une nitrone indolique de formule (I-5-bis) suivante: dans laquelle : 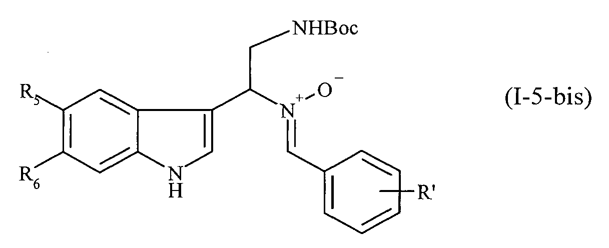
- R₅ et R₆ représentent indépendamment l'un de l'autre un groupe choisi parmi l'un des groupes suivants :
   * un atome d'hydrogène ;
   * un groupe alkyle comprenant de 1 à 4 atomes de carbone, notamment un groupe méthyle ;
   * un groupe trifluorométhyle ;
   * un groupe trifluorométhoxyle ;
   * un groupe hydroxyle ;
   * un groupe alcoxyle comprenant de 1 à 7 atomes de carbone, notamment un groupe méthoxyle ou un groupe benzyloxyle ;
   * un atome d'halogène, notamment Br, Cl, F ou I ;
   * un groupe amino NH₂ ;
   * un groupe *N*-alkylamino NHRₐ, Rₐ étant tel que défini ci-dessus ;
   * un groupe *N,N-*dialkylamino NRₐR_{b}, notamment un groupe *N*,*N-*diméthylamino, Rₐ et R_{b} étant tels que définis ci-dessus,
- R' représente un groupe choisi parmi l'un des groupes suivants :
   * un atome d'hydrogène ;
   * un groupe OH ;
   * un groupe alcoxyle comprenant de 1 à 4 atomes de carbone, notamment o-méthoxyle, *m*-méthoxyle et *p*-méthoxyle ;
   * un groupe NH₂ ;
   * un groupe *N*-alkylamino NHRₐ, Rₐ étant tel que défini ci-dessus ;
   * un groupe *N,N-*dialkylamino NRₐR_{b}, notamment un groupe *N*,*N-*diméthylamino, Rₐ et R_{b} étant tels que définis ci-dessus,
ledit procédé étant caractérisé en ce qu'il comprend une étape d'oxydation de préférence avec MnO₂ dans le toluène ou avec l'AMCPB dans le dichlorométhane ou de l'eau de javel dans le mélange DCM/eau, de préférence avec MnO₂ dans le toluène, de préférence à 100°C, d'un composé de formule (I-1-bis) suivante : 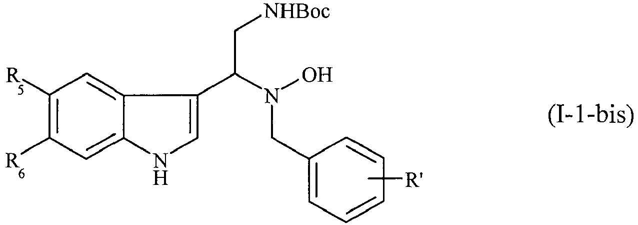 R₅, R₆ et R' étant tels que définis ci-dessus.

La présente invention concerne également un procédé de préparation 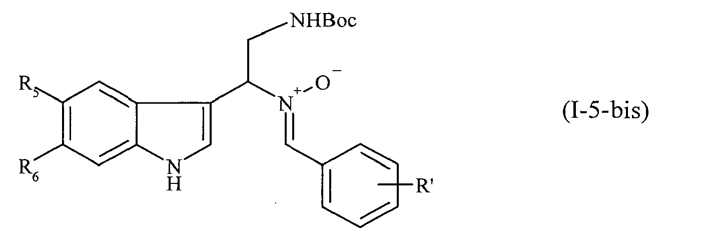 dans laquelle :
- R₅ et R₆ représentent indépendamment l'un de l'autre un groupe choisi parmi l'un des groupes suivants:
   * un atome d'hydrogène;
   * un groupe alcoxyle comprenant de 1 à 7 atomes de carbone ou un groupe benzyloxyle;
   * un atome d'halogène choisi parmi Br, Cl, F ou I;
- R' représente un groupe choisi parmi l'un des groupes suivants:
   * un atome d'hydrogène;
   * un groupe alcoxyle comprenant de 1 à 4 atomes de carbone;
ledit procédé étant caractérisé en ce qu'il comprend une étape d'oxydation avec MnO₂ dans le toluène ou avec l'AMCPB dans le dichlorométhane ou de l'eau de javel dans le mélange DCM/eau, à 100°C, d'un composé de formule (I-1-bis) suivante: 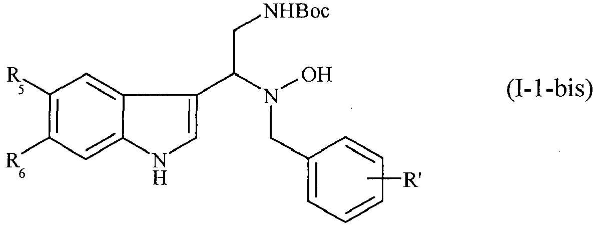 R₅, R₆ et R' étant tels que définis ci-dessus.

Les composés de formule (I-1-bis) correspondent à des composés de formule (I-1-a) dans laquelle R₁ = CH₂NHBoc et R" = H.

La présente description concerne également un procédé de préparation des *N-*(Boc)hydroxylamines indoliques *β*-aminées à partir de nitrones indoliques, ce qui correspond à un procédé de préparation d'un composé de formule (I-6-a) suivante : 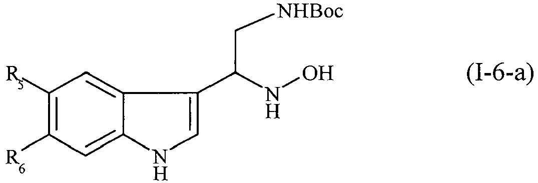 dans laquelle R₅ et R₆ représentent indépendamment l'un de l'autre un groupe choisi parmi l'un des groupes suivants :
* un atome d'hydrogène ;
* un groupe alkyle comprenant de 1 à 4 atomes de carbone notamment un groupe méthyle ;
* un groupe trifluorométhyle ;
* un groupe trifluorométhoxyle ;
* un groupe hydroxyle ;
* un groupe alcoxyle comprenant de 1 à 7 atomes de carbone, notamment un groupe méthoxyle ou un groupe benzyloxyle ;
* un atome d'halogène, notamment Br, Cl, F ou I ;
* un groupe amino NH₂ ;
* un groupe *N*-alkylamino NHRₐ, Rₐ étant tel que défini ci-dessus ;
* un groupe *N,N-*dialkylamino NRₐR_{b}, notamment un groupe *N*,*N-*diméthylamino, Rₐ et R_{b} étant tels que définis ci-dessus,
comprenant une étape de traitement avec NH₂OH.HCl (chlorhydrate d'hydroxylamine), de préférence dans le méthanol à température ambiante, d'un composé de formule (I-5-bis) suivante: 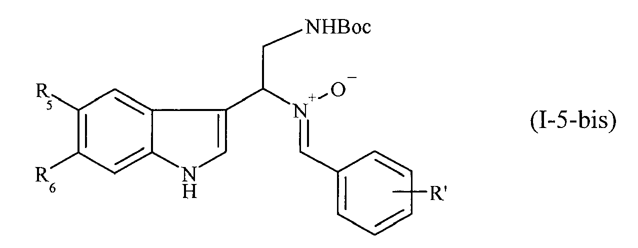
R₅ et R₆ étant tels que définis ci-dessus, et
R' représentant un groupe choisi parmi l'un des groupes suivants :
   * un atome d'hydrogène ;
   * un groupe OH ;
   * un groupe alcoxyle comprenant de 1 à 4 atomes de carbone, notamment *o*-méthoxyle, *m*-méthoxyle et *p*-méthoxyle ;
   * un groupe NH₂ ;
   * un groupe *N*-alkylamino NHRₐ, Rₐ étant tel que défini ci-dessus ;
   * un groupe *N,N-*dialkylamino NRₐR_{b}, notamment un groupe *N*,*N-*diméthylamino, Rₐ et R_{b} étant tels que définis ci-dessus.

La présente description concerne un procédé de préparation d'un composé de formule (I-6-a) telle que mentionné ci-dessus, dans lequel le composé de formule (I-5-bis) est obtenu par le procédé de préparation tel que défini ci-dessus à partir d'un composé de formule (I-1-bis).

La présente description concerne un procédé de préparation de *N-*(Boc)hydroxylamines indoliques β-aminées à partir de *N*-hydroxylamines indoliques, correspondant à la préparation d'un composé de formule (I-6-a) suivante : 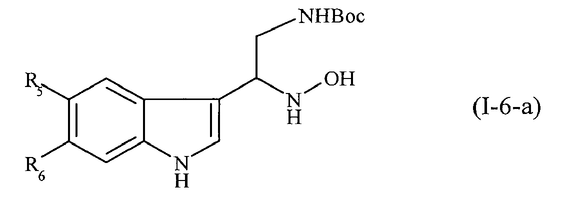 dans laquelle R₅ et R₆ représentent indépendamment l'un de l'autre un groupe choisi parmi l'un des groupes suivants :
* un atome d'hydrogène ;
* un groupe alkyle comprenant de 1 à 4 atomes de carbone notamment un groupe méthyle ;
* un groupe trifluorométhyle ;
* un groupe trifluorométhoxyle ;
* un groupe hydroxyle ;
* un groupe alcoxyle comprenant de 1 à 7 atomes de carbone, notamment un groupe méthoxyle ou un groupe benzyloxyle ;
* un atome d'halogène, notamment Br, Cl, F ou I ;
* un groupe amino NH₂ ;
* un groupe *N*-alkylamino NHRₐ, Rₐ étant tel que défini ci-dessus ;
* un groupe *N,N-*dialkylamino NRₐR_{b}, notamment un groupe *N*,*N-*diméthylamino, Rₐ et R_{b} étant tels que définis ci-dessus,
comprenant les étapes suivantes :
- une étape d'oxydation de préférence avec MnO₂ de préférence dans le toluène à 100°C, d'un composé de formule (I-1-bis) suivante : 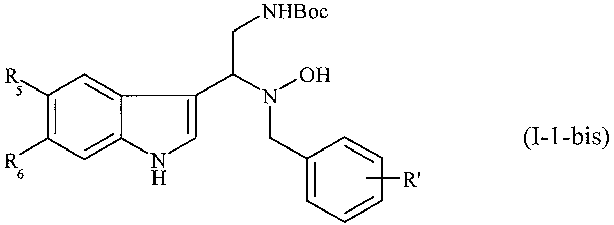 dans laquelle :
   - R₅ et R₆ sont tels que définis ci-dessus, et
   - R' représente un groupe choisi parmi l'un des groupes suivants :
      * un atome d'hydrogène ;
      * un groupe OH ;
      * un groupe alcoxyle comprenant de 1 à 4 atomes de carbone, notamment *o*-méthoxyle, *m*-méthoxyle et *p*-méthoxyle ;
      * un groupe NH₂ ;
      * un groupe *N*-alkylamino NHRₐ, Rₐ étant tel que défini ci-dessus ;
      * un groupe *N,N-*dialkylamino NRₐR_{b}, notamment un groupe *N*,*N-*diméthylamino, Rₐ et R_{b} étant tels que définis ci-dessus,
   afin d'obtenir un composé de formule (I-5-bis) suivante : 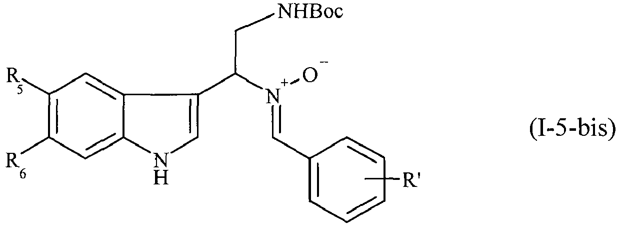 R₅, R₆ et R' étant tels que définis ci-dessus,
- et une étape de traitement avec NH₂OH.HCl, de préférence dans le méthanol à température ambiante, du composé de formule (I-5-bis) tel qu'obtenu à l'issue de l'étape précédente, afin d'obtenir un composé de formule (I-6-a).

La présente invention concerne un procédé de préparation d'un composé de formule (I-6-a) suivante : 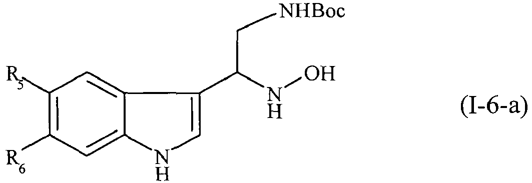 dans laquelle R₅ et R₆ représentent indépendamment l'un de l'autre un groupe choisi parmi l'un des groupes suivants :
* un atome d'hydrogène ;
* un groupe alcoxyle comprenant de 1 à 7 atomes de carbone ou un groupe benzyloxyle ;
* un atome d'halogène choisi parmi Br, Cl, F ou I ;
comprenant une étape de traitement avec NH₂OH.HCl (chlorhydrate d'hydroxylamine), dans le méthanol à température ambiante, d'un composé de formule (I-5-bis) suivante: 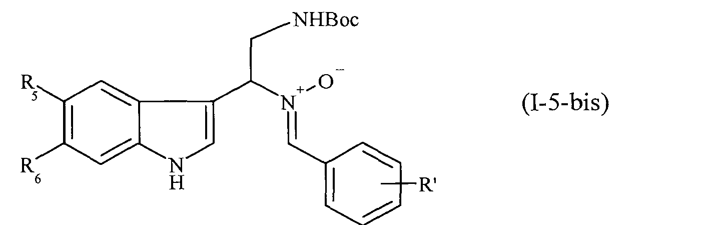
R₅ et R₆ étant tels que définis ci-dessus, et
R' représentant un groupe choisi parmi l'un des groupes suivants:
   * un atome d'hydrogène;
   * un groupe alcoxyle comprenant de 1 à 4 atomes de carbone;
le composé de formule (I-5-bis) étant obtenu par le procédé de préparation décrit précédemment.

La présente description concerne un procédé de préparation de 1,2-diamines indoliques monoprotégées à partir des *N*-(Boc)hydroxylamines indoliques *β*-aminées, correspondant à la préparation d'un composé de formule (I-7-a) suivante : 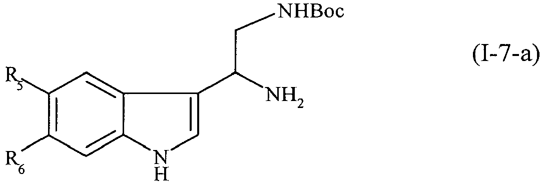 dans laquelle R₅ et R₆ représentent indépendamment l'un de l'autre un groupe choisi parmi l'un des groupes suivants :
* un atome d'hydrogène ;
* un groupe alkyle comprenant de 1 à 4 atomes de carbone notamment un groupe méthyle ;
* un groupe trifluorométhyle ;
* un groupe trifluorométhoxyle ;
* un groupe hydroxyle ;
* un groupe hydroxyméthyle (-CH₂OH) ;
* un groupe alcoxyle comprenant de 1 à 7 atomes de carbone, notamment un groupe méthoxyle ou un groupe benzyloxyle ;
* un atome d'halogène, notamment Br, Cl, F ou I ;
* un groupe amino NH₂ ;
* un groupe *N*-alkylamino NHRₐ, Rₐ étant tel que défini ci-dessus ;
* un groupe *N*,*N*-dialkylamino NRₐR_{b}, notamment un groupe *N*,*N-*diméthylamino, Rₐ et R_{b} étant tels que définis ci-dessus,
ledit procédé comprenant une étape de traitement avec TiCl₃/HCl, de préférence dans le méthanol à température ambiante, d'un composé de formule (I-6-a) suivante : 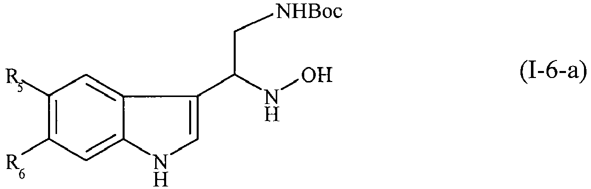 R₅ et R₆ étant tels que définis ci-dessus.

La présente description concerne également un procédé de préparation d'un composé de formule (I-7-a) tel que défini ci-dessus, dans lequel le composé de formule (I-6-a) est obtenu par le procédé de préparation susmentionné à partir d'un composé de formule (I-1-bis).

La présente description concerne également la préparation de 1,2-diamines indoliques monoprotégées à partir de *N*-hydroxylamines indoliques, ce qui correspond à un procédé de préparation d'un composé de formule (I-7-a) suivante : 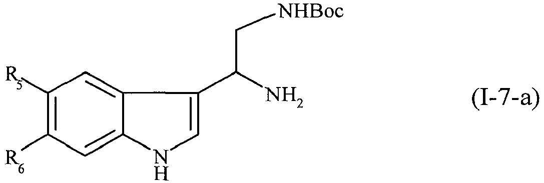 dans laquelle R₅ et R₆ représentent indépendamment l'un de l'autre un groupe choisi parmi l'un des groupes suivants :
* un atome d'hydrogène ;
* un groupe alkyle comprenant de 1 à 4 atomes de carbone notamment un groupe méthyle ;
* un groupe trifluorométhyle ;
* un groupe trifluorométhoxyle ;
* un groupe hydroxyle ;
* un groupe alcoxyle comprenant de 1 à 7 atomes de carbone, notamment un groupe méthoxyle ou un groupe benzyloxyle ;
* un atome d'halogène, notamment Br, Cl, F ou I ;
* un groupe amino NH₂ ;
* un groupe *N*-alkylamino NHRₐ, Rₐ étant tel que défini ci-dessus ;
* un groupe *N,N-*dialkylamino NRₐR_{b}, notamment un groupe *N*,*N-*diméthylamino, Rₐ et R_{b} étant tels que définis ci-dessus,
ledit procédé comprenant les étapes suivantes :
- une étape d'oxydation de préférence avec MnO₂, de préférence dans le toluène à 100°C, d'un composé de formule (I-1-bis) suivante : 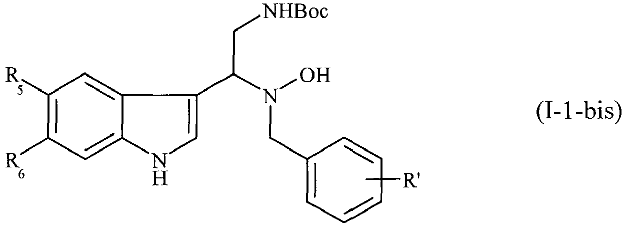 dans laquelle :
   - R₅ et R₆ sont tels que définis ci-dessus ; et
   - R' représente un groupe choisi parmi l'un des groupes suivants :
      * un atome d'hydrogène ;
      * un groupe OH ;
      * un groupe alcoxyle comprenant de 1 à 4 atomes de carbone, notamment *o*-méthoxyle, *m*-méthoxyle et *p*-méthoxyle ;
      * un groupe NH₂ ;
      * un groupe *N*-alkylamino NHRₐ, Rₐ étant tel que défini ci-dessus ;
      * un groupe *N*,*N*-dialkylamino NRₐR_{b}, notamment un groupe *N*,*N-*diméthylamino, Rₐ et R_{b} étant tels que définis ci-dessus,
   afin d'obtenir un composé de formule (I-5-bis) suivante : 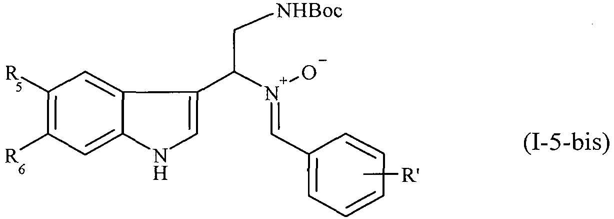 R₅, R₆ et R' étant tels que définis ci-dessus,
- une étape de traitement avec NH₂OH.HCl, de préférence dans le méthanol à température ambiante, du composé de formule (I-5-bis) tel qu'obtenu à l'issue de l'étape précédente, afin d'obtenir un composé de formule (I-6-a) suivante : 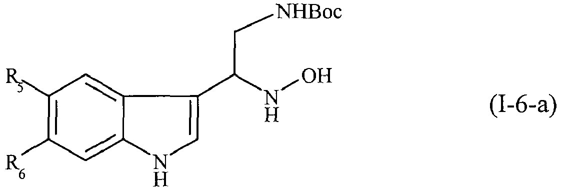 R₅ et R₆ étant tels que définis ci-dessus,
- et une étape de traitement avec TiCl₃/HCl, de préférence dans le méthanol à température ambiante, du composé de formule (I-6-a) tel qu'obtenu à l'issue de l'étape précédente, afin d'obtenir un composé de formule (I-7-a).

La présente invention concerne un procédé de préparation d'un composé de formule (I-7-a) suivante : 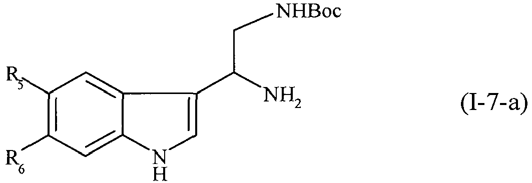 dans laquelle R₅ et R₆ représentent indépendamment l'un de l'autre un groupe choisi parmi l'un des groupes suivants:
* un atome d'hydrogène;
* un groupe alcoxyle comprenant de 1 à 7 atomes de carbone ou un groupe benzyloxyle;
* un atome d'halogène choisi parmi Br, Cl, F ou I ;
ledit procédé comprenant une étape de traitement avec TiCl₃/HCl, dans le méthanol à température ambiante, d'un composé de formule (I-6-a) suivante: 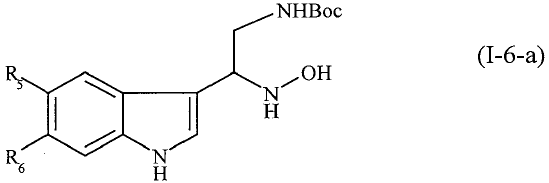 R₅ et R₆ étant tels que définis ci-dessus,
le composé de formule (I-6-a) étant obtenu par le procédé de préparation décrit précédemment.

La présente description concerne également un procédé de préparation de dichlorhydrates de 1,2-diamines indoliques à partir de 1,2-diamines indoliques monoprotégées, ce qui correspond à un procédé de préparation d'un composé de formule (I-8-a) suivante: 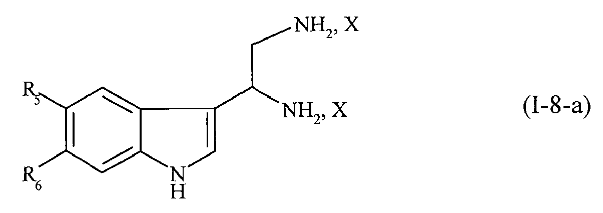 dans laquelle R₅ et R₆ représentent indépendamment l'un de l'autre un groupe choisi parmi l'un des groupes suivants :
* un atome d'hydrogène ;
* un groupe alkyle comprenant de 1 à 4 atomes de carbone notamment un groupe méthyle ;
* un groupe trifluorométhyle ;
* un groupe trifluorométhoxyle ;
* un groupe hydroxyle ;
_{*} un groupe alcoxyle comprenant de 1 à 7 atomes de carbone, notamment un groupe méthoxyle ou un groupe benzyloxyle ;
* un atome d'halogène, notamment Br, Cl, F ou I ;
* un groupe amino NH₂ ;
* un groupe *N*-alkylamino NHRₐ, Rₐ étant tel que défini ci-dessus ;
* un groupe *N,N*-dialkylamino NRₐR_{b}, notamment un groupe *N*,*N-*diméthylamino, Rₐ et R_{b} étant tels que définis ci-dessus,
ledit procédé comprenant une étape de traitement avec HCl dans le MeOH d'un composé de formule (I-7-a) suivante : 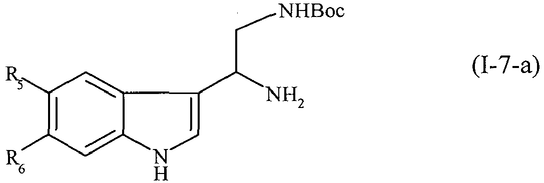 R₅ et R₆ étant tels que définis ci-dessus.

La présente description concerne également un procédé de préparation d'un composé de formule (I-8-a) tel que défini ci-dessus, dans lequel le composé de formule (I-7-a) est obtenu par le procédé de préparation tel que défini ci-dessus à partir d'un composé de formule (I-1-bis).

La présente description concerne également un procédé de préparation de dichlorhydrates de 1,2-diamines indoliques à partir de *N*-hydroxylamines indoliques, ce qui correspond à la préparation d'un composé de formule (I-8-a) suivante : 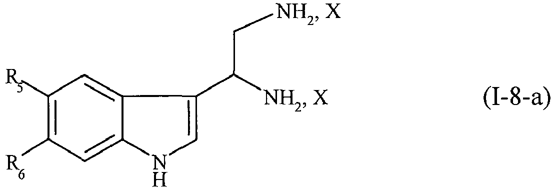 dans laquelle R₅ et R₆ représentent indépendamment l'un de l'autre un groupe choisi parmi l'un des groupes suivants :
* un atome d'hydrogène ;
* un groupe alkyle comprenant de 1 à 4 atomes de carbone notamment un groupe méthyle ;
* un groupe trifluorométhyle ;
* un groupe trifluorométhoxyle ;
* un groupe hydroxyle ;
* un groupe alcoxyle comprenant de 1 à 7 atomes de carbone, notamment un groupe méthoxyle ou un groupe benzyloxyle ;
* un atome d'halogène, notamment Br, Cl, F ou I ;
* un groupe amino NH₂ ;
* un groupe *N*-alkylamino NHRₐ, Rₐ étant tel que défini ci-dessus ;
* un groupe *N,N-*dialkylamino NRₐR_{b}, notamment un groupe *N*,*N-*diméthylamino, Rₐ et R_{b} étant tels que définis ci-dessus,
ledit procédé comprenant les étapes suivantes :
- une étape d'oxydation de préférence avec MnO₂, de préférence dans le toluène à 100°C, d'un composé de formule (I-1-bis) suivante : 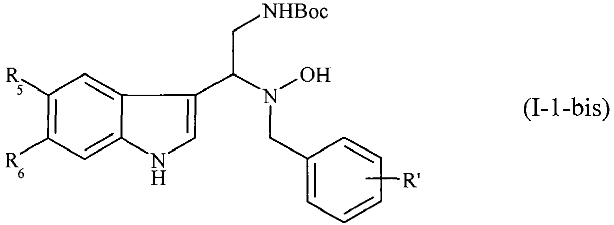 dans laquelle :
   - R₅ et R₆ sont tels que définis ci-dessus ; et
   - R' représente un groupe choisi parmi l'un des groupes suivants :
      * un atome d'hydrogène ;
      * un groupe OH ;
      * un groupe alcoxyle comprenant de 1 à 4 atomes de carbone, notamment *o*-méthoxyle, *m*-méthoxyle et *p*-méthoxyle ;
      * un groupe NH₂ ;
      * un groupe *N*-alkylamino NHRₐ, Rₐ étant tel que défini ci-dessus ;
      * un groupe *N,N-*dialkylamino NRₐR_{b}, notamment un groupe *N*,*N-*diméthylamino, Rₐ et R_{b} étant tels que définis ci-dessus,
   afin d'obtenir un composé de formule (I-5-bis) suivante : 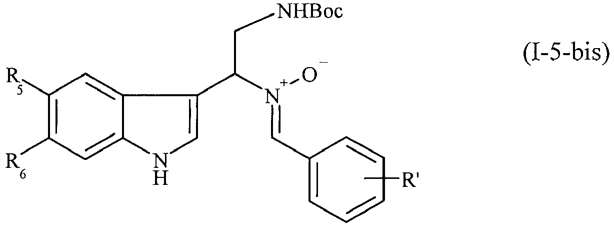 R₅, R₆ et R' étant tels que définis ci-dessus,
- une étape de traitement avec NH₂OH.HCl, de préférence dans le méthanol à température ambiante, du composé de formule (I-5-bis) tel qu'obtenu à l'issue de l'étape précédente, afin d'obtenir un composé de formule (I-6-a) suivante : 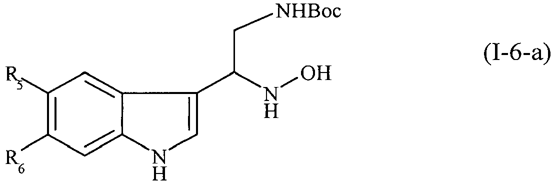 R₅ et R₆ étant tels que définis ci-dessus,
- une étape de traitement avec TiCl₃/HCl, de préférence dans le méthanol à température ambiante, du composé de formule (I-6-a) tel qu'obtenu à l'issue de l'étape précédente, afin d'obtenir un composé de formule (I-7-a) suivante : 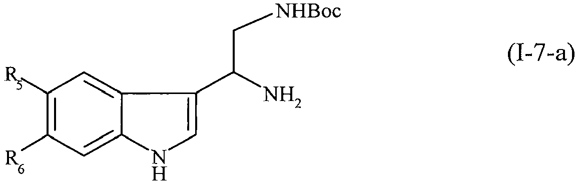 R₅ et R₆ étant tels que défini ci-dessus,
- et une étape de traitement avec HCl dans le MeOH du composé de formule (I-7-a) tel qu'obtenu à l'issue de l'étape précédente, afin d'obtenir un composé de formule (I-8-a).

La présente invention concerne un procédé de préparation d'un composé de formule (I-8-a) suivante: 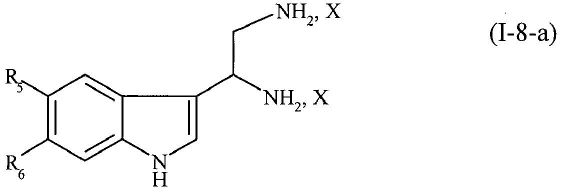 dans laquelle R₅ et R₆ représentent indépendamment l'un de l'autre un groupe choisi parmi l'un des groupes suivants:
* un atome d'hydrogène;
* un groupe alcoxyle comprenant de 1 à 7 atomes de carbone ou un groupe benzyloxyle;
* un atome d'halogène choisi parmi Br, Cl, F ou I;
ledit procédé comprenant une étape de traitement avec HCl dans le MeOH d'un composé de formule (I-7-a) suivante: 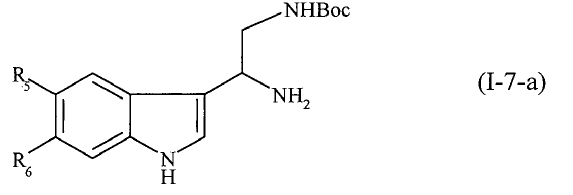 R₅ et R₆ étant tels que définis ci-dessus,
le composé de formule (I-7-a) étant obtenu par le procédé de préparation décrit précédemment.

La présente description concerne également la préparation de *N*-hydroxylamines primaires indoliques β-aminées à partir de *N*-(Boc)hydroxylamines indoliques *β-*aminées, ce qui correspond à la préparation d'un composé de formule (I-9-a) suivante : 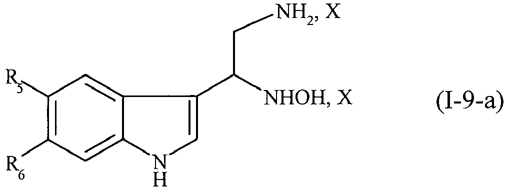 dans laquelle :
- X représente HCl, HCOOH ou HOOCCOOH ;
- R₅ et R₆ représentent indépendamment l'un de l'autre un groupe choisi parmi l'un des groupes suivants :
   * un atome d'hydrogène ;
   * un groupe alkyle comprenant de 1 à 4 atomes de carbone notamment un groupe méthyle ;
   * un groupe trifluorométhyle ;
   * un groupe trifluorométhoxyle ;
   * un groupe hydroxyle ;
   * un groupe alcoxyle comprenant de 1 à 7 atomes de carbone, notamment un groupe méthoxyle ou un groupe benzyloxyle ;
   * un atome d'halogène, notamment Br, Cl, F ou I ;
   * un groupe amino NH₂ ;
   * un groupe *N*-alkylamino NHRₐ, Rₐ étant tel que défini ci-dessus ;
   * un groupe *N,N-*dialkylamino NRₐR_{b}, notamment un groupe *N*,*N-*diméthylamino, Rₐ et R_{b} étant tels que définis ci-dessus,
ledit procédé comprenant une étape de traitement avec HCl sec, de préférence dans le méthanol à température ambiante, d'un composé de formule (I-6-a) suivante : 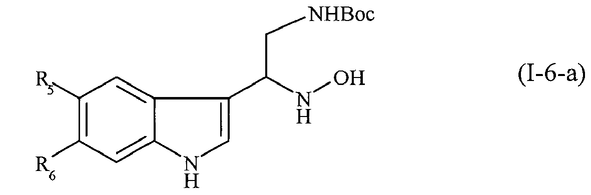 R₅ et R₆ étant tels que définis ci-dessus.

La présente description concerne également un procédé de préparation d'un composé de formule (I-9-a) tel que défini ci-dessus, caractérisé en ce que le composé de formule (I-6-a) est obtenu par le procédé de préparation tel que défini ci-dessus à partir d'un composé de formule (I-1-bis).

La présente description concerne également la préparation de *N*-hydroxylamines primaires indoliques *β*-aminées à partir de *N*-hydroxylamines indoliques, ce qui correspond à un procédé de préparation d'un composé de formule (I-9-a) suivante : 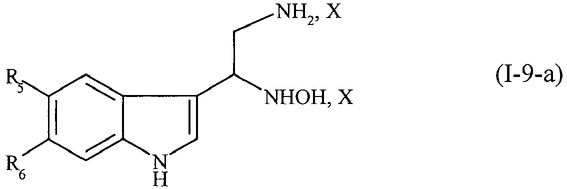 dans laquelle :
- X représente HCl, HCOOH ou HOOCCOOH ;
- R₅ et R₆ représentent indépendamment l'un de l'autre un groupe choisi parmi l'un des groupes suivants :
   * un atome d'hydrogène ;
   * un groupe alkyle comprenant de 1 à 4 atomes de carbone notamment un groupe méthyle ;
   * un groupe trifluorométhyle ;
   * un groupe trifluorométhoxyle ;
   * un groupe hydroxyle ;
   * un groupe alcoxyle comprenant de 1 à 7 atomes de carbone, notamment un groupe méthoxyle ou un groupe benzyloxyle ;
   * un atome d'halogène, notamment Br, Cl, F ou I ;
   * un groupe amino NH₂ ;
   * un groupe *N*-alkylamino NHRₐ, Rₐ étant tel que défini ci-dessus ;
   * un groupe *N,N-*dialkylamino NRₐR_{b}, notamment un groupe *N*,*N-*diméthylamino, Rₐ et R_{b} étant tels que définis ci-dessus,
ledit procédé comprenant les étapes suivantes :
- une étape d'oxydation de préférence avec MnO₂, de préférence dans le toluène à 100°C, d'un composé de formule (I-1-bis) suivante : 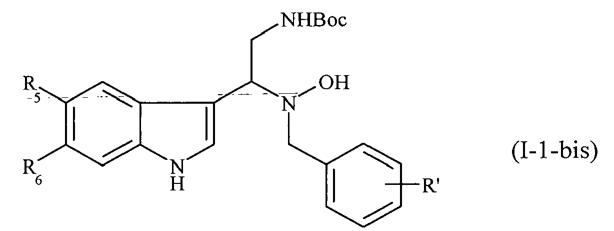 dans laquelle :
   - R₅ et R₆ sont tels que définis ci-dessus ; et
   - R' représente un groupe choisi parmi l'un des groupes suivants :
      * un atome d'hydrogène ;
      * un groupe OH ;
      * un groupe alcoxyle comprenant de 1 à 4 atomes de carbone, notamment *o*-méthoxyle, *m*-méthoxyle et *p*-méthoxyle ;
      * un groupe NH₂ ;
      * un groupe *N*-alkylamino NHRₐ, Rₐ étant tel que défini ci-dessus ;
      * un groupe *N,N-*dialkylamino NRₐR_{b}, notamment un groupe *N*,*N-*diméthylamino, Rₐ et R_{b} étant tels que définis ci-dessus,
   afin d'obtenir un composé de formule (I-5-bis) suivante : 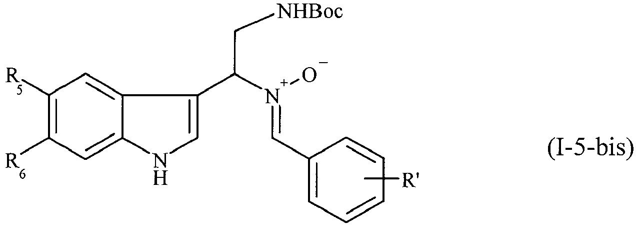 R₅, R₆ et R' étant tels que définis ci-dessus,
- une étape de traitement avec NH₂OH.HCl, de préférence dans le méthanol à température ambiante, du composé de formule (I-5-bis) tel qu'obtenu à l'issue de l'étape précédente, afin d'obtenir un composé de formule (I-6-a) suivante : 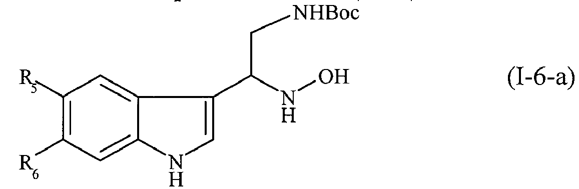 R₅ et R₆ étant tels que définis ci-dessus,
- et une étape de traitement avec HCl sec, de préférence dans le méthanol à température ambiante, du composé de formule (I-6-a) tel qu'obtenu à l'issue de l'étape précédente, afin d'obtenir un composé de formule (I-9-a).

La présente invention concerne un procédé de préparation d'un composé de formule (I-9-a) suivante : 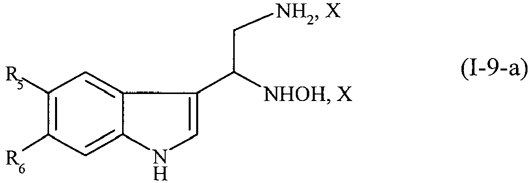 dans laquelle :
- X représente HCl, HCOOH ou HOOCCOOH;
- R₅ et R₆ représentent indépendamment l'un de l'autre un groupe choisi parmi l'un des groupes suivants:
   * un atome d'hydrogène;
   * un groupe alcoxyle comprenant de 1 à 7 atomes de carbone ou un groupe benzyloxyle;
   * un atome d'halogène choisi parmi Br, Cl, F ou I;
ledit procédé comprenant une étape de traitement avec HCl sec, dans le méthanol à température ambiante, d'un composé de formule (I-6-a) suivante: 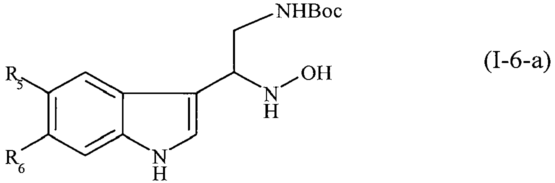 R₅ et R₆ étant tels que définis ci-dessus,
le composé de formule (I-6-a) étant obtenu par le procédé de préparation décrit précédemment.

La présente invention concerne également un procédé de préparation tel que défini ci-dessus, pour la préparation d'un composé de formule (I-5-bis), (I-6-a), (I-7-a), (I-8-a) ou (I-9-a), caractérisé en ce que R₅ ou R₆ représente un atome de brome.

### PARTIE EXPÉRIMENTALE - CHIMIE

Des stratégies de synthèse originales qui permettent d'accéder à des nouvelles classes d'alcaloïdes marins bio-actifs ont été développées. Ces stratégies de synthèse sont axées sur la réaction de nitrones vis-à-vis de noyaux indoliques. Cette réaction, qui conduit aux *N*-hydroxylamines indoliques β-aminée, a été décrite dans 2 publications : J.-N. Denis, H. Mauger, Y. Vallée Tetrahedron Lett. 1997, 38, 8515-8518 ; H. Chalaye-Mauger, J.-N. Denis, M.-T. Averbuch-Pouchot, Y.Vallée Tetrahedron 2000, 56, 791-804.

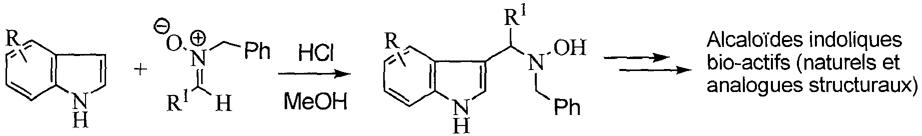

Les intermédiaires de synthèse, issus de cette méthodologie inédite, sont des dérivés indoliques possédant des structures originales. Ces composés, synthétisés *sans a priori biologique,* ont été testés pour évaluer leur activité biologique potentielle, non encore explorée. Son criblage sur deux souches bactériennes sensibles - *Escherichia coli* (Gram négatif) et *Staphylococcus aureus* (Gram positif) - a montré que certains dérivés indoliques cités ci-dessus présentaient une activité antibiotique sur *Staphylococcus aureus.* La similitude des structures de certaines touches obtenues a permis de les classer en plusieurs familles.

La validation chimique des molécules obtenues a été effectuée : la plupart d'entre elles sont stables. Les réactions ainsi que leurs structures sont décrites ci-dessous.

### SYNTHÈSES DES COMPOSÉS INDOLIQUES

### N-hydroxylamines indoliques

Les *N*-benzylhydroxylamines indoliques de structure générale (I-1-a) (avec R'=R"=H) ont été obtenues par réaction des nitrones avec des noyaux indoliques selon une procédure générale rapportée dans la littérature (J.-N. Denis, H. Mauger, Y. Vallée Tetrahedron Lett. 1997, 38, 8515-8518 ; H. Chalaye-Mauger, J.-N. Denis, M.-T Averbuch-Pouchot, Y.Vallée Tetrahedron 2000, 56, 791-804) et résumée ci-dessous :

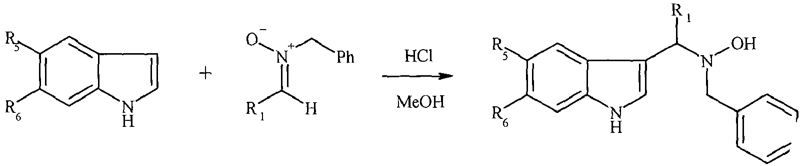

Parmi ces composés, on a notamment synthétisé les composés suivants (les composés (1), (2) et (3) ne font pas partie de l'invention):

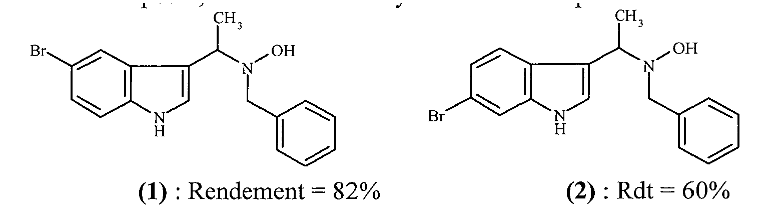

Le composé **(1)** est un composé de formule (I-1-a) dans laquelle R' = R" = H ; R₁ = CH₃ ; R₅ = Br et R₆ = H.

Le composé **(2)** est un composé de formule (I-1-a) dans laquelle R' = R" = H ; R₁ = CH₃ ; R₅ = H et R₆ = Br.

La mise en oeuvre de cette réaction a également permis de préparer la *N*-hydroxylamine indolique de structure :

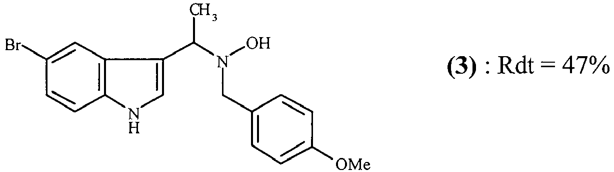

Le composé **(3)** est un composé de formule (I-1-a) dans laquelle R' = *p*-méthoxyle ; R" = H ; R₁ = CH₃ ; R5 = Br et R₆ = H.

Plus particulièrement, les *N*-hydroxylamines indoliques β-aminées de structure (I-1-a) dans laquelle R'=R"=H et R₁ = CH₂NHBoc sont issues d'une réaction entre une nitrone α-aminée et les noyaux indoliques, selon le schéma réactionnel suivant :

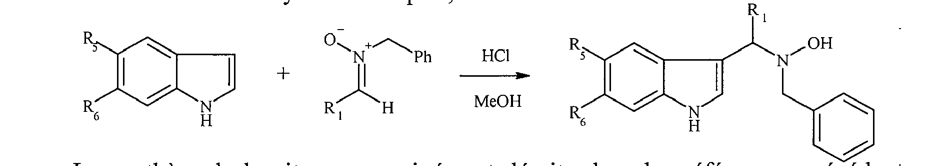

La synthèse de la nitrone α-aminée est décrite dans les références précédentes. Elle est préparée selon la procédure de A. Dondoni, S. Franco, F. Junquera, F. Merchan, P. Merino, T. Tejero Synth. Commun. 1994, 24, 2537-2550.

Parmi ces composés, on a notamment synthétisé les composés suivants :

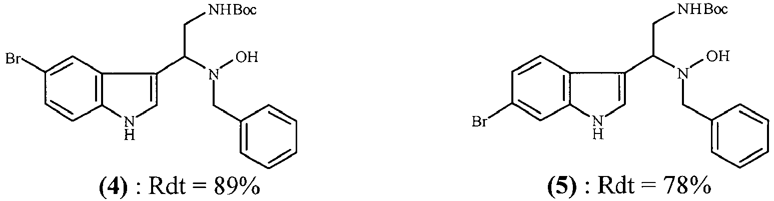

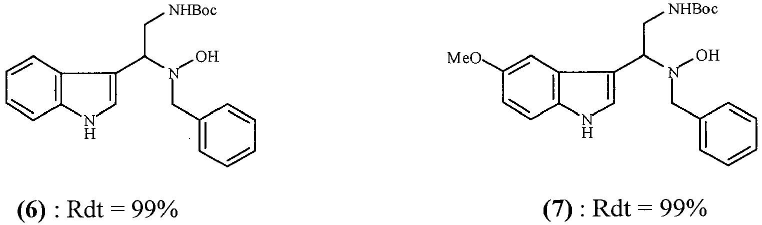

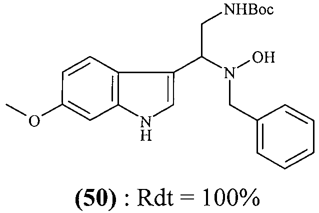

Le composé **(4)** est un composé de formule (I-1-a) dans laquelle R' = R" = H ; R₁ = CH₂NHBoc ; R₅ = Br et R₆ = H.

Le composé **(5)** est un composé de formule (I-1-a) dans laquelle R' = R" = H ; R₁ = CH₂NHBoc ; R₅ = H et R₆ = Br.

Le composé **(6)** est un composé de formule (I-1-a) dans laquelle R' = R" = H ; R₁ = CH₂NHBoc ; R₅ = R₆ = H.

Le composé **(7)** est un composé de formule (I-1-a) dans laquelle R' = R" = H ; R₁ = CH₂NHBoc ; R₅ = OMe et R₆ = H.

Le composé **(50)** est un composé de formule (I-1-a) dans laquelle R' = R" = H ; R₁ = CH₂NHBoc ; R₅ = H et R₆ = OMe.

La mise en oeuvre de cette réaction a également permis de préparer les *N*-hydroxylamines indoliques β-aminée de structure :

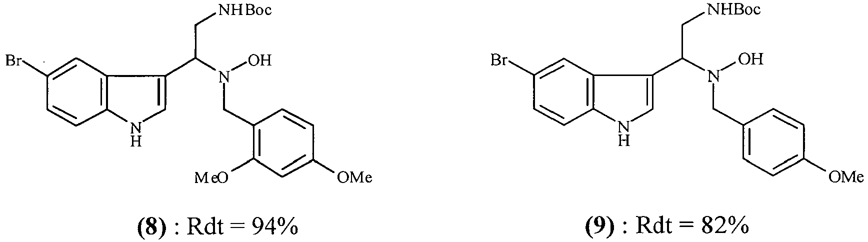

Le composé **(8)** est un composé de formule (I-1-a) dans laquelle R' et R" forment ensemble un groupe *o*,*p*-diméthoxyle ; R₁ = CH₂NHBoc ; R₅ = Br et R₆ = H.

Le composé (9) est un composé de formule (I-1-a) dans laquelle R' = *p*-méthoxyle ; R" = H ; R₁ = CH₂NHBoc ; R₅ = Br et R₆ = H.

Les composés **(37), (38)** (Rdt = 88%), **(39)** (Rdt = 65%), **(40)** (Rdt = 52%), **(41)** (Rdt = 74%) et **(42)** (Rdt = 44%) tels que décrits ci-dessus sont obtenus selon la même procédure.

Les *N*-(Boc)hydroxylamines indoliques β-aminées de structure (I-2-a) ont été obtenues par application de la méthodologie décrite dans l'article : X. Guinchard, Y. Vallée, J.-N. Denis Org. Lett. 2005, 7, 5147-5150 et dont la réaction impliquant les noyaux indoliques est représentée ci-dessous :

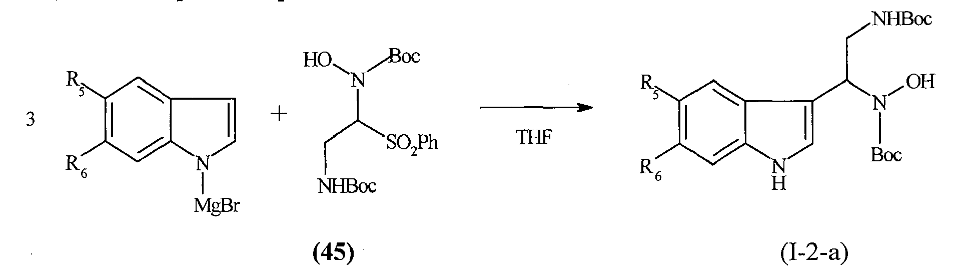

Parmi cette famille de composés, les composés suivants ont été synthétisés :

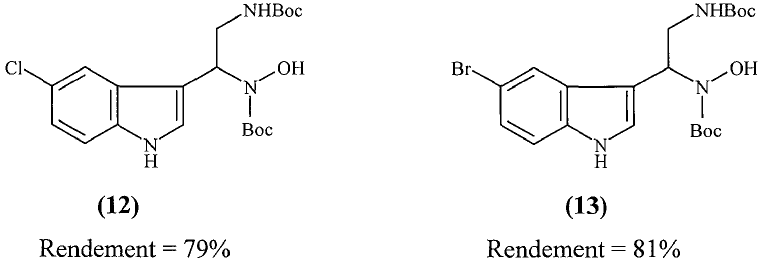

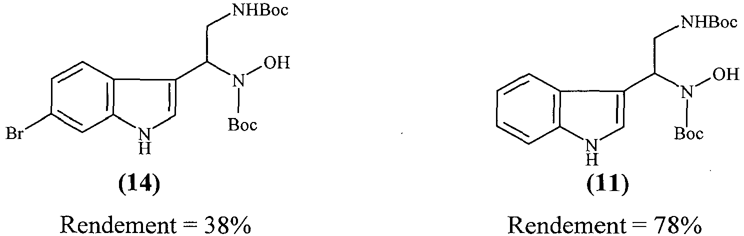

Le composé **(11)** est un composé de formule (I-2-a) dans laquelle R₅ et R₆ = H.

Le composé **(12)** est un composé de formule (I-2-a) dans laquelle R₅ = Cl et R₆ = H.

Le composé **(13)** est un composé de formule (I-2-a) dans laquelle R₅ = Br et R₆ = H.

Le composé **(14)** est un composé de formule (I-2-a) dans laquelle R₅ = H et R₆ = Br.

Les *N*-(Boc)hydroxylamines indoliques β-aminées de structure (I-11-a) ont été obtenues par la réaction analogue suivante :

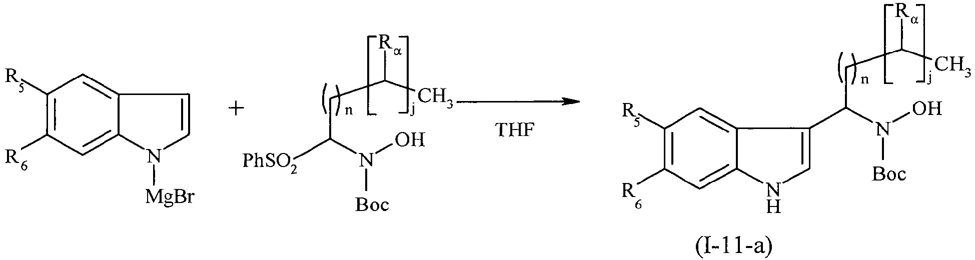

Parmi cette famille de composés, le composé suivant qui ne fait pas partie de l'invention a été synthétisé :

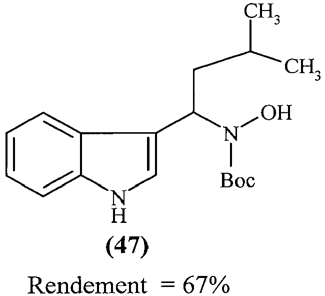

Le composé (47) est un composé de formule (I-11-a) dans laquelle R₅ et R₆ = H, R_{α} = Me, n et j = 1.

### Amines indoliques protégées,

Les *N*-benzylhydroxylamines indoliques de structure (I-1-a) ont ensuite été transformées en amines indoliques protégées de structure (I-3-a) ou (I-4-a) correspondantes par réaction avec le trichlorure de titane aqueux (TiCl₃) en milieu méthanolique acide, selon les deux schémas réactionnels suivants : 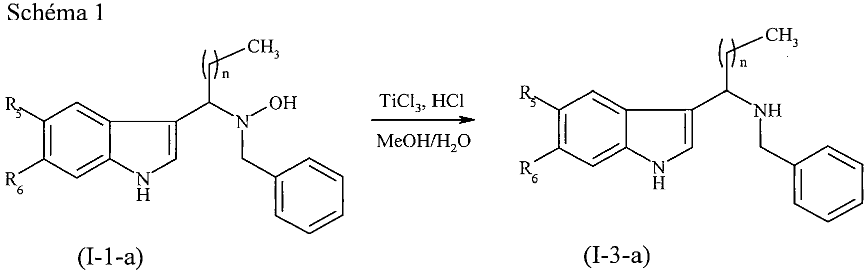 avec R₁ = un groupement alkyle : -(CH₂)ₙ-CH₃ 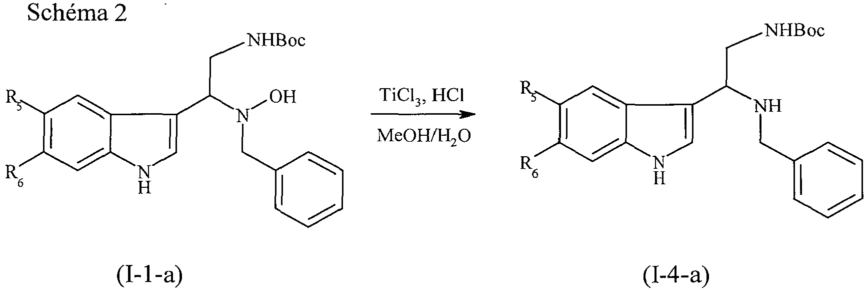 avec R₁ = un groupement CH₂NHBoc

Parmi cette famille de composés, les amines indoliques suivantes ont été synthétisées :

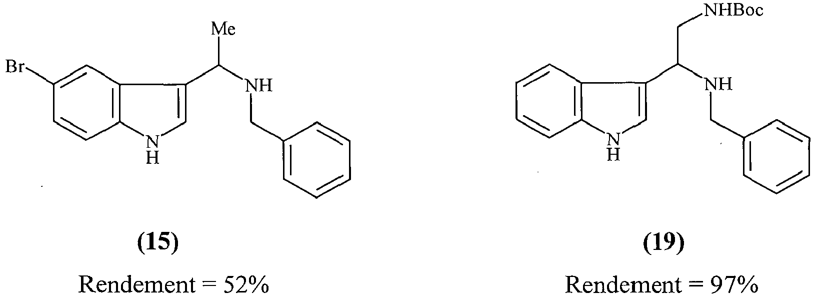

Le composé **(15)** est un composé de formule (I-3-a) dans laquelle n = 0 ; R₅ = Br et R₆ = H.

Le composé **(19)** est un composé de formule (I-4-a) dans laquelle R₅ = R₆ = H.

On applique le même procédé pour synthétiser les composés de formules **(16), (17)** et **(18).**

Le composé **(16)** est un composé de formule (I-3-a) dans laquelle n = 1 ; R₅ = R₆ = H (rendement = 88%).

Le composé **(17)** est un composé de formule (I-4-a) dans laquelle R₅ = OMe et R₆ = H (rendement = 87%).

Le composé **(18)** est un composé de formule (I-4-a) dans laquelle R₅ = Br et R₆ = H (rendement = 95%).

### 1,2-Diamines indoliques monoprotégées

Les diamines indoliques de structure (I-4-a) sont ensuite débenzylées par hydrogénation catalytique en présence du réactif de Pearlman (Pd(OH)₂) dans le mélange méthanol / acide acétique dans un rapport allant de 98/2 à 96/4 à température ambiante pendant 14-16 heures pour conduire aux diamines monoprotégées de structure (I-7-a).

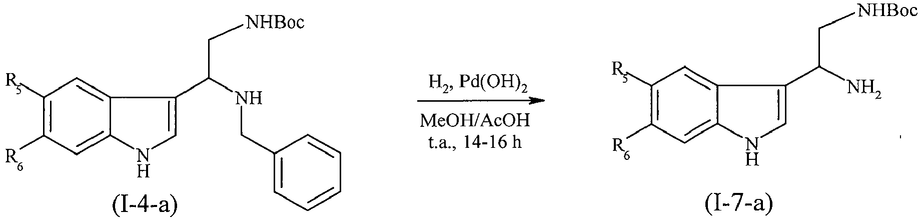

Parmi ces familles de composés, les diamines indoliques suivantes ont été synthétisées:

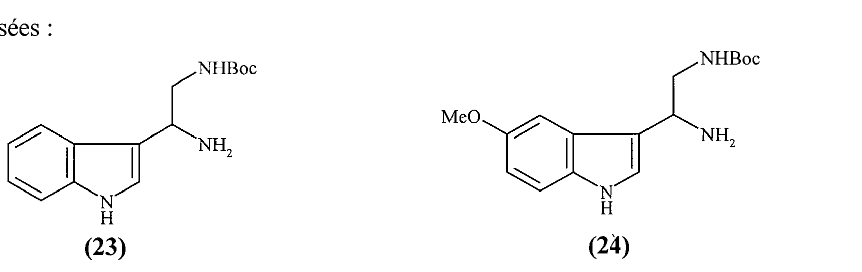

Les diamines monoprotégées de structure (I-7-a) peuvent être obtenues directement à partir des *N*-benzylhydroxylamines indoliques *β*-aminées de structure (I-1-a) (avec R' et R" = H et R₁ = CH₂NHBoc) dans les conditions expérimentales décrites ci-dessus en prolongeant le temps de réaction à 40-60 heures.

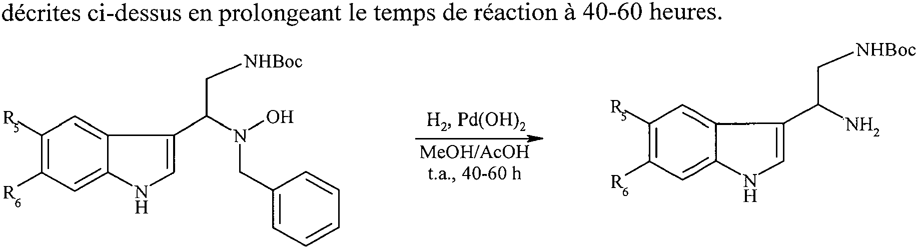

On a ainsi préparé les composés **(23)** et **(24)** tels que définis ci-dessus avec les rendements respectifs de 90% et 88%.

Les 1,2 diamines indoliques monoprotégées bromées de formule (I-7-a), dans laquelle R₅ ou R₆ représente un atome de brome, ont été préparées selon la stratégie de synthèse décrite ci-dessous :

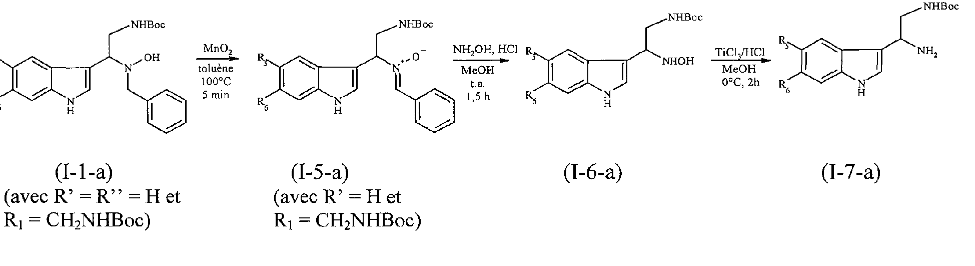

La réaction des *N*-hydroxylamines indoliques de structure (I-1-a) avec le dioxyde de manganèse (MnO₂) dans le toluène à 100-120°C a conduit aux nitrones indoliques de structure (I-5-a). L'hydroxylamine indolique de structure (I-1-a) avec R₅ = Br et R₆ = H a été également oxydée par l'acide m-chloroperbenzoïque (MCPBA) dans le dichlorométhane pour conduire à la nitrone correspondante de structure (I-5-a) avec R₅ = Br et R₆ = H avec 70% de rendement.

En appliquant la synthèse décrite ci-dessus, on a obtenu les produits suivants :

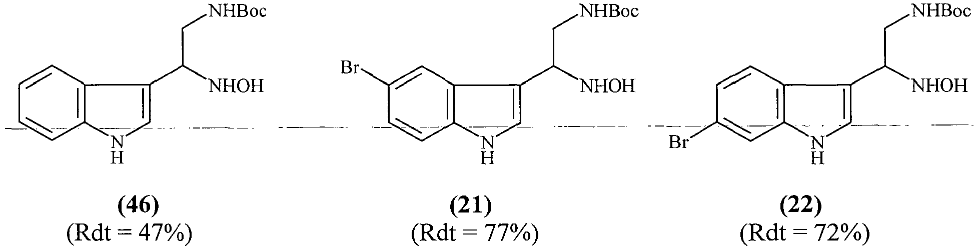

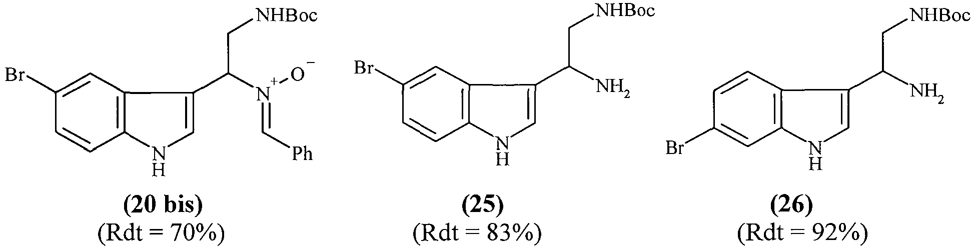

La nitrone indolique de structure **(20)** a été préparée par oxydation de l'hydroxylamine indolique correspondante avec MnO₂ dans des conditions expérimentales très similaires décrites ci-dessus (température utilisée : 120°C).

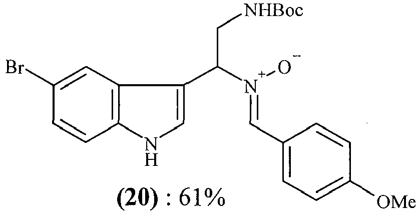

Les nitrones indoliques de structure (I-5-a) ont ensuite été transformées en β-(*N-*Boc)amino *N*-hydroxylamines indoliques de structure (I-6-a) par réaction avec le chlorhydrate d'hydroxylamine (HONH₂.HCl) dans le méthanol, notamment pour obtenir les composés **(46), (21)** et **(22)** susmentionnés.

Les β-(*N*-Boc)amino *N*-hydroxylamines indoliques de structure (I-6-a) ont ensuite été mises en réaction avec une solution aqueuse de TiCl₃ en milieu méthanolique acide pour conduire aux 1,2-diamines indoliques monoprotégées de structure (I-7-a). Cette réaction a permis d'obtenir notamment les composés **(25)** et **(26)** susmentionnés.

### Dichlorhydrates de 1,2 diamines indoliques

Le traitement des 1,2-diamines indoliques monoprotégées de structure (I-7-a) par l'acide chlorhydrique dans le méthanol a conduit aux dichlorhydrates de 1,2-diamines correspondantes de structure (I-8-a) avec X = HCl.

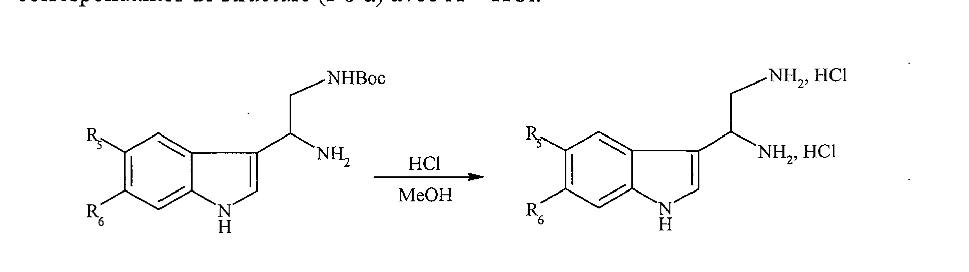

Ce procédé a permis de préparer les composés suivants :

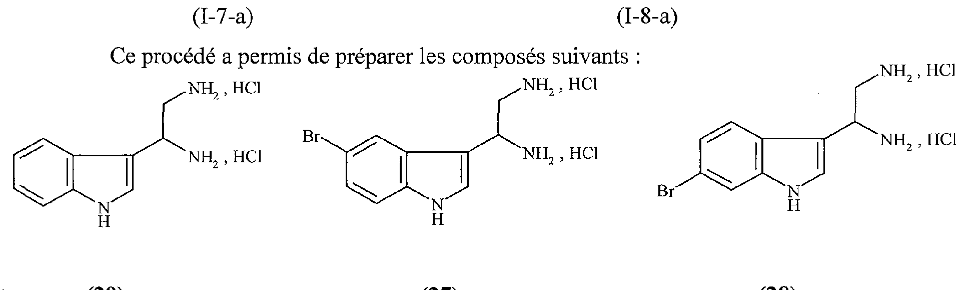

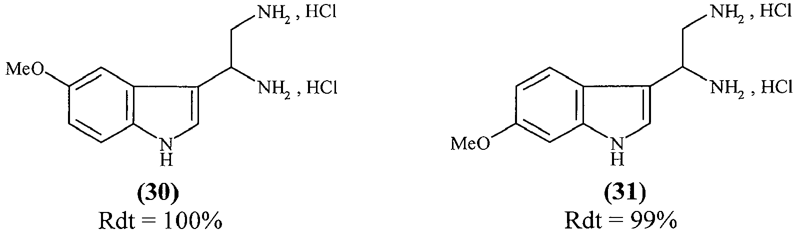

### N-hydroxylamines primaires indoliques β-aminées

Les β-(*N*-Boc)amino *N*-hydroxylamines indoliques de structure (I-6-a) ont été déprotégées par l'acide chlorhydrique dans le méthanol pour conduire aux N-hydroxylamines primaires indoliques β-aminées de structure (I-9-a).

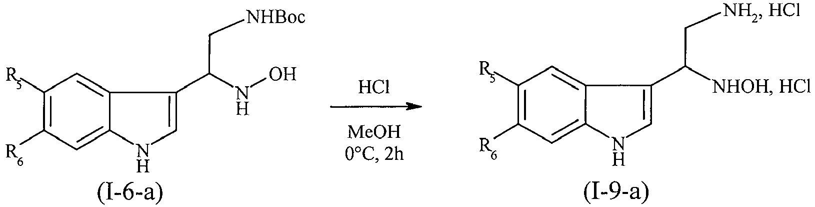

On a ainsi préparé les composés suivants :

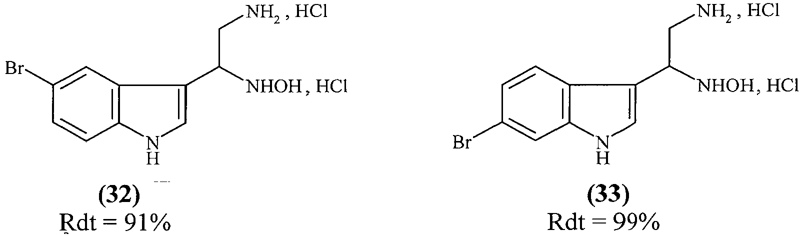

### 1,2-Di-(N-Boc)amines indoliques

Les *N*-hydroxylamines indoliques de structure (I-2-a) ont été réduites par réaction avec le diiodure de samarium en présence d'eau dans le THF à température ambiante pour conduire aux 1,2-di-(*N*-Boc)amines indoliques de structure (I-10-a).

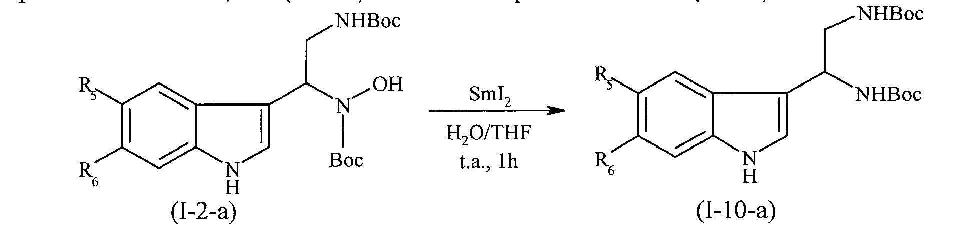

Ce procédé a permis de préparer les composés suivants :

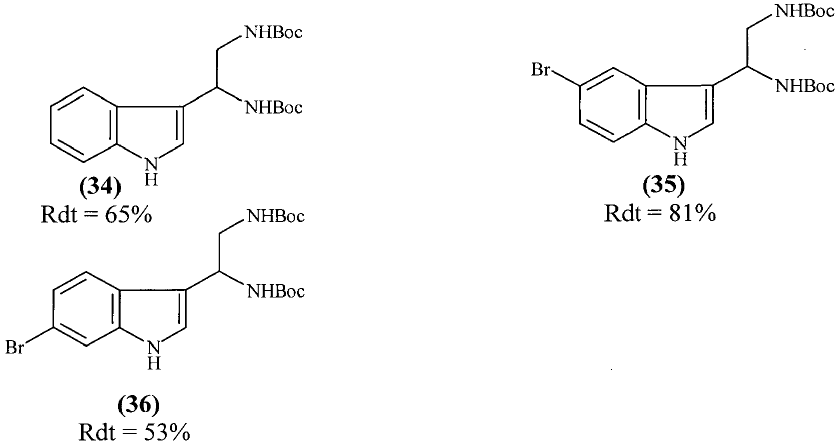

### PARTIE EXPÉRIMENTALE DÉTAILLÉE

### - 1-[N-(benzyl)amino]-2-[N'-(tert-butoxycarbonyl)amino]-1-(5'-méthoxy-3'-indolyl) éthane = composé (17)

Dans un ballon muni d'une agitation magnétique et placé sous argon, on met 2,2 g (5,5 mmol) d'hydroxylamine indolique (7) en solution dans 65 mL de méthanol. On ajoute 2 équivalents de trichlorure de titane en solution aqueuse à 15% (12,1 mL ; 16,05 mmol) et on laisse réagir 15 minutes à température ambiante. Le mélange réactionnel est alors traité par une solution aqueuse d'hydroxyde de sodium à 20% jusqu'à pH basique, puis le méthanol est éliminé à l'évaporateur rotatif. La phase aqueuse est alors extraite trois fois par de l'acétate d'éthyle. Les phases organiques réunies sont lavées par une solution aqueuse saturée en chlorure de sodium, séchées sur sulfate de magnésium anhydre puis les solvants sont éliminés à l'évaporateur rotatif. Le résidu est purifié par chromatographie sur colonne de gel de silice (éluant : acétate d'éthyle). La diamine est obtenue sous forme de solide blanc (1,83 g ; 6,63 mmol).
**Rdt :** 87%.
**IR (film) :** 3420, 3320, 2980, 2930, 2825, 1690, 1485, 1370, 1255, 1165, 1035, 920, 800, 695 cm⁻¹.
**¹H RMN (CDCl₃, 300 MHz) :** δ = 1,42 (s; 9H; (CH₃)₃C) ; 1,84 (s; 1H; NH) ; 3,38-3,60 (m; 2H; CH₂N) ; 3,74 (AB_{q}; *J*_{AB}= 13,2 Hz; δ_{A}- δ_{B}= 25,1 Hz; 2H; CH₂Ph) ; 3,81 (s; 3H; CH₃O) ; 4,10 (t; *J=* 6,0 Hz; 1H; CHN) ; 4,94 (s large; 1H; NHBoc) ; 6,84 (dd; *J=* 2,4 Hz et 8,7 Hz; 1 H; CH indol) ; 7,04 (s; 1 H; H indol) ; 7,14 (s; 1 H; H indol) ; 7,10-7,40 (m; 6H; 5H arom et 1H indol) ; 8;53 (s; 1H; NH indol).
**¹³C RMN (CDCl₃;** 75,5 **MHz) :** δ = 28,5 ((CH₃)₃C); 45,5 (CH₂N); 51,5 (CH₂Ph); 54,8 (CHN); 56,0 (CH₃O); 79,4 ((CH₃)₃C); 101,4 (CH arom); 112,2 (CH arom); 112,6 (CH arom); 115,7 (C arom); 123,2 (CH arom); 126,9 (C arom); 127,0 (CH arom); 128,3 (CH arom); 128,5 (CH arom); 131,9 (C arom); 140,7 (C arom); 154,0 (C arom); 156,4 (C=O).

### - 1-amino-[2-N'-(tert-butoxycarbonyl)amino]-1-(5'-méthoxy-3'-indolyl) éthane = composé (24)

Dans un ballon muni d'une agitation magnétique et placé sous argon, on met 485 mg (1,18 mmol) d'hydroxylamine indolique (7) en solution dans 24 mL de méthanol et 0,5 mL d'acide acétique et 195 mg de catalyseur de Pearlmann Pd(OH)₂. L'argon est remplacé par de l'hydrogène et on laisse réagir 2 jours à température ambiante. Le mélange réactionnel est alors filtré sur célite, puis traité par une solution aqueuse d'hydroxyde de sodium à 20% jusqu'à pH basique. Le méthanol est alors éliminé à l'évaporateur rotatif. La phase aqueuse résultante est extraite trois fois par de l'acétate d'éthyle. Les phases organiques réunies sont lavées par une solution aqueuse saturée en chlorure de sodium, séchées sur sulfate de magnésium anhydre puis les solvants sont éliminés à l'évaporateur rotatif. Le résidu est purifié par chromatographie sur colonne de gel de silice (éluant : acétate d'éthyle). La diamine est obtenue sous forme de solide blanc (317 mg, 1,04 mmol).
**Rdt :** 88%.
**Pf :** 131°C.
**IR (film) :** 3296, 2972, 2927, 2830, 1686, 1634, 1505, 1466, 1369, 1273, 1253, 1163, 1027, 801 cm⁻¹.
**¹H RMN (CDCl₃, 300 MHz) :** δ = 1,43 (s; 9H; C(CH₃)₃) ; 2,74 (s large; 2H; NH₂) ; 3,31-3,54 (m; 2H; CH₂N) ; 4;34 (s large; 1H; CHN) ; 5,10 (s large; 1H; NHBoc) ; 6,83 (dd; *J*= 2,0 et 8,5 Hz; 1 H; H indol) ; 7,02 (s; 1 H; H indol) ; 7,11 (s; 1 H; H indol) ; 7,21 (d; *J*= 9,0 Hz; 1H; H indol) ; 8,75 (s large; 1H; NH indol).
**¹³C RMN (CDCl₃; 75,5 MHz) :** δ = 28,3 (C(CH₃)₃); 47,1 (CH₂); 48,5 (CHN); 55,9 (OCH₃); 79,3 (C(CH₃)₃); 101,0 (CH indol); 112,1 (CH indol); 112,3 (CH indol); 117,2 (C indol); 122,0 (CH indol); 126,2 (C indol); 131,7 (C indol); 153,9 (C indol); 156,3 (C=O). **SMBR (IC) :** m/z= 306 [(M+H)⁺]; 289; 233.
**Anal Calculée for C₁₆H₂₃N₃O₃ :** C; 62,95; H; 7,54; N; 13,77
**Trouvée :** C; 62,74; H; 7,59; N; 13,74.

### - Dichlorhydrate de 1,2-diamino-1-(5'-méthoxy-3'-indolyl) éthane = composé (30)

Une solution méthanolique d'acide chlorhydrique est préparée à 0°C par réaction de 0,33 mL (363 mg ; 4,62 mmol) de chlorure d'acétyle fraîchement distillé avec 1,5 mL de méthanol sec. Cette solution est agitée pendant 15 minutes à 0° C. Une solution de diamine (1,31 g; 0,33 mmol) de structure **(24)** dans 0,5 mL de méthanol est additionnée lentement à la solution acide. Le milieu réactionnel résultant est agité pendant une heure supplémentaire, puis le méthanol est éliminé lentement à l'évaporateur rotatif sans élever la température du bain thermostaté. Le chlorhydrate de diamine est alors obtenu sous forme de solide pourpre (940 mg ; 0,33 mmol).
**Rdt :** 100%.
**IR (KBr) :** 3386, 2947, 1628, 1550, 1512, 1454, 1273, 1163, 1111, 1026, 807 cm⁻¹.
**¹H RMN** (**CD₃OD**, **300 MHz) :** δ = 3,77 (d; *J*= 7,4 Hz; 2H; CH₂N); 3,88 (s; 3H; CH₃O); 5,14 (t; *J*= 7,6 Hz; 1H; CHN); 6,86 (dd; *J*= 2,2 et 8,8 Hz; 1H; H indol); 7,32 (d; *J=* 2,4 Hz; 1H; H indol); 7,38 (dd; *J=* 0,5 et 8,8 Hz; 1H; H indol); 7,63 (d; *J*= 3,0 Hz; 1H; H indol); 8,22 (s large; 2H; NH₂); 8,70 (s large; 2H; NH₂).
**¹³C RMN (MeOD; 75,5 MHz) :** δ = 42,5 (CH₂); 47,3 (CHN); 56,3 (CH₃O); 100,9 (CH indol); 106,8 (C indol); 113,8 (CH indol); 114,1 (CH indol); 127,0 (C indol); 127,0 (CH indol); 127,1 (CH indol); 133,5 (C indol); 156,0 (C indol).
**SMBR (IC) :** m/z= 213; 174; 162; 148 (5-méthoxyindole).

### - N-oxyde de N-(benzylidène)-2-[-N'-(tert-butoxycarbonyl)amino]-(5'-bromo-3'-indolyl) éthanamine = composé (20 bis)

L'hydroxylamine indolique **(4)** (2,00 g ; 5,45 mmol) est mise en solution dans 30 mL de toluène anhydre sous atmosphère inerte. Cette solution est portée à 100 °C, puis le dioxyde de manganèse (1,90 g ; 5,45 mmol) est additionné en une fois. Le milieu réactionnel résultant est agité durant 5 minutes, puis est refroidi. Il est alors filtré sur célite afin d'éliminer les sels de manganèse. Le toluène est éliminé à l'évaporateur rotatif, puis le résidu est purifié par chromatographie sur colonne de gel de silice (éluant : Et₂O). Le produit est alors obtenu pur sous forme de solide blanc (1,40 g ; 3,05 mmol).
**Rdt :** 70%.
**Pf :** 128 °C.
**IR (KBr) :** 3419, 3299, 3075, 2977, 2929, 1696, 1513, 1453, 1363, 1254, 1164, 887, 801 cm⁻¹.
**¹H RMN (CDCl₃, 300 MHz) :** δ= 1,42 (s; 9H; C(CH₃)₃); 3,72-3,83 (m; 1H; 1H of CH₂N); 3,93-4,10 (m; 1H; 1H of CH₂N); 5,39 (s large; 1H; NHBoc); 5,54 (s large; 1H; CHN); 6,99 (d; *J=* 8,6 Hz; 1H; H arom); 7,12 (dd; *J=* 1,7 et 8,6 Hz; 1H; H arom); 7,16 (d; *J*= 2,4 Hz; 1H; H arom); 7,30-7,45 (m; 3H; H arom); 7,61 (s; 1H; CH=N); 7,77 (d; *J*= 1,6 Hz; 1H; H arom); 8,15-8,30 (m; 2H; H arom); 9,57 (s; 1H; NH).
**¹³C RMN (CDCl₃; 75,5 MHz) :** δ= 28,5 ((CH₃)₃C); 43,0 (CH₂N); 71,6 (CHN); 80,0 ((CH₃)₃C); 109,3 (C arom); 113,3 (CH arom); 113,5 (C arom); 121,1 (CH arom); 125,3 (CH arom); 125,7 (CH arom); 127,8 (C arom); 128,7 (CH arom); 129,1 (CH arom); 130,2 (C arom); 131,0 (CH arom); 134,9 (C arom); 135,5 (CH=N); 156,5 (C=O). **SMBR (IC, NH₃+isobutane) :** m/z= 458 et 460 [(M+H)⁺], 298 et 300 , 281 et 283.

### - 1-N-(hydroxy)amino-2-[N'-(tert-butoxycarbonyl)amino]-1-(5'-bromo-3'-indolyl) éthane = composé (21)

La nitrone indolique **(20 bis)** (1,28 g ; 2,79 mmol) est mise en solution dans 10 mL de méthanol sec sous atmosphère inerte à température ambiante. On additionne à cette solution du chlorhydrate d'hydroxylamine (0,99 g ; 14,00 mmol) et la solution résultante est agitée à température ambiante durant une heure puis diluée dans du chloroforme. Le solide résultant est éliminé par filtration sur célite. Le filtrat est évaporé sous vide. Le résidu est alors dissous dans l'éther diéthylique, puis traité par une solution aqueuse saturée en hydrogénocarbonate de sodium. La phase aqueuse est alors extraite deux fois par de l'éther. Les phases organiques réunies sont lavées par une solution aqueuse saturée en chlorure de sodium, séchées sur sulfate de magnésium anhydre et évaporées. Le solide résultant est purifié par chromatographie sur colonne de gel de silice (éluant : Et₂O) ce qui a conduit au produit pur sous forme de solide blanc (790 mg, 2,14 mmol).
**Rdt :** 77%.
**Pf** : 87 °C.
**IR (KBr) :** 3419, 3307, 2977, 2936, 1692, 1516, 1456, 1366, 1254, 1172, 805 cm⁻¹.
**¹H RMN (CDCl₃, 300 MHz) :** δ = 1,43 (s; 9H; (CH₃)₃C); 3,35-3,65 (m; 2H; CH₂); 4,25 (t; *J=* 5,4 Hz; 1H; CHN); 5,11 (s large; 1H; NHBoc); 6,98 (d; *J=* 1,7 Hz; 1H; H indol); 7,12 (d; *J=* 8,7 Hz; 1H; H indol); 7,19 (dd; *J=* 1,7 et 8,6 Hz; 1H; H indol); 7,72 (s; 1H; H indol); 9,05 (s; 1H; NH indol).
**¹³C RMN (CDCl₃; 75,5 MHz) :** δ= 28,3 ((CH₃)₃C); 42,5 (CH₂N); 58,6 (CHN); 79,9 ((CH₃)₃C); 112,4 (C indol); 112,8 (C indol); 112,9 (CH indol); 121,7 (CH indol); 124,0 (CH indol); 124,9 (CH indol); 128,0 (C indol); 134,7 (C indol); 157,1 (C=O).
**SMBR (IC; NH₃+isobutane) :** m/z= 370 et 372 [(M+H)⁺]; 298 et 300; 281 et 283. **SMHR (ESI) Calculée pour C₁₅H₂₀N₃O₃⁷⁹BrNa** : 392,0586, **Trouvée :** 392,0591 [(M+Na)⁺] .

### - 1-amino-2-[N'-(tert-butoxycarbonyl)amino]-1-(5'-bromo-3'-indolyl) éthane = composé (25)

A une solution de 556 mg (1,50 mmol) d'hydroxylamine primaire de formule **(21)** dans le méthanol à température ambiante et sous atmosphère inerte, on additionne 3,53 mL de trichlorure de titane 15% aqueux (16,05 mmol) et on laisse réagir 15 minutes à température ambiante. Le mélange réactionnel est alors traité par une solution aqueuse d'hydroxyde de sodium à 20% jusqu'à pH basique, puis le méthanol est éliminé à l'évaporateur rotatif. La phase aqueuse est alors extraite trois fois par l'acétate d'éthyle. Les phases organiques réunies sont lavées par une solution aqueuse saturée en chlorure de sodium, séchées sur sulfate de magnésium anhydre puis les solvants sont éliminés à l'évaporateur rotatif. Le résidu est purifié par chromatographie sur colonne de gel de silice (éluant : acétate d'éthyle). La diamine est obtenue sous forme de solide blanc (438 mg ; 1,24 mmol).
**Rdt :** 83%.
**Pf :** 151 °C.
**IR (film) :** 3423, 3296, 2977, 2925, 1692, 1508, 1456, 1363, 1280, 1250, 1164, 287 cm⁻¹. **¹H RMN (MeOD, 300 MHz) :** δ= 1,41 (s, 9H, C(CH₃)₃); 3,20-3,50 (m, 2H, CH₂N); 4,28 (dd, *J*= 5.9 et 7.3 Hz, 1H, CHN); 7,18 (dd, *J*= 1.8 et 8.6 Hz, 1H, H indol); 7,26 (d, *J*= 8.5 Hz, 1H, H indol); 7,26 (s, 1H, H indol); 7,81 (d, *J=* 1.5 Hz, 1H, H indol).
**¹³C RMN (MeOD, 75.5 MHz) :** δ= 28,7 (C(CH₃)₃); 48,9 (CH₂); 49,4 (CHN); 80,1 (C(CH₃)₃); 113,1 (C indol); 114,0 (CH indol); 117,6 (C indol); 122,3 (CH indol); 124,2 (CH indol); 125,3 (CH indol); 129,2 (C indol); 136,7 (C indol); 158,5 (C=O).
**SMBR (ESI) :** m/z= 354 et 356 [(M+H)⁺].
**SMHR (ESI) Calculée pour C₁₅H₂₁N₃O₂⁷⁹Br :** 354,0817, **Trouvée :** 354,0837 [(M+H)⁺].

### - Dichlorhydrate de 1,2-diamino-1-(5'-bromo-3'-indolyl) éthane = composé (27)

Une solution d'acide chlorhydrique est préparée à 0°C par réaction de 931 mg (11,8 mmol) de chlorure d'acétyle fraîchement distillé avec 5,0 mL de méthanol sec. Cette solution est agitée pendant 15 minutes à 0°C. Une solution de diamine **(25)** (300 mg ; 0,85 mmol) dans 0,3 mL de méthanol est additionnée lentement à la solution acide. Le milieu réactionnel résultant est agité pendant deux heures supplémentaires, puis le méthanol est éliminé lentement à l'évaporateur rotatif sans élever la température du bain thermostaté. Le chlorhydrate de diamine est alors obtenu sous forme de solide brun (275 mg ; 11,8 mmol).
**Rdt :** 100%.
**Pf :** 235 °C (déc).
**IR (film) :** 3223, 2919, 1610, 1505, 1464, 1329, 1119, 973, 885 cm⁻¹.
**¹H RMN (MeOD, 300 MHz) :** δ= 3,72 (d, *J=* 7,6 Hz, 2H, CH₂); 5,05 (t, *J=* 7,0 et 7,9 Hz, 1H, CHN); 7,33 (dd, *J=* 1,7 et 8,7 Hz, 1H, H indol); 7,43 (d , *J=* 8,7 Hz, 1H, H indol); 7,75 (s, 1H, H indol); 8,00 (d, *J*= 1,7 Hz, 1H, H indol).
**¹³C RMN (MeOD, 75,5 MHz) :** δ= 42,4 (CH₂); 46,7 (CHN); 106,9 (C indol); 114,5 (C indol); 114,8 (CH indol); 121,8 (CH indol); 126,6 (CH indol); 128,2 (CH indol); 128,5 (C indol); 136,9 (C indol).

### - Dichlorhydrate de 1-(N-hydroxy)-1,2-diamino-1-(5'-bromo-3'-indolyl) éthane = composé (32)

Une solution d'acide chlorhydrique est préparée à 0°C par réaction de 490 mg (6,24 mmol) de chlorure d'acétyle fraîchement distillé avec 2,6 mL de méthanol sec. Cette solution est agitée pendant 15 minutes à 0°C. Une solution de β-(*N*-Boc)amino *N*-hydroxylamine indolique **(21)** (166 mg, 0,45 mmol) dans 0,2 mL de méthanol est additionnée lentement à la solution acide. Le milieu réactionnel résultant est agité pendant deux heures supplémentaires, puis le méthanol est éliminé lentement à l'évaporateur rotatif sans élever la température du bain thermostaté. Le chlorhydrate de diamine est alors obtenu sous forme de solide brun (140 mg, 0,41 mmol).
**Rdt :** 91%.
**IR (KBr) :** 3405, 2924, 1615, 1507, 1457, 1322, 888 cm⁻¹.
**¹H RMN** (**CD₃OD**, **300 MHz) :** δ= 3,79 (dd; *J=* 9,0 et 13,2 Hz; 1H; H de CH₂); 3,95 (dd; *J=* 5,5 et 13,2 Hz; 1H; H de CH₂); 5,23 (dd; *J=* 5,5 et 9,0 Hz; 1H; CHN); 7,35 (dd; *J=* 1,8 et 8,7 Hz; 1H; H indol); 7,44 (d; *J*= 8,7 Hz; 1H; H indol); 7,80 (s; 1H; H indol); 7,98 (d; *J=* 1,8 Hz; 1H; H indol).
**¹³C RMN** (**CD₃OD; 75,5 MHz) :** δ= 40,1 (CH₂N); 56,6 (CHN); 103,1 (C indol); 114,6 (C indol); 114,8 (CH indol); 122,0 (CH indol); 126,6 (CH indol); 129,1 (C indol); 129,5 (CH indol); 136,6 (C indol).

### - 1-[N-(tert-butoxycarbonyl)-N-(hydroxy)amino]-2-[N-(tert-butoxycarbonyl)]-1-(5'-bromo-3'-indolyl) éthane = composé (13)

Une solution de bromure de 5-bromo-3'-indolylmagnésium est préparée à -78°C par réaction de 212 mg de 5-bromoindole en solution dans 3 mL de THF avec 0,36 mL de bromure de méthylmagnésium (solution 3M dans THF, 1,08 mmol). Cette solution est agitée 15 minutes à -78 °C puis on y additionne une solution de 150 mg (0,36 mmol) de sulfone de structure **(45)** dans 2 mL de THF. Le mélange réactionnel résultant est alors agité durant une nuit au cours de laquelle la température évolue lentement jusqu'à atteindre -5°C. La réaction est alors stoppée par ajout d'une solution aqueuse saturée en chlorure d'ammonium. Le mélange hétérogène résultant est alors extrait par l'acétate d'éthyle trois fois. Les phases organiques réunies sont lavées par une solution aqueuse saturée en chlorure de sodium, séchées sur sulfate de magnésium anhydre puis les solvants sont éliminés à l'évaporateur rotatif. Le résidu est purifié par chromatographie sur colonne de gel de silice (éluant : dichlorométhane puis acétate d'éthyle). Le composé est alors obtenu sous forme de solide blanc (137 mg ; 0,29 mmol).
**Rdt :** 81%.
**IR (film) :** 3324, 2972, 2914, 2846, 1684, 1519, 1461, 1392, 1367, 1287, 1255, 1167, cm⁻¹. **¹H RMN (CDCl₃, 300 MHz) :** δ= 1,46 (s; 18H; C(CH₃)₃); 3,23 (dt; *J=* 4,7 et 14,7 Hz; 1H; CH de CH₂); 3,77-3,95 (m; 1H; CH de CH₂); 5,18-5,28 (m; 1H; NHBoc); 5,38 (dd; *J=* 3,8 et 11,3 Hz; 1H; CHN); 7,10 (dt; *J=* 1,3 et 7,8 Hz; 1H; H indol); 7,08-7,19 (m; 2H; H indol); 7,78 (d; *J*= 1 Hz; 1H; CH indol); 8,05 (s; 1H; OH); 8,91 (s; 1H; NH indol).
**¹³C RMN (CDCl₃; 75,5 MHz) :** δ= 28,3 (2 C(CH₃)₃); 41,1 (CH₂); 55,1 (CHN); 80,6 (C(CH₃)₃); 81,6 (C(CH₃)₃); 112,3 (C indol); 112,8 (C indol); 112,8 (CH indol); 121,5 (CH indol); 124,4 (CH indol); 124,7 (CH indol); 128,0 (C indol); 134,4 (C indol); 156,6 (C=O); 158,0 (C=O).
**SMBR (ESI) :** m/z= 476 et 478 [(M+Li)⁺]; 492 et 494 [(M+Na)⁺]; 945; 947 et 949 [(dimère+Li)⁺]; 961; 963 et 965 [(dimère+Na)⁺].
**Anal Calculée pour C₂₀H₂₈BrN₃O₅ :** C; 51,08; H; 6,00; N; 8,94.
**Trouvée :** C; 50,87; H; 6,13; N; 8,73.

### - 1-[N-(tert-butoxycarbonyl)amino]-2-[N-(tert-butoxycarbonyl)]amino-1-(5'-bromo-3'-indolyl) éthane = composé (35)

L'hydroxylamine **(13)** (423 mg ; 0,90 mmol) est dissoute dans 5 mL de THF sous atmosphère inerte. On additionne alors 16 équivalents d'eau correctement dégazée (0,26 mL ; 14,4 mmol) puis 4 équivalents de diiodure de samarium en solution 1M dans le THF (36 mL, 3,6 mmol). La réaction est agitée à température ambiante durant une heure, puis stoppée par réaction avec le dioxygène de l'air ambiant, puis par ajout d'une solution aqueuse saturée en thiosulfate de sodium. Le milieu réactionnel résultant est extrait trois fois par l'acétate d'éthyle. Les phases organiques réunies sont lavées par une solution aqueuse saturée en chlorure de sodium, séchées sur sulfate de magnésium anhydre puis les solvants sont éliminés à l'évaporateur rotatif. Le résidu est purifié par chromatographie sur colonne de gel de silice (éluant : Et₂O/pentane, 1:1). La diamine est obtenue sous forme de solide blanc (332 mg, 0,73 mmol).
**Rdt:** 81%.
**IR (film) :** 3313, 2978, 2932, 2874, 1694, 1513, 1455, 1394, 1369, 1251, 1166 cm⁻¹.
**¹H RMN (CDCl₃, 300 MHz) :** δ= 1,45 (s; 9H; C(CH₃)₃); 1,46 (s; 9H; C(CH₃)₃); 3,45-3,70 (m; 2H; CH₂); 4,90-5,10 (m; 2H; 2 NHBoc); 5,10-5,20 (s large; 1H; CHN); 6,98 (s; 1H; H indol); 7,21 (dd; *J*= 8,6 et 18,7 Hz; 1H; H indol); 7,24 (d; *J*= 9,3 Hz; 1H; H indol); 7,73 (d; *J=* 1,7 Hz; 1H; CH indol); 8,76 (s; 1H; NHBoc).
**¹³C RMN (CDCl₃; 75,5 MHz) :** δ= 28,4 (C(CH₃)₃); 29,0 (C(CH₃)₃); 45,0 (CH₂); 48,7 (CHN); 79,8 (C(CH₃)₃); 112,9 (CH indol); 113,0 (C indol); 121,6 (CH indol); 122,1 (C indol); 122,9 (CH indol); 125,2 (CH indol); 127,5 (C indol); 135,2 (C indol); 156,0 (C=O); 156,6 (C=O),
**SMBR (ESI) :** m/z= 476 et 478 [(M+Na)⁺].
**Anal Calculée for C₁₅H₁₆N₂OS :** C; 52,87; H; 6,22; N; 9,25.
**Trouvée :** C; 52,94; H; 6,43; N; 9,01.

### - 1-N-(hydroxy)amino-2-[N'-(tert-butoxycarbonyl)amino]-1-(indol-3'-yl)éthane = composé (46)

5 équivalents de chlorhydrate d'hydroxylamine (260 mg, 3,69 mmol) sont ajoutés sous argon et sous agitation à une solution de nitrone indolique de formule (I-5-a) avec R₁ = CH₂NHBoc, R₅ = R₆ = R' = H (280 mg, 0,73 mmol) dans le méthanol (3 mL). Le milieu réactionnel est alors agité une heure à température ambiante puis dilué avec du chloroforme. Le solide blanc obtenu est filtré sur célite. Les solvants du filtrat sont évaporés sous vide. Le résidu est dissous dans l'éther diéthylique et cette solution est lavée à l'eau et par une solution aqueuse saturée en NaHCO₃. La phase aqueuse est extraite deux fois à l'éther diéthylique. Les phases organiques sont réunies, lavées par une solution aqueuse saturée en NaCl, séchées sur MgSO₄, filtrées et les solvants sont évaporés. Le produit brut est purifié par chromatographie sur colonne de silice (Eluant: Et₂O) pour donner un solide blanc, le 1-*N*-(hydroxy)amino-2-[*N*'-(*tert-*butoxycarbonyl)amino]-1-(indol-3'-yl)éthane (100 mg, 0,34 mmol).
**Rdt:** 47%.
**¹H RMN (CDCl₃, 300 MHz) :** δ= 1,43 (s, 9H, C(CH₃)₃); 3,4-3,6 (m, 1H, H du CH₂); 3,6-3,8 (m, 1H, H du CH₂); 4,37 (t, *J*= 5,2 Hz, 1H, CHN); 5,04 (s large, 1H, NHBoc); 7,00 (s, 1H, H indol); 7,09 (t, *J=* 7,0 Hz, 1H, H indol); 7,15 (t, *J=* 7,0 Hz, 1H, H indol); 7,29 (d, *J*= 7,9 Hz, 1H, H indol); 7,58 (d, *J*= 7,8 Hz, 1H, H indol); 8,66 (s, 1H, NH indol).
**¹³C RMN (CDCl₃, 75,5 MHz):** δ= 28,4 (C(CH₃)₃); 42,5 (CH₂), 58,5 (CHN); 79,7 (C(CH₃)₃); 111,4 (CH indol); 112,5 (C indol); 118,9 (CH indol); 119,6 (CH indol); 122,2 (CH indol); 122,8 (CH indol); 126,2 (C indol); 136,0 (C indol); 157,0 (C=O). **LRMS (DCI, NH₃+isobutane):** m/z= 292 [(M+H)⁺], 279, 203.

### - 1-[N-(tert-butoxycarbonyl)-N-(hydroxy)amino]-1-(indol-3'-yl)-3-methylpropane = composé (47) qui ne fait pas partie de l'invention

Une solution de bromure de méthylmagnésium 3M dans l'éther (194 µL, 0,58 mmol) est ajoutée à -78 °C sous atmosphère inerte à une solution d'indole (34 mg, 0,29 mmol) et de *tert*-butyl 3-méthyl-1-(phénylsulfonyl)butyl-*N*-hydroxycarbamate (100 mg, 0,29 mmol) dans 1 mL de THF anhydre. le milieu réactionnel est agité 3 heures à cette température, puis est réchauffé à -10°C et est agité pendant la nuit. La réaction est stoppée par ajout d'une solution aqueuse saturée en chlorure d'ammonium. Le mélange est extrait trois fois par de l'acétate d'éthyle. Les phases organiques sont lavées par une solution aqueuse saturée en NaCl et séchées sur sulfate de magnésium anhydre. Après évaporation des solvants, le résidu est purifié par chromatographie sur colonne de silice (éluant : Et₂O:pentane 1:1) pour donner le 1-[*N*-(*tert*-butoxycarbonyl)-*N-*(hydroxy)amino]- 1-(indol-3'-yl)-3-methylpropane sous la forme d'une poudre blanche (62 mg, 0,195 mmol).
**Rdt:** 67%.
**IR(KBr):** 3410, 3194, 3056, 2947, 2866, 1695, 1617, 1251, 1167, 1138, 1093 cm⁻¹. **¹H** RMN **(CDCl₃, 300 MHz):** δ= 0,99 (d, *J*= 6,3 Hz, 3H); 1,01 (d, *J*= 6,3 Hz, 3H); 1,48 (s, 9H, C(CH₃)₃); 1,65-1,80 (m, 2H); 2,10-2,25 (m, 1H); 5,55 (dd, *J*= 5,1 et 9,9 Hz, 1H); 6,20 (s, 1H); 7,14 (dt, *J*= 1,5 et 8,1 Hz, 1H); 7,12-7,22 (m, 1H); 7,22 (d, *J*= 2,7 Hz, 1H); 7,30-7,36 (m, 1H); 7,72 (d, *J=* 7,5 Hz, 1H); 8,20 (s, 1H).
**¹³C RMN (CDCl₃,** 75,5 **MHz):** δ= 22,0 (CH₃); 23,1 (CH₃); 25,0 (CH); 28,3 (C(CH₃)₃); 40,7 (CH₂); 53,0 (CHN); 81,7 (C(CH₃)₃); 111,1 (CH indol); 114,8 (Cq); 119,3 (CH indol); 119,6 (CH indol); 122,1 (CH indol); 123,1 (CH indol); 126,6 (Cq); 135,9 (Cq); 156,4 (C=O).
**LRMS (ESI):** m/z= 341 [(M+Na)⁺].
**Calculée pour C₁₈H₂₆N₂O₃** C: 67,90. H : 8,23. N : 8,80.
**Trouvée :** C: 68,12. H : 8,44. N : 8,77.

### -1-[N-benzyl-N-(hydroxy)amino]-2-[N'-(tert-butoxycarbonyl)amino]-1-(6'-méthoxyindol-3'-yl)éthane = composé (50)

Une solution d'acide chlorhydrique est préparée à 0°C par réaction du chlorure d'acétyle fraichement distillé (533 mg, 6,80 mmol) avec 5 mL de méthanol anhydre. Cette solution est agitée à cette température pendant 15 min puis une solution d'oxyde de *N-*[2-(benzyloxy)éthylidène]benzylamine (898 mg, 3,40 mmol) et de 6-méthoxyindole (500 mg, 3,40 mmol) dans 10 mL de méthanol y est ajouté. La réaction est agitée à 0 °C pendant une heure jusqu'à conversion totale. Une solution aqueuse saturée en NaHCO₃ est alors ajoutée. Le milieu est extrait trois fois au CH₂Cl₂ et les phases organiques sont réunies, lavées par une solution aqueuse saturée en NaCl et séchées sur MgSO₄ anhydre. Les solvants sont évaporés sous vide. le produit brut est purifié par trituration avec du pentane. Un solide blanc est obtenu (1,38 g, 3,39 mmol).
**Rdt:** 100%.
**Pf:** 120 °C.
**IR (film):** 3405, 3354, 2979, 2934, 2837, 1686, 1628, 1505, 1454, 1369, 1253, 1163, 1027, 911, 807, 736 cm⁻¹.
**¹H RMN (CDCl₃, 300 MHz):** δ= 1,50 (s, 9H, C(CH₃)₃); 3,50-3,70 (m, 2H, CH₂N); 3,74 (AB_{q}, *J*_{AB}= 14,1 Hz, δ_{A}-δ_{B}= 69,2 Hz, 2H, CH₂Ph); 3,82 (s, 3H, CH₃O), 4,06 (t, *J*= 5,7 Hz, 1H, CHN); 4,80-5,00 (large s, 1H, NHBoc); 6,48 (s, 1H, OH); 6,79 (dd, *J=* 5,2 et 7,8 Hz, 1H, H arom); 6,84 (s, 1H, H arom); 7,07 (s, 1H, H arom); 7,15-7,32 (m, 5H, H arom); 7,53 (d, *J*= 8,7 Hz, 1H, H arom); 8,22 (s, 1H, NH indol).
**¹³C RMN (CDCl₃, 75,5 MHz):** δ**=** 28,5 (C(CH₃)₃); 44,0 (CH₂); 53,6 (CH₃); 60,6 (CH₂Ph); 79,7 (C(CH₃)₃); 94,6 (CH arom); 112,2 (C arom); 120,3 (CH arom); 121,4 (C arom); 122,1 (CH arom); 126,7 (CH arom); 128,0 (CH arom); 128,6 (CH arom); 136,8 (C arom); 139,0 (C arom); 156,6 (C arom); 157,7 (C=O).
**LRMS (ESI):** m/z= 412 [(M+H)⁺], 434 [(M+Na)⁺].
**HRMS (ESI):** calculé pour C₂₀H₃₀N₃O₄: 412,2236. Trouvé: 412,2238 [(M+H)⁺].

### - Composé (48)

Une solution d'ester succinique d'acide 9-fluorenylméthoxycarbonyl-aminoxyacétique (246 mg, 1,2 mmol) dans le DMF (3 mL) est ajoutée à température ambiante à une solution de 2-[benzyloxy]-1-(indol-3'-yl)éthylamine (133 mg, 0,5 mmol) dans le DMF (12 mL) sous argon et sous agitation. Le mélange est agité 30 minutes à température ambiante puis le DMF est évaporé sous vide. Le résidu est purifié par chromatographie sur colonne de silice (éluant, EtOAc/CH₂Cl₂: 1/1) pour donner un solide blanc (40 mg, 0,071 mmol).
**Rdt:** 14%.
**¹H RMN (CDCl₃, 300 MHz):** δ= 3,85-3,94 (m, 2H, CH₂); 3,95-4,04 (m, 1H, CH); 4,21 (d, *J*= 7,5 Hz, 2H, CH₂); 4,30-4,35 (m, 2H, CH₂); 4,53 (s, 2H, CH₂); 5,61-5,72 (m, 1H, CH); 6,95-7,11 (m, 2H, H indol); 7,11 (d, *J*= 2,3 Hz, 1H, H indol); 7,15-7,50 (m, 13H, H arom); 7,62 (d, *J*= 7,8, 1H, H indol); 7,65-7,79 (m, 3H, H arom); 7,96 (d, *J*= 8,3, 1H, NH); 8,09 (s, 1H, NH); 8,16 (s large, 1H, NH indol).

### SYNTHESES DE MOLECULES SELON LA DESCRIPTION

### Groupement R

(cf. *"*Studies in the Protection of Pyrrole and Indole Derivatives" ; D. Dhanak, C. B. Reese J. Chem. Soc., Perkin Trans. I 1986, 2181-2186 et références citées; T. W. Greene, P. M. Wuts, « Protective Groups in Organic Synthesis », Third Edition, John Wiley & Sons, Inc., 1999 : chapitre 7, pages 615-631)

### 1-alkylindoles (R = groupe alkyle)

Les 1-alkylindoles peuvent être préparés par réaction des noyaux indoliques avec l'iodure d'alkyle adéquat en présence d'hydroxyde de potassium dans l'acétone à température ambiante selon la procédure décrite dans Y. Kikugawa, Y. Miyake Synthesis 1981, 461-462.

*Procédure* pour la synthèse du **1-éthylindole :** à une solution refroidie d'indole (496 mg, 4,24 mmol) dans 12 mL d'acétone est additionné l'hydroxyde de potassium en poudre (1,19 g ; 21,2 mmol). Après quelques minutes de réaction, l'iodure d'éthyle (1,32 g ; 8,46 mmol) est additionné sous vive agitation et la réaction est laissée agiter pendant 10 minutes à température ambiante. Du benzène est alors additionné au mélange réactionnel et les produits insolubles sont éliminés par filtration sous vide. La solution benzénique résultante est lavée avec une solution aqueuse saturée en NaCl et séchée sur Na₂SO₄ anhydre. Le benzène est évaporé sous vide et le résidu est purifié par chromatographie sur gel de silice pour conduire au 1-éthylindole (541 mg ; 3,73 mmol) avec 88% de rendement.

Le **1-méthylindole** a été préparé selon la procédure décrite dans K. T. Potts, J. E. Saxton Org. Synth., John Wiley & Sons, Inc., Coll. Vol. V, 1973, 769-771.

Les **1-alkylindoles** peuvent également être préparés selon une procédure décrite dans W. E. Noland, C. Reich J. Org. Chem. 1967, 32, 828-832.

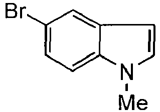

*Procédure :* Le 5-bromo 1-méthylindole (R = Me) est préparé par métallation du 5-bromoindole par l'amidure de sodium dans l'ammoniac/éther en présence de nitrate de ferrique suivi de la réaction du 5-bromo-1-sodioindole obtenu avec l'iodure de méthyle dans l'éther (rendement : 32%).

### 1-benzylindoles (R = groupe benzyle)

Le **1-benzylindole** a été préparé par réaction de l'indole avec le chlorure de benzyle en présence d'hydroxyde de potassium dans l'acétone à température ambiante selon la procédure décrite dans Y. Kikugawa, Y. Miyake Synthesis 1981, 461-462.

Il peut également être synthétisé selon une des procédures rapportées dans T. W. Greene, P. M. Wuts, « Protective Groups in Organic Synthesis », Third Edition, John Wiley & Sons, Inc., 1999 : chapitre 7, pages 620-621 ou dans l'article Y. Murakami, T. Watanabe, A. Kobayashi, Y. Yokoyama Synthesis 1984, 738-740.

### 1-(hydroxyméthyl)indoles

Le **1-(hydroxyméthyl)indole** est préparé par réaction du 1-(pivaloyloxy-méthyl)indole avec du méthylate de sodium dans le méthanol à température ambiante (rendement : 30%) selon la procédure décrite dans la publication suivante : D. Dhanak, C. B. Reese J. Chem. Soc., Perkin Trans. I 1986, 2181-2186. Il a été caractérisé par spectrométrie de masse et par RMN ¹H et ¹³C.

La préparation du **1-(pivaloyloxyméthyl)indole** (POM-indole) est décrite dans cette même publication. Il est préparé par *N*-métallation de l'indole avec l'hydrure de sodium dans le THF à température ambiante suivi de la réaction du 1-sodioindole intermédiairement obtenu avec le pivalate de chlorométhyle dans le THF (rendement : 65%). Ses caractéristiques physiques et spectrales sont décrites dans la publication.

### 1-(2-chloroéthyl)indoles

Les **1-(2-chloroéthyl)indoles** peuvent être synthétisés selon la procédure décrite dans M. A. de la Mora, E. Cuevas, J. M. Muchowski, R. Cruz-Almanza Tetrahedron Lett. 2001, 42, 5351-5353.

*Synthèse du 1-(2-chloroéthyl)indole :* à une solution d'indole (1,17 g ; 0,1 mole) dans 10 mL de DMF mise sous atmosphère d'azote, on additionne NaH (0,253 g ; 0,11 mole de NaH 60% en suspension dans l'huile minérale, préalablement lavé par du pentane), puis le mélange réactionnel est agité pendant 30 minutes à température ambiante. Le 1-bromo 2-chloroéthane est alors ajouté. Après 2 heures de réaction, le mélange réactionnel est traité par addition lente d'eau et extrait par de l'acétate d'éthyle. La phase organique est lavée par de l'eau puis par une solution aqueuse saturée en NaCl, séchée sur Na₂SO₄ anhydre, filtrée et évaporée sous vide. Le résidu obtenu est purifié par chromatographie sur gel de silice. Le 1-(2-chloroéthyl)indole est obtenu pur avec 80% de rendement.

Les **1-(2-chloroéthyl)indoles** peuvent être transformés en **1-(2-azidoéthyl)indoles** correspondants par réaction avec l'azoture de sodium dans le DMSO selon le mode opératoire décrit dans M. A. de la Mora, E. Cuevas, J. M. Muchowski, R. Cruz-Almanza Tetrahedron Lett. 2001, 42, 5351-5353.

### 1-aminoindoles (R = NH₂)

Ils peuvent être préparés par *N*-amination de noyaux indoliques avec la monochloramine (NH₂Cl) selon la procédure décrite dans J. Hynes, Jr., W. W. Doubleday, A. J. Dyckman, J. D. Godfrey, J. A. Grosso, S. Kiau, K. Leftheris, J. Org. Chem. 2004, 69, 1368-1371. La préparation de la monochloramine est décrite.

L'utilisation du réactif d'amination - H₂NOSO₃H (HOSA) - est décrite dans J. T. Klein, L. Davis, G. E. Olsen, G. S. Wong, F. P. Huger, C. P. Smith, W. W. Petko, M. Cornfeldt, J. C. Wilker, R. D. Blitzer, E. Landau, V. Haroutunian, L. L. Martin, R. C. Effland J. Med. Chem. 1996, 39, 570-581.

Une procédure permettant de préparer les **1-aminoindoles** par réaction des indoles avec l'acide *O*-hydroxylamino sulfonique (HOSA) a fait l'objet d'une demande internationale (WO 2005/035496).

### 1-(N-alkylamino)indoles (R = NHRₐ)

Ils peuvent être préparés par réaction d'aldéhydes avec des 1-aminoindoles suivie de la réduction des 1-(alkylidèneamino)indoles intermédiairement obtenus avec NaBH₄ selon la procédure décrite dans M. Somei, M. Natsume Tetrahedron Lett. 1974, 3605-3608.

### 1-(N,N-dialkylamino)indoles (R = NRₐR_{b})

Ils peuvent être préparés selon une procédure décrite dans M. Watanabe, T. Yamamoto, M. Nishiyama Angew. Chem. Int. Ed. 2000, 39, 2501-2504 ou dans M. Watanabe, T. Yamamoto, M. Nishiyama Eur. Pat. Appl. 2000, 23 pages, 1035114 A2 20000913.

### 1-(N-alkyl- ou N,N-dialkyl-aminoalkyl)indoles

### Synthèse des 1-(N,N-dialkylaminométhyl)indoles

Les **1-(*N*,*N*-dialkylaminométhyl)indoles** peuvent être préparés selon la procédure décrite dans B. E. Love, B. T. Nguyen Synlett 1998, 1123-1125 par réaction des noyaux indoliques avec les 1-(*N*,*N*-dialkylaminométhyl)benzotriazoles adéquats en milieu basique.

*Procédure :* à une suspension de *t*-butylate de potassium dans le THF à 0°C mise sous atmosphère d'azote, on additionne une solution de 1 équivalent d'indole dans le THF puis une solution de 1 équivalent de 1-(*N,N*-dialkylaminométhyl)benzotriazole. Le mélange réactionnel est remonté à température ambiante et laissé agiter à cette température pendant 2 heures. La solution résultante est diluée dans de l'éther, lavée 4 fois avec de l'eau et 1 fois avec une solution aqueuse saturée en NaCl. La phase éthérée est séchée sur MgSO₄ anhydre, filtrée et évaporée sous vide pour donner le 1*-*(*N,N* dialkylaminométhyl)indole. Les données spectroscopiques de 5 composés sont décrites dans l'article en particulier du **1-(*N*,*N*-diméthylaminométhyl)indole** (R = -CH₂-NMe₂).

La synthèse de **1-(*N*,*N*-dialkylaminométhyl)benzotriazoles** est décrite dans : A. R. Katritzky, B. Pilarski, L. Urogdi Org. Prep. Proced. Int. 1989, 21, 135- et dans A. R. Katritzky, K. Yannakopoulou, P. Lue, D. Rasala, L. Urogdi J. Chem. Soc., Perkin Trans. 1 1989, 225-233.

Le **1-(*N*,*N*-diméthylaminométhyl)indole** (isogramine) est également préparé selon la procédure décrite dans A. R. Katritzky, P. Lue, Y.-X. Chen J. Org. Chem. 1990, 55, 3688-3691 par réaction de Mannich en faisant réagir l'indole avec le formaldéhyde et la *N*,*N*-diméthylamine dans l'eau à 0°C.

### 1-(N,N-dibenzylaminométhyl)indoles

Les **1-(*N*,*N*-dibenzylaminométhyl)indoles** peuvent être préparés selon la procédure décrite dans B. E. Love, B. T. Nguyen Synlett 1998, 1123-1125.

La synthèse du **1-(*N*,*N*-dibenzylaminométhyl)benzotriazole** est rapportée dans A. R. Katritzky, K. Yannakopoulou, P. Lue, D. Rasala, L. Urogdi J. Chem. Soc., Perkin Trans. 1 1989, 225-233 et décrite dans J. R. L. Smith, J. S. Sadd J. Chem. Soc., Perkin Trans. 1 1975, 1181-1184. Rendement: 64%. Ses caractéristiques physiques sont décrites dans la publication.

### Synthèse des 1-(N,N-dialkylaminoéthyl)indoles

Les **1-(*N*,*N*-dialkylaminoéthyl)indoles** peuvent être préparés par réaction du 1-sodioindole approprié (préparé *in situ* par réaction du noyau indolique avec NaH dans l'hexaméthylphosphoramide (HMPA)) avec le chlorure de 2-(dialkylamino)éthyle selon la procédure décrite dans R. A. Glennon, J. M. Jacyno, R. Young, J. D. McKenney, D. Nelson J. Med. Chem. 1984, 27, 41-45. Ils peuvent être isolés sous la forme sels d'oxalate.

**L'oxalate du 5-méthoxy-1-(*N*,*N*-diméthylaminoéthyl)indole** est préparé selon la procédure suivante : à une suspension de NaH (1,5 g d'une dispersion 50% dans l'huile minérale et lavé au pentane) dans 20 mL d'HMPA, on ajoute le 5-méthoxyindole (4,4 g, 30 mmol) en solution dans 25 mL d'HMPA. Après addition complète, le mélange réactionnel est agité pendant 2 heures à température ambiante. Une solution de chlorure de 2-(*N,N-*diméthylamino)éthyle (6,4 g, 60 mmol) dans le toluène est alors ajoutée puis le mélange réactionnel résultant est laissé agiter pendant la nuit à température ambiante. Il est ensuite traité par une solution aqueuse saturée de NH₄Cl (450 mL) et la phase aqueuse est extraite par de l'éther. Les phases éthérées sont rassemblées, lavées avec de l'eau et séchées sur MgSO₄ anhydre. Le solvant est évaporé sous vide et le 5-méthoxy-1-(*N,N*-diméthylaminoéthyl)indole est obtenu avec 65% de rendement (6,2 g). Sans purification, il est converti en sel d'oxalate : pf 179-180°C après recristallisation par le méthanol. Les caractéristiques RMN ¹H du 5-méthoxy-1-(*N*,*N*-diméthylaminoéthyl)indole sont décrites dans la publication.

Les **1-(*N*,*N*-dialkylaminoalkyl)indoles** peuvent être préparés selon cette procédure en utilisant le chlorure 2-(*N,N-*dialkylamino)alkyle adéquat.

### 1-arylsulfonylindoles (R = SO₂Ar)

### Synthèse du 1-(phénylsulfonyl)indole (R = SO₂Ph)

Le **1-(phénylsulfonyl)indole** est préparé selon les conditions opératoires décrites dans S. Roy, G. W. Gribble Tetrahedron Lett. 2005, 46, 1325-1328, par réaction de l'indole avec le chlorure de phénylsulfonyle (PhSO₂Cl) en milieu basique (NaOH) en présence d'une quantité catalytique de (*n*-Bu)₄NHSO₄ dans le CH₂Cl₂ à 0°C pendant 1 heure puis à température ambiante pendant 4 heures (rendement : 89%).

### Synthèse du 1-(p-toluènesulfonyl)indole (R = SO₂C₆H₄-pMe)

Le **1-(*p*-toluènesulfonyl)indole** a été préparé selon la procédure décrite dans Y. Kikugawa Synthesis 1981, 460-461, par réaction de l'indole avec le chlorure de *p-*toluènesulfonyle en présence d'hydroxyde de potassium dans le diméthoxyéthane à température ambiante pendant 30 minutes. Rendement : 89%.

Le **1-(*p*-toluènesulfonyl)indole** peut également être préparé par métallation de l'indole par l'hydrure de sodium dans le THF suivi de la réaction du 1-sodioindole intermédiairement obtenu avec l'anhydride triflique dans le THF selon la procédure décrite dans E. V. Sadanandan, S. K. Pillai, M. V. Lakshmikantham, A. D. Billimoria, J. S. Culpepper, M. P. Cava J. Org. Chem. 1995, 60, 1800-1805.

Autres références : T. W. Greene, P. M. Wuts, « Protective Groups in Organic Synthesis », Third Edition, John Wiley & Sons, Inc., 1999 : chapitre 7, pages 615-617 et références citées.

### Synthèse des 1-alcoxycarbonylindoles et 1-alkylcarbonylindoles

### Synthèse du 1-(t-butoxycarbonyl)indole (R = Boc)

Le **1-(*t*-butoxycarbonyl)indole** peut être préparé selon la procédure décrite : D. Dhanak, C. B. Reese J. Chem. Soc., Perkin Trans. I 1986, 2181-2186 : l'hydrure de sodium, en suspension dans l'huile minérale, (0,77 g, 25,6 mmol) est lavé 2 fois avec du pentane puis est mis en suspension dans 10 mL de THF sous atmosphère d'azote à température ambiante. A cette suspension est alors ajoutée une solution d'indole (1,01 g, 8,6 mmol) dans 8,2 mL de THF. La réaction terminée (dégagement de H₂), on ajoute ensuite le carbonate de *t*-butyle et de phényle (2,0 g, 10,25 mmol) goutte à goutte. Le mélange réactionnel résultant est agité pour une période supplémentaire de 12 heures à température ambiante. 15 mL d'eau sont alors ajoutés puis le mélange résultant est extrait 3 fois par de l'éther. La phase organique obtenue est séchée sur MgSO₄ anhydre, filtrée puis évaporée sous vide. Le résidu est purifié par distillation (point éb. : 84° C/0,25 mm Hg) pour conduire à 1,465 g (78%). Les caractéristiques spectrales sont décrites dans la publication.

Le **1-(*t*-butoxycarbonyl)indole** peut également être préparé selon les conditions opératoires décrites dans l'article S. Roy, G. W. Gribble Tetrahedron Lett. 2005, 46, 1325-1328, par réaction de l'indole avec le dicarbonate de di-*t*-butyle (Boc₂O) en présence de *N,N-*diméthylaminopyridine (DMAP) dans le THF à température ambiante pendant 14 heures (rendement : 98%).

Autre publication : L. Grehn, U. Ragnarsson Angew. Chem. Int. Ed. Engl. 1984, 23, 296-301 ((Boc)₂O, MeCN, DMAP, rendement : 82%). Le point d'ébullition et la RMN ¹H sont décrits.

**1-(*t*-butoxycarbonyl)-5-méthoxyindole :** P. Zhang, R. Liu, J. M. Cook Tetrahedron Lett. 1995, 36, 9133-9136 ((Boc)₂O, MeCN, DMAP, rendement : 98%).

Autres références : T. W. Greene, P. M. Wuts, « Protective Groups in Organic Synthesis », Third Edition, John Wiley & Sons, Inc., 1999 : chapitre 7, pages 617-618 et références citées.

### Synthèse du 1-hydroxy, des 1-alkyloxyindoles et du 1-benzyloxyindole

### 1-hydroxyindole (R = OH)

Le 1-hydroxyindole peut être préparé en 2 étapes à partir de l'indole en le réduisant avec le triéthylsilane dans l'acide trifluoroacétique puis en traitant l'indoline obtenue par une solution aqueuse d'H₂O₂ (30%) en présence d'une quantité catalytique (20%) de tungstate de sodium dihydrate (Na₂WO₄.H₂O) dans le méthanol à 0°C selon une procédure décrite dans M. Somei, F. Yamada, T. Kurauchi, Y. Nagahama, M. Hasegawa, K. Yamada, S. Teranishi, H. Sato, C. Kaneko Chem. Pharm. Bull. 2001, 49, 87-96. Cette procédure a été utilisée dans la préparation, à partir de dérivés de la tryptamine, de plusieurs composés oxygénés en position 5 du noyau indolique comme par exemple la sérotonine, la *N*-méthylsérotonine et la 5-méthoxy-*N*-méthyltryptamine.

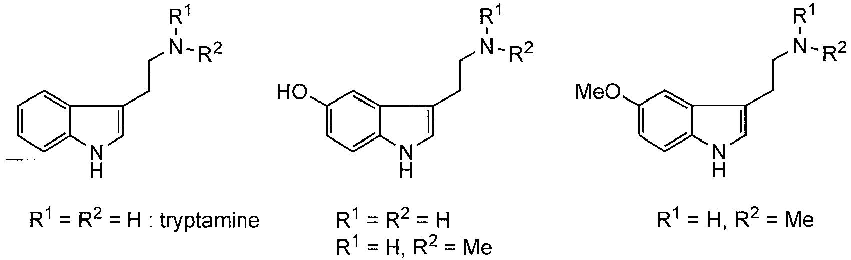

### 1-méthoxyindole (R = OMe)

La synthèse ainsi que les caractéristiques physiques et spectrales du **1-méthoxyindole** sont rapportées dans R. M. Acheson, P. G. Hunt, D. M. Littlewood, B. A. Murrer, H. E. Rosenberg J. Chem. Soc., Perkin Trans. I 1978, 1117-1125.

### Synthèse du 1-acétylindole et du 1-benzoylindole

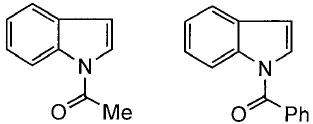

Les **1-acétylindole** et **1-benzoylindole** ont été préparés selon la procédure décrite dans Y. Kikugawa Synthesis 1981, 460-461, par réaction de l'indole avec respectivement le chlorure d'acétyle et le chlorure de benzoyle en présence d'hydroxyde de potassium dans le diméthoxyéthane à température ambiante pendant 20 minutes. Rendements 77 et 83%.

### Synthèse des 1-trialkylsilylindoles

Les **1-trialkylsilylindoles** peuvent être préparés selon les procédures rapportées dans : T. W. Greene, P. M. Wuts, « Protective Groups in Organic Synthesis », Third Edition, John Wiley & Sons, Inc., 1999 : chapitre 7, page 620 et/ou selon les procédures décrites ci-dessous.

### 1-(diméthyl-t-butylsilyl)indole

Le **1-(diméthyl-*t*-butylsilyl)indole** est préparé par traitement de l'indole par l'hydrure de sodium dans le THF à température suivi de la réaction du 1-sodioindole avec le chlorure de diméthyl-*t*-butylsilyle dans le THF selon la procédure décrite dans D. Dhanak, C. B. Reese J. Chem. Soc., Perkin Trans. I 1986, 2181-2186. Le rendement est de 79%. Les caractéristiques physiques et spectrales sont décrites dans cet article.

Le **1-(diméthyl-*t*-butylsilyl)indole** peut également être préparé selon une procédure décrite dans l'article : P. Ashworth, B. Broadbelt, P. Jankowski, P. Kocienski, A. Pimm, R. Bell Synthesis 1995, 199-206 par réaction de l'indole avec NaHMDS dans le THF à -78°C pendant 1 heure suivi de la réaction du 1-sodioindole intermédiairement obtenu avec le chlorure de diméthyl-*t*-butylsilyle dans le THF à -78°C pendant 1,5 heure ou selon le mode opératoire décrit dans l'article Y. Hirai, K. Yokota, T. Momose Heterocycles 1994, 39, 603-612.

### Groupement R₃

### Synthèse des 2-halogénoindoles

### Synthèse des 2-chloro-, 2-bromo- et des 2-iodoindoles

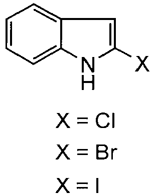

Les **2-halogénoindoles** sont préparés à partir de l'indole selon les modes opératoires décrits dans l'article suivant : J. Bergman, L. Venemalm J. Org. Chem. 1992, 57, 2495-2497. Leurs caractéristiques physiques et spectrales sont également décrites dans l'article.

Le **2-iodoindole** est préparé selon la procédure suivante : A une solution d'indole (1,17 g, 10 mmol) dans 20 mL de THF anhydre à -70°C, on additionne, goutte-à-goutte, le *n*-butyllithium (4,2 mL, 2,5 M en solution dans l'hexane). La suspension résultante est laissée sous agitation à cette température pendant 30 minutes puis on fait buller du CO₂ gazeux pendant 10 minutes. La solution devenue claire est ensuite laissée sous agitation pendant 10 minutes. Le solvant et l'excès de CO₂ sont évaporés sous vide puis le résidu solide résultant est mis en solution dans 20 mL de THF anhydre et refroidi à - 70°C. Le *t*-butyllithium (6,2 mL ; 1,7 M en solution dans le pentane) est alors additionné goutte-à-goutte puis la solution résultante est laissée sous agitation pendant 1 heure. Le 1,2-diiodoéthane (2,82 g ; 10 mmol) est alors additionné. Le mélange réactionnel est laissé à -78°C pendant 1 heure puis traité par 1 mL d'eau. La solution résultante est laissée remonter à température ambiante puis elle est traitée par une solution aqueuse saturée en NH₄Cl. Elle est extraite par de l'éther. La phase organique obtenue est lavée par une solution aqueuse saturée en NaCl, séchée sur MgSO₄ anhydre et évaporée sous vide. Le résidu solide obtenu est purifié par chromatographie flash sur gel de silice (hexane/éther, 4/1) conduisant au 2-iodoindole (2,19 g ; 9 mmol) avec 90% de rendement. Les données physiques et spectrales sont décrites dans l'article.

Le **2-bromoindole** est préparé selon la procédure précédente en utilisant comme réactif d'halogénation le 1,2-dibromotétrachloroéthane (3,26 g; 10,0 mmol). La purification par chromatographie flash sur gel de silice (pentane/éther, 4/1) a conduit à un solide qui a ensuite été trituré avec de l'hexane. Le 2-iodoindole est obtenu sous forme de cristaux avec 87% de rendement (1,70 g ; 8,7 mmol). Les données physiques et spectrales sont décrites dans l'article.

Le **2-chloroindole** est préparé selon la procédure précédente en utilisant comme réactif d'halogénation l'hexachloroéthane (2,37 g ; 10,0 mmol). Le 2-chloroindole (1,37 g ; 9 mmol) est obtenu avec 90% de rendement après purification par chromatographie sur colonne de gel de silice. Les données physiques et spectrales sont décrites dans l'article.

### Groupements R₄, R₅, R₆, R₇

### Synthèse des alkylindoles

Les **5-, 6- et 7-méthylindoles** sont commercialisés par Alfa Aesar, a Johnson Matthey Company.

Le **6-méthylindole** est préparé (rendement : 55%) selon la procédure générale décrite dans A. Walkington, M. Gray, F. Hossner, J. Kitteringham, M. Voyle Synth. Commun. 2003, 33, 2229-2233.

### Procédure pour la synthèse du 6-méthylindole :

*Etape 1* : à une suspension de carbonate de potassium (6,08 g ; 44 mmol) dans 5 mL de DMF, est additionné le cyanoacétate de benzyle (3,50 g ; 20 mmol). Le carbonate est lavé avec 2 mL de DMF. Une solution de 3-nitro-4-chlorotoluène (3,41 g, 20 mmol) dans 5 mL de DMF est ajoutée et le mélange réactionnel résultant est agité à 70°C pendant 24 heures. Après refroidissement à température ambiante, il est traité par une solution aqueuse d'HCl 5N (12,5 mL). Le mélange est réparti entre de l'éther (100 mL) et de l'eau (50 mL) puis la phase organique est re-extraite par de l'éther. Les phases éthérées sont lavées par de l'eau, séchées sur MgSO₄ anhydre, filtrées sous vide et évaporées. Le résidu est purifié par chromatographie sur gel de silice (éluant : CH₂Cl₂/pentane, 1/1) pour conduire au 2-cyano-2-(2-nitro-4-méthylphényl)éthanoate de benzyle avec un rendement de 75%.
*Etape 2 :* un mélange de 2-cyano-2-(2-nitro-4-méthylphényl)éthanoate de benzyle (13 mmol), de palladium 5% (0,84 g), d'eau (4 mL) et d'éthanol (40 mL) est hydrogéné sous 50 psi pendant 18 heures à 25°C. Le catalyseur est éliminé par filtration sur célite et le solide est lavé par de l'éthanol. Le filtrat obtenu est évaporé sous vide et le résidu est purifié par chromatographie sur colonne de gel de silice (éluant : CH₂Cl₂/pentane, 2/1) pour conduire au 6-méthylindole avec 74% de rendement.

Le **7-méthylindole** est préparé selon la procédure générale décrite dans A. P. Dobbs, M. Voyle, N. Whittall Synlett 1999, 1594-1596. Rendement : 71%.

### Synthèse d'indoles disubstitués

Le **5-méthyl-6-trifluorométhylindole et** le **5-méthyl-6-bromoindole** sont préparés selon la procédure générale décrite dans A. P. Dobbs, M. Voyle, N. Whittall Synlett 1999, 1594-1596. Rendements respectivement de 36 et 34%.

### Synthèse des trifluorométhylindoles

Le **4-trifluorométhylindole** est préparé selon la procédure générale décrite dans K. J. Krishna, R. Jain, A. Dandia, S. Saroj, N. Ahmed J. Heterocyclic Chem. 1989, 26, 1799-1802.

Le **5-trifluorométhylindole** est préparé selon la procédure générale décrite dans A. Walkington, M. Gray, F. Hossner, J. Kitteringham, M. Voyle Synth. Commun. 2003, 33, 2229-2233. Rendement : 84%.

Le **6-trifluorométhylindole** est commercial chez la Société Alfa Aesar. Sa synthèse (rendement : 85%) à partir du 4-chloro-3-nitro-1-trifluorométhylbenzène (commercial notamment chez Sigma-Algrich), ses caractéristiques physiques et spectrales sont décrites dans A. Walkington, M. Gray, F. Hossner, J. Kitteringham, M. Voyle Synth. Commun. 2003, 33, 2229-2233. Cette méthode permet d'obtenir des quantités de produits de l'ordre de 100 kg (dans l'article : 8,36 kg, 79%).

Le **7-trifluorométhylindole** est préparé selon la procédure générale décrite dans A. P. Dobbs, M. Voyle, N. Whittall Synlett 1999, 1594-1596. Rendement : 56%.

Autre publication : Y. Murakami, T. Watanabe, T. Hagiwara, Y. Akiyama, N. Kondo, H. Ishii Chem. Pharm. Bull. 1995, 43(8), 1281-1286.

### Synthèse des hydroxyindoles

Les **4- et 5-hydroxyindoles** sont commercialisés par Alfa Aesar.

Les **hydroxyindoles** peuvent être préparé par réaction des méthoxyindoles correspondants avec BBr₃ dans le CH₂Cl₂ selon P. Zhang, R. Liu, J. M. Cook Tetrahedron Lett. 1995, 36, 9133-9136 ou A. M. Felix J. Org. Chem. 1974, 39, 1427-1429.

Synthèse du **5,6-dihydroxyindole :** sa synthèse en 3 étapes à partir du 3,4-dihydroxybenzaldéhyde est décrite dans L. Novellino, M. d'Hischia, G. Prota Synthesis 1999, 793-796. Cette méthode a permis d'accéder au **5,6-dibenzyloxyindole** et au **5,6-diacétoxyindole.** Leurs données physiques et spectrales sont décrites dans la publication.

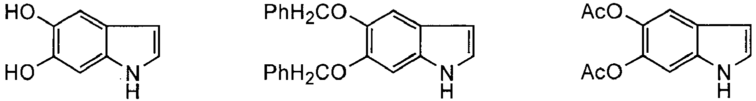

Synthèse du **4,6,7-triméthoxyindole :** sa synthèse à partir du 2,4,5-triméthoxybenzaldéhyde est décrite dans E. V. Sadanandan, S. K. Pillai, M. V. Lakshmikantham, A. D. Billimoria, J. S. Culpepper, M. P. Cava J. Org. Chem. 1995, 60, 1800-1805. Ses données physiques et spectrales sont décrites dans la publication.

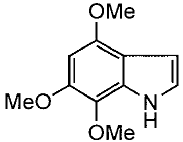

### Synthèse des alcoxyindoles

### Méthoxyindoles

Les **4-, 5- et 6-méthoxyindoles** sont commercialisés par Alfa Aesar, a Johnson Matthey Company.

Le **6-méthoxyindole** est préparé (rendement : 39%) selon la procédure générale décrite dans A. Walkington, M. Gray, F. Hossner, J. Kitteringham, M. Voyle Synth. Commun. 2003, 33, 2229-2233.

### N-Benzyloxyindoles

Les **4-, 5- et 6-benzyloxyindoles** sont commercialisés par Alfa Aesar, a Johnson Matthey Company.

Les ***N*-benzyloxyindoles** peuvent être préparés selon une procédure décrite dans T. W. Greene, P. M. Wuts, « Protective Groups in Organic Synthesis », Third Edition, John Wiley & Sons, Inc., 1999, chapitre 3, pages 266-269.

### Halogénoindoles

Les **4-, 5-, 6- et 7-bromoindoles** sont commercialisés par Alfa Aesar, a Johnson Matthey Company. Leur synthèse est effectuée selon le mode opératoire décrit dans M. P. Moyer, J. F. Shiurba, H. Rapoport J. Org. Chem. 1986, 51, 5106-5110.

Le **4,7-dibromoindole** est préparé selon la procédure générale décrite dans A. P. Dobbs, M. Voyle, N. Whittall Synlett 1999, 1594-1596. Rendement : 69%.

Les **4-, 5- et 6-chloroindoles** sont commercialisés par Alfa Aesar, a Johnson Matthey Company.

Les **5-, 6- et 7-fluoroindoles** sont commercialisés par Alfa Aesar, a Johnson Matthey Company.

Le 6-iodoindole a été préparé en 3 étapes à partir du 6-nitroindole selon K. Kato, M. Ono, H. Akita Tetrahedron Lett. 1997, 38, 1805-1808. Le 4-iodoindole et 5-iodoindole pourraient être préparés selon cette procédure à partir des nitroindoles commerciaux respectifs.

Le 7-iodoindole a été préparé en 4 étapes à partir de l'indole selon une procédure décrite dans Y. Yamada, S. Arima, C. Okada, A. Akiba, T. Kai, Y. Harigaya Chem. Pharm. Bull. 2006, 54, 788-794. Cette procédure permet de préparer le 7-chloroindole et le 7-bromoindole.

### Dihalogénoindoles

Monobromation en position 2 du noyau indolique ou dibromation en positions 2 et 6 du noyau indolique : A. G. Mistry, K. Smith, M. R. Bye Tetrahedron Lett. 1986, 27, 1051-1054. Dans cet article, les auteurs montrent que les 3-méthyl- (à l'abri de la lumière) et 3-cyanométhyl-indoles réagissent avec 1 équivalent ou 2 équivalents de *N*-bromosuccinimide (NBS) dans le dichlorométhane à 20°C pour conduire respectivement aux **2-bromoindoles** ou aux **2,6-dibromoindoles** correspondants (rendements : 77-96%)

### Aminoindoles

Les **4-, 5-, 6- et 7-aminoindoles** sont commercialisés chez Alfa Aesar, a Johnson Matthey Company.

Ils peuvent être préparés par réduction des nitroindoles correspondants. La synthèse du **5-aminoindole** est décrite dans J. I. DeGraw Can. J. Chem. 1966, 44, 387-393 : à un mélange de 25,0 g de 5-nitroindole, 1,2 g de palladium 10% sur charbon et 200 mL d'éthanol absolu est additionné lentement 25 mL d'hydrazine (98-100%). Après addition, le mélange réactionnel résultant est ensuite porté au reflux pendant 2 heures. Le catalyseur est éliminé par filtration et le filtrat est évaporé pour conduire à un résidu solide jaune. Les cristaux sont lavés avec 200 ml d'eau et ils sont séchés sous vide pour conduire à 16,5 g (81%) de 5-aminoindole. Pt fusion : 131-134°C.

### N-monoalkylamino- et N,N-dialkylaminoindoles

Le groupe ***N,N-*diméthylamino** pourrait être préparé :
- directement à partir de **l'amine** correspondante par réaction avec NaBH₃CN, CH₂O en milieu méthanolique acide (AcOH) : K. S. Jandu, V. Barrett, M. Brockwell, D. Cambridge, D. R. Farrant, C. Foster, H. Giles, R. C. Glen, A. P. Hill, H. Hobbs, A. Honey, G. R. Martin, J. Salmon, D. Smith, P. Woollard, D. L Selwood J Med. Chem. 2001, 44, 681-693 ou
- à partir du groupe ***N*-méthylamino :** M. Kurosu, S. S. Dey, D. C. Crick Tetrahedron Lett. 2006, 47, 4871-4875. Cette méthode permet la synthèse d'amines tertiaires différemment substituées (Rₐ ≠ R_{b}) par *N*-alkylation d'amines secondaires.

### Monoalkylation

La monoalkylation des amines primaires peut être réalisée selon la procédure décrite dans R. N. Salvatore, A. S. Nagle, S. E. Schmidt, K. W. Jung Org. Lett. 1999, 1, 1893-1896.

**Revue :** « Synthesis of Secondary Amines », R. N. Salvatore, C. H. Yoon, K. W. Jung Tetrahedron 2001, 57, 7785-7811.

Référence pour la synthèse du groupe ***N*-éthylamino** à partir de l'amine primaire par amination réductrice : 1,0 équivalent de MeCHO, 0°C, 2 heures puis 2,0 équivalents de NaBH₄, 0°C, 1heure : K. C. Nicolaou, R. D. Groneberg, N. A. Stylianides, T. Miyazaki J. Chem. Soc., Chem. Commun. 1990, 1275-1277.

### Synthèse des N,N-diméthylaminoindoles

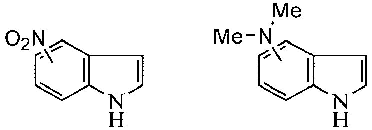

Les ***N,N*-diméthylaminoindoles** peuvent être préparés à partir des **nitroindoles** correspondants selon un mode opératoire décrit dans l'article suivant : J. I. DeGraw *Can.*

J. Chem. 1966, 44, 387-393. Les 4-nitro-, 5-nitro- et 7-nitroindoles sont commercialisés par Aldrich. Les 4-, 5-, 6- et 7-nitroindoles sont commercialisés par Alfa Aesar.

Le **5-(*N,N-*diméthylamino)indole** est préparé à partir du 5-nitroindole selon la procédure suivante :
*Synthèse du 5-aminoindole :* à un mélange de 25,0 g de **5-nitroindole,** 1,2 g de palladium 10% sur charbon et 200 mL d'éthanol absolu sont additionnés lentement 25 mL d'hydrazine (98-100%). Après addition, le mélange réactionnel résultant est ensuite porté au reflux pendant 2 heures. Le catalyseur est éliminé par filtration et le filtrat est évaporé pour conduire à un résidu solide jaune. Les cristaux sont lavés avec 200 ml d'eau et ils sont séchés sous vide pour conduire à 16,5 g (81%) de 5-aminoindole. Pt fusion : 131-134°C.
*Synthèse du picrate de 5-indolyltriméthylammonium :* à un mélange de 2 g (15,5 mmol) de 5-aminoindole dans 32 mL d'eau et contenant 5,7 g (68 mmol) de bicarbonate de sodium, est additionné 6,2 mL de diméthylsulfate en 12 minutes sous agitation. La solution résultante est agitée pendant 10 nouvelles minutes, chauffée à 65°C et versée dans une solution chaude de 3,6 g (15,7 mmol) d'acide picrique dans 240 mL d'eau. Le précipité cristallin jaune résultant est laissé pendant 2 heures. Il est récupéré, lavé avec de l'eau puis avec de l'éther et séché pour donner 5,2 g (85%) de picrate. Sa recristallisation par l'éthanol à 75% a conduit à 4,3 g (75%) de picrate de 5-indolyltriméthylammonium pur (point de fusion : 171-173°C). L'analyse centésimale est décrite dans la publication.
*Synthèse du chlorure de 5-indolyltriméthylammonium :* un mélange de 300 g d'une résine Dowex 2 (chlorure), 46 g de picrate de 5-indolyltriméthylammonium et 1200 mL de méthanol à 83% est agité pendant 17 heures à température ambiante. La résine est éliminée par filtration et lavée avec 100 mL de méthanol. Le filtrat est évaporé sous vide pour conduire à un résidu sirupeux qui est dissous dans 120 mL d'isopropanol chaud et dilué dans 800 mL d'acétone. Le résidu qui précipite est éliminé par décantation et le surnageant est dilué dans 700 mL supplémentaire d'acétone. La solution est laissée pendant 3 jours pour conduire à des cristaux beige qui sont récupérés, lavés avec de l'acétone et séchés pour donner 13,5 g (57%) de produit (point de fusion : 200-201°C). L'analyse centésimale est décrite dans la publication.
*Synthèse du 5-(N,N-diméthylamino)indole :* à une solution de 1,54 g (67 mmol) de sodium dans 270 mL de *n*-propanol est additionné 13 g de chlorure de 5-indolyltriméthylammonium et le mélange réactionnel est agité au reflux pendant 17 heures. Il est ensuite évaporé « à sec » sous vide et le résidu est traité par 100 mL d'eau et 100 mL d'éther. Après séparation des 2 phases, la phase aqueuse est extraite par de l'éther. Les phases éthérées sont réunies, lavées par de l'eau, séchées sur MgSO₄ anhydre et évaporées sous vide pour conduire à 8,2 g de résidu sirupeux. Ce dernier est distillé sous pression réduite pour conduire à 7,5 g (76%) de 5-(*N,N-*diméthylamino)indole sous forme d'un liquide clair (point éb. : 135-140°C sous 0,25 mm Hg). Le liquide se solidifie pour former des cristaux blancs (point fusion : 45-47°C). L'analyse centésimale est décrite dans la publication.

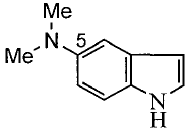

### N-(Boc)aminoindoles

Les *N*-(Boc)aminoindoles pourraient être préparés par réaction des aminoindoles avec le dicarbonate de di-*t*-butyle (Boc₂O) dans le THF à 60°C selon la procédure décrite dans G. Chelucci, I. Manca, G. A. Pinna Tetrahedron Lett. 2005, 46, 767-770.

### Groupement R₁

### Groupe -COCH₂ONH-GP

### Synthèse des précuseurs avec GP = Boc ou Fmoc

Les réactifs dont les structures sont décrites ci-dessous : 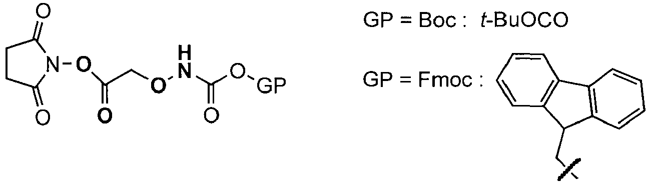 permettent l'introduction du groupe **-COCH₂ONH-GP.** Ils sont notamment préparés selon les références :
- **GP = Boc :** M. Kurono, M. Isobe Chem. Lett. 2004, 33, 452-453 ; M. Kurono, A. Shimomura, N. Chikusa Tetrahedron 2004, 60, 1773-1780 et S. Deroo, E. Defrancq, C. Moucheron, A. Kirsch-De Mesmaecker, P. Dumy Tetrahedron Lett. 2003, 44, 8379-8382, par protection de l'hémichlorhydrate de l'*O*-carboxyméthyl-hydroxylamine commercial ((H₂NOCH₂COOH)₂. HCl) avec le dicarbonate de di-*t*-butyle (Boc₂O) dans le dioxane en milieu basique (NaOH) (80%) suivi de la réaction de l'acide intermédiairement obtenu (BocNHOCH₂COOH) avec le *N*-hydroxysuccinimide en présence de dicyclohexylcarbodiimide (DCC) dans le CH₂Cl₂ (rendement : 90%) ;
- **GP = Fmoc :** L. Cipolla, M. Rescigno, A. Leone, F. Peri, B. La Ferla, F. Nicotra Bioorg. Med. Chem. 2002, 10, 1639-1646 par protection de l'hémichlorhydrate de l'*O-*carboxyméthyl-hydroxylamine commercial ((H₂NOCH₂COOH)₂. HCl) avec le chloroformiate de 9-fluorénylméthyle (Fmoc-Cl) dans le dioxane en présence de Na₂CO₃ (77%) suivi de la réaction de l'acide intermédiairement obtenu (FmocNHOCH₂COOH) avec le *N*-hydroxysuccinimide en présence de dicyclohexylcarbodiimide (DCC) dans un mélange acétate d'éthyle/dioxane (rendement : 93%). Les caractéristiques physiques et spectroscopiques des 2 composés sont décrites.

### PARTIE EXPÉRIMENTALE - BIOLOGIE

### Souches bactériennes

Les deux souches utilisées sont *Staphylococcus aureus* (ATCC 25923, souche à Gram-positif sensible) appelée S, et *S. aureus* SA-1199 (sans efflux), ainsi que son dérivé *S. aureus* SA-1199B (Kaatz, G. W., Seo, S.M., Antimicrob. Agents Chemother., 1995, 39, 2650-2655) présentant une résistance aux fluoroquinolones due à la surexpression de la pompe d'efflux NorA et appelée R NorA. Les bactéries sont cultivées à 37 °C dans les milieux Mueller-Hinton (MH, Bio Rad) ou Luria-Bertani (LB, Difco).

### Détermination de l'activité antibactérienne

Les expériences décrites ci-après sont faites en suivant les recommandations du Comité de l'Antibiogramme de la Société Française de Microbiologie (http://www.sfm.asso.fr/nouv/general.php Voir aussi Courvalin, P., Goldstein, F., Philippon, A., Sirot J. L'antibiogramme, 1985, M.P.C.-Videom editeur). L'activité antibactérienne des molécules a été déterminée en milieu liquide en utilisant un robot Biomek 2000 robot (Beckman) et des plaques de micro-titration. Le volume final dans chaque puits est de 200 µl. On ajoute un volume maximum de 5 µl d'une solution de la molécule dans le DMSO à chaque puits contenant 10⁶ UFC/ml (Unités Formant Colonies) de bactéries dans du milieu MH. La concentration finale de la molécule à tester doit être de 100 ou de 128 µg/ml Les plaques sont incubées dans une étuve à 37°C, et la croissance bactérienne est mesurée à 650 nm après 1 h, 2 h, 8 h et 24 h d'incubation. L'ampicilline est utilisée comme témoin positif et le même volume de DMSO que celui employé pour introduire la molécule (généralement de 2 à 5 µl) comme témoin négatif. Une molécule est qualifiée de très active si elle provoque dans ces conditions un arrêt complet de la croissance bactérienne, comme active si la croissance bactérienne n'excède pas 10% du témoin négatif, comme inactive dès que la croissance atteint 10% de celle du témoin négatif.

La Concentration Minimale Inhibitrice (CMI) des molécules très actives est déterminée par la méthode des dilutions sériées de raison 2 dans le milieu Mueller-Hinton, en présence de 10⁶ UFC de bactéries par ml. La CMI, exprimée en µg/ml, est définie comme la plus faible concentration de molécule ne permettant d'observer aucune croissance après 18h d'incubation à 37 °C. Les expériences sont répétées trois fois.

### Inhibition du mécanisme de la résistance

Les expériences sont menées comme plus haut, mais en utilisant la souche résistante, et en regardant l'effet du mélange de la molécule à tester et de l'antibiotique auquel la bactérie est résistante. Ainsi, on a utilisé la méthode des dilutions sériées, dans le milieu Mueller-Hinton, en partant également d'une concentration de molécule de 100 ou de 128 µg/ml. L'antibiotique utilisé a été la ciprofloxacine. Sa CMI sur cette souche résistante est de 16 µg/mL. Dans cette étude, l'activité inhibitrice spécifique de la pompe d'efflux NorA a été caractérisée par la CMI des composés en présence de la ciprofloxacine évaluée à des concentrations de 8 µg/mL (c/2) et de 4 µg/mL (c/4).

### RÉSULTATS BIOLOGIQUES

### A - Composés présentant une activité antibactérienne intrinsèque

| **Composé** | **CMI (µg/ml)** |
|---|---|
| (2) | 100 |
| (1) | 25 |
| (3) | 100 |
| (4) | 25 |
| (5) | 6 |
| (12) | 12,5 |
| (13) | 50 |
| (15) | 25 |
| (19) | 100 |
| (21) | 50 |
| (22) | 50 |
| (25) | 25 |
| (26) | 25 |
| (27) | 25 |
| (28) | 25 |
| (31) | 64 |
| (32) | 3 |
| (33) | 6 |
| (41) | 12,5 |
| (42) | 50 |
| (43) | 100 |
| (47) | 64 |

On peut également se référer aux composés suivants présentant une activité antibactérienne intrinsèque (tests effectués pour *S. Aureus*) :

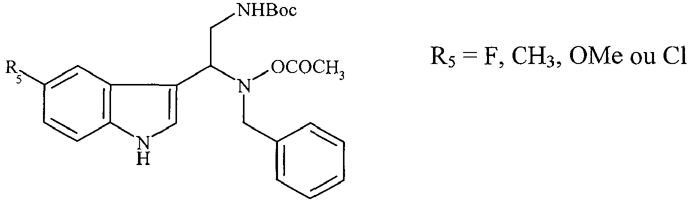

Ces composés sont des composés de formule (I) telle que définie ci-dessus dans laquelle : R = R₃ = R₄ = R₆ = R₇ = H ; R₁ = CH₂NHBoc ; R₂ = OCOCH₃ et B est un groupe de formule (B-1) dans laquelle a est une liaison simple ; A est un atome d'azote et R_{IV} = R' = R" = R"' = H.

Ces composés présentent une CMI inférieure ou égale à 10 µg/ml.

De même, les trois composés suivants présentent une CMI inférieure ou égale à 10 µg/ml :

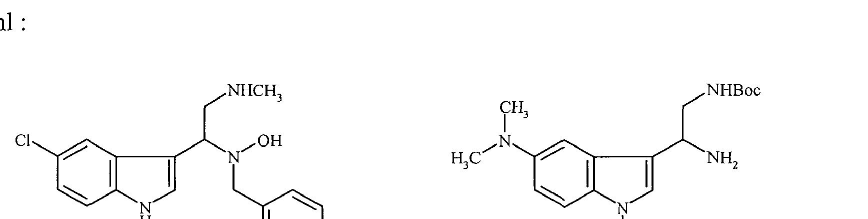

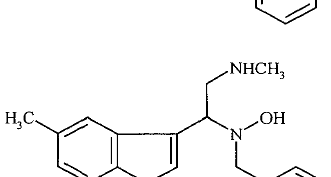

Parmi les composés de l'invention, on peut également citer les composés répondant à la formule suivante :

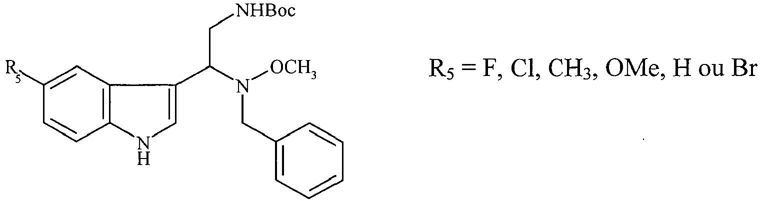

Ces composés sont des composés de formule (I) telle que définie ci-dessus dans laquelle : R = R₃ = R₄ = R₆ = R₇ = H ; R₁ = CH₂NHBoc ; R₂ = OCH₃ et B est un groupe de formule (B-1) dans laquelle a est une liaison simple ; A est un atome d'azote et R_{IV} = R' = R" = R"' = H.

Parmi les six composés susmentionnés, les composés dans lesquels R₅ est F, Cl, CH₃, OMe ou H présentent une CMI inférieure ou égale à 20 µg/ml, et le composé dans lequel R₅ est Br présente une CMI inférieure ou égale à 10 µg/ml.

On peut également citer des composés particuliers répondant à la formule (I-7-a) telle que définie ci-dessus :
- lorsque R₅ = H et R₆ = Cl, ou lorsque R₅ = Cl et R₆ = H, ou lorsque R₅ = NH₂ et R₆ = H, ou lorsque R₅ = H et R₆ = CH₃ ou lorsque R₅ = CH₃ et R₆ = H, les composés correspondants présentent une CMI inférieure ou égale à 20 µg/ml,
- tandis que lorsque R₅ = H et R₆ = OMe, ou lorsque R₅ = F, H ou OMe et R₆ = H, ou lorsque R₅ = H et R₆ = NH₂, ou lorsque R₅ = *N,N-*diméthylamino et R₆ = H, ou lorsque R₅ = H et R₆ = *N,N-*diméthylamino, ou lorsque R₅ = H et R₆ = CF₃, les composés correspondants présentent une CMI inférieure ou égale à 10 µg/ml.

On peut également citer des composés particuliers répondant à la formule (I-4) telle que définie ci-dessus dans laquelle R = R₃ = R₄ = R₇ = H :

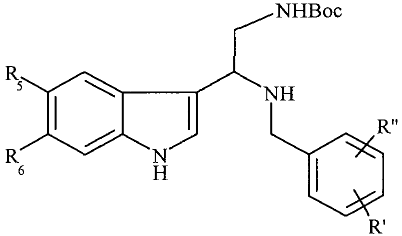

Les composés préférés sont les suivants :
* R' = *p*-méthoxyle et R" = H
   - R₅ = F et R₆ = H CMI ≤ 20 µg/ml
   - R₅ = OMe et R₆ = H CMI ≤ 10 µg/ml
   - R₅=H et R₆ = OMe CMI≤ 20 µg/ml
* R' et R" représentent ensemble un groupe di-*m*-méthoxyle
   - R₅=F et R₆=H CMI≤10 µg/ml
   - R₅=R₆=H CMI≤ 20 µg/ml
   - R₅=OMe et R₆=H CMI≤ 10 µg/ml
   - R₅=H et R₆=OMe CMI ≤10µg/ml
* R' et R" représentent ensemble un groupe *o*- et *p*-diméthoxy
   - R₅=R₆=H CMI≤ 10 µg/ml
   - R₅=F et R₆=H CMI≤10 µg/ml
   - R₅ =OMe et R₆=H CMI≤ 10 µg/ml

On peut également citer des composés particuliers répondant à la formule (I-4-a) telle que définie ci-dessus dans laquelle :
- R₅=CF₃ et R₆=H CMI≤ 20 µg/ml
- R₅=H et R6=CF₃ CMI≤ 20 µg/ml

On peut également citer des composés particuliers répondant à la formule (I-5-a) telle que définie ci-dessus (avec R' = H) dans laquelle :
- R₅=CF₃ et R₆=H CMI≤ 20 µg/ml
- R₅=H et R6=CF₃ CMI≤ 20 µg/ml

On peut également citer un composé particulier répondant à la formule (I-8-a) telle que définie ci-dessus (avec X = HCl) dans laquelle R₅ = CF₃ et R₆ = H dont la CMI est inférieure ou égale à 10 µg/ml.

### B - Composés présentant une activité d'inhibition de la pompe NorA

| **Composé** | **CMI (µg/ml) du composé en présence de ciprofloxacine à 8 µg/ml (c/2)** | **CMI (µg/ml) du composé en présence de ciprofloxacine à 4 µg/ml (c/4)** | |
|---|---|---|---|
| (1) | non déterminé | 1 | |
| (5) | non déterminé | 2 | |
| (6) | 2 | 4 | |
| (7) | <2 | 2 | |
| (8) | 8 | 8 | |
| (10) | non déterminé | 8 | |
| (11) | non déterminé | 16 | |
| (12) | non déterminé | 4 | |
| (20) | <3 | 3 | |
| (20 bis) | 1 | 1 | |
| (23) | 4 | 8 | |
| (24) | 4 | 8 | |
| (31) | non déterminé | 16 | |
| (36) | non déterminé | 8 | |
| (37) | > 8 | >8 | |
| (38) | > 8 | 16 | |
| (40) | > 8 | 16 | |
| (46) | non déterminé | 8 | |
| (47) | non déterminé | 32 | |
| (48) | non déterminé | 2 | |
| (50) | non déterminé | 8 | |

### CYTOTOXICITÉ

Dans le cadre de l'évaluation de l'activité antibiotique ou inhibitrice de pompe d'efflux, la cytotoxicité doit être minimale. En effet, dans la perspective d'un traitement médical chez l'homme ou l'animal, la substance administrée doit être sélective des cellules procaryotes.

Les résultats de la cytotoxicité *in vitro,* mesurée à une concentration de 10⁻⁵ M, sont décrits ci-dessous. La cytotoxicité a été mesurée sur 5 lignées cellulaires - KB (carcinome de la bouche humaine), MCF7 (carcinome du sein) et MCF7R (souche résistante), Véro (rein de singe) et HCT116 (tumeur colique humaine). Les composés (1), (38) et (40) ne font pas partie de l'invention

| | **Composés** | **Activité Antibactérienne (AA) et/ou Activité inhibitrice sur NorA (+ ciprofloxacine) (c/2 et c/4) (µg/mL)** | **Cytotoxicité moyenne (% inhibition)** |
|---|---|---|---|
| | 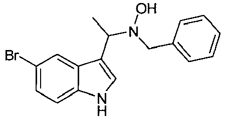 | | Cellules KB : 50% |
| **(1)** | | c/4 = 1 | Cellules Véro : 36% |
| | | | Cellules HCT116: 28% |
| | 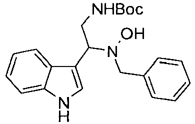 | | Cellules KB: 33,7% |
| | | c/2 = 2 | Cellules MCF7: 13,1% |
| **(6)** | | c/4 = 4 | Cellules MCF7R : 16% |
| | | | Cellules Véro : 27% |
| | | | Cellules HCT116 : 29% |
| | 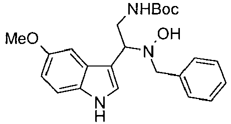 | | Cellules KB : 15,6% |
| | | c/2 <2 | Cellules MCF7 : 9,7% |
| **(7)** | | c/4 = 2 | Cellules MCF7R : 10,1 % |
| | | | Cellules Véro : 8% |
| | | | Cellules HCT116 : 10% |
| | 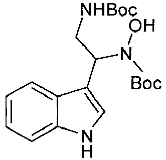 | | Cellules KB : 14% |
| **(11)** | | c/4 = 16 | Cellules Véro : 15% |
| | | | Cellules HCT116 : 22% |
| | 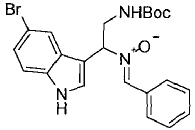 | | Cellules KB : 38,3% |
| **(20bis)** | | c/2 = 1 | Cellules MCF7 : 23,3% |
| | | c/4 = 1 | Cellules MCF7R: 38,1% |
| | 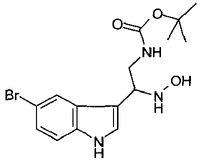 | | Cellules KB : 0% |
| **(21)** | | AA = 50 | Cellules Véro : 11% |
| | | | Cellules HCT116 : 28% |
| | 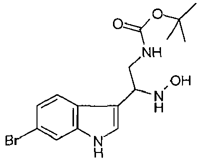 | | Cellules KB : 0% |
| **(22)** | | AA = 50 | Cellules Véro : 22% |
| | | | Cellules HCT116 : 37% |
| | 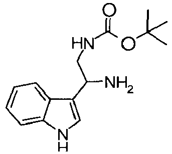 | | Cellules KB : 2% |
| **(23)** | | c/4 = 8 | Cellules Véro : 11% |
| | | | Cellules HCT116 : 5% |
| | 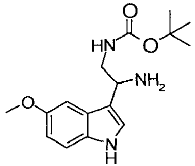 | | Cellules KB : 2% |
| **(24)** | | c/4 = 8 | Cellules Véro : 12% |
| | | | Cellules HCT116 : 13% |
| **(27)** | 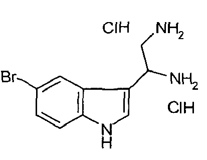 | | Cellules KB : 0% |
| | | AA = 25 | Cellules Véro : 14% |
| | | | Cellules HCT116 : 26% |
| **(31)** | 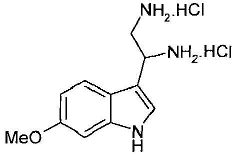 | c/4 = 16 | Cellules KB : 41% |
| | | | Cellules Véro : 27% |
| **(32)** | 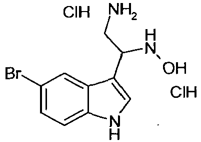 | AA = 3 | Cellules KB : 23% |
| | | | Cellules Véro : 20% |
| | 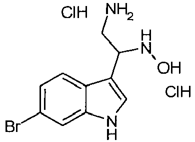 | | Cellules KB : 14% |
| **(33)** | | AA = 6 | Cellules Véro : 31% |
| | | | Cellules HCT116 : 37% |
| | 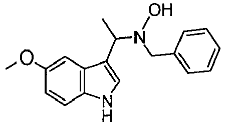 | | Cellules KB : 0% |
| **(38)** | | c/4=16 | Cellules Véro : 5% |
| | | | Cellules HCT116 : 15% |
| | 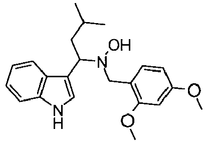 | | Cellules KB : 13% |
| **(40)** | | c/4 = 16 | Cellules Véro : 32% |
| | | | Cellules HCT116 : 46% |
| | 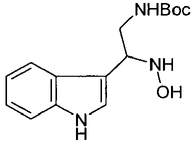 | | Cellules KB : 31% |
| **(46)** | | c/4 = 8 | Cellules Véro : 0% |
| | | | Cellules HCT116 : 39% |
| **(50)** | 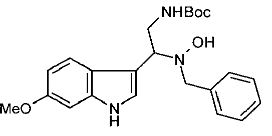 | c/4 = 8 | Cellules Véro : 42% |

## Revendications

1. Utilisation d'au moins un composé de formule (I) suivante : 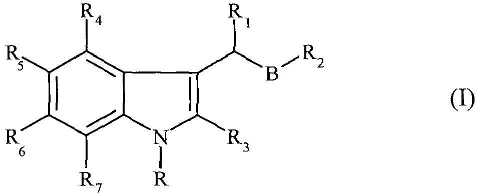 dans laquelle :
- R représente un atome d'hydrogène;
- R₃ représente un atome d'hydrogène;
- R₄ et R₇ repésentent un atome d'hydrogène;
- R₅ et R₆ représentent indépendamment l'un de l'autre un groupe choisi parmi l'un des groupes suivants:
* un atome d'hydrogène;
* un groupe alcoxyle comprenant de 1 à 7 atomes de carbone ou un groupe benzyloxyle;
* un atome d'halogène choisi parmi Br, Cl, F ou I;
- R₁ représente un groupe choisi parmi l'un des groupes suivants:
* un groupe -CH₂-NH-GP, GP représentant un groupe choisi parmi les groupes suivants : Boc, Cbz, ou Me₃SiCH₂CH₂OCO (Teoc);
* un groupe -CH₂-NH₂ ou -CH₂-NH₂.X, X représentant HCl, HCOOH ou HOOCCOOH;
- R₂ représente un groupe choisi parmi l'un des groupes suivants :
* un atome d'hydrogène;
* un groupe O⁻;
* un groupe OH;
* un groupe acyle comprenant de 1 à 10 atomes de carbone;
- B représente un groupe choisi parmi l'un des groupes suivants: 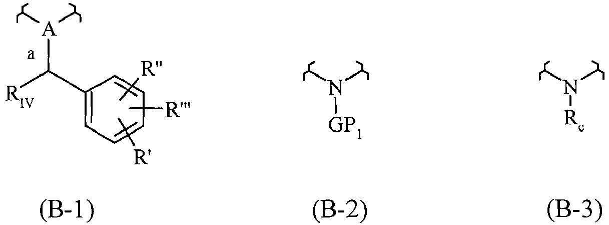
* a représentant une liaison simple ou une double liaison;
* A représentant N ou N⁺ ;
. a représentant une liaison simple lorsque A représente N;
. a représentant une double liaison lorsque A représente N⁺ et R₂ représente O⁻ ;
* R', R" et R"' représentant, indépendamment les uns des autres, un groupe choisi parmi l'un des groupes suivants:
* un atome d'hydrogène;
* un groupe alcoxyle comprenant de 1 à 4 atomes de carbone;
* R_{IV} représentant un atome d'hydrogène;
* GP₁ représentant un groupe Boc ou Cbz;
* R_{c} représentant un atome d'hydrogène;
lesdits composés de formule (I) pouvant être sous forme d'isomères optiques, à savoir sous forme d'énantiomères ou de mélanges de ces énantiomères, y compris le mélange racémique, ou sous forme, le cas échéant, de sels d'acides physiologiquement acceptables,
pour la préparation d'un médicament destiné au traitement de pathologies associées à des infections bactériennes.

2. Utilisation selon la revendication 1, d'un composé de formule (I-1) suivante : 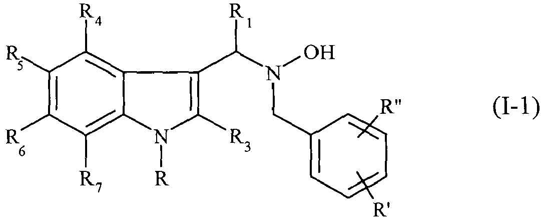 ledit composé de formule (I-1) étant **caractérisé en ce que**:
- R, R₃, R₄ et R₇ représentent un atome d'hydrogène;
- R₅ représente un atome d'hydrogène, un groupe méthoxyle, un atome de brome ou un atome de chlore;
- R₆ représente un atome d'hydrogène ou un atome de brome;
- R₁ représente CH₂NHBoc;
- R' et R" représentent indépendamment l'un de l'autre un atome d'hydrogène ou un groupe méthoxyle ou R' et R" représentent ensemble un groupe *o,p-*diméthoxyle ou un groupe di-*m*-méthoxyle.

3. Utilisation selon la revendication 1, d'un composé de formule (I-2-bis) suivante : 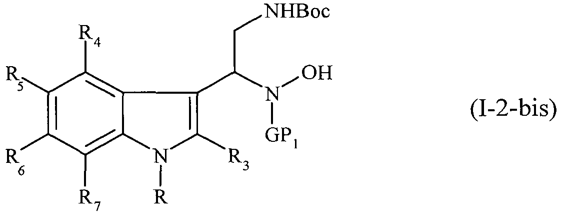 GP₁ représentant un groupe Boc,
ledit composé de formule (I-2-bis) étant **caractérisé en ce que** :
- R, R₃, R₄ et R₇ représentent un atome d'hydrogène;
- R₅ représente un atome d'hydrogène, un atome de brome ou un atome de chlore;
- R₆ représente un atome d'hydrogène ou un atome de brome.

4. Utilisation selon la revendication 1, d'un composé de formule (I-4) suivante : 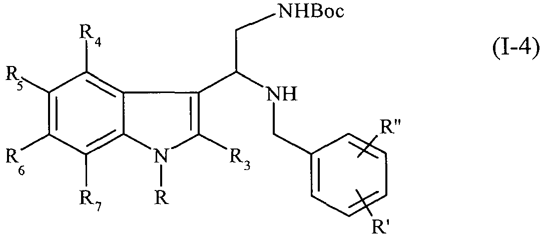 dans laquelle :
- R' et R" représentent un atome d'hydrogène;
- R, R₃, R₄ et R₇ représentent un atome d'hydrogène;
- R₅ représente un atome d'hydrogène, un groupe méthoxyle ou un atome de brome;
- R₆ représente un atome d'hydrogène ou un atome de brome.

5. Utilisation selon la revendication 1, d'un composé de formule formule (I) dans laquelle :
- R₂ représente O⁻ ;
- B représente un groupe de formule (B-1) avec R_{IV} = H;
- A représente N⁺ ; et
- a représente une double liaison.
- R, R₁, R₃, R₄, R₅, R₆, R₇, R', R" et R"' étant tels que définis dans la revendication 1,
les dits composés répondant à la formule (I-5'):

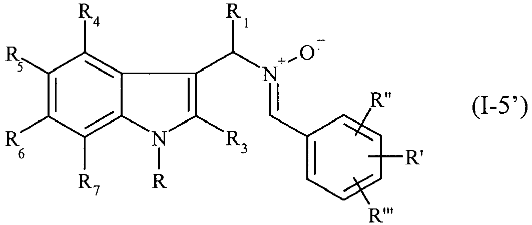

6. Utilisation selon la revendication 1, d'un composé de formule (I-5) suivante : 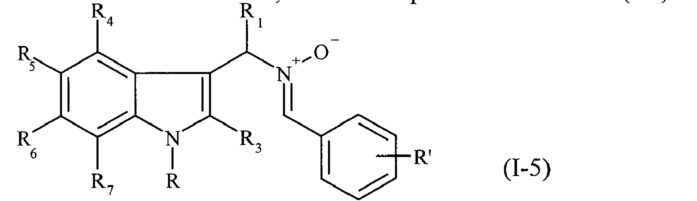 ledit composé de formule (I-5) étant **caractérisé en ce que**:
- R' représente un atome d'hydrogène ou un groupe *p*-méthoxyle;
- R, R₃, R₄ et R₇ représentent un atome d'hydrogène;
- R₁ représente un groupe CH₂NHBoc;
- R₅ représente un atome d'hydrogène ou un atome de brome;
- R₆ représente un atome d'hydrogène ou un atome de brome.

7. Utilisation selon la revendication 1, d'un composé de formule (I-6) suivante : 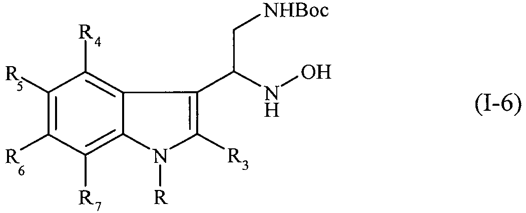 lesdits composés de formule (I-6) pouvant être sous forme, le cas échéant, de sels physiologiquement acceptables choisis parmi les chlorhydrates, les formiates ou les oxalates,
et étant **caractérisés en ce que**:
- R, R₃, R₄ et R₇ représentent un atome d'hydrogène;
- R₅ représente un atome d'hydrogène ou un atome de brome;
- R₆ représente un atome d'hydrogène ou un atome de brome.

8. Utilisation selon la revendication 1, d'un composé de formule (I-7) suivante : 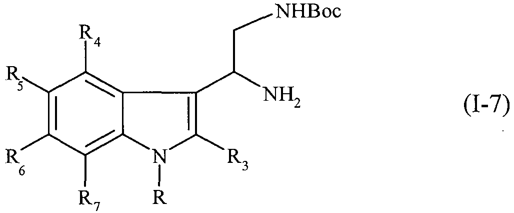 lesdits composés de formule (I-7) pouvant être sous forme, le cas échéant, de sels physiologiquement acceptables choisis parmi les chlorhydrates, les formiates ou les oxalates,
lesdits composés de formule (I-7) étant **caractérisés en ce que**:
- R, R₃, R₄ et R₇ représentent un atome d'hydrogène;
- R₅ représente un atome d'hydrogène, un groupe méthoxyle ou un atome de brome;
- R₆ représente un atome d'hydrogène, un groupe méthoxyle ou un atome de brome.

9. Utilisation selon la revendication 1, d'un composé de formule (I-8) suivante : 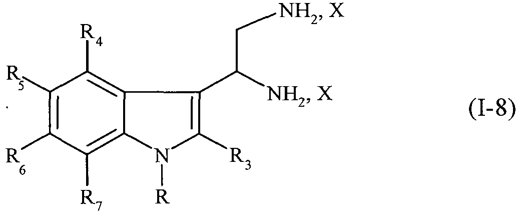 dans laquelle :
- R, R₃, R₄, R₅, R₆ et R₇ sont tels que définis dans la revendication 1 ; et
- X représente HCl, HCOOH ou HOOCCOOH.

10. Utilisation selon la revendication 1, d'un composé de formule (I-9) suivante : 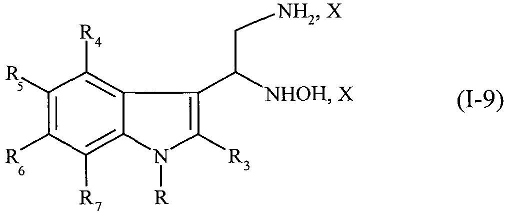 dans laquelle :
- R, R₃, R₄, R₅, R₆ et R₇ sont tels que définis dans la revendication 1 et
- X représente HCl, HCOOH ou HOOCCOOH.

11. Utilisation selon la revendication 1, d'un composé de formule (I-10) suivante : 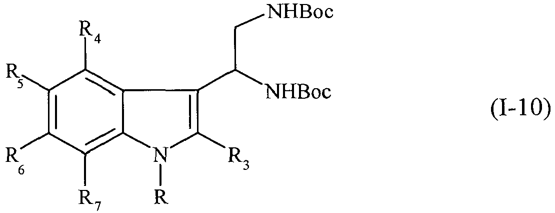 ledit composé de formule (I-10) étant **caractérisé en ce que**:
- R, R₃, R₄ et R₇ représentent un atome d'hydrogène;
- R₅ représente un atome d'hydrogène, un atome de brome ou un groupe méthoxyle;
- R₆ représente un atome d'hydrogène, un atome de brome ou un groupe méthoxyle.

12. Utilisation selon l'une quelconque des revendications 1 à 11, d'un composé répondant à l'une des formules suivantes :

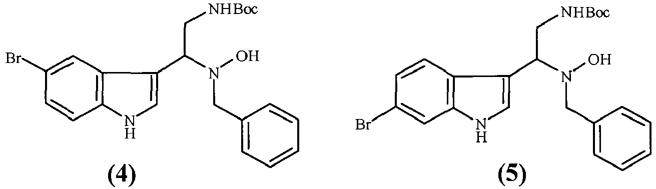

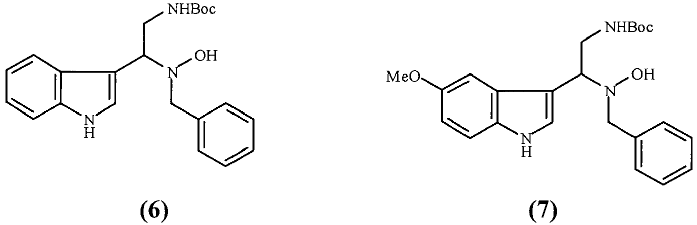

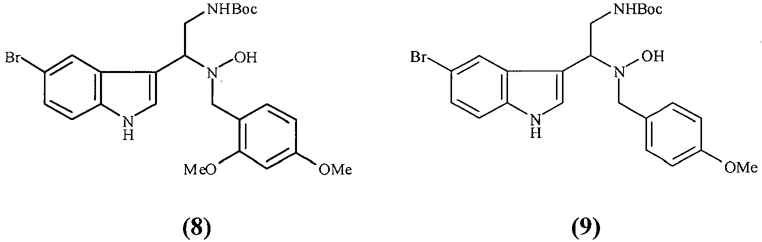

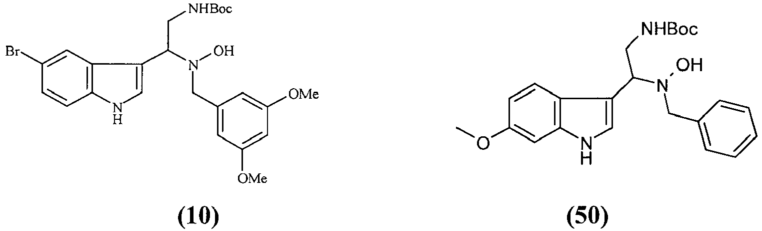

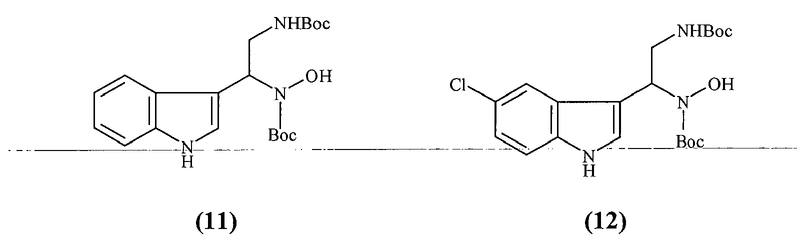

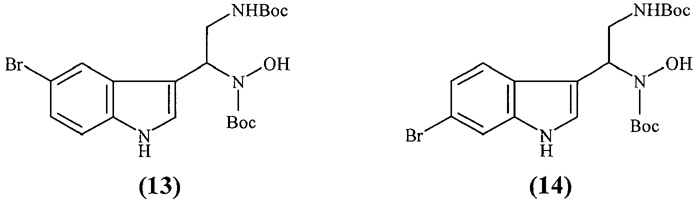

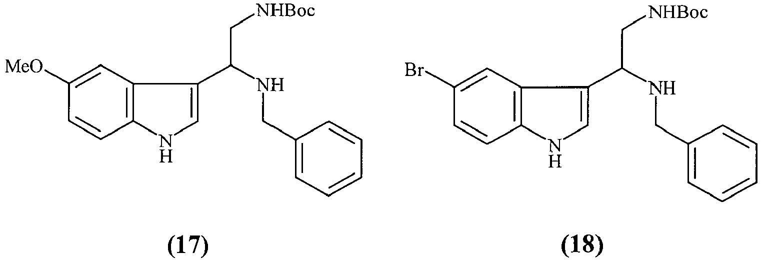

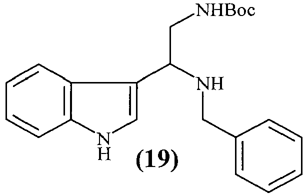

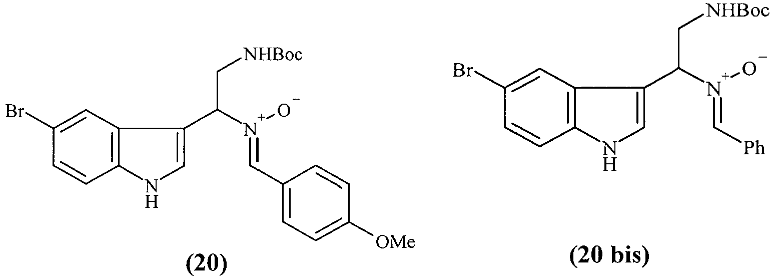

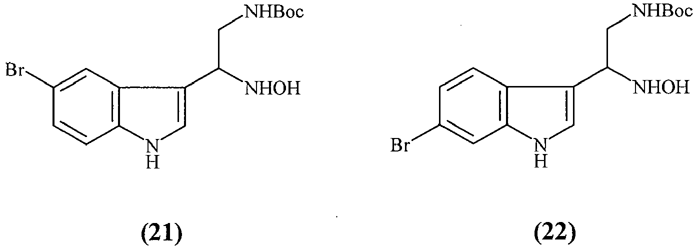

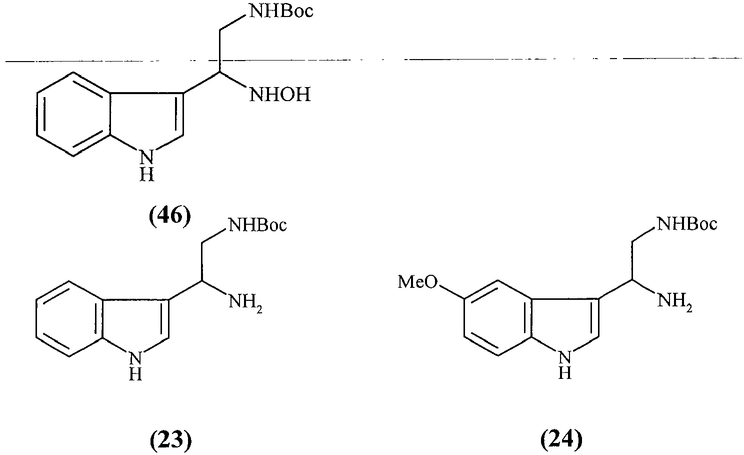

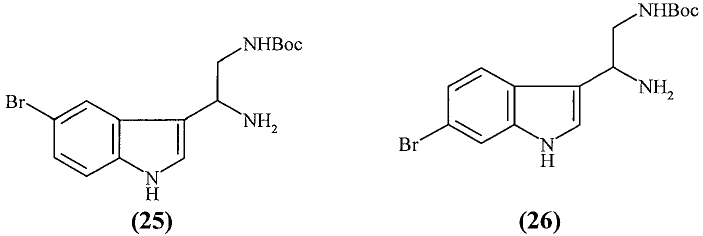

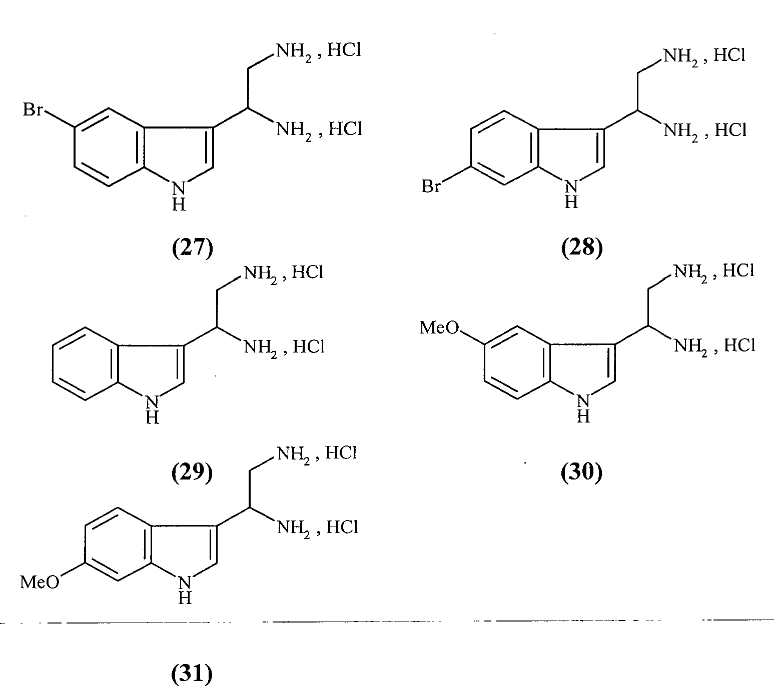

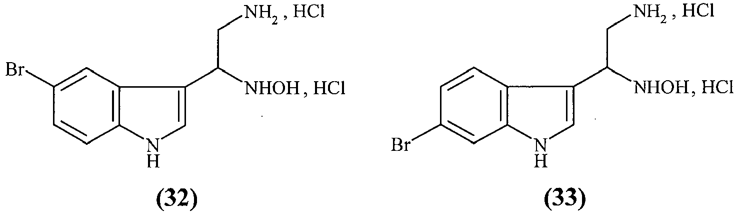

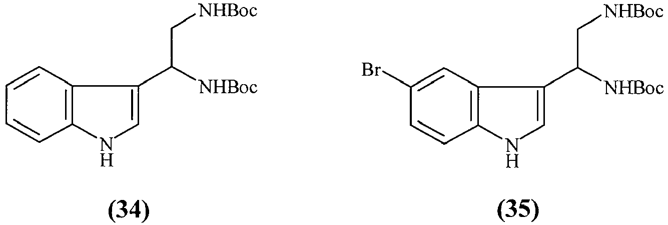

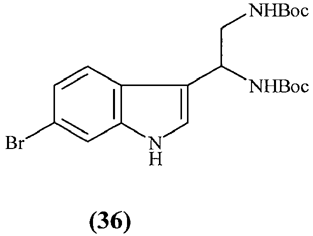

13. Utilisation selon l'une quelconque des revendications 1 à 12, d'au moins un composé de formule (I), en association avec un antibiotique de la famille des fluoroquinolones choisi parmi la ciprofloxacine, la norfloxacine, la péfloxacine, l'énofloxacine, l'ofloxacine, la lévofloxacine et la moxifloxacine, pour le traitement de pathologies associées à des infections bactériennes vis-à-vis desquelles il existe une résistance aux antibactériens,
ledit composé de formule (I) étant choisi parmi l'un des composés suivants:

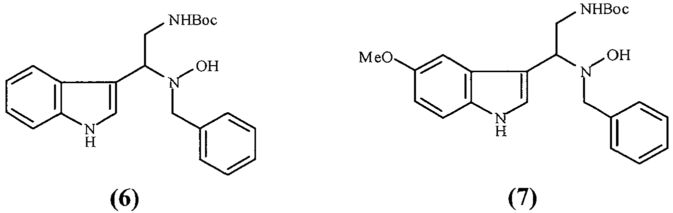

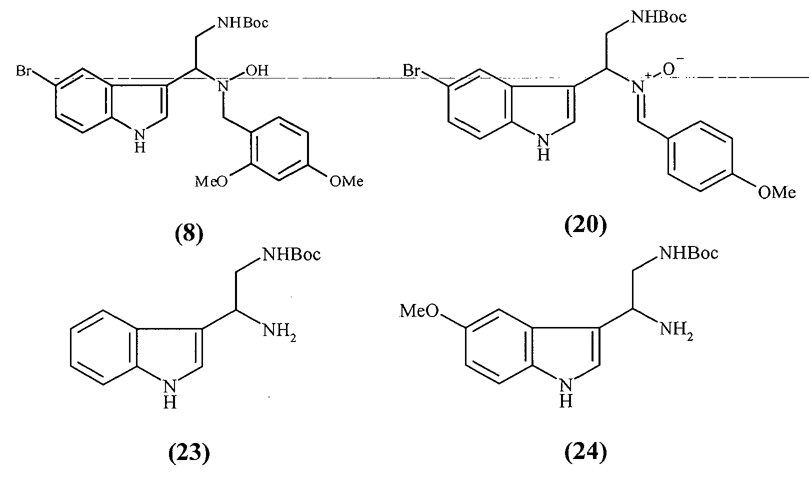

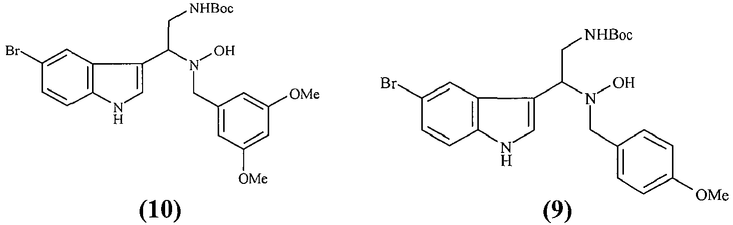

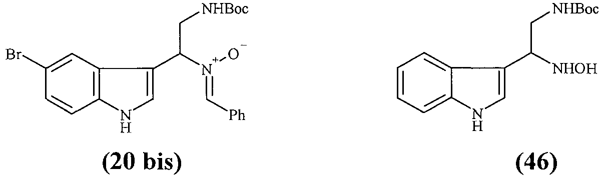

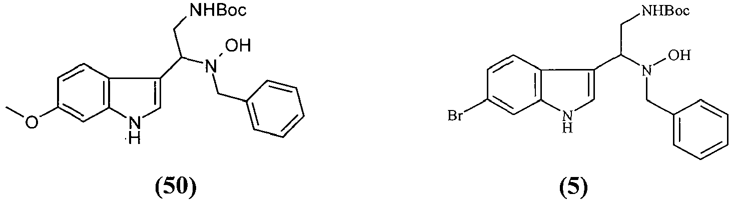

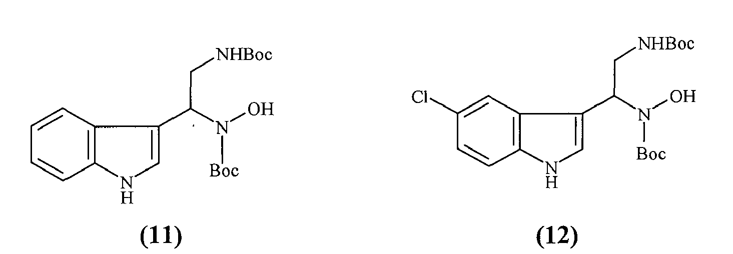

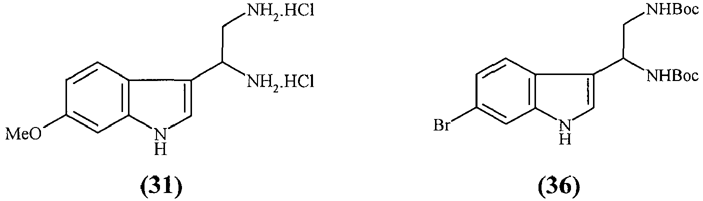

14. Composition pharmaceutique comprenant, en association avec un véhicule pharmaceutiquement acceptable :
- au moins un composé de formule (I) telle que définie dans l'une quelconque des revendications 1 à 12, et
- au moins un composé antibiotiquede la famille des fluoroquinolones, choisi parmi la ciprofloxacine, la norfloxacine, la péfloxacine, l'énofloxacine, l'ofloxacine, la lévofloxacine et la moxifloxacine,
ladite composition pharmaceutique étant utilisée pour une utilisation simultanée, séparée ou étalée dans le temps destiné au traitement de pathologies associées à des infections bactériennes vis-à-vis desquelles il existe une résistance aux antibactériens, ledit composé de formule (I) étant choisi parmi l'un des composés suivants:

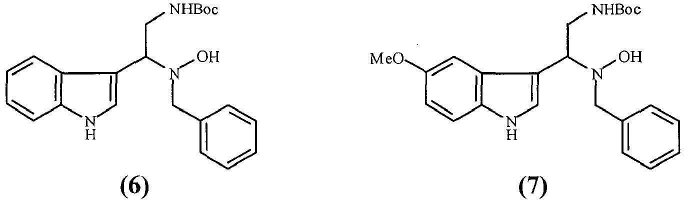

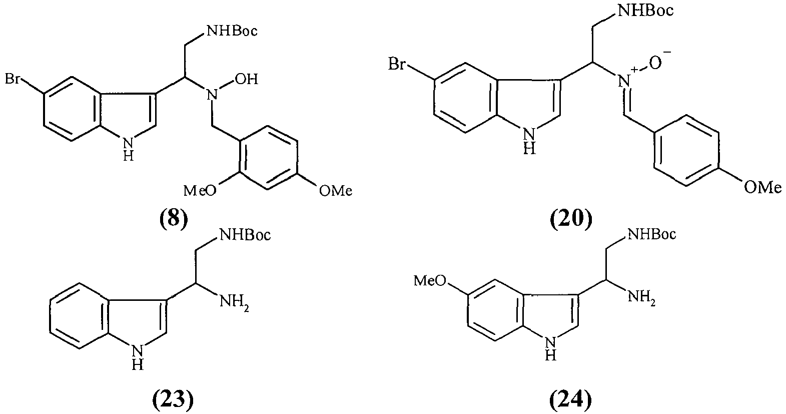

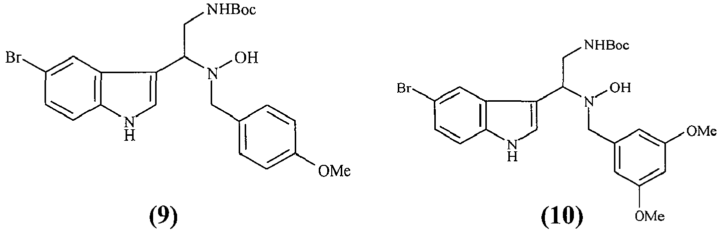

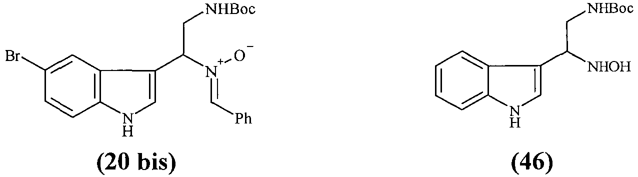

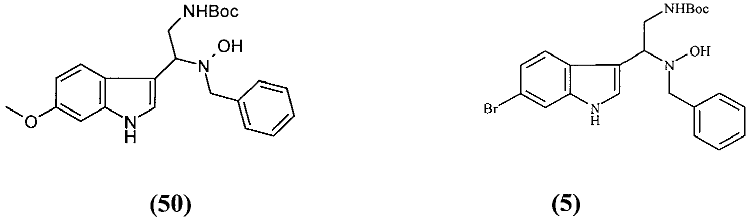

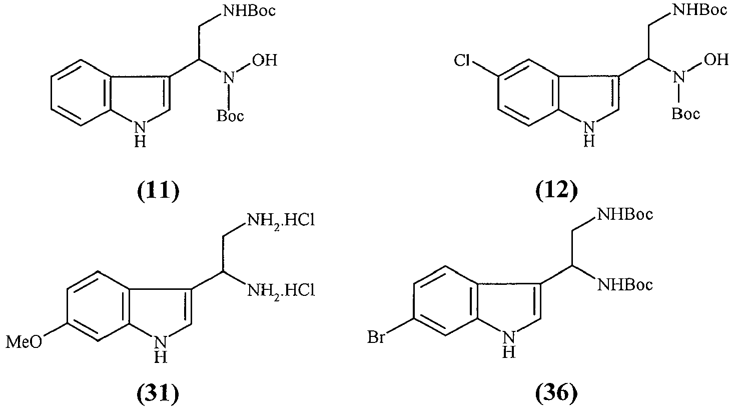

15. Composé de formule (I-6-a) suivante : 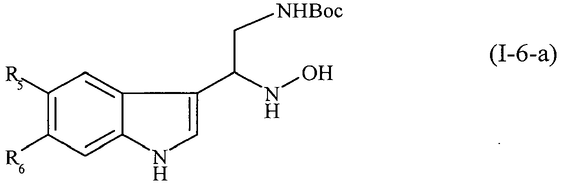 lesdits composés de formule (I-6-a) pouvant être sous forme d'isomères optiques, à savoir sous forme d'énantiomères ou de mélanges de ceséantiomères , y compris le mélange racémique, ou sous forme, le cas échéant, de sels d'acides physiologiquement acceptables choisis parmi les chlorhydrates, les formiates ou les oxalates (HOOCCOOH),
lesdits composés de formule (I-6-a) étant **caractérisés en ce que** :
- R₅ représente un atome d'hydrogène ou un atome de brome;
- R₆ représente un atome d'hydrogène ou un atome de brome.

16. Composé de formule (I-9-a) suivante : 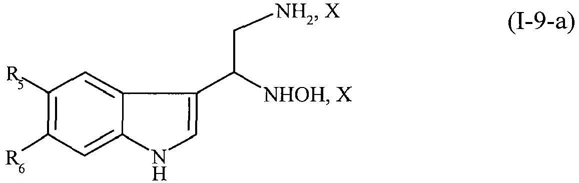 lesdits composés de formule (I-9-a) pouvant être sous forme d'isomères optiques, à savoir sous forme d'énantiomères ou de mélanges de ces énantiomères, y compris le mélange racémique, ou sous forme, le cas échéant, de sels d'acides physiologiquement acceptables choisis parmi les chlorhydrates, les formiates ou les oxalates (HOOCCOOH),
lesdits composés de formule (I-9-a) étant **caractérisés en ce que** :
- X représente HCl,
- R₅ représente un atome d'hydrogène ou un atome de brome;
- R₆ représente un atome d'hydrogène ou un atome de brome.

17. Composé de formule (I-10-a) suivante : 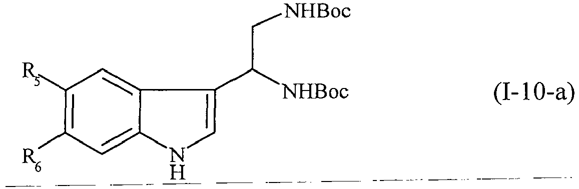 lesdits composés de formule (I-10-a) pouvant être sous forme d'isomères optiques, à savoir sous forme d'énantiomères ou de mélanges de ces énantiomères, y compris le mélange racémique,
lesdits composés de formule (I-10-a) étant **caractérisés en ce que**:
- R₅ représente un atome d'hydrogène ou un atome de brome;
- R₆ représente un atome d'hydrogène ou un atome de brome.

18. Composés répondant à l'une des formules suivantes:

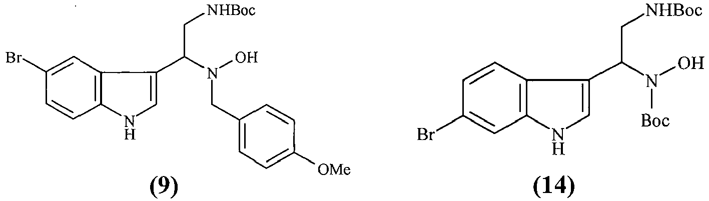

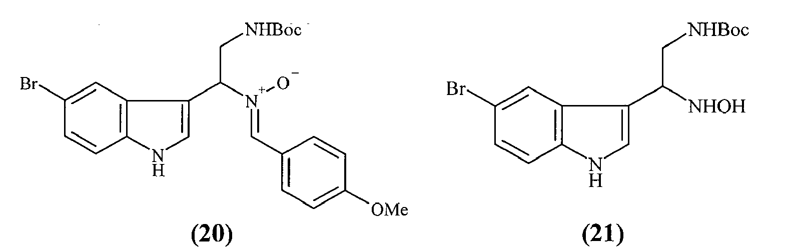

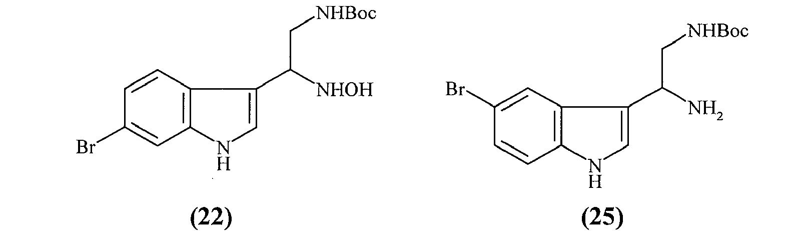

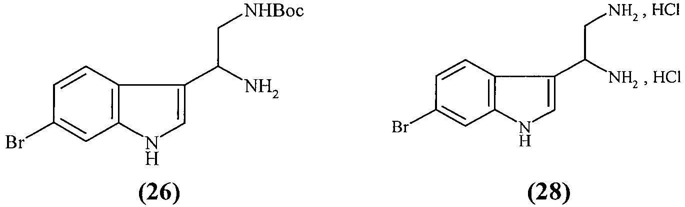

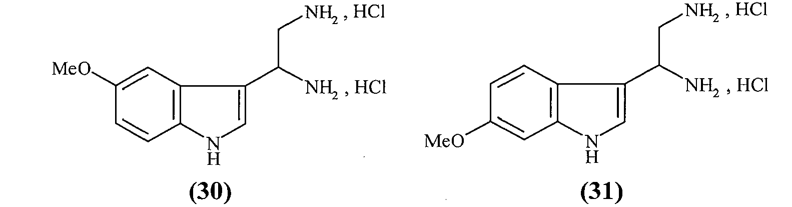

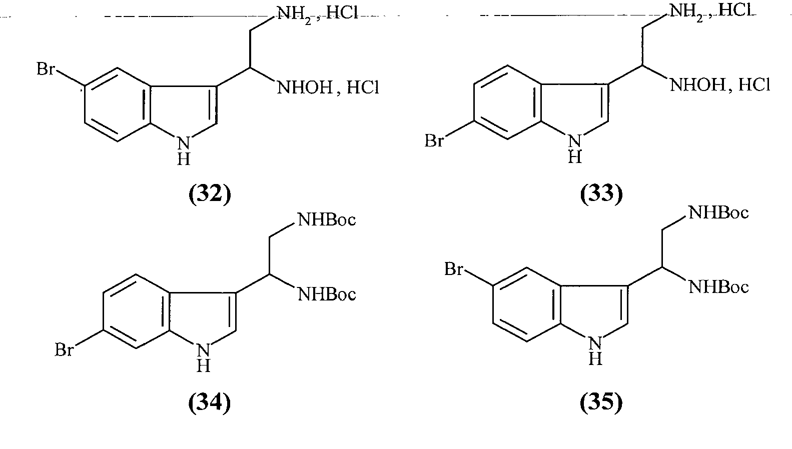

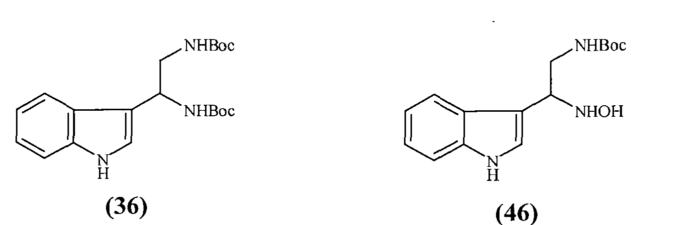

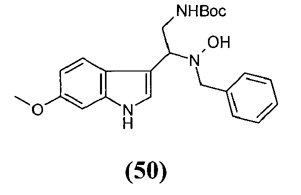

19. Composition pharmaceutique comprenant un composé selon l'une quelconque des revendications 15 à 18, en association avec un composé pharmaceutiquement acceptable.

20. Procédé de préparation d'un composé de formule (I-5-bis) suivante : 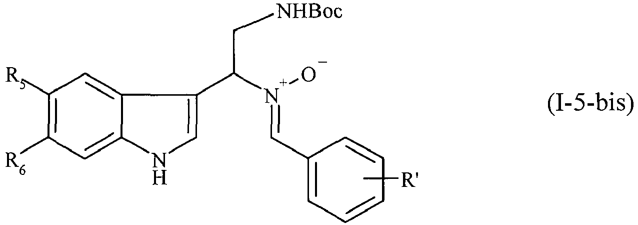 dans laquelle :
- R₅ et R₆ représentent indépendamment l'un de l'autre un groupe choisi parmi l'un des groupes suivants:
* un atome d'hydrogène;
* un groupe alcoxyle comprenant de 1 à 7 atomes de carbone ou un groupe benzyloxyle;
* un atome d'halogène choisi parmi Br, Cl, F ou I;
- R' représente un groupe choisi parmi l'un des groupes suivants:
* un atome d'hydrogène;
* un groupe alcoxyle comprenant de 1 à 4 atomes de carbone;
ledit procédé étant **caractérisé en ce qu'**il comprend une étape d'oxydation avec MnO₂ dans le toluène ou avec l'AMCPB dans le dichlorométhane ou de l'eau de javel dans le mélange DCM/eau, à 100°C, d'un composé de formule (I-1-bis) suivante: 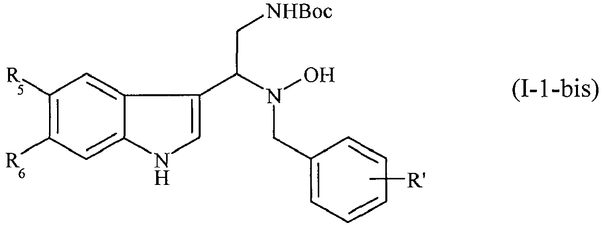 R₅, R₆ et R' étant tels que définis ci-dessus.

21. Procédé de préparation d'un composé de formule (I-6-a) suivante : 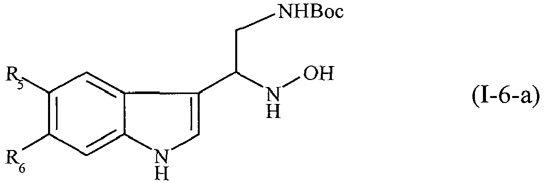 dans laquelle R₅ et R₆ représentent indépendamment l'un de l'autre un groupe choisi parmi l'un des groupes suivants :
* un atome d'hydrogène ;
* un groupe alcoxyle comprenant de 1 à 7 atomes de carbone ou un groupe benzyloxyle ;
* un atome d'halogène choisi parmi Br, Cl, F ou I ;
comprenant une étape de traitement avec NH₂OH.HCl (chlorhydrate d'hydroxylamine), dans le méthanol à température ambiante, d'un composé de formule (I-5-bis) suivante: 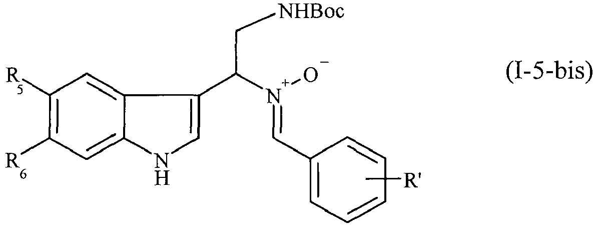
R₅ et R₆ étant tels que définis ci-dessus, et
R' représentant un groupe choisi parmi l'un des groupes suivants:
* un atome d'hydrogène;
* un groupe alcoxyle comprenant de 1 à 4 atomes de carbone;
le composé de formule (I-5-bis) étant obtenu par le procédé de préparation selon la revendication 20.

22. Procédé de préparation d'un composé de formule (I-7-a) suivante : 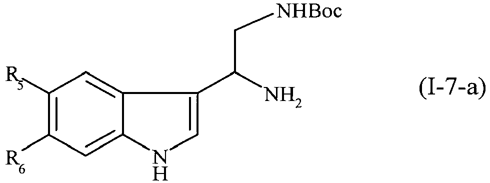 dans laquelle R₅ et R₆ représentent indépendamment l'un de l'autre un groupe choisi parmi l'un des groupes suivants:
* un atome d'hydrogène;
* un groupe alcoxyle comprenant de 1 à 7 atomes de carbone ou un groupe benzyloxyle;
* un atome d'halogène choisi parmi Br, Cl, F ou I ;
ledit procédé comprenant une étape de traitement avec TiCl₃/HCl, dans le méthanol à température ambiante, d'un composé de formule (I-6-a) suivante: 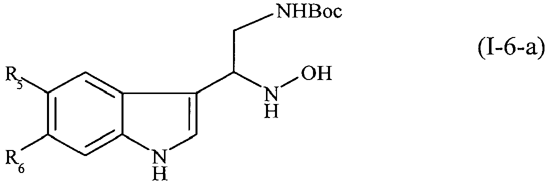 R₅ et R₆ étant tels que définis ci-dessus,
le composé de formule (I-6-a) étant obtenu par le procédé de préparation selon la revendication 21.

23. Procédé de préparation d'un composé de formule (I-8-a) suivante: 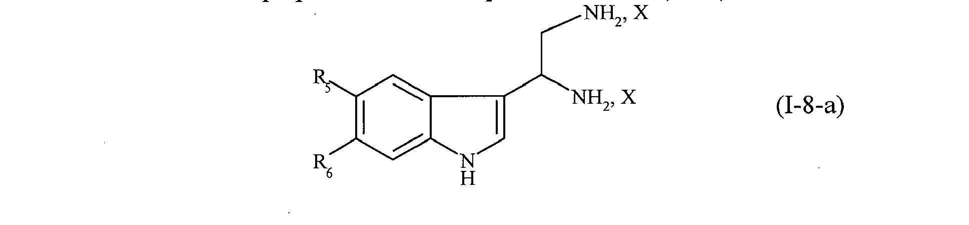 dans laquelle R₅ et R₆ représentent indépendamment l'un de l'autre un groupe choisi parmi l'un des groupes suivants:
* un atome d'hydrogène;
* un groupe alcoxyle comprenant de 1 à 7 atomes de carbone ou un groupe benzyloxyle;
* un atome d'halogène choisi parmi Br, Cl, F ou I;
ledit procédé comprenant une étape de traitement avec HCl dans le MeOH d'un composé de formule (I-7-a) suivante: 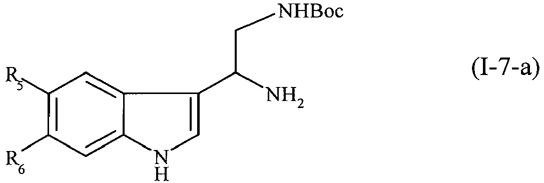 R₅ et R₆ étant tels que définis ci-dessus,
le composé de formule (I-7-a) étant obtenu par le procédé de préparation selon la revendication 22.

24. Procédé de préparation d'un composé de formule (I-9-a) suivante : 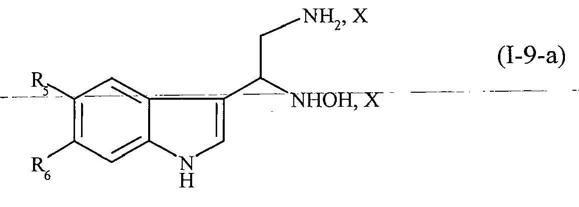 dans laquelle :
- X représente HCl, HCOOH ou HOOCCOOH;
- R₅ et R₆ représentent indépendamment l'un de l'autre un groupe choisi parmi l'un des groupes suivants:
* un atome d'hydrogène;
* un groupe alcoxyle comprenant de 1 à 7 atomes de carbone ou un groupe benzyloxyle;
* un atome d'halogène choisi parmi Br, Cl, F ou I;
ledit procédé comprenant une étape de traitement avec HCl sec, dans le méthanol à température ambiante, d'un composé de formule (I-6-a) suivante: 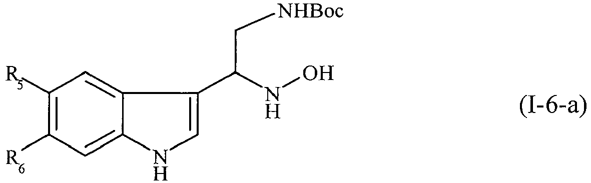 R₅ et R₆ étant tels que définis ci-dessus,
le composé de formule (I-6-a) étant obtenu par le procédé de préparation selon la revendication 21.

## Patentansprüche

1. Verwendung mindestens einer Verbindung mit der folgenden Formel (I): 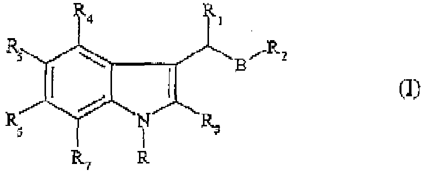 wobei:
- R für ein Wasserstoffatom steht;
- R₃ für ein Wasserstoffatom steht;
- R₄ und R₇ für ein Wasserstoffatom stehen;
- R₅ und R₆ unabhängig voneinander für eine Gruppe stehen, ausgewählt aus einer der folgenden Gruppen:
* einem wasserstoffatom;
* einer Alkoxylgruppe, die 1 bis 7 Kohlenstoffatome umfasst, oder einer Benzyloxylgruppe;
* einem Halogenatom, ausgewählt aus Br, Cl, F oder I;
- R₁ für eine Gruppe steht, ausgewählt aus einer der folgenden Gruppen:
* einer Gruppe -CH₂-NH-GP, wobei GP für eine Gruppe steht, ausgewählt aus den folgenden Gruppen: Boc, Cbz oder Me₃SiCH₂CH₂OCO (Teoc) ;
* einer Gruppe -CH₂-NH₂ oder -CH₂-NH₂.X, wobei X für HCl, HCOOH oder HOOCCOOH steht;
- R₂ für eine Gruppe steht, ausgewählt aus einer der folgenden Gruppen:
* einem Wasserstoffatom;
* einer Gruppe O⁻;
* einer Gruppe OH;
* einer Acylgruppe, die 1 bis 10 Kohlenstoffatome umfasst;
- B für eine Gruppe steht, ausgewählt aus einer der folgenden Gruppen: 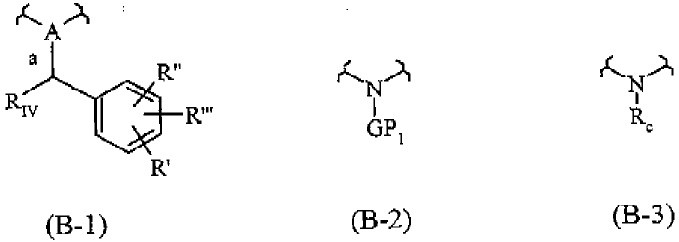
* wobei a für eine Einfachbindung oder eine Doppelbindung steht;
* A für N oder N⁺ steht;
. a für eine Einfachbindung steht, wenn A für N steht;
. a für eine Doppelbindung steht, wenn A für N⁺ steht und R₂ für O⁻ steht;
* R' R'' und R''' unabhängig voneinander für eine Gruppe stehen, ausgewählt aus den folgenden Gruppen:
* einem Wasserstoffatom;
* einer Alkoxylgruppe, die 1 bis 4 Kohlenstoffatome umfasst;
* R_{IV} für ein Wasserstoffatom steht,
* GP₁ für eine Gruppe Boc oder Cbz steht;
* R_{c} für ein Wasserstoffatom steht;
wobei die Verbindungen mit der Formel (I) in Form von optischen Isomeren vorliegen können, und zwar in Form von Enantiomeren oder von Gemischen dieser Enantiomere, einschließlich des racemischen Gemischs, oder gegebenenfalls in Form von Salzen physiologisch verträglicher Säuren,
zur Herstellung eines Medikaments, das zur Behandlung von Pathologien bestimmt ist, die mit bakteriellen Infektionen assoziiert sind.

2. Verwendung nach Anspruch 1 einer Verbindung mit der folgenden Formel (1-1): 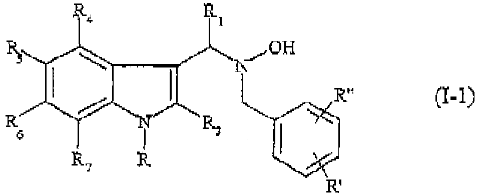 wobei die Verbindung mit der Formel (I-1) **dadurch gekennzeichnet ist, dass**:
- R, R₃, R₄ und R₇ für ein Wasserstoffatom stehen;
- R₅ für ein Wasserstoffatom, eine Methoxylgruppe, ein Bromatom oder ein Chloratom steht;
- R₆ für ein Wasserstoffatom oder ein Bromatom steht;
- R₁ für CH₂NHBoc steht,;
- R' und R'' unabhängig voneinander für ein Wasserstoffatom, oder eine Methoxylgruppe stehen oder R' und R" gemeinsam für eine *o,p-*Dimethoxylgruppe oder eine Di-*m*-methoxylgruppe stehen.

3. Verwendung nach Anspruch 1 einer Verbindung mit der folgenden Formel (I-2-bis) : 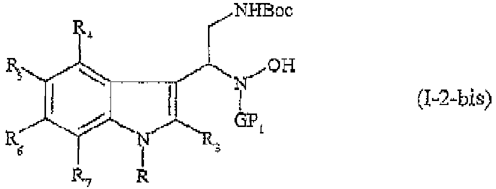 wobei GP₁ für eine Boc-Gruppe steht, wobei die Verbindung mit der Formel (I-2-bis) **dadurch gekennzeichnet ist, dass**:
- R, R₃, R₄ und R₇ für ein Wasserstoffatom stehen;
- R₅ für ein Wasserstoffatom, ein Bromatom oder ein Chloratom steht;
- R₆ für ein Wasserstoffatom, oder ein Bromatom steht.

4. Verwendung nach Anspruch 1 einer Verbindung mit der folgenden Formel (I-4) : 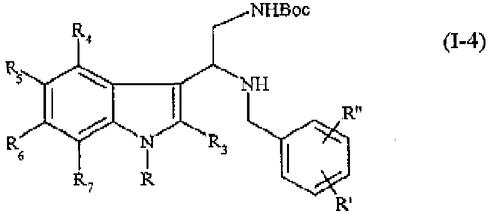 wobei:
- R' und R" für ein Wasserstoffatom, stehen;
- R, R₃, R₄ und R₇ für ein Wasserstoffatom stehen;
- R₅ für ein Wasserstoffatom, eine Methoxygruppe oder ein Bromatom steht;
- R₆ für ein Wasserstoffatom oder ein Bromatom steht.

5. Verwendung nach Anspruch 1 einer Verbindung mit der folgenden Formel (I), wobei:
- R₂ für O" steht;
- B für eine Gruppe mit der Formel (B-1) steht, worin R_{IV} = H;
- A für N⁻ steht; und
- a für eine Doppelbindung steht;
- R, R_{1,} R_{3,} R₄, R₅, R₆, R₇, R', R'' und R''' so
- sind, wie R₃, in Anspruch R₆, definiert. R'' und R''' so sind, wie in Anspruch 1 definiert,
wobei die Verbindungen der Formel (1-5') entsprechen:

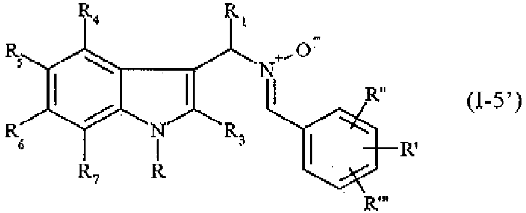

6. Verwendung nach Anspruch 1 einer Verbindung mit der folgenden Formel (I-5) : 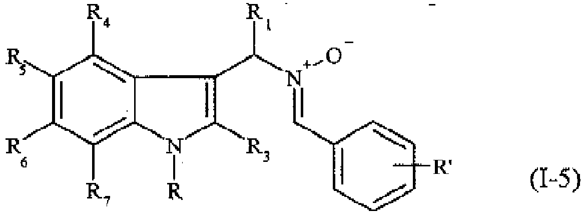 wobei die Verbindung mit der Formel (I-5) **dadurch gekennzeichnet ist, dass**:
- R' für ein Wasserstoffatom oder eine *p-*Methoxylgruppe steht;
- R, R₃, R₄ und R₇ für ein Wasserstoffatom stehen;
- R₁ für eine CH₂NHBoc-Gruppe steht;
- R₅ für ein Wasserstoffatom oder ein Bromatom steht;
- R₆ für ein Wasserstoffatom, oder ein Bromatom steht.

7. Verwendung nach Anspruch 1 einer Verbindung mit der folgenden Formel (I-6) : 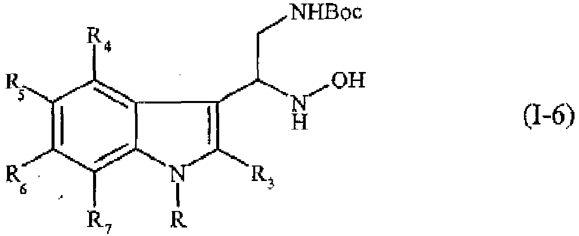 wobei die verbindungen mit der Formel (1-6) gegebenenfalls in Form von physiologisch verträglichen Salzen vorliegen können, ausgewählt aus Hydrochloriden, Formiaten oder Oxalaten,
und **dadurch gekennzeichnet sind, dass**:
- R, R₃, R₄ und R₇ für ein Wasserstoffatom stehen;
- R₅ für ein Wasserstoffatom, oder ein Bromatom steht;
- R₆ für ein wasserstoffatom oder ein Bromatom steht.

8. Verwendung nach Anspruch 1 einer Verbindung mit der folgenden Formel (I-7) : 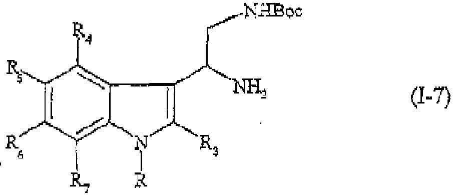 wobei die Verbindungen mit der Formel (I*-*7) gegebenenfalls in Form von physiologisch verträglichen Salzen vorliegen können, ausgewählt aus Hydrochloriden, Formiaten oder Oxalaten,
wobei die Verbindung mit der Formel (1-7) **dadurch gekennzeichnet ist, dass**:
- R, R₃, R₄ und R₇ für ein Wasserstoffatom stehen;
- R₅ für ein Wasserstoffatom, eine Methoxylgruppe oder ein Bromatom steht;
- R₆ für ein Wasserstoffatom, eine Methoxylgruppe oder ein Bromatom steht.

9. Verwendung nach Anspruch 1 einer Verbindung mit der folgenden Formel (I-8) : 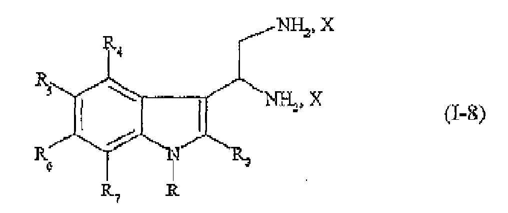 wobei;
- R, R₃, R₄, R₅, R₆ und R₇ so sind, wie in Anspruch 1 definiert; und
- X für HCl, HCOOH oder HOOCCOOH steht.

10. Verwendung nach Anspruch 1 einer Verbindung mit der folgenden Formel (I-9): 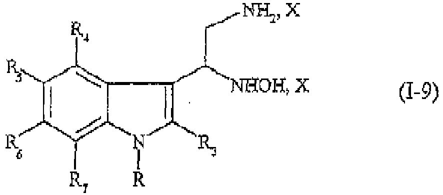 wobei :
- R, R₃, R_{4,} R₅, R₆ und R₇ so sind, wie in Anspruch 1 definiert; und
- X für HCl, HCOOH oder HOOCCOOH steht.

11. Verwendung nach Anspruch 1 einer Verbindung mit der folgenden Formel (I-10) : 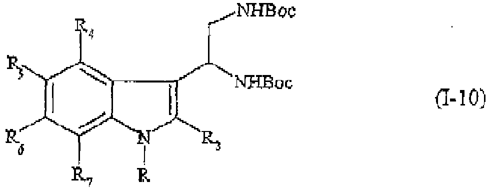 wobei die Verbindung mit der Formel (I-10) **dadurch gekennzeichnet ist, dass**:
- R, R₃, R₄ und R₇ für ein Wasserstoffatom stehen;
- R₅ für ein wasserstoffatom, ein Bromatom oder eine Methoxylgruppe steht;
- R₆ für ein Wasserstoffatom, ein Bromatom oder eine Methoxylgruppe steht.

12. Verwendung nach einem der Ansprüche 1 bis 11 einer Verbindung, die einer der folgenden Formeln entspricht:

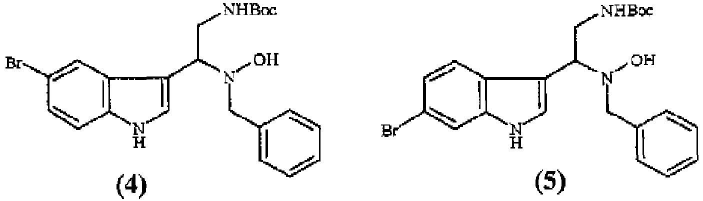

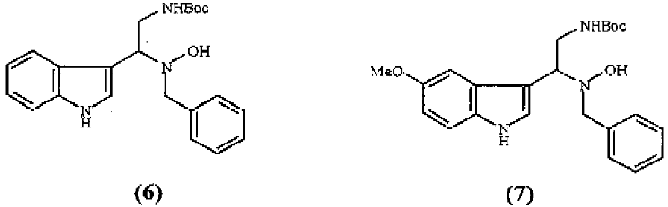

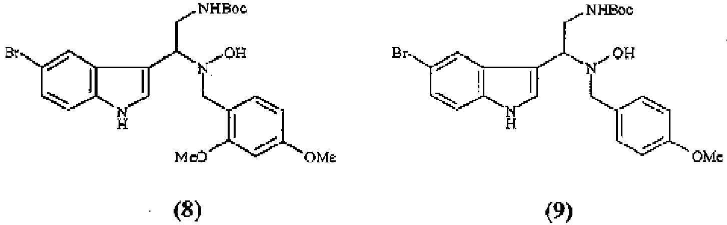

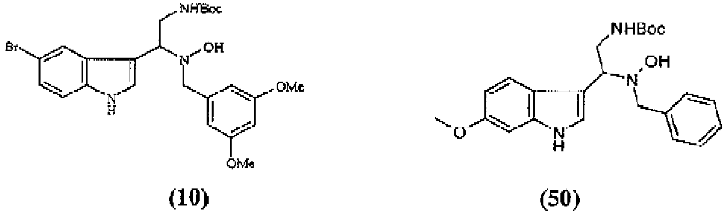

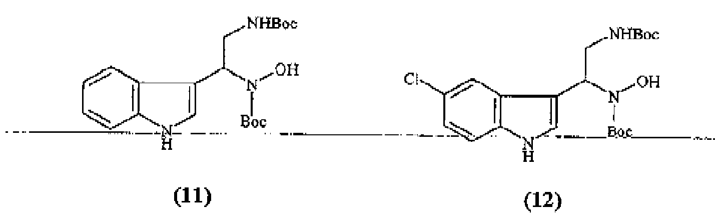

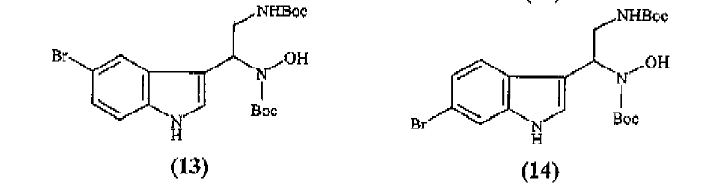

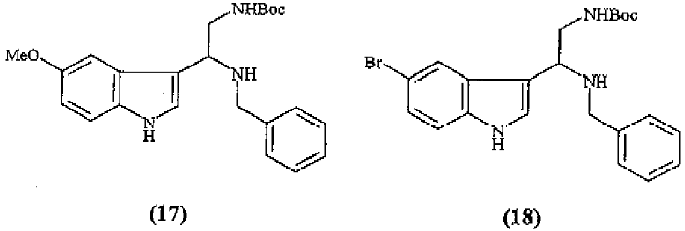

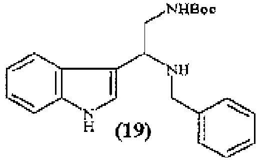

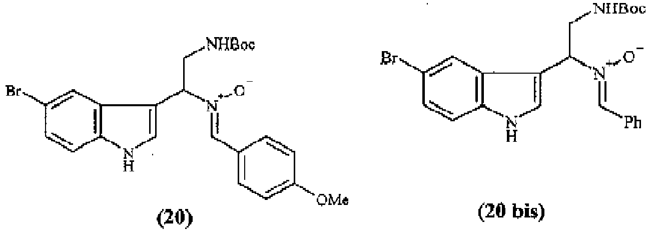

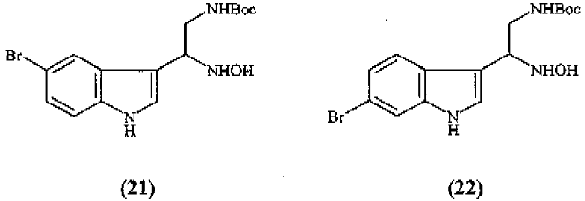

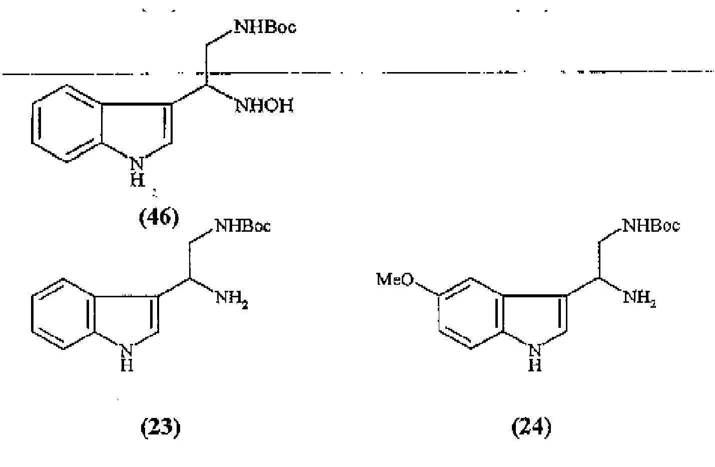

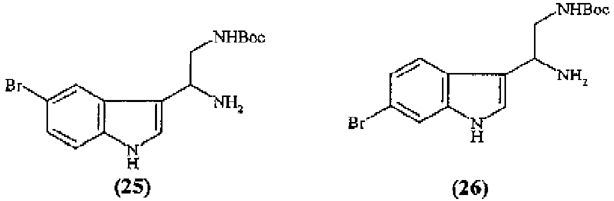

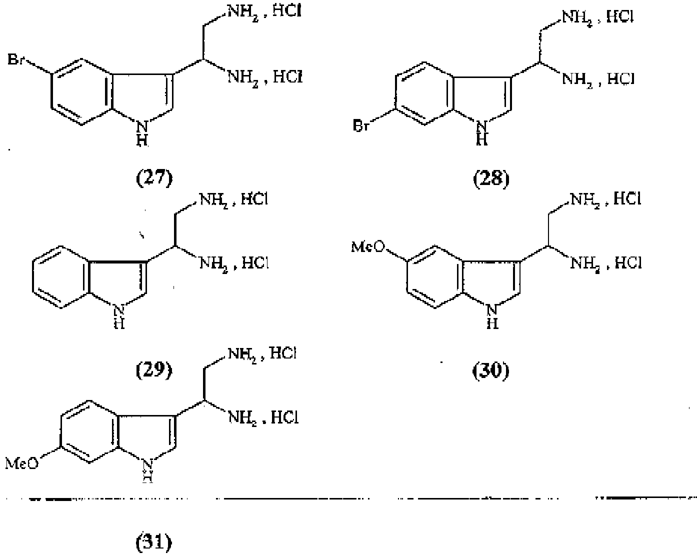

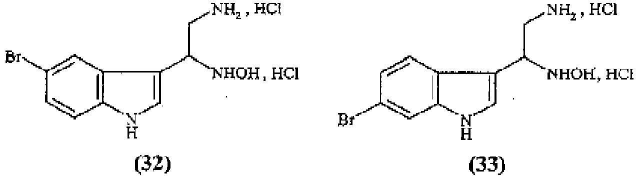

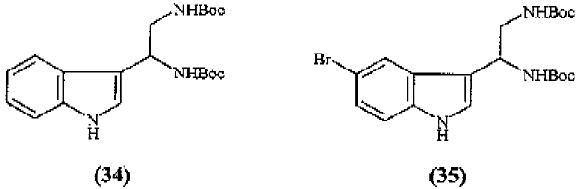

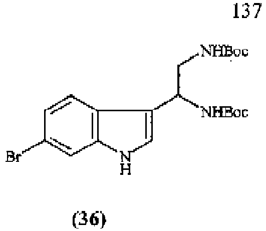

13. Verwendung nach einem der Ansprüche 1 bis 12 mindestens einer Verbindung mit der Formel (I), in Assoziation mit einem Antibiotikum der Familie der Fluorchinolone, ausgewählt aus Ciprofloxacin, Norfloxacin, Pefloxacin, Enofloxacin, Ofloxacin, Levofloxacin und Moxifloxacin, zur Behandlung von Pathologien, die mit bakteriellen Infektionen assoziiert sind, bei denen eine Resistenz gegen antibakterielle Mittel vorhanden ist,
wobei die Verbindung mit der Formel (I) aus einer der folgenden Verbindungen ausgewählt ist:

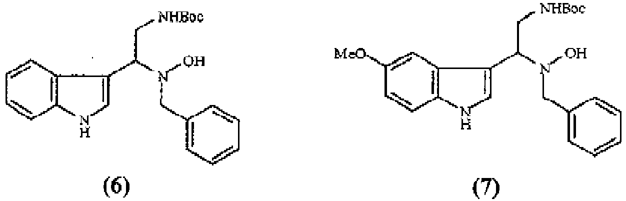

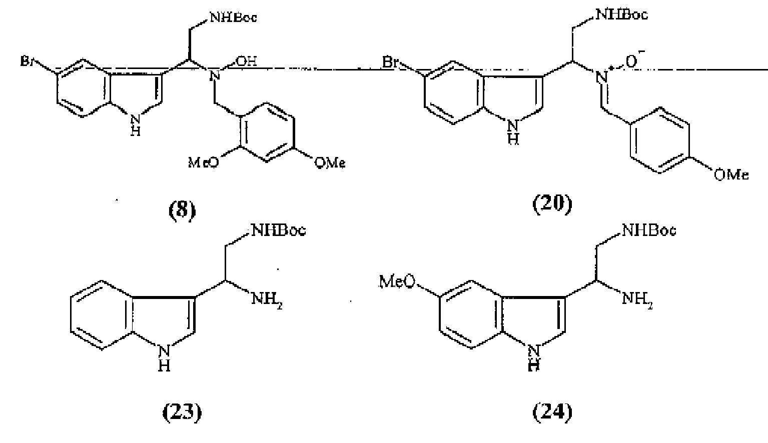

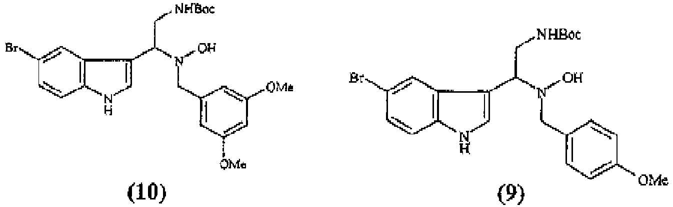

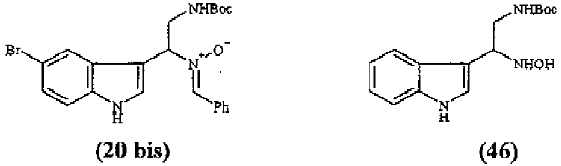

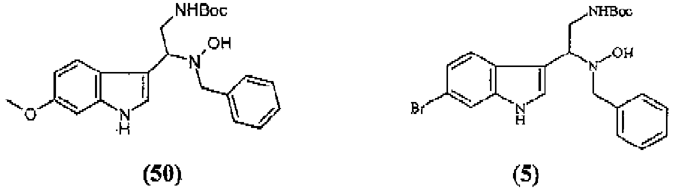

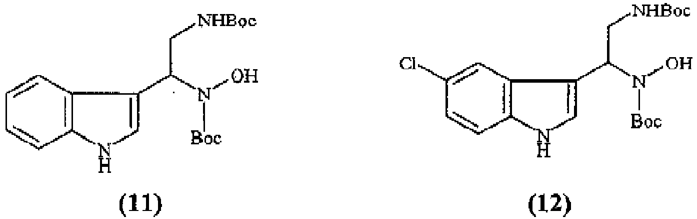

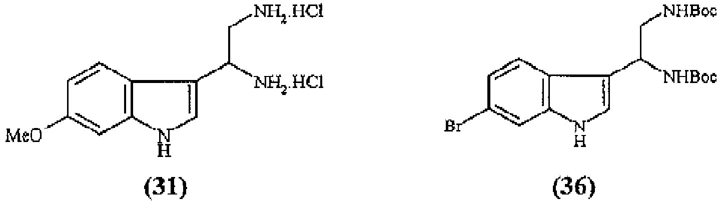

14. Pharmazeutische Zusammensetzung, umfassend, in Assoziation mit einem pharmazeutisch verträglichen Vehikel:
- mindestens eine Verbindung mit der Formel (I), wie in einem der Ansprüche 1 bis 12 definiert, und
- mindestens eine antibiotische Verbindung der Familie der Fluorchinolone, ausgewählt aus Ciprofloxacin, Norfloxacin, Pefloxacin, Enofloxacin, Ofloxacin, Levofloxacin und Moxifloxacin,
wobei die pharmazeutische Zusammensetzung für eine gleichzeitige, getrennte oder verzögerte Verwendung verwendet wird, die zur Behandlung von Pathologien bestimmt ist, die mit bakteriellen Infektionen assoziiert sind, bei denen eine Resistenz gegen antibakterielle Mittel vorhanden ist, wobei die Formel (I) aus einer der folgenden Verbindungen ausgewählt ist:

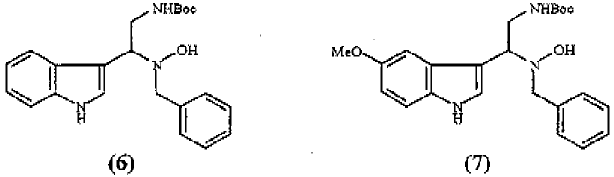

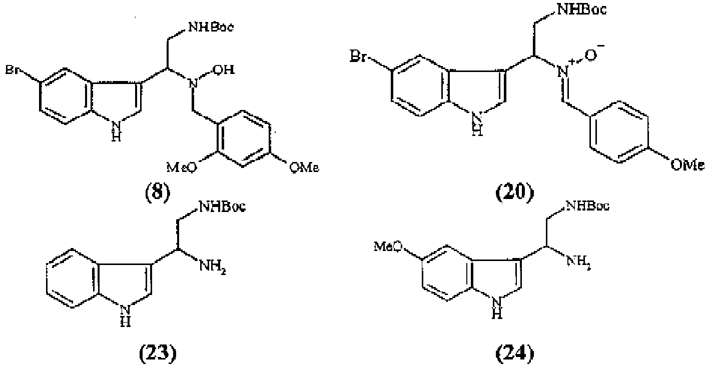

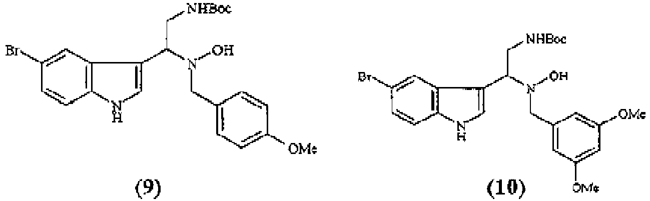

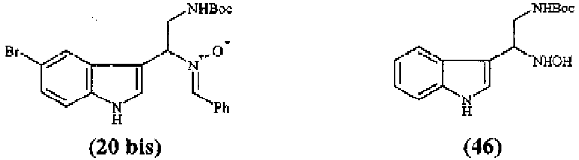

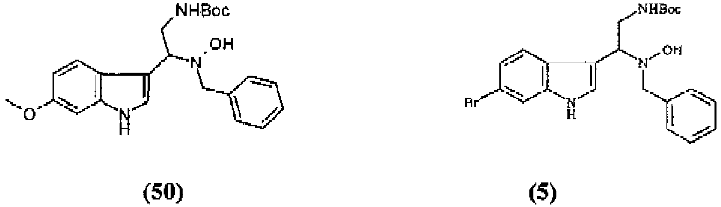

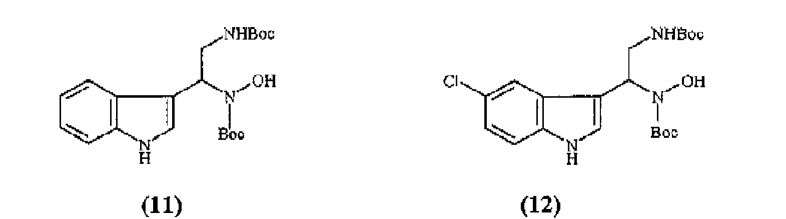

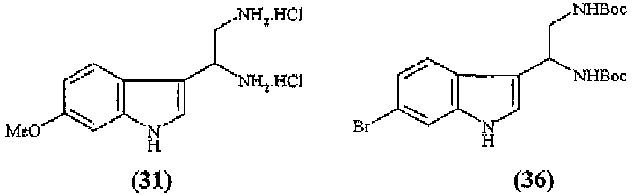

15. Verbindung mit der folgenden Formel (I-6-a): 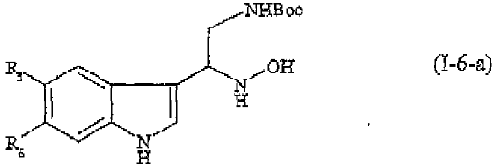 wobei die Verbindungen mit der Formel (I-6-a) in Form von optischen Isomeren vorliegen können, und zwar in Form von Enantiomeren oder von Gemischen dieser Enantiomere, einschließlich des racemischen Gemischs, oder gegebenenfalls in Form von Salzen physiologisch verträglicher Säuren, ausgewählt aus Hydrochloriden, Formiaten oder Oxalaten (HOOCCOOH),
wobei die Verbindung mit der Formel (I-6-a) **dadurch gekennzeichnet ist, dass**:
- R₅ für ein Wasserstoffatom oder ein Bromatom steht;
- R₆ für ein Wasserstoffatom oder ein Bromatom steht.

16. Verbindung mit der folgenden Formel (I-9-a): 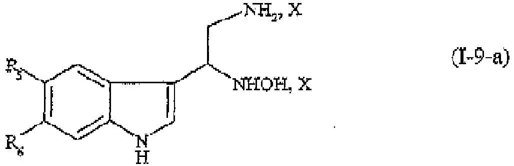 wobei die Verbindungen mit der Formel (I-9-a) in Form von optischen Isomeren vorliegen können, und zwar in Form von Enantiomeren oder von Gemischen dieser Enantiomere, einschließlich des racemischen Gemischs, oder gegebenenfalls in Form von Salzen physiologisch verträglicher Säuren, ausgewählt aus Hydrochloriden, Formiaten oder Oxalaten (HOOCCOCH),
wobei die Verbindung mit der Formel (I-9-a) **dadurch gekennzeichnet ist, dass**:
- X für HCl steht,
- R₅ für ein Wasserstoffatom oder ein Bromatom steht;
- R₆ für ein Wasserstoffatom oder ein Bromatom steht.

17. Verbindung mit der folgenden Formel (I-10-a) : 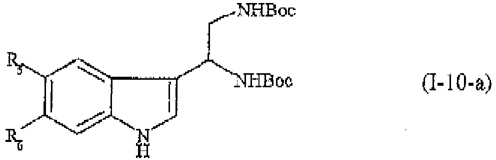 wobei die Verbindungen mit der Formel (I-10-a) in Form von optischen Isomeren vorliegen können, und zwar in Form von Enantiomeren oder Gemischen dieser Enantiomere, einschließlich des racemischen Gemischs, wobei die Verbindung mit der Formel (I-10-a) **dadurch gekennzeichnet ist, dass**:
- R₅ für ein Wasserstoffatom oder ein Bromatom steht;
- R₆ für ein Wasserstoffatom oder ein Bromatom steht.

18. Verbindungen, die einer der folgenden Formeln entsprechen:

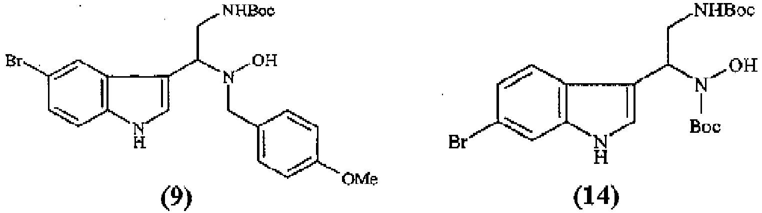

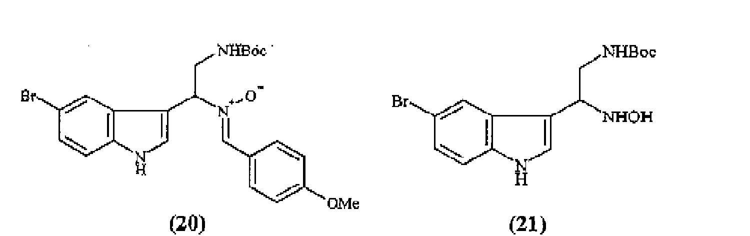

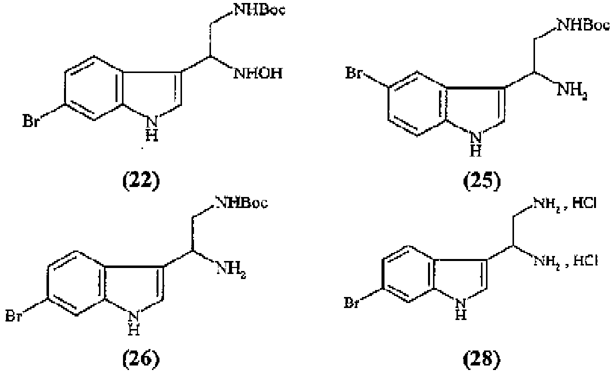

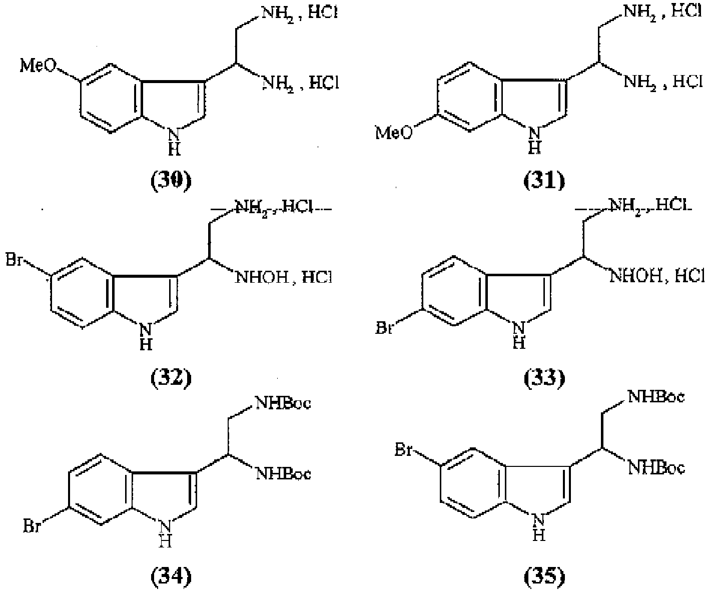

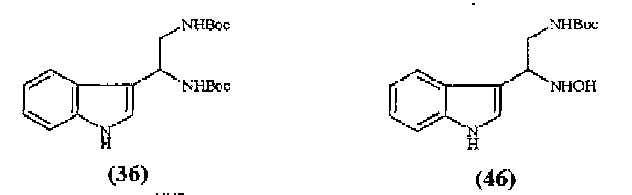

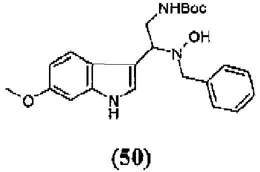

19. Pharmazeutische Zusammensetzung, umfassend eine Verbindung nach einem der Ansprüche 15 bis 18 in Assoziation mit einer pharmazeutisch verträglichen Verbindung.

20. Verfahren zur Herstellung einer Verbindung mit der folgenden Formel (I-5-bis) : 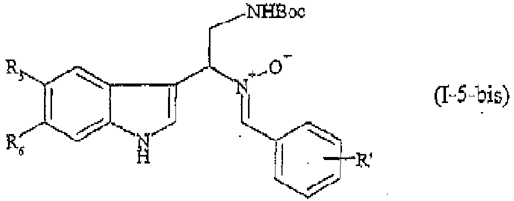 wobei:
- R₅ und R₆ unabhängig voreinander für eine Gruppe stehen, ausgewählt aus einer der folgenden Gruppen:
* einem Wasserstoffatom;
* einer Alkoxylgruppe, die 1 bis 7 Kohlenstoffatome umfasst, oder einer Benzyloxylgruppe;
* einem Halogenatom, ausgewählt aus Br, Cl, F oder I;
- R' für eine Gruppe steht, ausgewählt aus einer der folgenden Gruppen:
* einem wasserstoffatom;
* einer Alkoxylgruppe, die 1 bis 4 Kohlenstoffatome umfasst;
wobei das Verfahren **dadurch gekennzeichnet ist, dass** es einen Schritt zur Oxidation mit MnO₂ in Toluol oder mit AMCPB in Dichlormethan oder Javelwasser in dem DCM/Wasser-Gemisch bei 100 °C einer Verbindung mit der folgenden Formel (I-1-bis) umfasst: 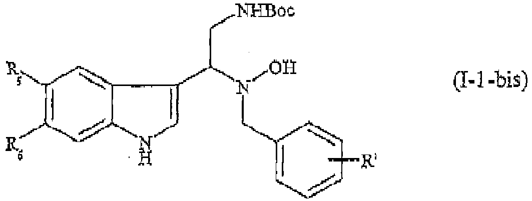 wobei R₅, R₆ und R' so sind, wie oben definiert.

21. Verfahren zur Herstellung einer Verbindung mit der folgenden Formel (I-6-a) : 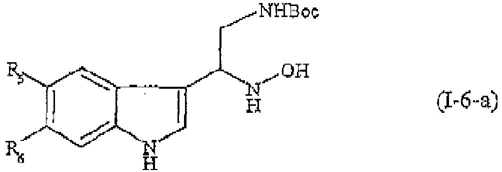 wobei R₅ und R₆ unabhängig voneinander für eine Gruppe stehen, ausgewählt aus einer der folgenden Gruppen;
* einem Wasserstoffatom;
* einer Alkoxylgruppe, die 1 bis 7 Kohlenstoffatome umfasst, oder einer Benzyloxylgruppe;
* einem Halogenatom, ausgewählt aus Br, Cl, F oder I;
umfassend einen Schritt zur Behandlung mit NH₂OH.HCl (Hydroxylaminhydrochlorid) in Methanol bei Umgebungstemperatur einer Verbindung mit der folgenden Formel (I-5-bis) : 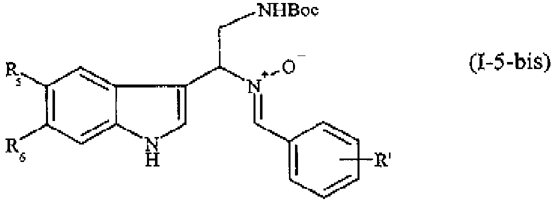 wobei R₅ und R₆ so sind, wie oben definiert, und
R' für eine Gruppe steht, ausgewählt aus einer der folgenden Gruppen:
* einem wasserstoffatom;
* einer Alkoxylgruppe, die 1 bis 4 Kohlenstoffatome umfasst;
wobei die Verbindung mit der Formel (I-5-bis) durch das Herstellungsverfahren nach Anspruch 20 erhalten wird.

22. Verfahren zur Herstellung einer Verbindung mit der folgenden Formel (I-7-a): 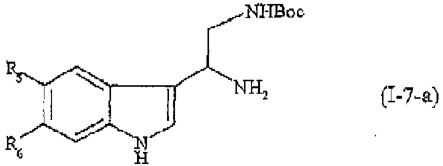 wobei R₅ und R₆ unabhängig voneinander für eine Gruppe stehen, ausgewählt aus einer der folgenden Gruppen:
* einem Wasserstoffatom;
* einer Alkoxygruppe, die 1 bis 7 Kohlenstoffatome umfasst, oder einer Benzyloxylgruppe;
* einem Halogenatom, ausgewählt aus Br, Cl, F oder I;
wobei das Verfahren einen Schritt zur Behandlung mit TiCl₃/HCl in Methanol bei Umgebungstemperatur einer Verbindung mit der folgenden Formel (I-6-a) umfasst: 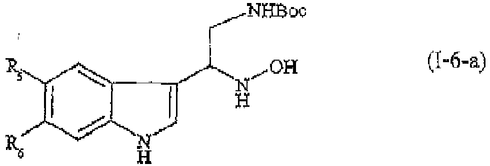 wobei R₅ und R₆ sind, wie oben definiert,
wobei die Verbindung mit der Formel (I-6-a) durch das Herstellungsverfahren nach Anspruch 21 erhalten wird.

23. Verfahren zur Herstellung einer Verbindung mit der folgenden Formel (I-8-a) : 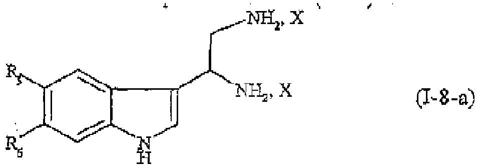 wobei R₅ und R₆ unabhängig voneinander für eine Gruppe stehen, ausgewählt aus einer der folgenden Gruppen:
* einem Wasserstoffatom;
* einer Alkoxylgruppe, die 1 bis 7 Kohlenstoffatome umfasst, oder einer Benzyloxylgruppe;
* einem Halogenatom, ausgewählt aus Br, Cl, F oder I;
wobei das Verfahren einen Schritt zur Behandlung mit HCl in MeOH einer Verbindung mit der folgenden Formel (1-7-a) umfasst: 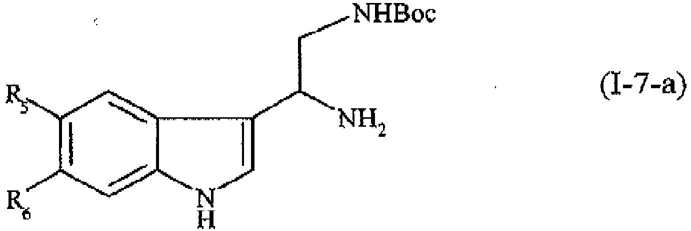 wobei R₅ und R₆ so sind, wie oben definiert,
wobei die Verbindung mit der Formel (I-7-a) durch das Herstellungsverfahren nach Anspruch 22 erhalten wird.

24. Verfahren zur Herstellung einer Verbindung mit der folgenden Formel (I-9-a): 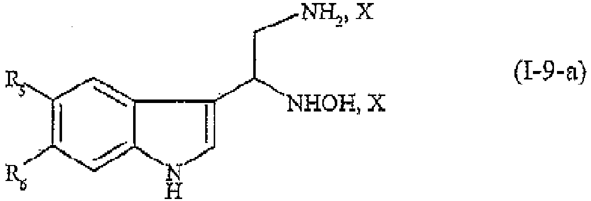 wobei:
- X für HCl, HCOOH oder HOOCCOOH steht;
- R₅ und R₆ unabhängig voneinander für eine Gruppe stehen, ausgewählt aus einer der folgenden Gruppen:
* einem Wasserstoffatom;
* einer Alkoxylgruppe, die 1 bis 7 Kohlenstoffatome umfasst, oder einer Benzyloxylgruppe;
* einem Halogenatom, ausgewählt aus Br, Cl, F oder I;
wobei das Verfahren einen Schritt zur Behandlung mit trockener HCl in Methanol bei Umgebungstemperatur einer Verbindung mit der folgenden Formel (I-6-a) umfasst: 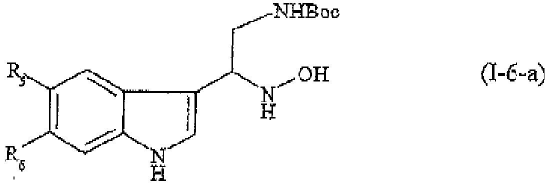 wobei R₅ und R₆ so sind, wie oben definiert,
wobei die Verbindung mit der Formel (I-6-a) durch das Herstellungsverfahren nach Anspruch 21 erhalten wird.

## Claims

1. Use of at least one compound of formula (1) below : 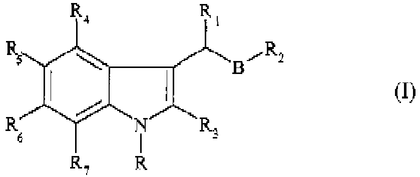 in which:
- R represents a hydrogen atom;
- R₃ represents a hydrogen atom;
- R₄ and R₇ repesent a hydrogen atom;
- R₅ and R₆ represent independently of each other a group chosen from one of the following groups:
* a hydrogen atom;
* a alkoxyl group comprising 1 to 7 carbon atoms or a benzyloxyl group;
* a halogen atom chosen from Br, Cl, F or I;
- R₁ represents a group chosen from one of the following groups:
* a -CH₂.NH-GP group, GP representing a group chosen from the following groups : Boc. Cbz, or Me₃SiCH₂CH₂OCO (Teoc);
* a -CH₂-NH₂ or -CH₂-NH₂.X group, X representing HCl, HCOOH or HOOCCOOH;
- R₂ represents a group chosen from one of the following groups :
* a hydrogen atom;
* an O⁻ group;
* an OH group;
* an acyl group comprising 1 to 10 carbon atoms ;
- B represents a group chosen from one of the following groups : 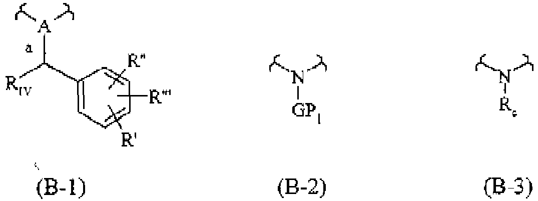
* a representing a single bond or a double bond;
* A representing N or N⁺;
. a representing a single bond when A represents N;
. a representing a double bond when A represents N⁺ and R₂ represents O";
* R', R" and R'" representing, independently of each other, a group chosen from one of the following groups:
* a hydrogen atom;
* an alkoxyl group comprising 1 to 4 carbon atoms;
* R_{IV} representing a hydrogen atom;
* GP₁ representing a Boc or Cbz group;
* R_{c} representing a hydrogen atom;
said compounds of formula (I) being possibly in the form of optical isomers, namely in the form of enantiomers or mixtures of enentiomers, including racemic mixtures, or in the form, if appropriate, of salts of physiologically acceptable acids,
for the preparation of a medicament intended for the treatment of pathologies associated with bacterial infections.

2. Use according to claim 1, of a compound of formula (I-1) below : 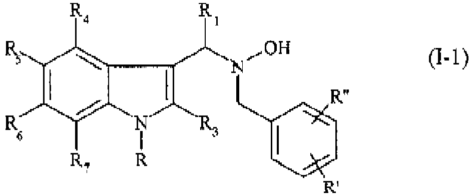 said compound of formula (1-1) being **characterized in that**:
- R, R₃, R₄ and R₇ represent a hydrogen atom;
- R₅ represents a hydrogen atom, a methoxyl group, a bromine atom or a chlorine atom;
- R₆ represents a hydrogen atom or a bromine atom;
- R₁ represents a CH₂NHBoc group;
- R' and R" represent independently of each other a hydrogen atom or a methoxyl group or R' and R" together represent a *o,p*-dimethoxyl group or a di-*m*-methoxyl group.

3. Use according to claim 1, of a compound of formula (I-2-bis) below: 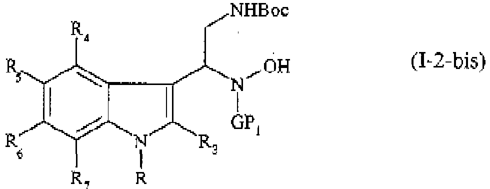 GP₁ representing a Boc group,
said compound of formula (I-2-bis) being **characterized in that**:
- R, R₃, R₄ and R₇ represent a hydrogen atom;
- R₅ represents a hydrogen atom, a bromine atom or a chlorine atom;
- R₆ represents a hydrogen atom or a bromine atom.

4. Use according to claim 1, of a campoud of formula (1-4) below : 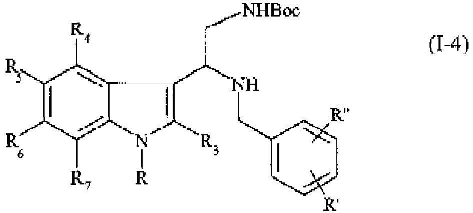 in which:
- R' and R" represent a hydrogen atom;
- R, R₃, R₄ and R₇ represent a hydrogen atom;
- R₅ represents a hydrogen atom, a methoxyl group or a bomine atom ;
- R₆ represents a hydrogen atom or a bromine atom.

5. Use according to claim 1, of a compoung of formula (I) in which :
- R₂ represents O⁻;
- B represents a group of formula (B-1) with R_{Iv} = H;
- A represents N⁺ ; and
- a represents a double bond.
- R, R_{1,} R₃, R₄, R₅, R₆, R₇, R', R" and R'" being as defined in claim 1, said compounds corresponding to the formula (I-5'):

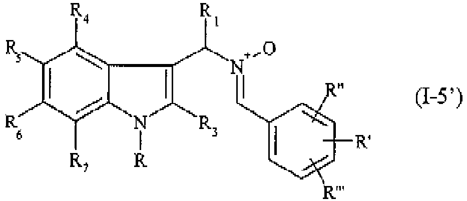

6. Use according to claim 1, of a compound of formula (I-5) below : 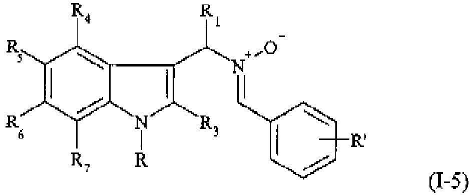 said compound of formula (I-5) being characteized in that:
- R' represents a hydrogen aom or a *p*-methoxyl group;
- R, R₃, R₄ and R₇ represent a hydrogen atom;
- R₁ represents a CH₂NHBoc group;
- R₅ represents a hydrogen atom or a bromine atom;
- R₆ represents a hydrogen atom or a bromine atom.

7. Use according to claim 1, of a compound of formula (1-6) below : 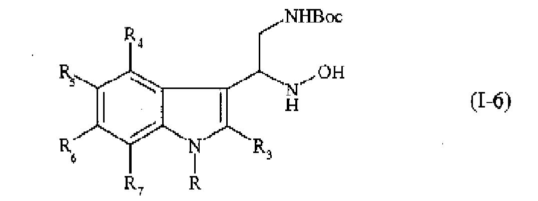 said compounds of formula (1-6) being possibly in the form, if appropriate, of physiologically acceptable salts chosen from hydrochlorides, formiates or oxalates,
and being **characterized in that**:
- R, R₃, R₄ and R₇ represent a hydrogen atom;
- R₅ represents a hydrogen atom or a bromine atom;
- R₆ represents a hydrogen atom or a bromine atom.

8. Use according to claim 1, of a compound of formula (1-7) below : 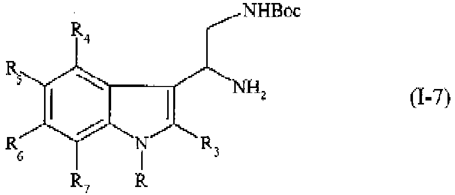 said compounds of formula (I-7) being possibly in the form, if appropriate, of physiologically acceptable salts chosen from hydrochlorides, formiates or oxalates, said compounds of formula (I-7) being **characterized in that**:
- R, R₃, R₄ and R₇ represent a hydrogen atom;
- R₅ represents a hydrogen atom, a methoxyl group or a bromine atom;
- R₆ represents a hydrogen atom, a methoxyl group or a bromine atom.

9. Use according to claim 1, of a compound of formula (I-8) below : in which : 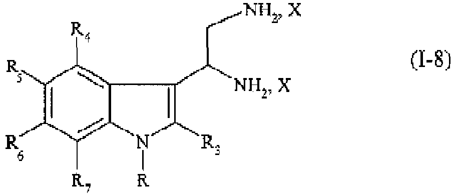
- R, R₃, R₄, R₅, R₆ and R₇ are as defined in claim 1 ; and
- X represents HCl, HCOOH or HOOCCOOH.

10. Use according to claim 1, of a compound of formula (1-9) below : in which : 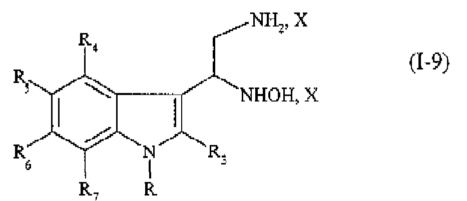
- R, R₃, R₄, R₅, R₆ and R₇ are as defined in claim 1; and
- X represents HCl, HCOOH or HOOCCOOH.

11. Use according to claim 1, of a compound of formula (I-10) below : 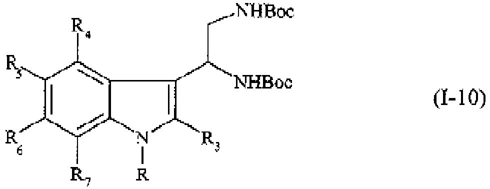 said compound of formula (I-10) being **characterised in that**:
- R, R₃, R₄ and R₇ represent a hydrogen atom;
- R₅ represents a hydrogen atom, a bromine atom or a methoxyl group ;
- R₆ represents a hydrogen atom, a bromine atom or a methoxyl group.

12. Use according to anyone of claims 1 to 11, of a compound corresponding to one of the following formulae :

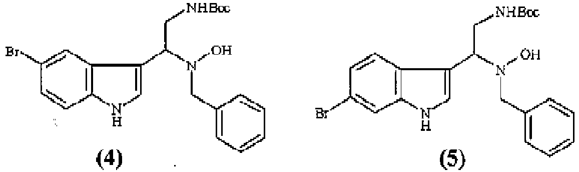

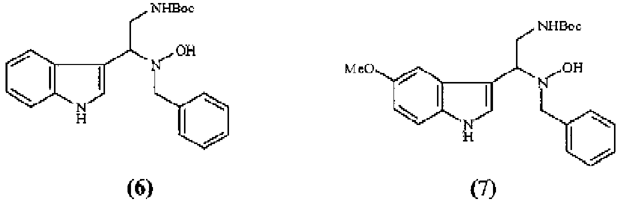

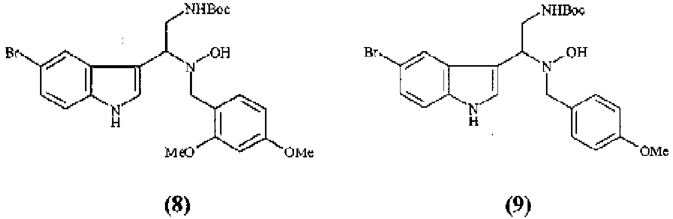

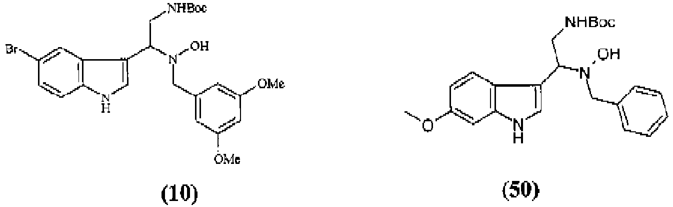

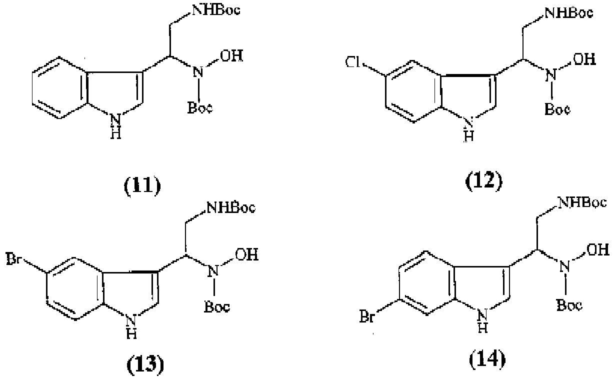

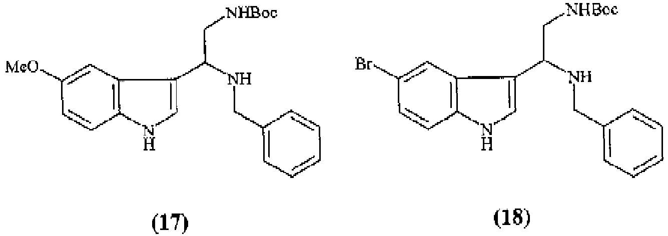

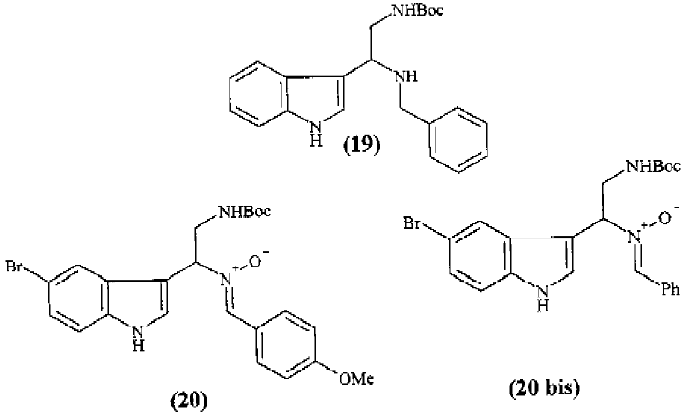

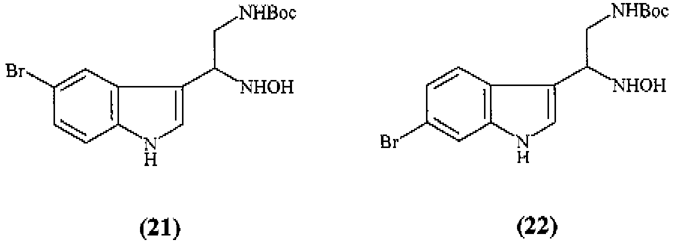

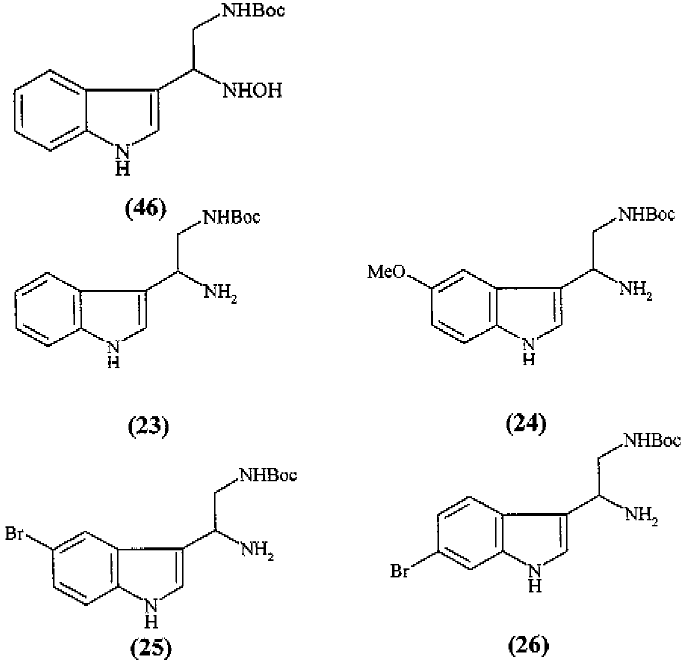

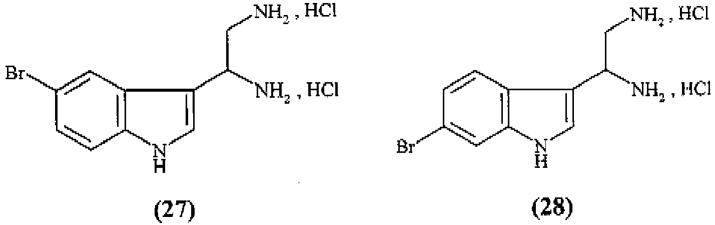

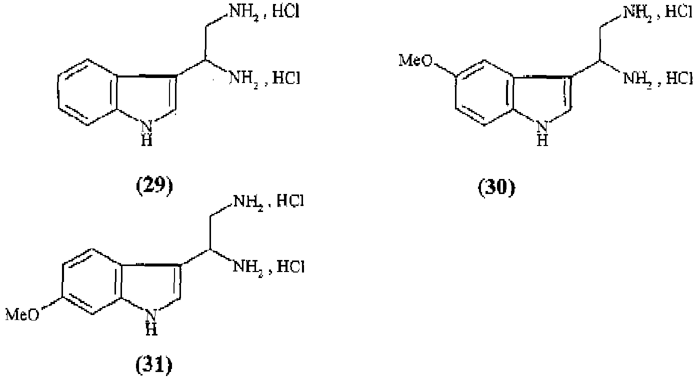

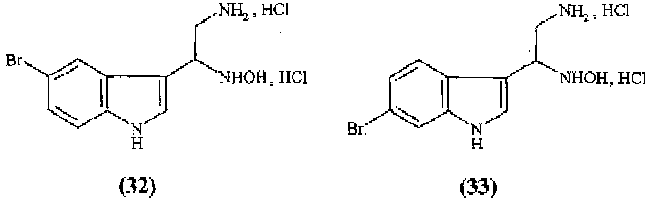

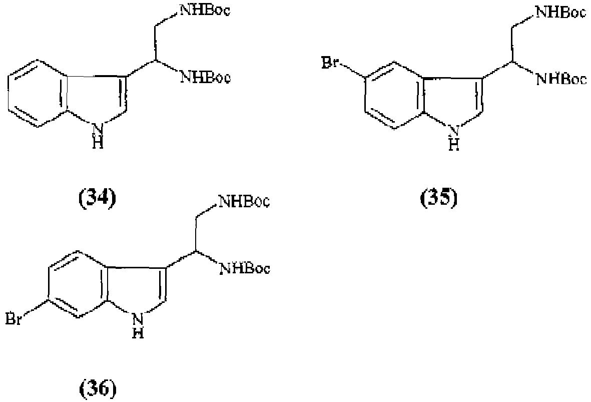

13. Use according to anyone of claims 1 to 12, of at least one compound of formula (I), in association with an antibiotic of the family of the fluoroquinolones chosen from ciprofloxacin, norfloxacin, pefloxacin, enofloxacin, ofloxacin, levofloxacin and moxifloxacin, for the treatment of pathologies associated with bacterial infections vis-à-vis which a resistance to the antibacterials exists,
said compound of formula (I) being chosen from one of the following compounds :

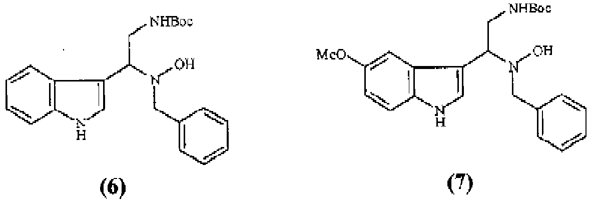

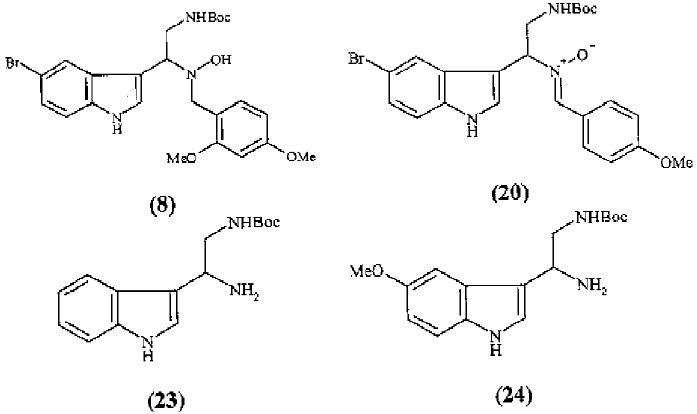

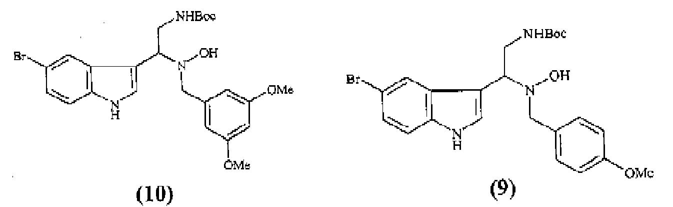

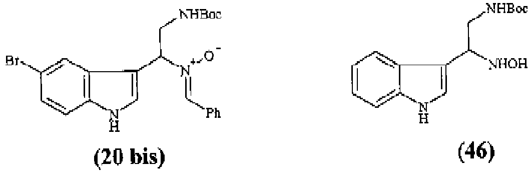

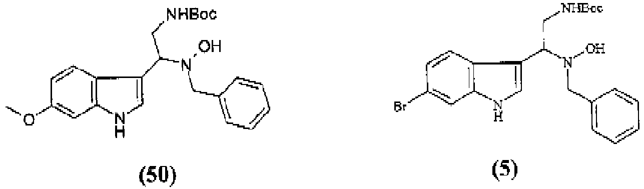

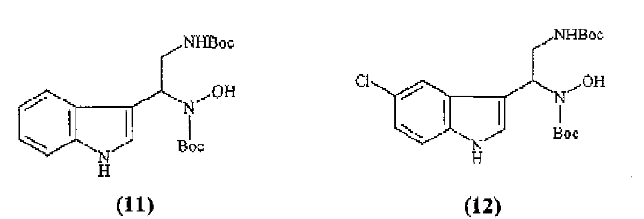

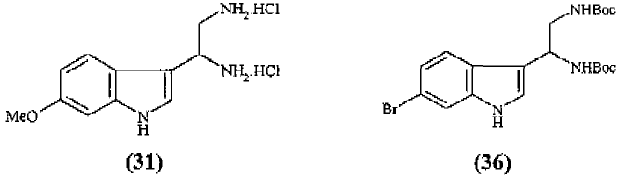

14. Pharmaceutical composition comprising, in combination with a pharmaceutically acceptable vehicle:
- at least one compound of formula (I) as defined in anyone of claims 1 to 12, and
- at least one antibiotic compound of the family of the fluoroquinolones, chosen from ciprofloxacin, norfloxacin, pefloxacin, enofloxacin, ofloxacin, levofloxacin and moxifloxacin,
said pharmaceutical composition being used for simultaneous or separate or spread over time use intended for the treatment of pathologies associated with bacterial infections vis-à-vis which a resistance to the antibacterials exists,
said compound of formula (I) being chosen from one of the following compounds.

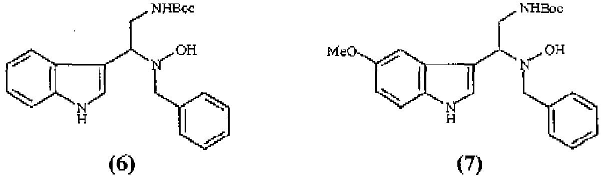

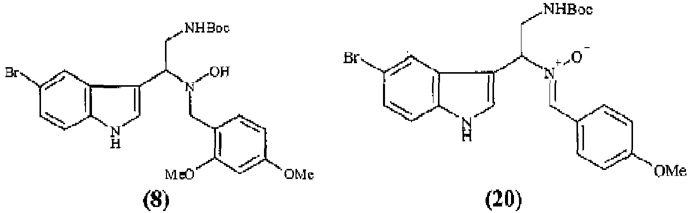

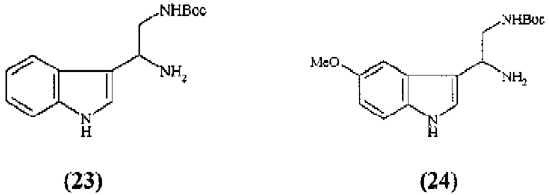

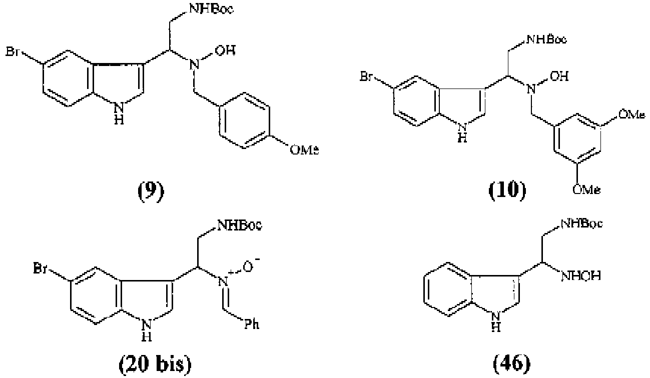

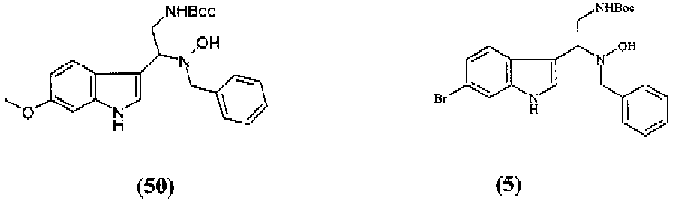

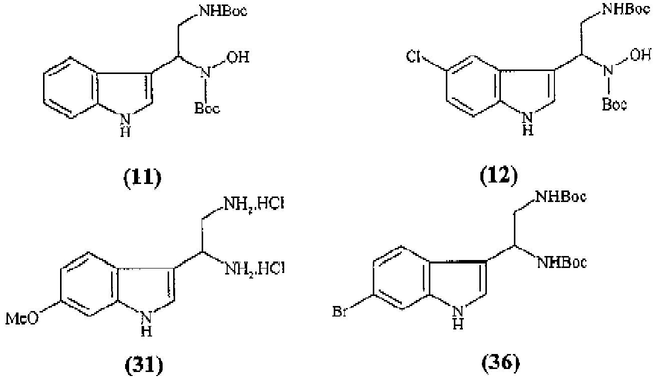

15. Compound of formula (I-6-a) below : 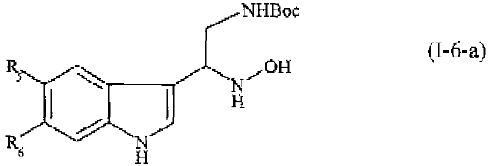 said compounds of formula (I-6-a) being possibly in the form of optical isomers, namely in the form of enantiomers or a mixtures of said enantiomers, including a racemic mixture, or in the form, if appropriate, ophysiologicahy acceptable acid salts chosen from hydrochlorides, formiates or oxalates (HOOCCOOH),
said compounds of formula (I-6-a) being **characterized in that**:
- R₅ represents a hydrogen atom or a bromine atom;
- R₆ represents a hydrogen atom or a bromine atom.

16. Compound of formula (I-9-a) below : 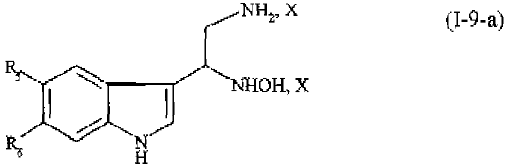 said compounds of formula (I-9-a) being possibly in the form of optical isomers, namely in the form of enantiomers or a mixture of said enantiomers, including a racemic mixture, or in the form, if appropriate, of physiologically acceptable acid salts chosen from hydrochlorides, formiates or oxalates (HOOCCOOH),
said compounds of formula (I-9-a) being **characterized in that**:
- X represents HCl,
- R₅ represents a hydrogen atom or a bromine atom;
- R₆ represents a hydrogen atom or a bromine atom.

17. Compound of formula (I-10-a) below : 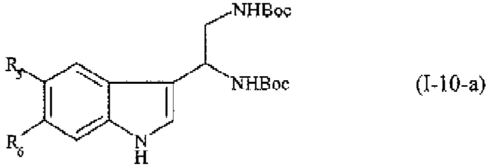 said compounds of formula (I-10-a) being possibly in the form of opical isomers, namely in the form of enantiomers or a mixture of said enantiomers, including a racemic mixture,
said compounds of formula (I-10-a) being **characterized in that**:
- R₅ represents a hydrogen atom or a bromine atom;
- R₆ represents a hydrogen atom or a bromine atom.

18. Compound corresponding to one of the following formulae :

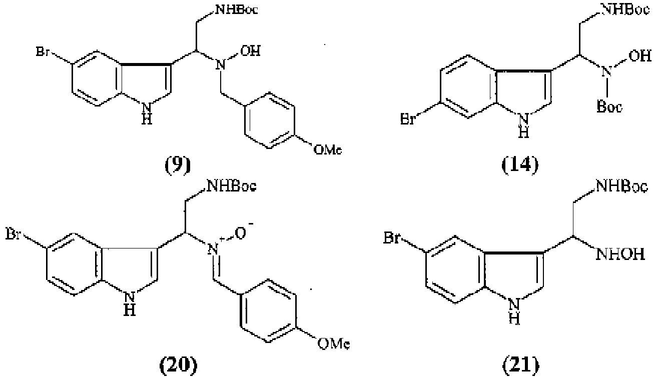

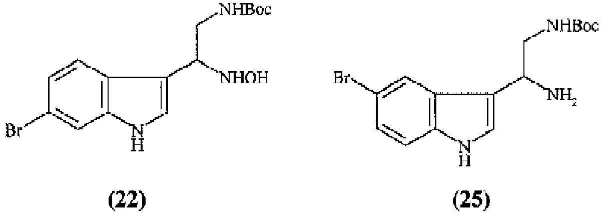

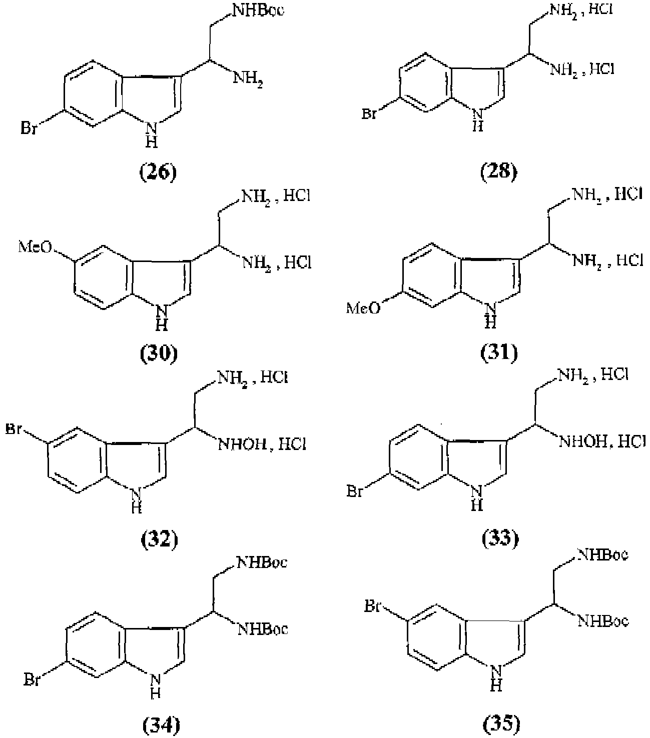

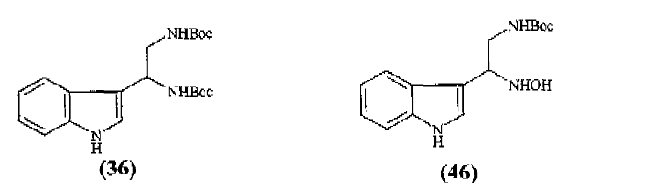

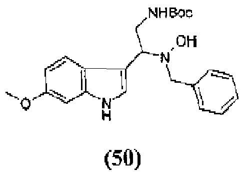

19. Pharmaceutical composition comprising a compound according to anyone of claims 15 to 18, in combination with a pharmaceutically acceptable compound.

20. Process for the preparation of a compound of formula (I-5-bis) below : 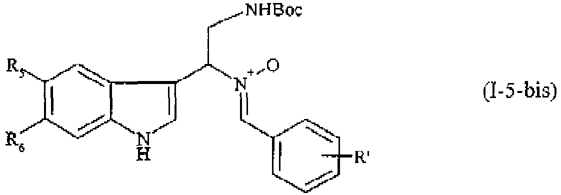 in which:
R₅ and R₆ represent independently of each other a group chosen from one of the following groups:
* a hydrogen atom;
* an alcoxyl group comprising 1 to 7 carbon atoms or a benzyloxyl group;
* a halogen atom chosen from Br, Cl, F or I;
- R' represents a group chosen from one of the following groups:
* a hydrogen atom;
* an alcoxyl group comprising 1 to 4 carbon atoms;
said process being **characterized in that** it comprises an oxidation step with MnO₂ in toluene or with AMCPB in dichloromethane or Javel water in a DCM/water mixture, at 100°C, of a compound of formula (I-1-bis) below: 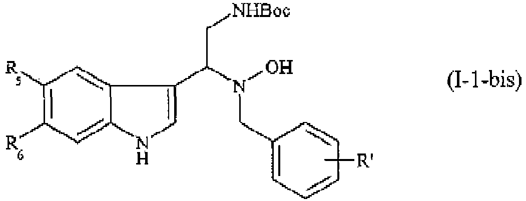 R₅, P₆ and R' being as defined above.

21. Process for the preparation of a compoundof formula (I-6-a) below; 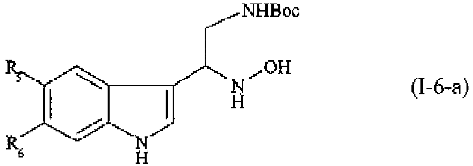 in which R₅ and R₆ represent independently of each other a group chosen from one of the following groups ;
* a hydrogen atom ;
* an alcoxyl group comprising 1 to 7 carbon atoms or a benzyloxyl group ;
* a halogen atom chosen from Br, Cl, F or I;
comprising a step of treatment with NH₂OH.HCl (hydroxylamine hydrochloride), in methanol at ambient temperature, of a compound of formula (I-5-bis) below: 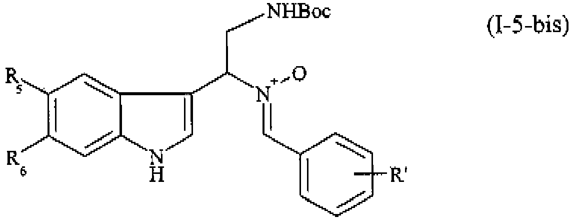
R₅ and R₆ being as defined above, and
R⁷ representing a group chosen from one of the following groups:
* a hydrogen atom;
* an alcoxyl group comprising 1 to 4 carbon atoms;
said compound of formula (I-5-bis) being obtained by the preparation process according to claim 20.

22. Process for the preparation of a compound of formula (I-7-a) below : 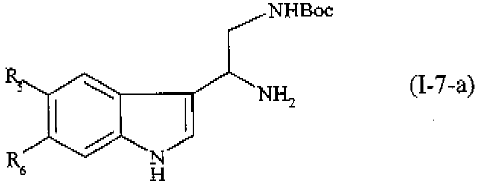 in which R₅ and R₆ represent independently of each other a group chosen from one of the following groups:
* a hydrogen atom;
* an alcoxyl group comprising 1 to 7 carbon atoms or a benzyloxyl group ;
* a halogen atom chosen from Br, Cl, F or I;
said process comprising a step of treatment with TiCl₃/HCl, in methanol at ambient temperature, of a compound of formula (I-6-a) below: 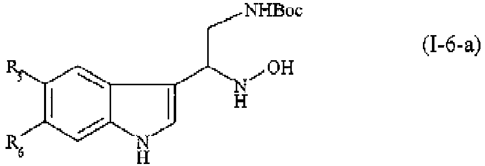 R₅ and R₆ being as defined above,
said compound of formula (I-6-a) being obtained by the preparation process according to claim 21.

23. Process for the preparation of a compound of formula (I-8-a) bellow : 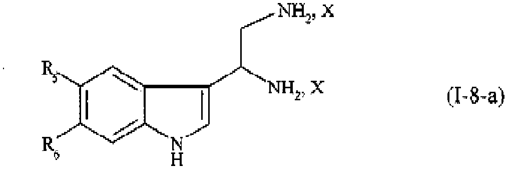 in which R₅ and R₆ represent independently of each other a group chosen from one of the following groups :
* a hydrogen atom ;
* an alcoxyl group comprising 1 to 7 carbon atoms or a benzyloxyl group ;
* a halogen atom chosen from Br, Cl, F or I;
said process comprising a step of treatment with HCl in MeOH of a compound of formula (I-7-a) below: 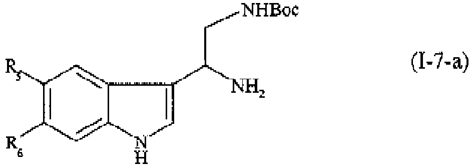 R₅ and R₆ being as defined above,
said compound of formula (I-7-a) being obtained by the preparation process according to claim 22.

24. Process for the preparation of a compound of formula (I-9-a) below : 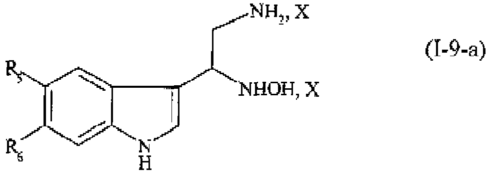 in which :
- X represents HCl, HCOOH or HOOCCOOH;
- R₅ and R₆ represent independently of each other a group chosen from one of the following groups:
* a hydrogen group;
* an alcoxyl group comprising 1 to 7 carbon atoms or a benzyloxyl group;
* a halogen atom chosen from Br, Cl, F or I;
said process comprising a step of treatment with dry HCl, in methanol at ambient temperature, of a compound of formula (I-6-a) below: 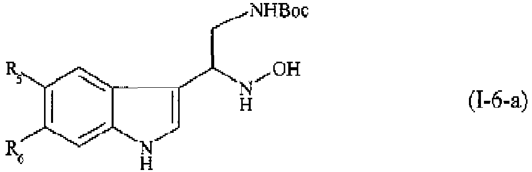 R₅ and R₆ being as defined above,
said compound of formula (I-6-a) being obtained by the preparation process according to claim 21.
